# EUROPEAN PATENT SPECIFICATION

(11) **EP 1 856 059 B1**
(45) Date of publication and mention of the grant of the patent: **19.05.2010**
(21) Application number: 06709612.3
(22) Date of filing: 03.02.2006
(51) Int. Cl.: C07D 239/22, C07D 401/12, C07D 403/14, A61K 31/513, A61P 9/00

(54) **MULTIMERS OF PYRIMIDINONE DERIVATIVES AND THEIR USE AS HUMAN NEUTROPHIL ELASTASE INHIBITORS**
MULTIMERE VON PYRIMIDINONDERIVATEN UND DEREN VERWENDUNG ALS INHIBITOREN DER HUMANEN NEUTROPHILELASTASE
MULTIMERES DE DERIVES PYRIMIDINONE ET LEUR UTILISATION COMME INHIBITEURS DE L'ELASTASE NEUTROPHILE HUMAINE

(30) Priority: 03.02.2005 GB 0502258
(43) Date of publication of application: 21.11.2007
(73) Proprietor: Argenta Discovery Limited, Harlow, Essex CM19 5TR (GB)
(72) Inventor: FINCH, Harry, Argenta Discovery LTD, Harlow, Essex CM19 5TR (GB); EDWARDS, Christine, Harlow, Essex CM19 5TR (GB); RAY; Nicholas, Charles, Harlow, Essex CM19 5TR (GB); O'CONNOR, Elizabeth, Anne, Harlow, Essex CM19 5TR (GB); FITZGERALD, Mary, F., Harlow, Essex CM19 5TR (GB)
(74) Representative: Perry, Robert Edward
(86) International application number: PCT/GB2006/000361
(87) International publication number: WO 2006/082412

(56) References cited:
- WO-A-2004/024700
- WO-A-2004/024701
- WO-A-2005/082863
- WO-A-2005/082864
- HEATHER L HANDL ET AL..: "HITTING MULTIPLE TARGETS WITH MULTIMERIC LIGANDS" EXPERT OPINION IN THERAPEUTIC TARGETS, vol. 8, no. 6, 2004, pages 565-586, XP009069513 ASHLEY PUBLICATIONS, GB cited in the application

## Description

### Field of the Invention

This invention relates to heterocyclic compounds and their use in therapy.

### Background to the invention

Human neutrophil elastase is a 32 kDa serine proteinase found in the azurophilic granules of neutrophils. It has a role in the degradation of a wide range of extracellular matrix proteins, including fibronectin, laminin, proteoglycans, Type III and Type IV collagens as well as elastin (Bieth, G. In Regulation of Matrix accumulation, Mecham, R. P. (Eds), Academic Press, NY, USA 1986, 217-306). HNE has long been considered to play an important role in homeostasis through repair and disposal of damaged tissues via degradation of the tissue structural proteins. It is also relevant in the defence against bacterial invasion by means of degradation of the bacterial body. In addition to its effects on matrix tissues, HNE has been implicated in the upregulation of IL-8 gene expression and also induces IL-8 release from the epithelial cells of the lung. In animal models of Chronic Obstructive Pulmonary Disease induced by tobacco smoke exposure both small molecule inhibitors and protein inhibitors of HNE inhibit the inflammatory response and the development of emphysema (Wright, J. L. et al. Am. J. Respir. Crit. Care Med. 2002, 166, 954-960; Churg, A. et al. Am. J. Respir, Crit. Care Med. 2003, 168, 199-207). Thus, HNE may play a role both in matrix destruction and in amplifying inflammatory responses in chronic respiratory diseases where neutrophil influx is a characteristic feature. Indeed, HNE is believed to play a role in several pulmonary diseases, including chronic obstructive pulmonary disease (COPD), cystic fibrosis (CF), acute respiratory distress syndrome (ARDS), pulmonary emphysema, pneumonia, severe asthma, sarcoidosis, bronchiectasis and lung fibrosis. It is also implicated in several cardiovascular diseases in which tissue remodelling is involved, for example, in heart failure and the generation of ischaemic tissue injury following acute myocardial infarction. Elevated HNE levels are also correlated with the severity of inflammation in inflammatory bowel disease (Silberer H et al, Clin Lab. 2005;51 (3-4):117-26) and may play a role in impaired mucosal repair in patients with ulcerative colitis.

COPD is an umbrella term encompassing three different pathological conditions, all of which contribute to limitation of airflow: chronic bronchitis, emphysema and small-airway disease. Generally all three will exist to varying extents in patients presenting with COPD, and all three may be due to neutrophil-mediated inflammation, as supported by the increased number of neutrophils observed in bronchoalveolar leakage (BAL) fluids of COPD patients (Thompson, A. B.; Daughton, D.; et al. Am. Rev. Respir. Dis. 1989, 140, 1527-1537). The major pathogenic determinant in COPD has long been considered to be the protease-anti-protease balance (also known as the "elastase:anti-elastase hypothoesis"), in which an imbalance of HNE and endogenous anti-proteases such as α1-antitrypsin (α₁-AT), Secretory leukocyte protease inhibitor (SLPI) and pre-elafin leads to the various inflammatory disorders of COPD. Individuals that have a genetic deficiency of the protease inhibitor α1-antitrypsin (α₁-AT) develop emphysema that inceases in severity over time (Laurrell, C.B.; Erikkson, S Scand. J. Clin. Invest> 1963 15, 132-140). An excess of HNE is therefore destructive, leading to the breakdown of pulmonary morphology with loss of elasticity and destruction of alveolar attachments of airways in the lung (emphysema) whilst simultaneously increasing microvasular permeability and mucus hypersecretion (chronic bronchitis).

Multimeric ligands consist of multiple binding domains which are tethered together through a suitable scaffold. Hence individual binding domains are linked together into a single molecule, increasing the probability that the multimer will bind simultaneously with multiple active sites resulting in high-affinity interactions (Handl, H.L. et al. Expert Opin. Ther. Targets 2004, 8, 565-586; Han, Y.F. et al. Bloorg. Med. Chem. Letts. 1999, 7, 2569-2575). Also, multiple binding intereactions with relatively high off-rates can combine to yield an overall low off-rate for the mutlimeric ligand. Thus, a molecule consisting of a suitable linker and ligands may be expected to show advantage over the monomeric ligands alone in terms of potency and/or duration of action. Multimeric compounds are unlikely to be orally bioavailable (as predicted by Lipinski's "Rule of 5") which may be advantageous where an inhaled route of administration to the lungs is targeted, since even after inhaled administration, a large proportion of drug is likely to enter the GI tract. Thus such compounds may be expected to show reduced systemic exposure after inhalation administration and hence an improved toxicity profile over orally administered therapies.

Monomers of formula (II) are described as inhibitors of human neutrophil elastase in WO2004/024700, WO2004/024701, GB2392910, WO2005/082863 and WO2005/082864.

### Summary of the Invention

A first aspect of the invention is a compound (which may be described as a dimer) of formula (I):

(M)-(L)-(M) (I)

wherein L is a linker and M is a group of formula (II) 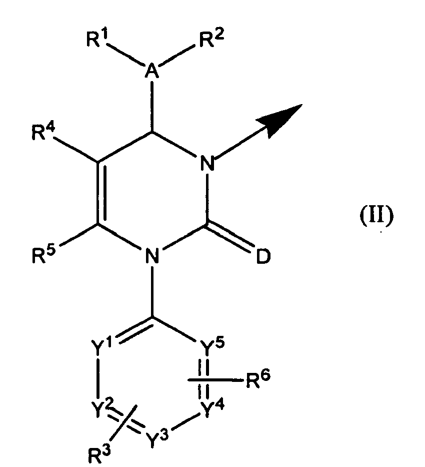 wherein
A is aryl or heteroaryl;
D is oxygen or sulphur;
R¹, R² and R³ are independently each hydrogen, halogen, nitro, cyano, alkyl, hydroxyl or alkoxy, wherein alkyl and alkoxy can be further substituted with one to three identical or different radicals selected from the group consisting of halogen, hydroxyl and alkoxy;
R⁴ is trifluoromethylcarbonyl, alkylcarbonyl, alkoxycarbonyl, alkenoxycarbonyl, hydroxycarbonyl, aminocarbonyl, arylcarbonyl, heteroarylcarbonyl or cyano, wherein alkylcarbonyl, alkoxycarbonyl and aminocarbonyl can be further substituted with one to three identical or different radicals selected from the group consisting of cycloalkyl, hydroxyl, alkoxy, alkoxycarbonyl, hydroxycarbonyl, aminocarbonyl, cyano, amino, heteroaryl, heterocycloalkyl and tri-(alkyl)-silyl, and wherein heteroarylcarbonyl and heteroaryl can be further substituted with alkyl;
or R⁴ represents a group of formula (VIII) 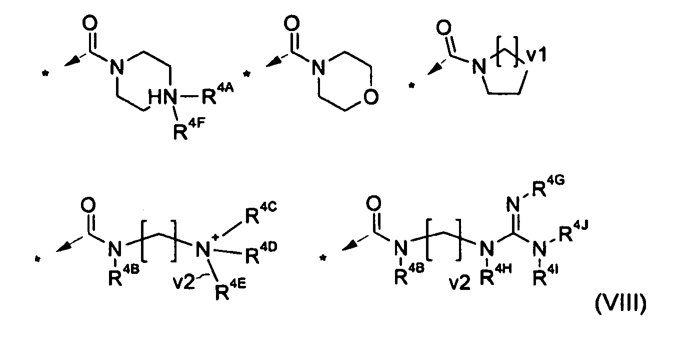 wherein
R^{4A}, R^{4B}, R^{4G}, R^{4H}, R^{4I} and R^{4J} are independently hydrogen or alkylk, or R^{4H} and R^{4I} may be joined together with the nitrogen atom to which they are attached to form a ring;
R^{4F} is a lone pair or R^{4F} is alkyl and the nitrogen atom to which it is attached is quaternary and carries a positive charge;
R^{4C}, R^{4D} and R^{4E} are alkyl, or any two of R^{4C}, R^{4D} or R^{4E} may be joined together with the nitrogen atom to which they are attached to form a ring, optionally containing a further heteroatom selected from oxygen or nitrogen;
v1 is 1-3;
v2 is 1-6;
R⁵ is alkyl, which can be substituted with one to three identical or different radicals selected from the group consisting of halogen, hydroxy, alkoxy, alkenoxy, alkenoxy, alkylthio, amino hydroxycarbonyl, alkoxycarbonyl and the radical -O-(alkyl)-O-(alkyl); or R⁵ is amino;
R⁶ is halogen, nitro, cyano, alkyl, hydroxy or alkoxy, wherein alkyl and alkoxy can be further substituted with one or three identical or different radicals selected from the group consisting of halogen, hydroxyl and alkoxy; and
Y¹, Y², Y³, Y⁴ and Y⁵ are independently each CH, CR³, CR⁶ or N, with the proviso that the ring in which they are comprised contains no more than 2 N atoms;
or a pharmaceutically acceptable salt, solvate or N-oxide thereof.

The following preferences apply:
L is a linker group of Formula (III),

-L^{a}-R⁷-WL^{b}-R⁷-L^{a}- (III)

wherein;
L^{a} is a bond or group -C(O)-;
L^{b} is a bond or group -C(O)-;
R⁷ is an alkyklene or cycloalkylene group;
W is a bond or is selected from the following divalent radicals

-(O-R^{8A})ₘ₁-O-

-N(R^{9A})-(O-R^{8A})ₘ₁-R^{8A}-N(R^{9A})-

-N(R^{9A})-R^{8B}-N(R^{9B})(R^{9C})-R^{8B}-N(R^{9A})-

-N(R^{9A})-R^{8B}-N(R^{10B})C(=NR^{10A})(NR^{10C})-R^{8B}-N(R^{9A})-

-N(R^{9A})-R^{8B}-N(R^{9A})-

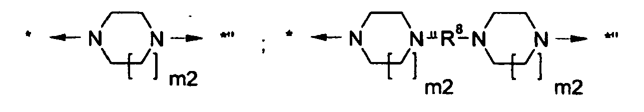

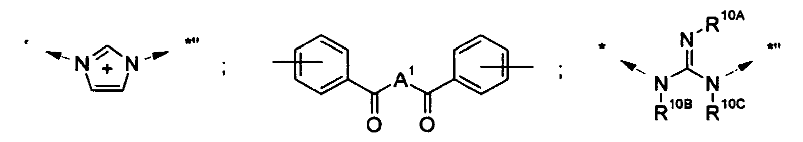

wherein;
m1 is 1-4;
R^{8A} is an alkylene or cycloalkylene group;
R^{8B} is an alkylene or cycloalkylene group, or a group of Formula A²;
R^{9A} is hydrogen or alkyl;

One of R^{9B} or R^{9C} is a lone pair and the other is hydrogen or alkyl, or R^{9B} and R^{9C} are both alkyl, in which case the nitrogen to which they are attached is quaternary and carries a positive charge. Additionally, R^{9B} and R^{9C} may be joined together with the nitrogen to which they are attached to form a ring;
R^{10A} is hydrogen or alkyl;
R^{10B} and R^{10C} are independently hydrogen or alkyl, or alternatively R^{10B} and R^{10C} may be joined together to form a ring;
m2 is 1-3;
A¹ is selected from the groups -N(R^{9A})-R⁸-N(R^{9B})(R^{9C})-R⁸-N(R^{9A})-, -N(R^{9A})-R⁸-N(R^{10B})C(=NR^{10A})(NR^{10C})-R⁸-N(R^{9A})-;
A² is selected from the groups of Formula (...)

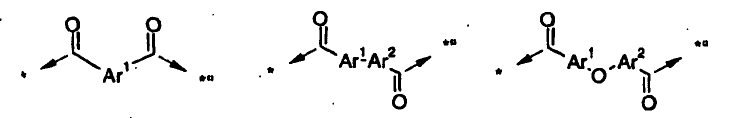

Wherein Ar¹, Ar² are independently an aryl or heteroaryl group.

It Will be appreciate that any compound of the invention may be used in the form of a prodrug.

Compounds of the invention may be useful in the treatment or prevention of diseases in which HNE is implicated, for example chronic obstructive pulmonary disease (COPD), chronic bronchitis, lung fibrosis, pneumonia, acute respiratory distress syndrome (ARDS), pulmonary emphysema, smoking-induced emphysema, severe asthma, sarcoidosis, bronchiectasis, cystic fibrosis, inflammatory bowel disease; ulcerative colitis and Crohn's disease.

Another aspect of the invention is a pharmaceutical composition comprising a compound of the invention and a pharmaceutically acceptable carrier or excipient

Another aspect of the invention is the use of a compound of the invention for the manufacture of a medicament for the treatment or prevention of a disease or condition in which HNE Is implicated. Thus, compounds of the invention may be used in a method of therapy, for the treatment of a patient suffering from a condition or disease as defined above.

### Description of Preferred Embodiments

"Alkylcarbonyl" means a-CO-alkyl group in which the alkyl group is as described herein. Exemplary acyl groups include -COCH₃ and -COCH(CH₃)₂.

"Acylamino" means a -NR-acyl group in which R and acyl are as described herein. Exemplary acylamino groups include -NHCOCH₃ and -N(CH₃)COCH₃.

"Alkenoxy" means an -O-alkenyl group in which alkenyl is as described below. Exemplary groups includes -O-allyl (-OCH₂CH=CH₂)

"Alkenoxycarbonyl" means a -COO-alkenyl group which alkenyl is as described below. Exemplary groups includes -C(O)O-allyl.

"Alkoxy" and "alkyloxy" means an -O-alkyl group in which alkyl is as described below. Exemplary alkoxy groups include methoxy (-OCH₃) and ethoxy (-OC₂H₅).

"Alkoxycarbonyl" means a -COO-alkyl group in which alkyl is as defined below. Exemplary alkoxycarbonyl groups include methoxycarbonyl and ethoxycarbonyl.

"Alkyl" or "lower alkyl", as a group or part of a group, refers to a straight or branched chain saturated hydrocarbon group having from 1 to 12, preferably 1 to 6, carbon atoms, in the chain. Exemplary alkyl groups include methyl, ethyl, 1-propyl and 2-propyl.

"Alkenyl" as a group or part of a group refers to a straight or branched chain hydrocarbon group having from 1 to 12, preferably 1 to 6, carbon atoms and one carbon-carbon double bond in the chain. Exemplary alkenyl groups include ethenyl, 1-propenyl, and 2-propenyl.

"Alkylamino" means a -NH-alkyl group in which alkyl is as defined above. Exemplary alkylamino groups include methylamino and ethylamino.

"Alkylene means an -alkyl- group in which alkyl is as defined previously. Exemplary alkylene groups include -CH₂-, -(CH₂)₂- and -C(CH₃)HCH₂-.

"Alkenylene" means an -alkenyl- group in which alkenyl is as defined previously. Exemplary alkenylene groups include -CH=CH-, -CH=CHCH₂-, and -CH₂CH=CH-.

"Alkylthio" means a -S-alkyl group in which alkyl is as defined above. Exemplary alkylthio groups include methylthio and ethylthio.

"Amino" means a -NR¹R² group where R¹ and R² may be independently a hydrogen atom, alkyl, aryl, arylalkyl, alkenyl, alkynyl, heteroaryl or heterocycloalkyl group. (i.e. The amino group may be primary, secondary or tertiary). Exemplary amino groups include -NH₂, NHCH₃, -NHPh, -N(CH₃)₂, etc.

"Aminocarbonyl" means a -CO-NRR group in which R is as herein described. Exemplary aminocarbonyl groups include -CONH₂, -CONHCH₃ and -CONH-phenyl.

"Aminoalkyl" means an alkyl-NH₂ group in which alkyl is as previously described. Exemplary aminoalkyl groups include -CH₂NH₂.

"Ammonium" means a quarternary nitrogen group -N⁺R¹R²R³ where R¹, R² and R³ are alkyl, aryl, alkenyl, arylalkyl, heteroaryl, heterocycloalkyl, and the nitrogen atom carries a formal positive charge.

"Aryl" as a group or part of a group denotes an optionally substituted monocyclic or multicyclic aromatic carbocyclic moiety of from 6 to 14 carbon atoms, preferably from 6 to 10 carbon atoms, such as phenyl or naphthyl. The aryl group may be substituted by one or more substituent groups.

"Arylalkyl" means an aryl-alkyl- group in which the aryl and alkyl moieties are as previously described. Exemplary arylalkyl groups include benzyl, phenethyl and naphthlenemethyl.

"Arylalkyloxy" means an aryl-alkyloxy- group in which the aryl and alkyloxy moieties are as previously described. Preferred arylalkyloxy groups contain a C₁₋₄ alkyl moiety. Exemplary arylalkyl groups include benzyloxy.

"Arylcarbonyl" means an aromatic ring joined to a carbonyl group -(C=O). Exemplary groups include benzoyl (-C(O)Ph).

"Aryloxy" means an -O-aryl group in which aryl is described above. Exemplary aryloxy groups include phenoxy.

"Cyclic amine" means an optionally substituted 3 to 8 membered monocyclic cycloalkyl ring system where one of the ring carbon atoms is replaced by nitrogen, and which may optionally contain an additional heteroatom selected from O, S or NR (where R is as described herein). Exemplary cyclic amines include pyrrolidine, piperidine, morpholine, piperazine and *N*-methylpiperazine. The cyclic amine group may be substituted by one or more substituent groups.

"Cycloalkyl" means an optionally substituted saturated monocyclic or bicyclic ring system of from 3 to 12 carbon atoms, preferably from 3 to 8 carbon atoms, and more preferably from 3 to 6 carbon atoms. Exemplary monocyclic cycloalkyl rings include cyclopropyl, cyclopentyl, cyclohexyl and cycloheptyl. The cycloalkyl group may be substituted by one or more substituent groups.

"Cyloalkylene" means means an optionally substituted saturated monocyclic or bicyclic ring system of from 3 to 12 carbon atoms, preferably from 3 to 8 carbon atoms, and more preferably from 3 to 6 carbon atoms, as a bivalent radical. Exemplary cycloalkylene groups include cyclohexane-1,4-diyl.

"Cycloalkylalkyl" means a cycloalkyl-alkyl- group in which the cycloalkyl and alkyl moieties are as previously described. Exemplary monocyclic cycloalkylalkyl groups include cyclopropylmethyl, cyclopentylmethyl, cyclohexylmethyl and cycloheptylmethyl. "Dendrimer" means a multifunctional core group with a branching group attached to each functional site. Each branching site can be attached to another branching molecule and this process may be repeated multiple times.

"Halo" or "halogen" means fluoro, chloro, bromo, or iodo.

"Haloalkoxy" means an -O-alkyl group in which the alkyl is substituted by one or more halogen atoms. Exemplary haloalkyl groups include trifluoromethoxy and difluoromethoxy.

"Haloalkyl" means an alkyl group which is substituted by one or more halo atoms. Exemplary haloalkyl groups include trifluoromethyl.

"Heteroaryl" as a group or part of a group denotes an optionally substituted aromatic monocyclic or multicyclic organic moiety of from 5 to 14 ring atoms, preferably from 5 to 10 ring atoms, in which one or more of the ring atoms is/are element(s) other than carbon, for example nitrogen, oxygen or sulfur. Examples of such groups include benzimidazolyl, benzoxazolyl, benzothiazolyl, benzofuranyl, benzothienyl, furyl, imidazolyl, indolyl, indolizinyl, isoxazolyl, isoquinolinyl, isothiazolyl, oxazolyl, oxadiazolyl, pyrazinyl, pyridazinyl, pyrazolyl, pyridyl, pyrimidinyl, pyrrolyl, quinazolinyl, quinolinyl, tetrazolyl, 1,3,4-thiadiazolyl, thiazolyl, thienyl and triazolyl groups. The heteroaryl group may be substituted by one or more substituent groups. The heteroaryl group may be attached to the remainder of the compound of the invention by any available carbon or nitrogen atom.

"Heteroarylcarbonyl" means a heteroaryl group attached to a carbonyl group - C(O)-. Exemplary groups are pyridine-2-carbonyl, thiophene-2-carbonyl.

"Heteroaryloxy" means a heteroaryloxy- group in which the heteroaryl is as previously described. Exemplary heteroaryloxy groups include pyridyloxy.

"Heterocycloalkyl" means: (i) an optionally substituted cycloalkyl group of from 4 to 8 ring members which contains one or more heteroatoms selected from O, S or NR; (ii) a cycloalkyl group of from 4 to 8 ring members which contains CONR and CONRCO (examples of such groups include succinimidyl and 2-oxopyrrolidinyl). The heterocycloalkyl group may be substituted by one or more substituent groups. The heterocycloalkyl group may be attached to the remainder of the compound by any available carbon or nitrogen atom.

"Heterocycloalkylalkyl" means a heterocycloalkyl-alkyl- group in which the heterocycloalkyl and alkyl moieties are as previously described.

"Hydroxycarbonyl" means a group -COOH.

"Pharmaceutically acceptable salt" means a physiologically or toxicologically tolerable salt and include, when appropriate, pharmaceutically acceptable base addition salts and pharmaceutically acceptable acid addition salts. For example (i) where a compound of the invention contains one or more acidic groups, for example carboxy groups, pharmaceutically acceptable base addition salts that may be formed include sodium, potassium, calcium, magnesium and ammonium salts, or salts with organic amines, such as, diethylamine, *N*-methyl-glucamine, diethanolamine or amino acids (e.g. lysine) and the like; (ii) where a compound of the invention contains a basic group, such as an amino group, pharmaceutically acceptable acid addition salts that may be formed include hydrochlorides, hydrobromides, phosphates, acetates, citrates, lactates, tartrates, malonates, methanesulphonates and the like. "Pharmaceutically acceptable salt" also means quaternary ammonium salts. In this case, the acceptable salts may be chlorides, bromides, iodides, mesylates, tosylates, succinates and the like.

It will be understood that, as used herein, references to the compounds of the invention are meant to also include the pharmaceutically acceptable salts.

"Prodrug" refers to a compound which is convertible *in vino* by metabolic means (e.g. by hydrolysis, reduction or oxidation) to a compound of the invention. For example an ester prodrug of a compound of the invention containing a hydroxy group may be convertible by hydrolysis *in vivo* to the parent molecule. Suitable esters of compounds of the invention containing a hydroxy group, are for example acetates, citrates, lactates, tartrates, malonates, oxalates, salicylates, propionates, succinates, fumarates, maleates, methylene-bis-β-hydroxynaphthoates, gentisates, isethionates, di-*p*-toluoyltartrates, methanesulfonates, ethanesulfonates, benzenesulfonates, p-toluenesulfonates, naphthalene bis sulfonates, cyclohexylsulfamates and quinates. As another example an ester prodrug of a compound of the invention containing a carboxy group may be convertible by hydrolysis *in vivo* to the parent molecule. Examples of ester prodrugs are those described by F. J. Leinweber, Drug Metab. Res., 1987, 18, 379.

It will be understood that, as used in herein, references to the compounds of the invention are meant to also include the prodrug forms.

"Saturated" pertains to compounds and/or groups which do not have any carbon-carbon double bonds or carbon-carbon triple bonds.

The cyclic groups referred to above, namely, aryl, heteroaryl, cycloalkyl, and heterocycloalkyl, may be substituted by one or more substituent groups. Suitable optional substituent groups include acyl (e.g. -COCH₃), alkoxy (e.g., -OCH₃), alkoxycarbonyl (e.g. -COOCH₃), alkylamino (e.g. -NHCH₃), alkylsulfinyl (e.g. -SOCH₃), alkylsulfonyl (e.g. - SO₂CH₃), alkylthio (e.g. -SCH₃), -NH₂, aminoacyl (e.g. -CON(CH₃)₂), aminoalkyl (e.g. - CH₂NH₂), arylalkyl (e.g. -CH₂Ph or -CH₂-CH₂-Ph), cyano, dialkylamino (e.g. -N(CH₃)₂), halo, haloalkoxy (e.g. -OCF₃ or -OCHF₂), haloalkyl (e.g. -CF₃), alkyl (e.g. -CH₃ or - CH₂CH₃), -OH, -CHO, -NO₂, aryl (optionally substituted with alkoxy, haloalkoxy, halogen, alkyl or haloalkyl), heteroaryl (optionally substituted with alkoxy, haloalkoxy, halogen, alkyl or haloalkyl), heterocycloalkyl, aminoacyl (e.g. -CONH₂, -CONHCH₃), aminosulfonyl (e.g. -SO₂NH₂, -SO₂NHCH₃), acylamino (e.g. -NHCOCH₃), sulfonylamino (e.g. -NHSO₂CH₃), heteroarylalkyl, cyclic amine (e.g. morpholine), aryloxy, heteroaryloxy, arylalkyloxy (e.g. benzyloxy) and heteroarylalkyloxy.

Alkylene or alkenylene groups may be optionally substituted. Suitable optional substituent groups include alkoxy (e.g., -OCH₃), alkylamino (e.g. -NHCH₃), alkylsulfinyl (e.g. -SOCH₃), alkylsulfonyl (e.g. -SO₂CH), alkylthio (e.g. -SCH), -NH₂, aminoalkyl (e.g. - CH₂NH₂), arylalkyl (e.g. -CH₂Ph or -CH₂-CH₂-Ph), cyano, dialkylamino (e.g. -N(CH₃)₂), halo, haloalkoxy (e.g. -OCF₃ or -OCHF₂), haloalkyl (e.g. -CF₃), alkyl (e.g. -CH₃ or - CH₂CH₃, -OH, -CHO, and -NO₂.

Compounds of the invention may exist in one or more geometrical, optical, enantiomeric, diastereomeric and tautomeric forms, including but not limited to *cis-* and trans-forms, *E-* and *Z*-forms, *R*-, *S*- and *meso*-forms, keto-, and enol-forms. Unless otherwise stated a reference to a particular compound includes all such isomeric forms, including racemic and other mixtures thereof. Where appropriate such isomers can be separated from their mixtures by the application or adaptation of known methods (e.g. chromatographic techniques and recrystallisation techniques). Where appropriate such isomers may be prepared by the application of adaptation of known methods (e.g. asymmetric synthesis).

Certain compounds and combinations of substituents are preferred. Certain preferences are given in the subclaims.

Each M is the same or different. In formula (II), the arrow denotes the point of attachment of M to the linker L. Preferably, L is a group of Formula (III) as defined herein.

In a preferred embodiment, A is a phenyl ring.

In a preferred embodiment D is an oxygen atom

In another preferred embodiment, the groups M have the stereochemistry shown below (R) configuration;

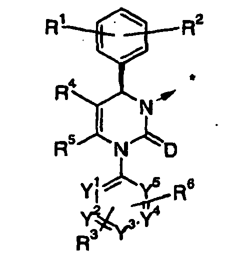

In one preferred embodiment R⁶ is a haloalkyl group.

In one preferred embodiment, Y¹-Y⁵ are carbon atoms.

in a preferred embodiment, L^{a} is a bond.

In a further preferred embodiment, W is the radical -N(R^{9A}) - R^{8B} - N(R^{9B})(R^{9C}) - R^{8B} - N(R^{9A})-.

In another preferred embodiment, W is the radical -N(R^{9A}) - R^{8B} - N(R^{10B})C(=NR^{10A})(NR^{10C})- R^{8B} - N(R^{9A})-.

In yet another preferred embodiment R⁵ is an alkyl group.

In yet another preferred embodiment R⁵ is a methyl group.

In one embodiment R⁴ is alkoxycarbonyl

In a further embodiment R⁴ is alkoxycarbonyl wherein the alkoxy group is substituted with a hydroxyl group.

In a further embodiment R⁴ is alkoxycarbonyl wherein the alkoxy group is substituted with an amino group.

In a further embodiment R⁴ is alkoxycarbonyl wherein the alkoxy group is substituted with an ammonium group.

Preferred compounds of the invention are of Examples 1 to 76, more preferably of Examples 1 to 17, or of Examples 22, 24, 33, 35, 36, 38, 41, 46, 65, 69, 70, 71 and 76.

Preferred compounds of the invention include:

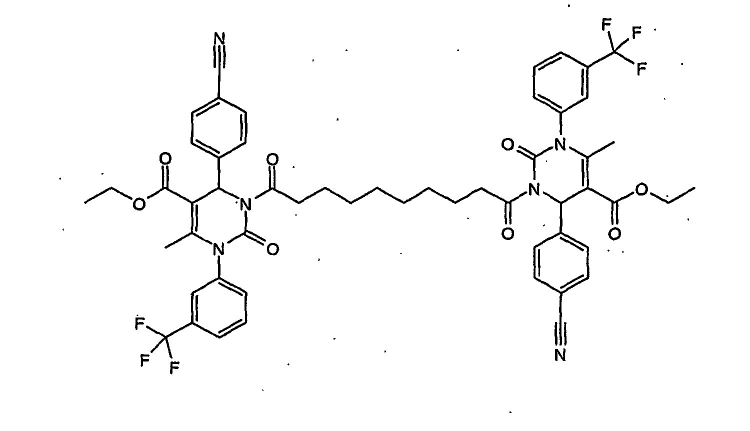

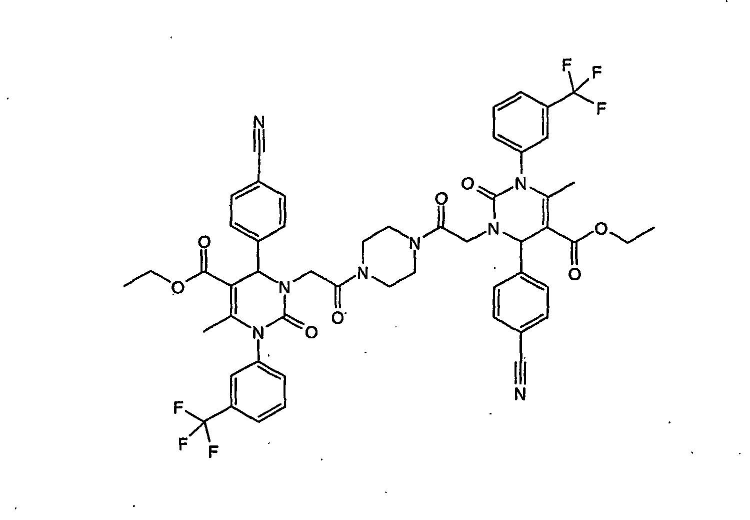

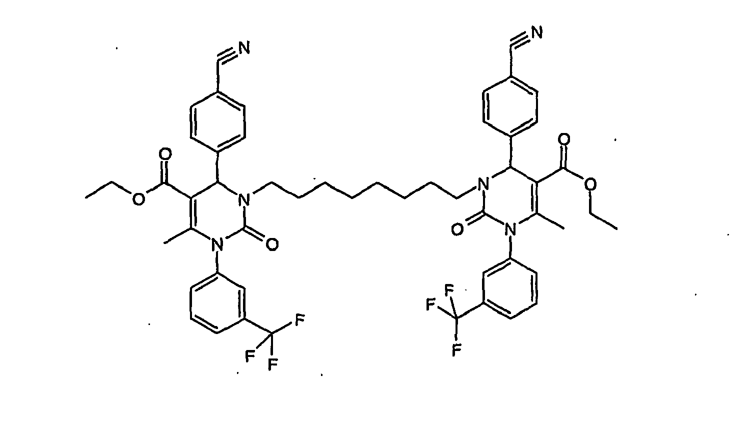

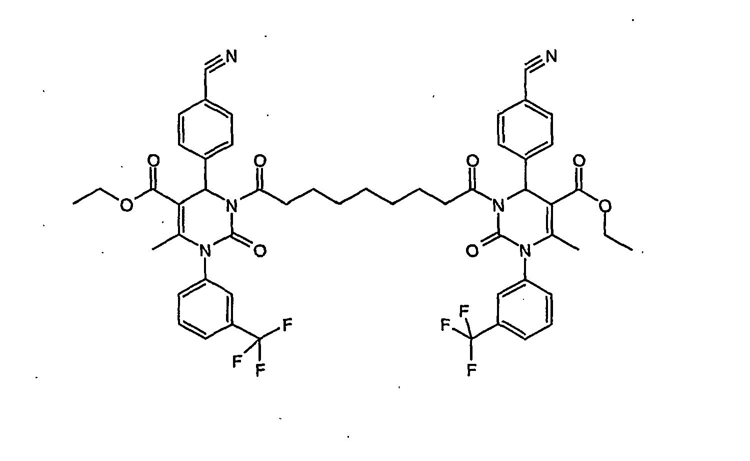

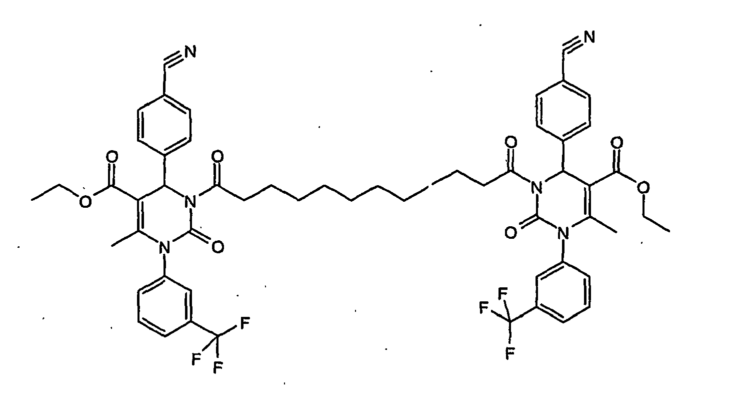

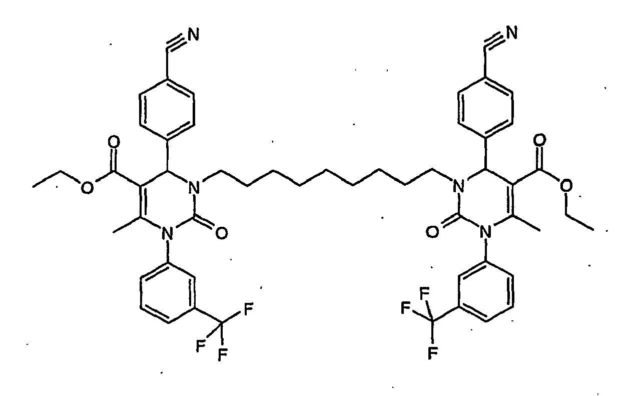

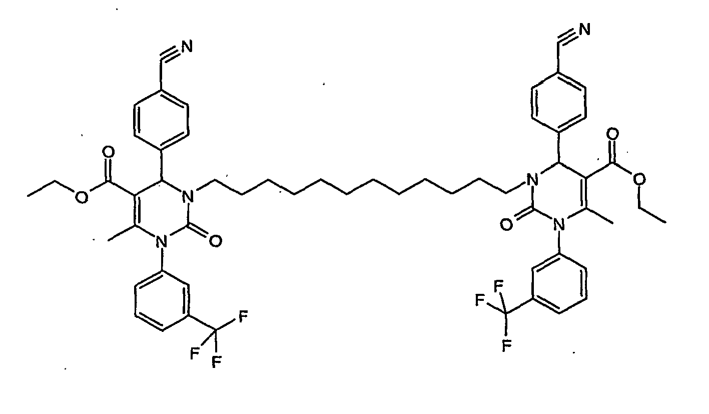

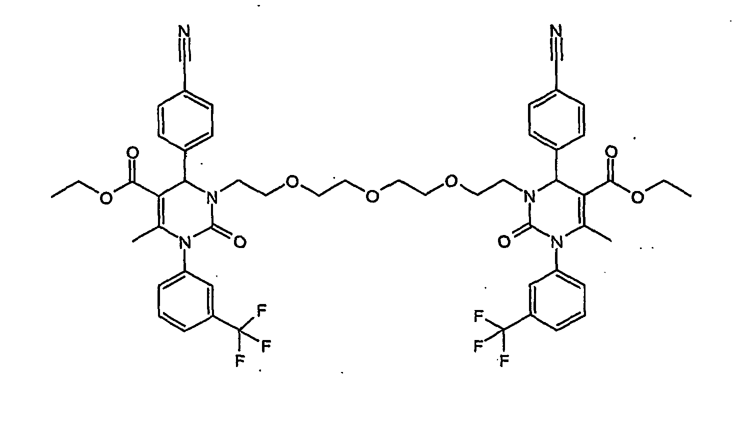

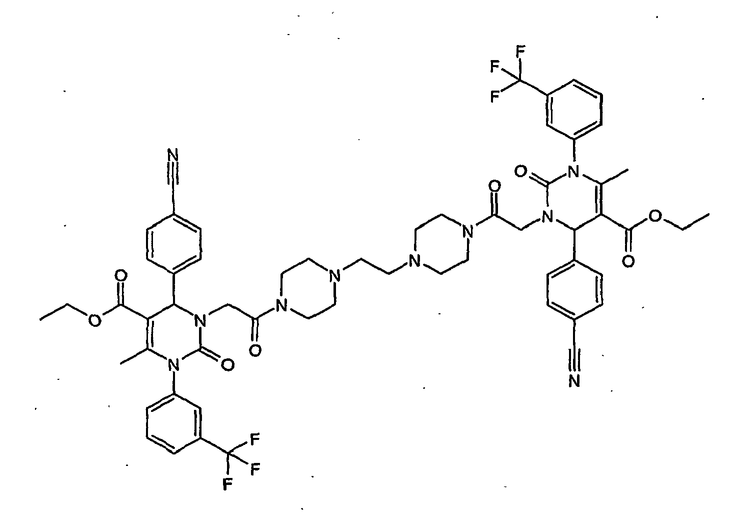

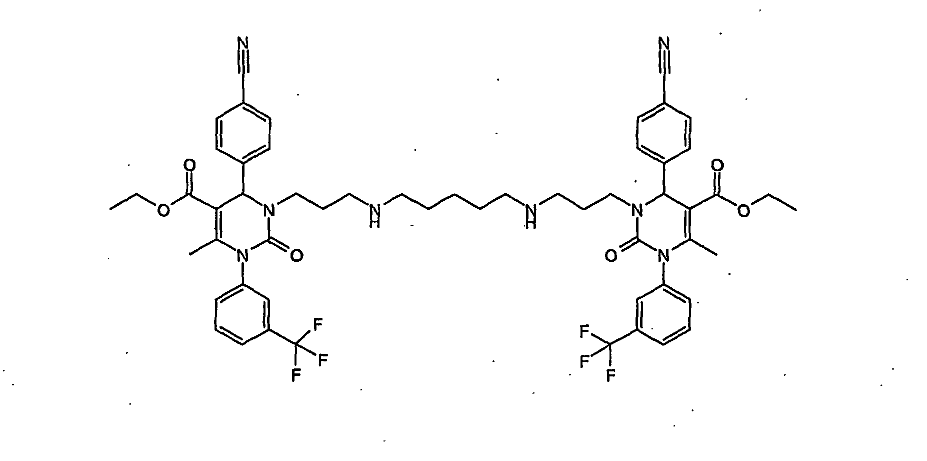

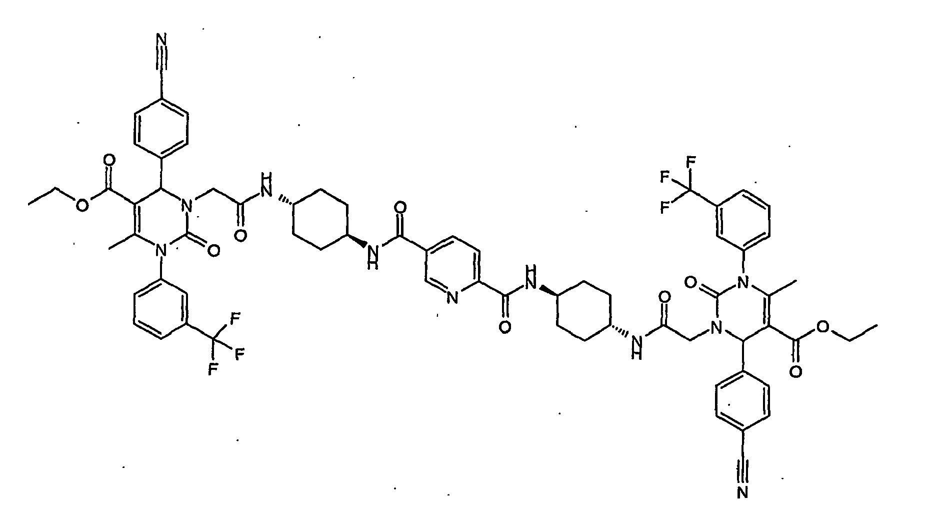

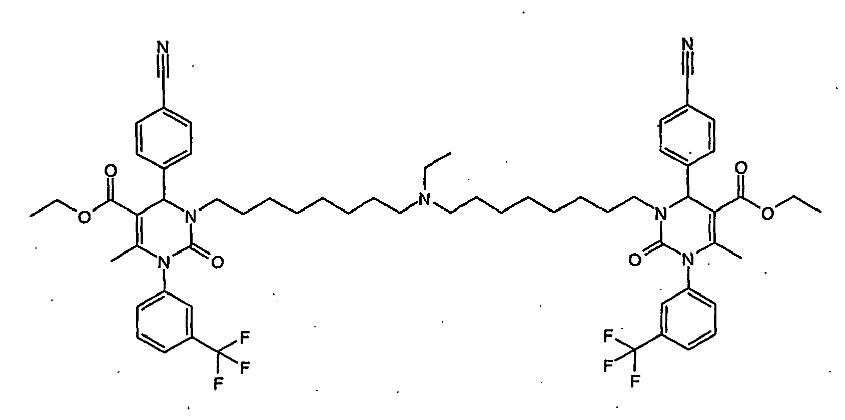

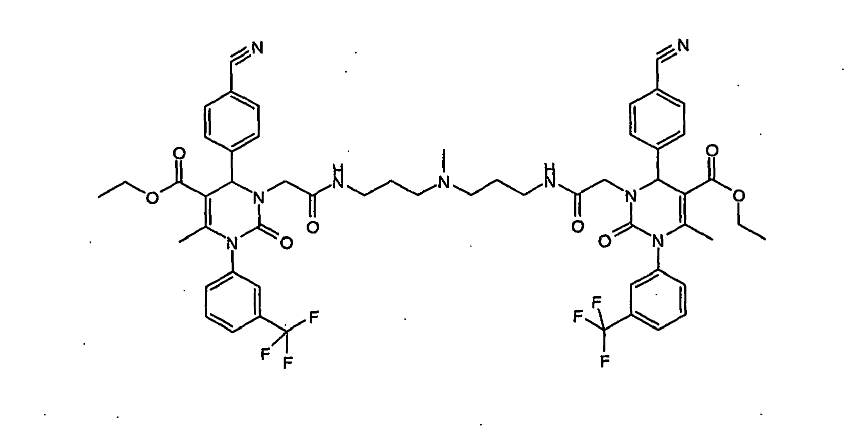

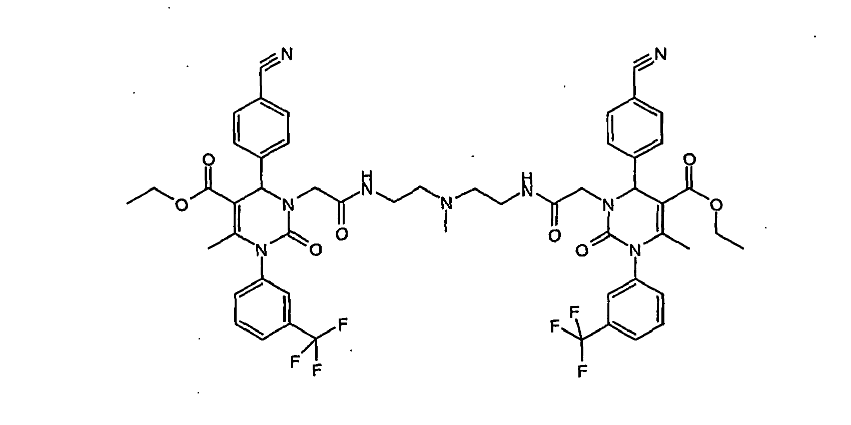

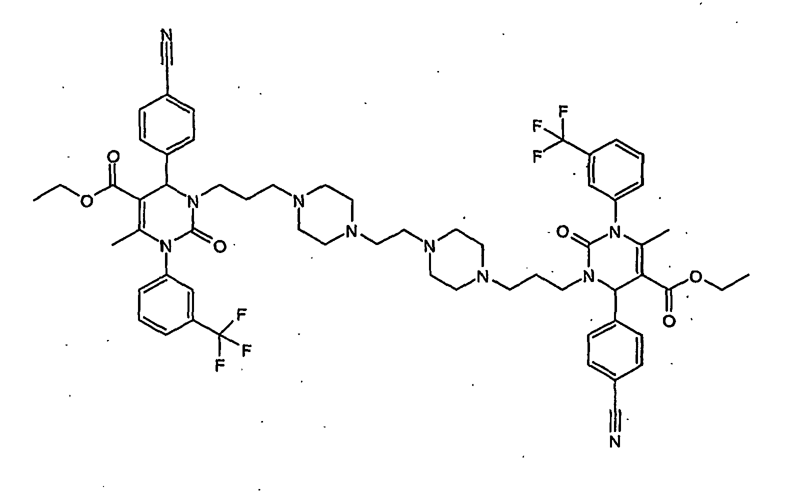

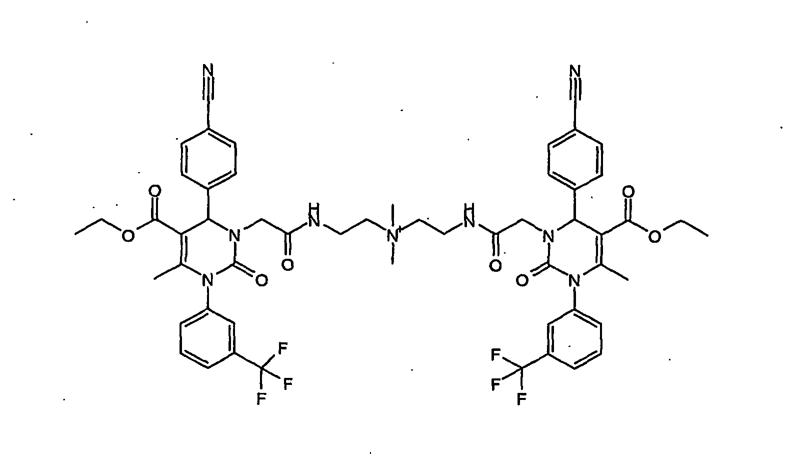

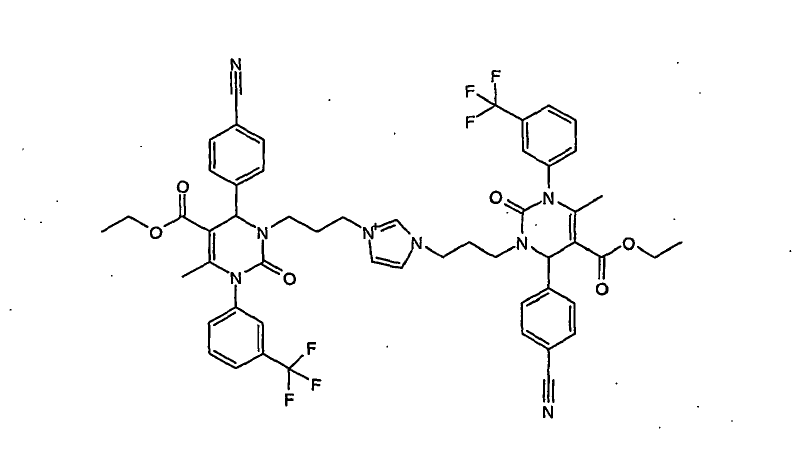

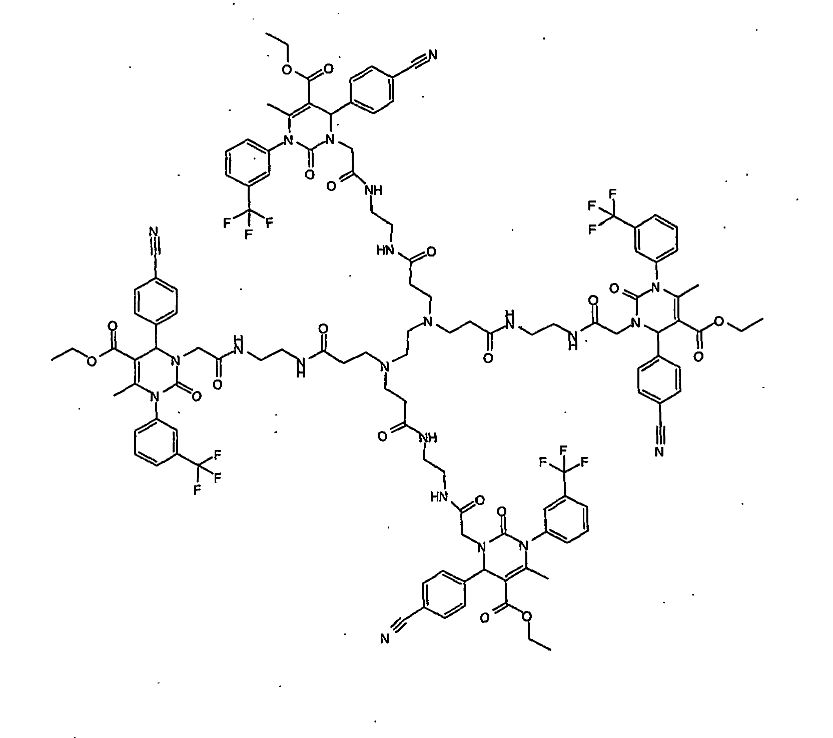

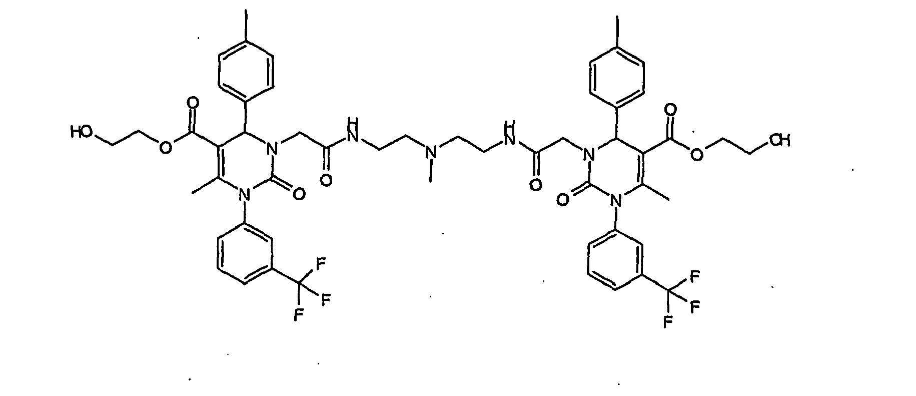

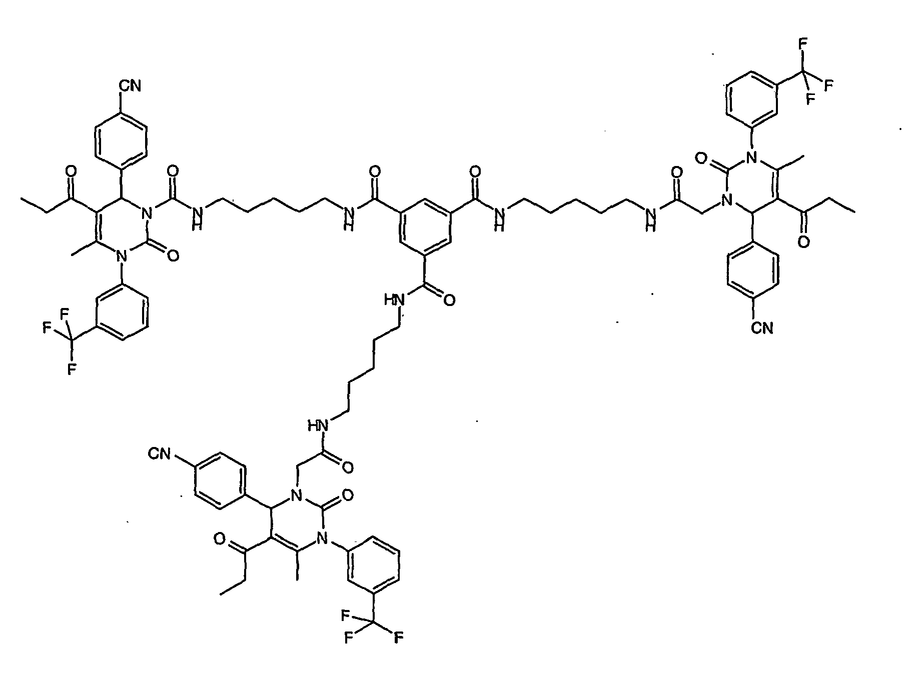

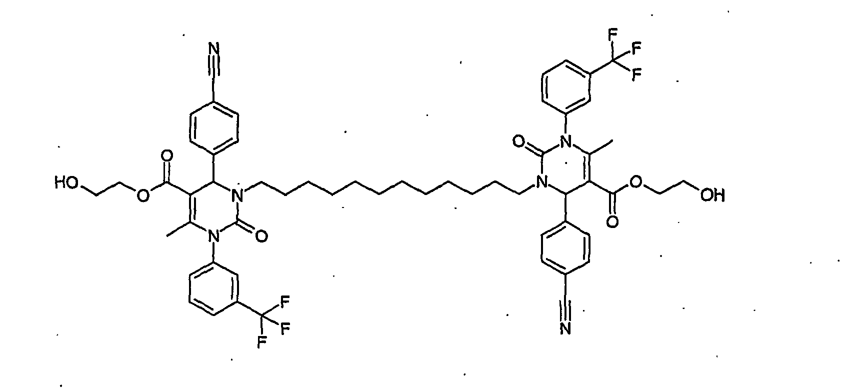

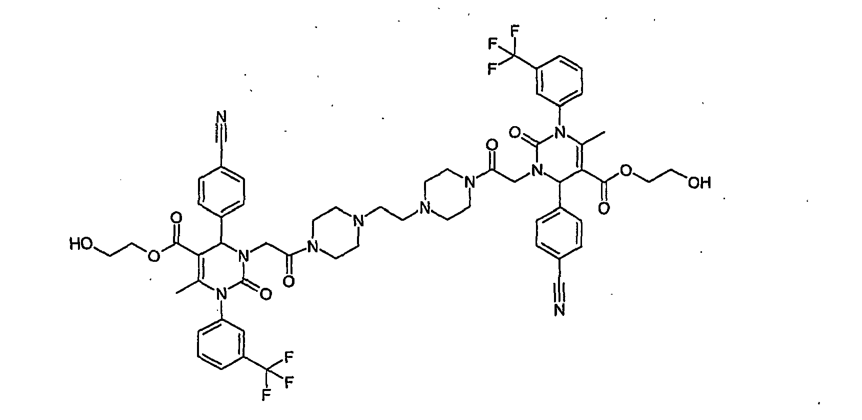

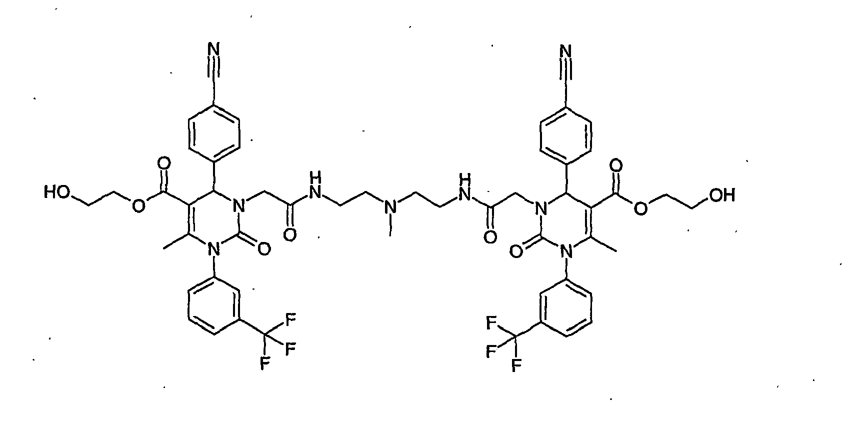

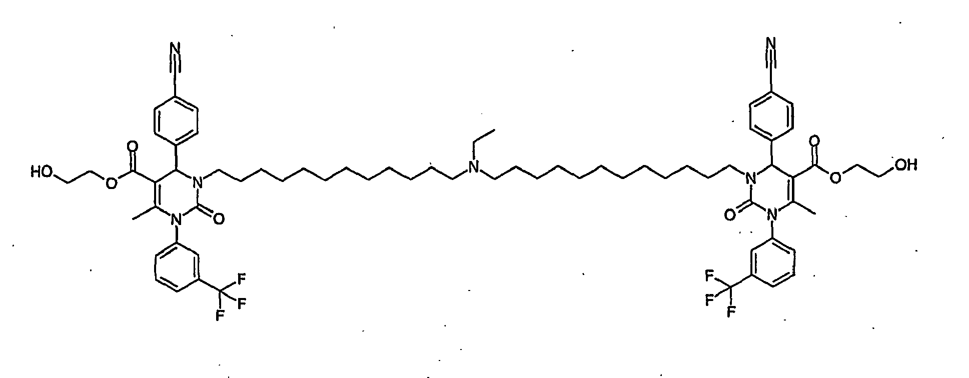

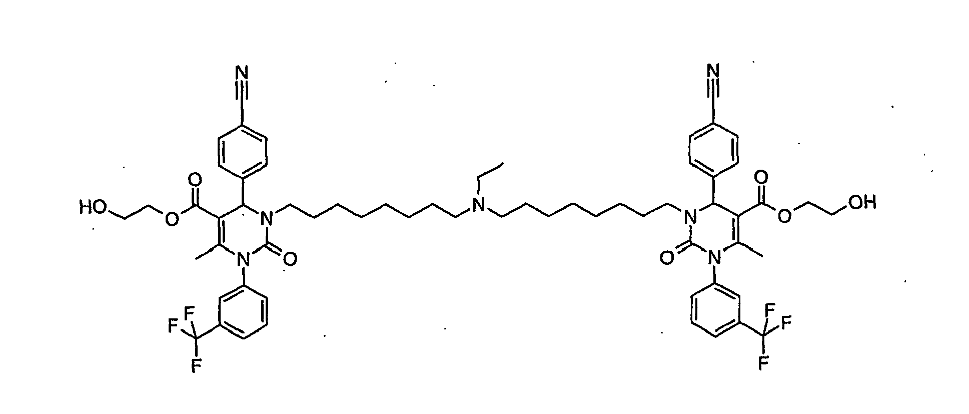

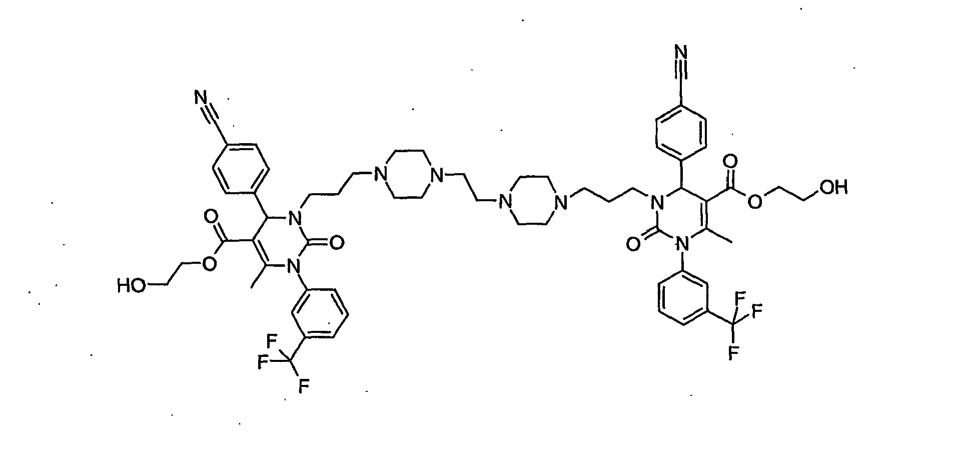

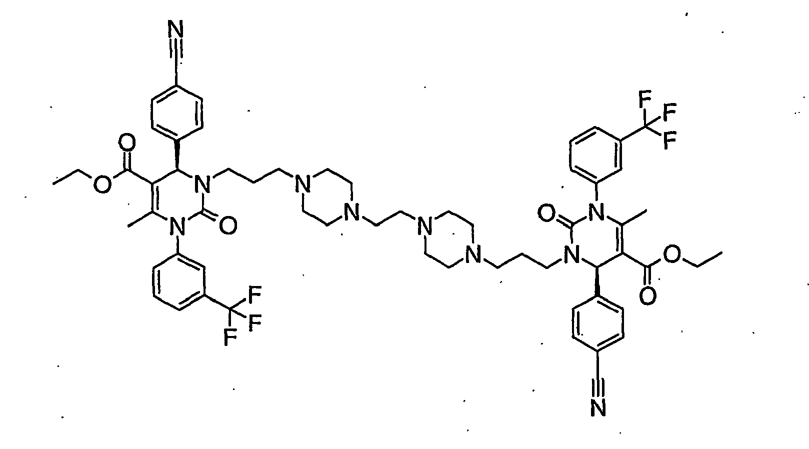

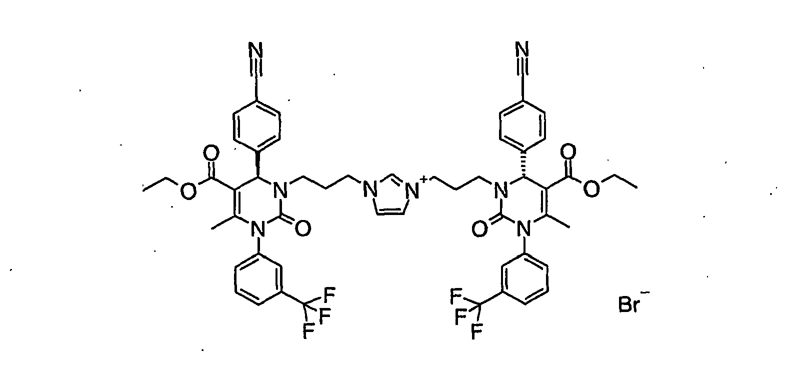

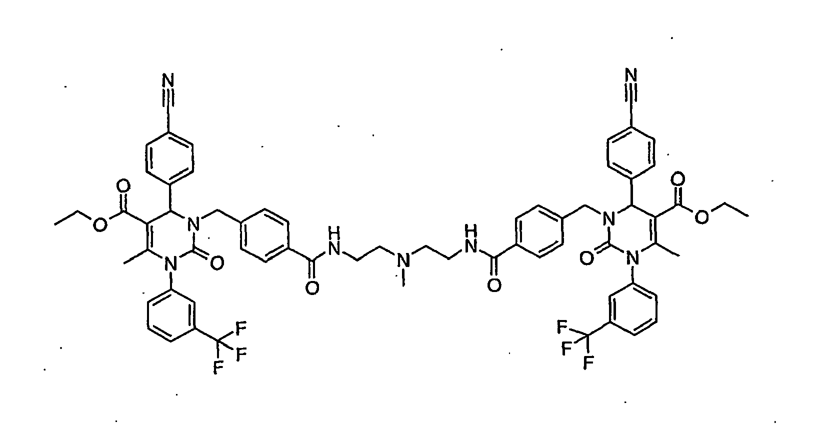

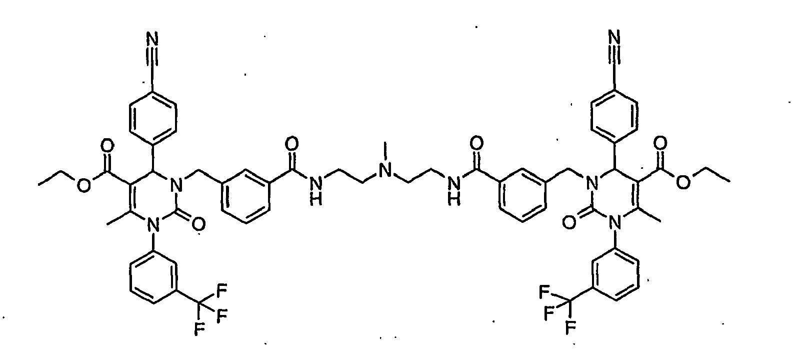

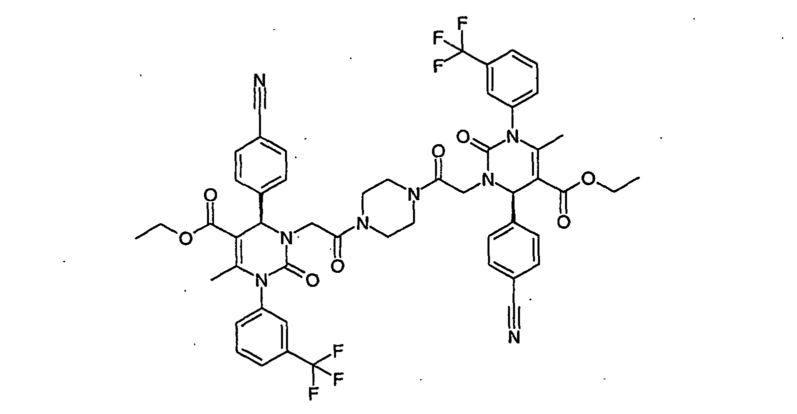

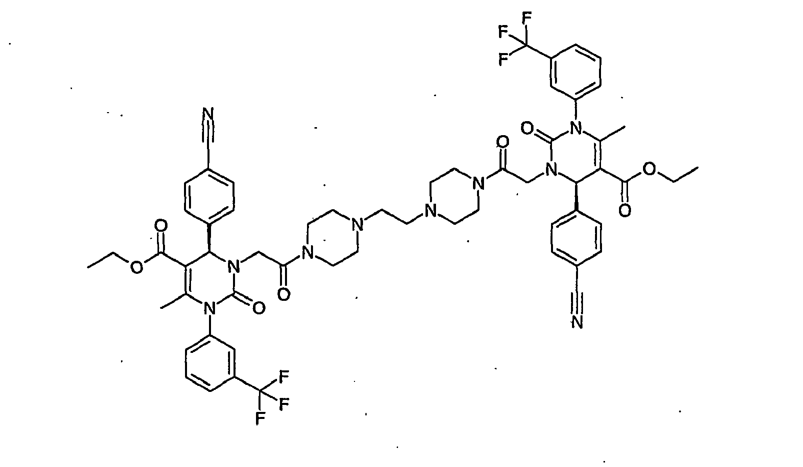

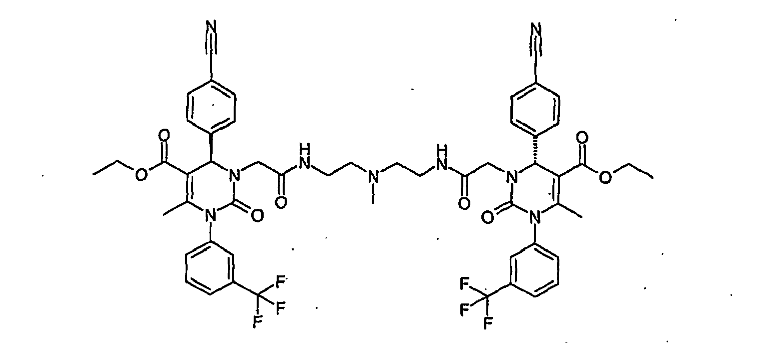

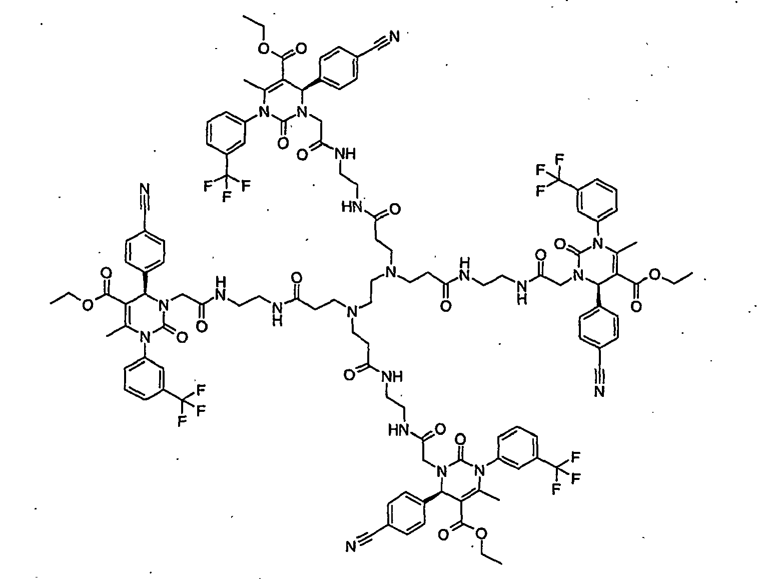

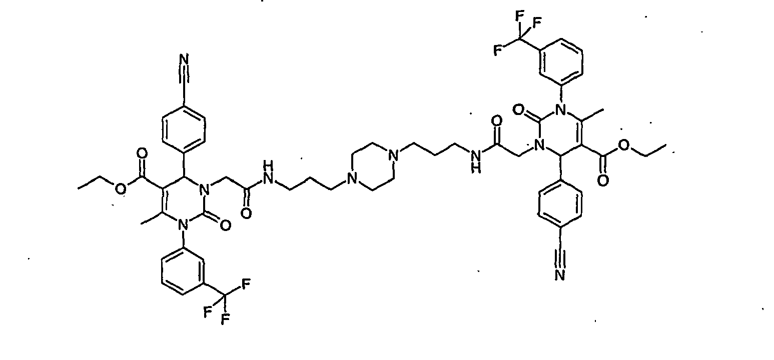

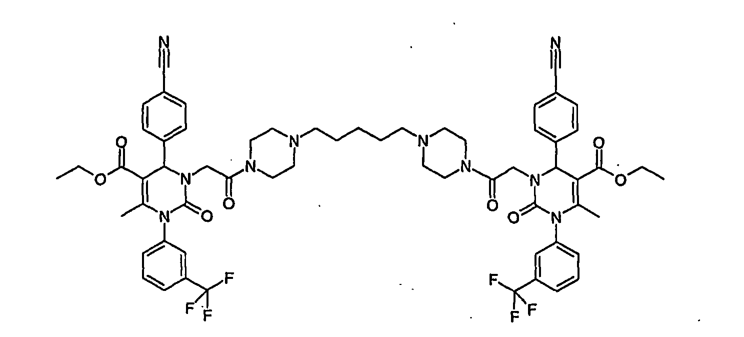

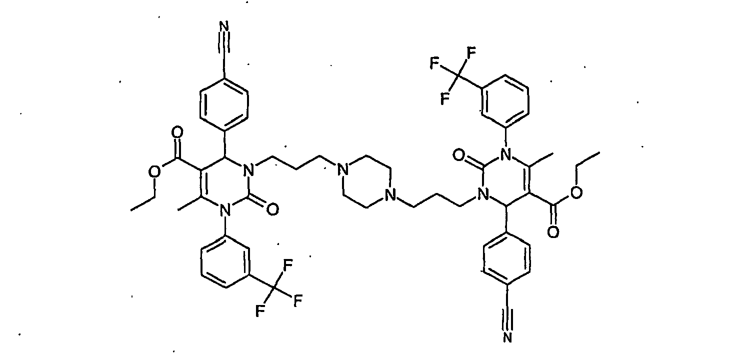

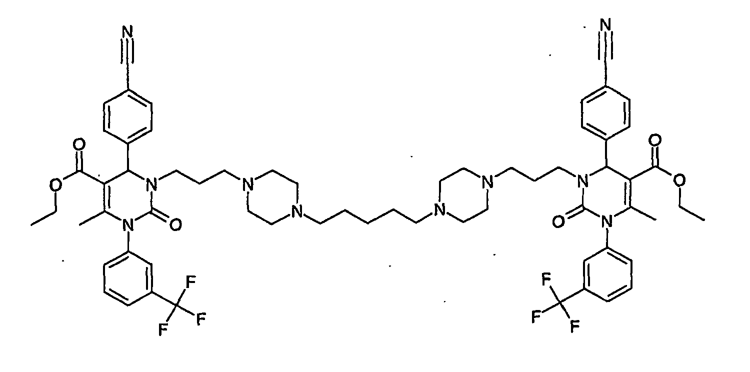

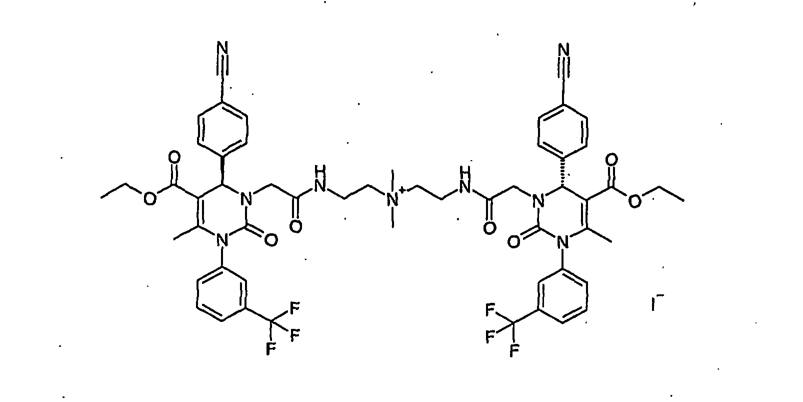

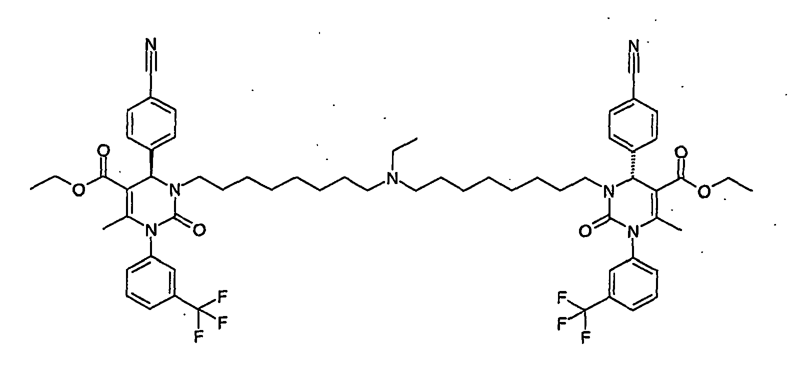

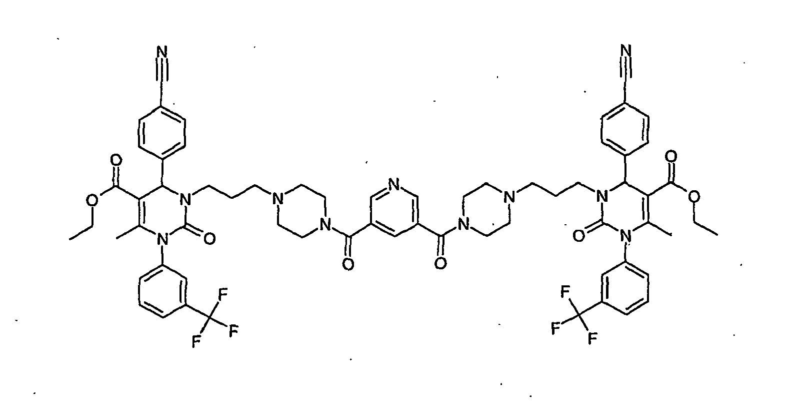

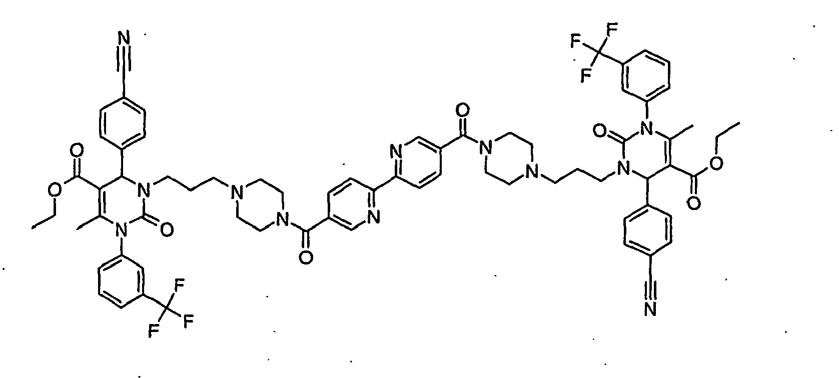

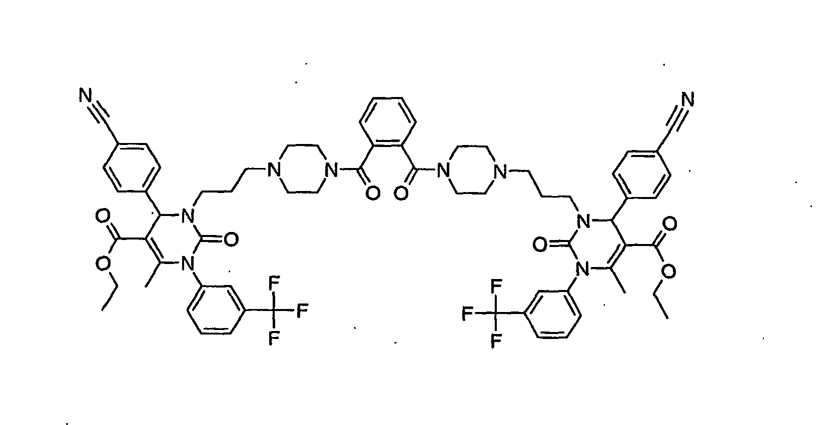

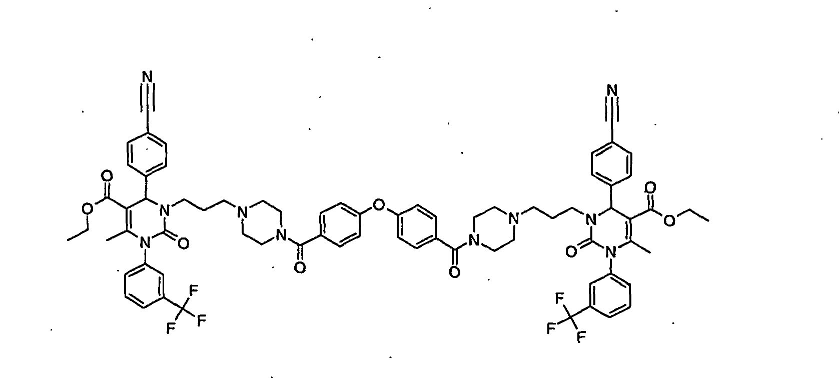

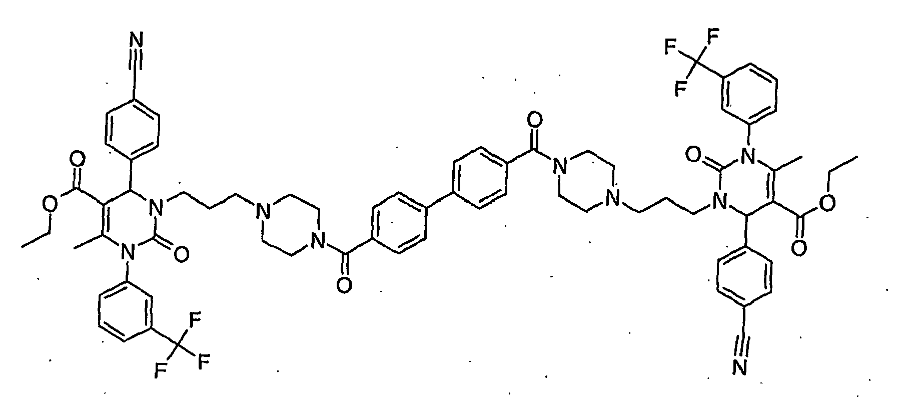

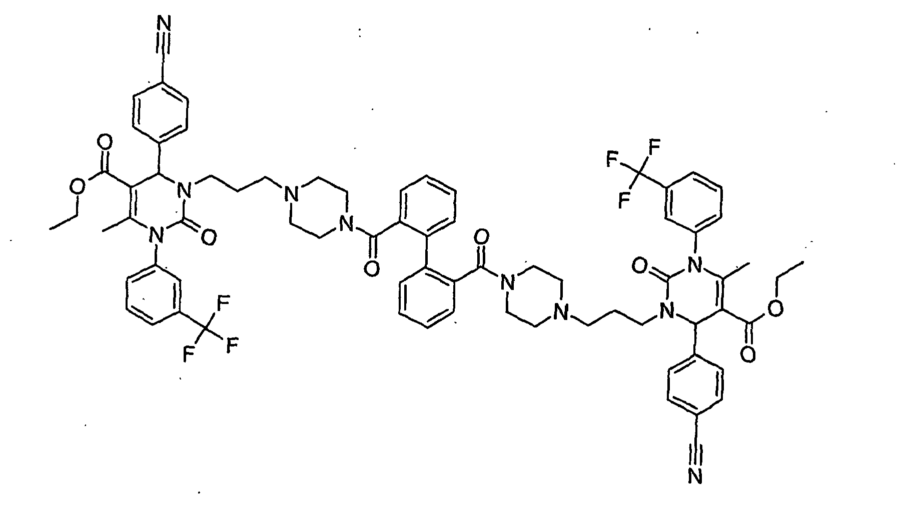

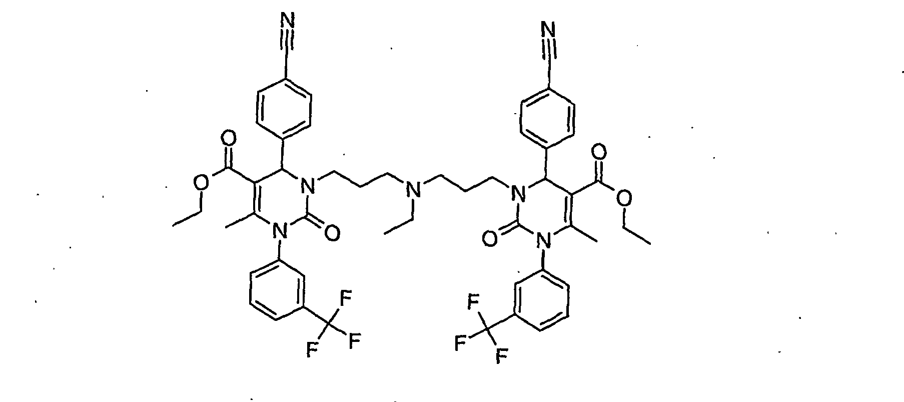

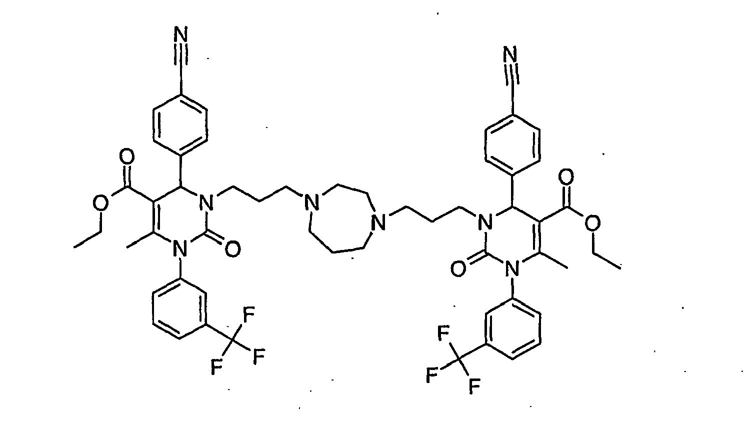

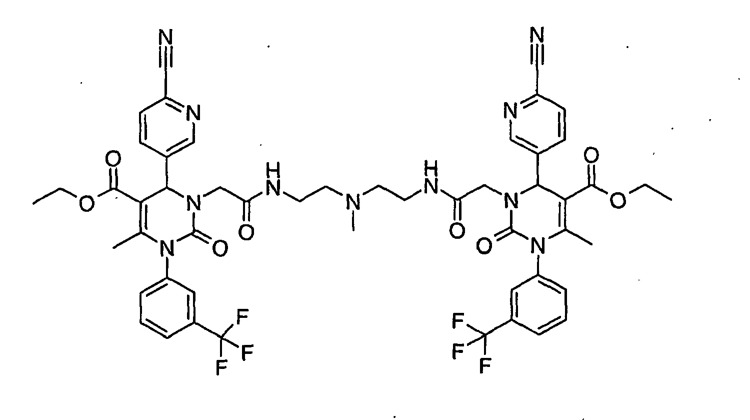

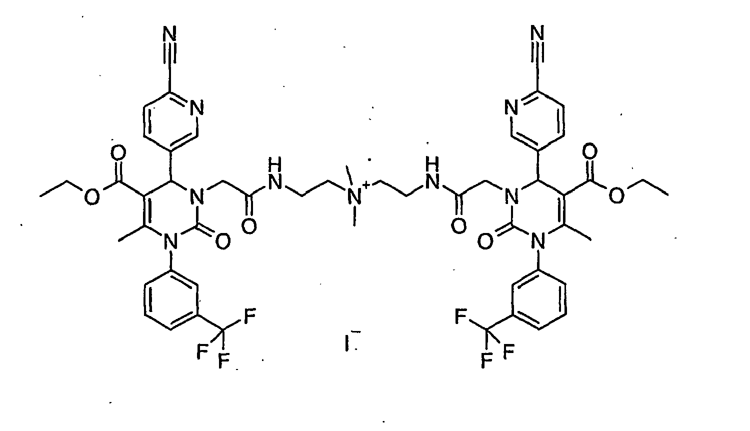

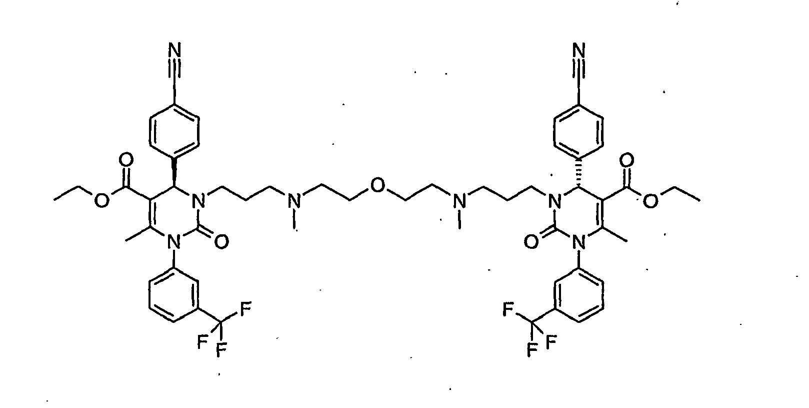

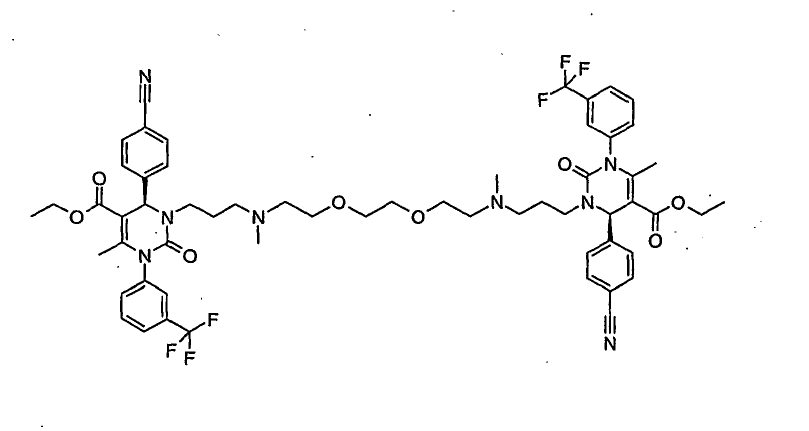

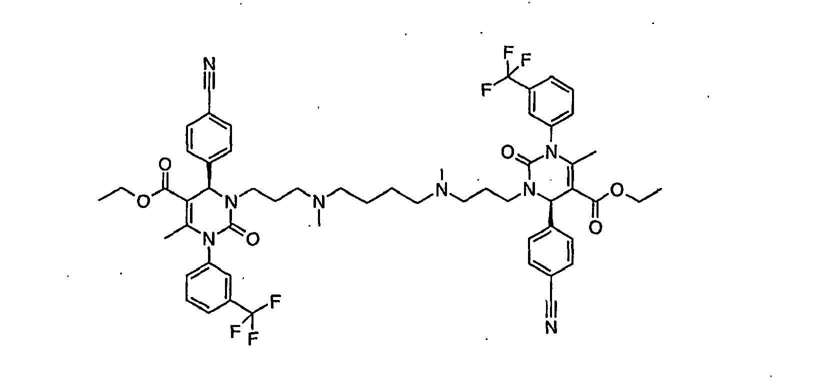

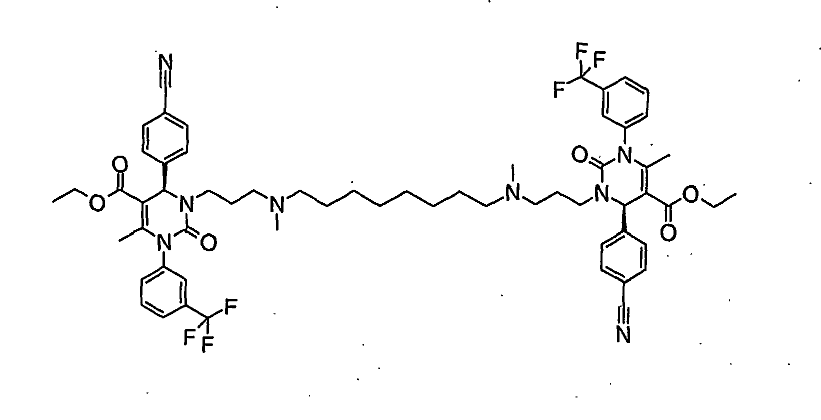

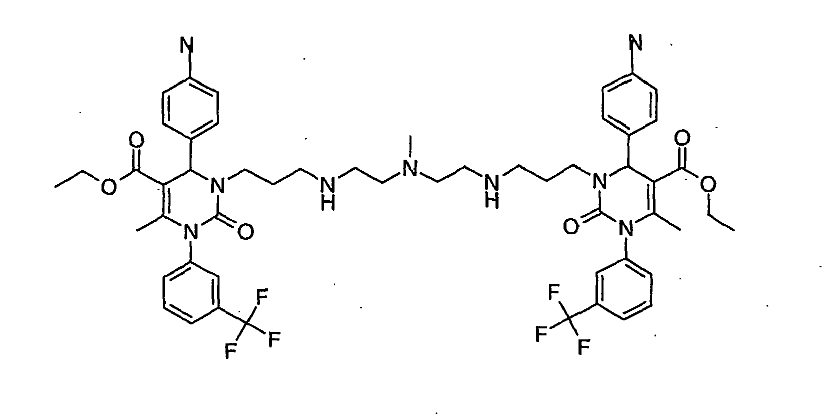

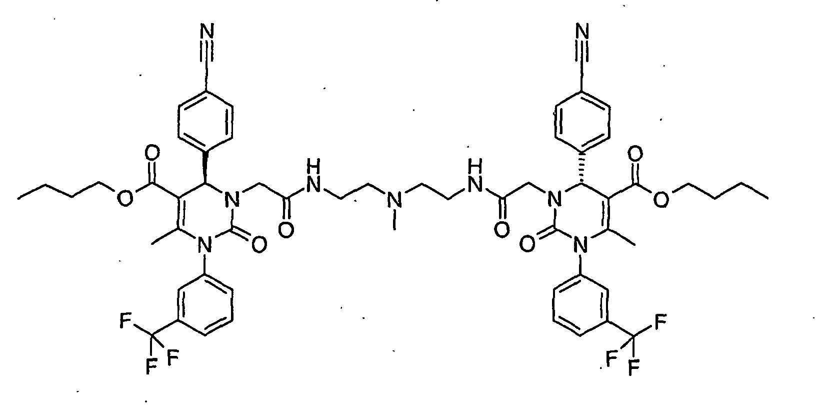

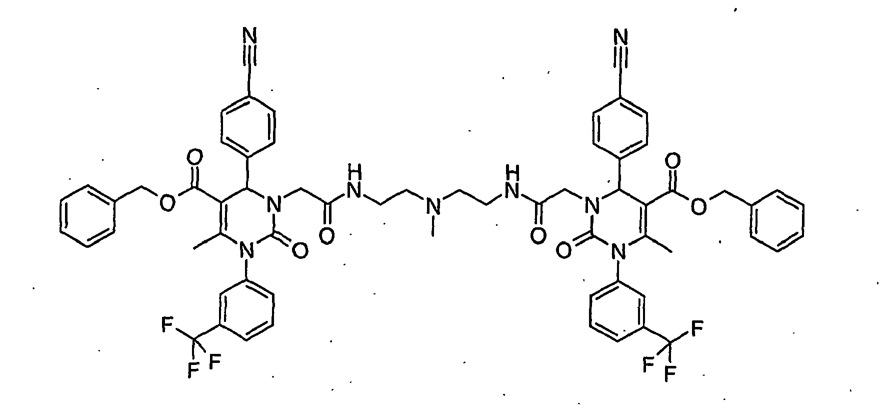

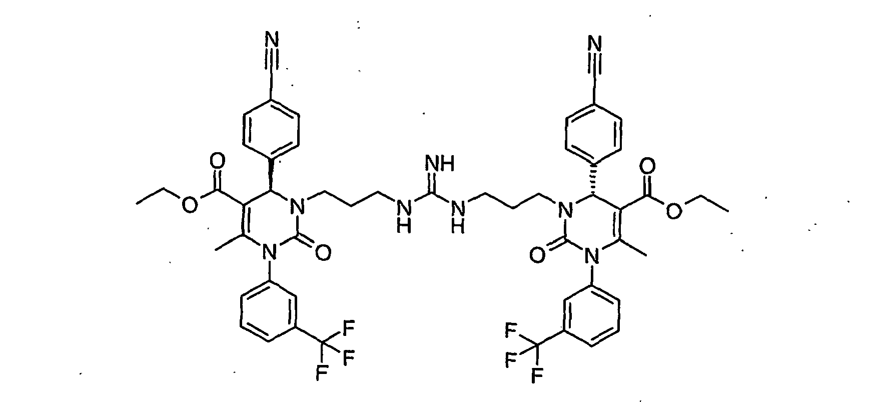

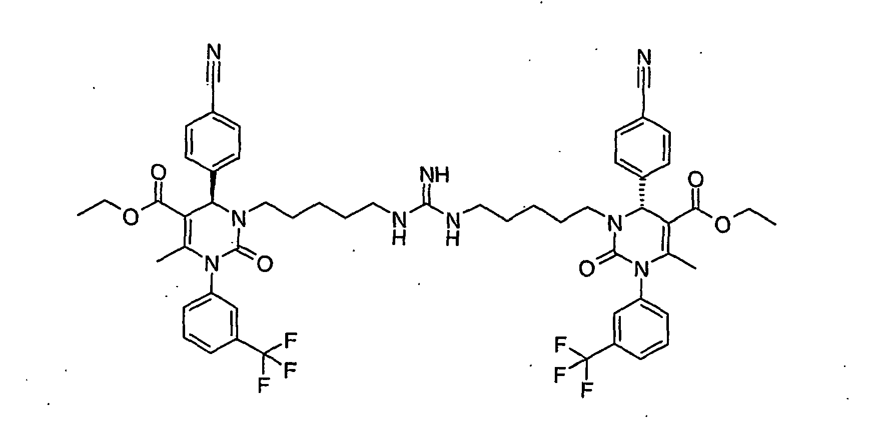

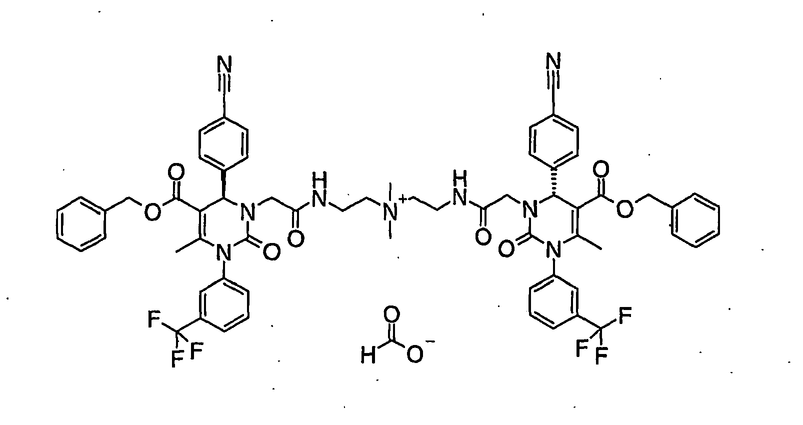

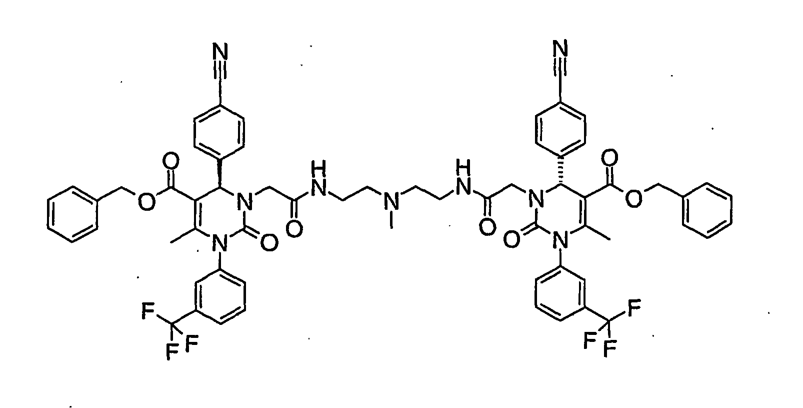

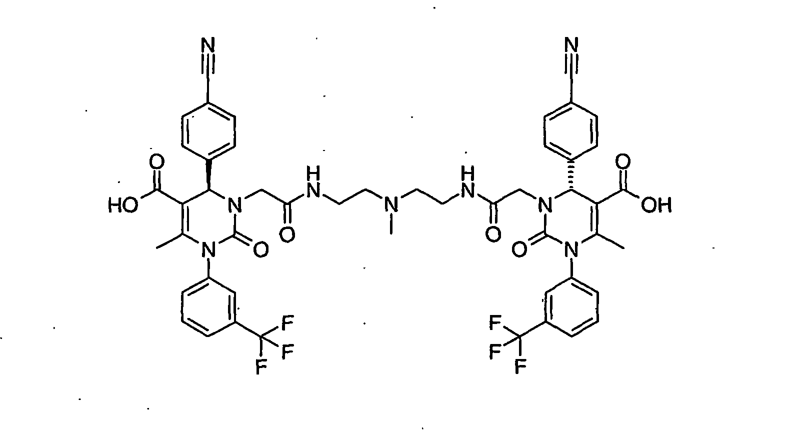

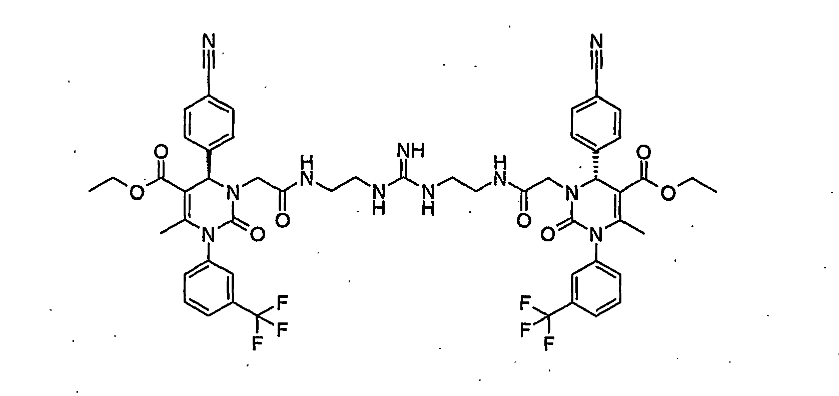

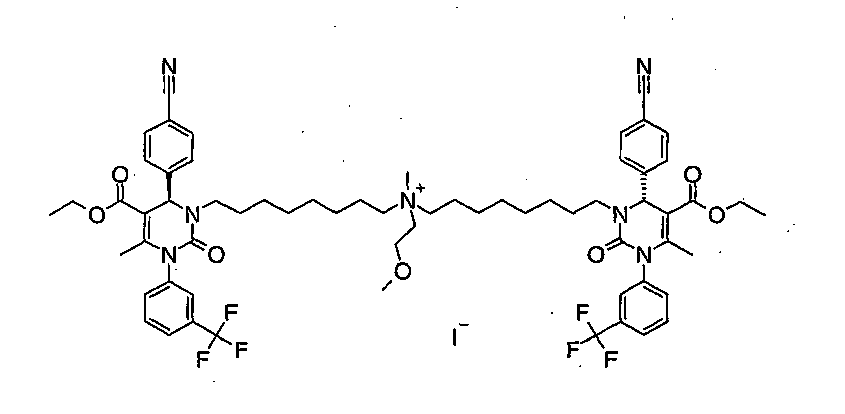

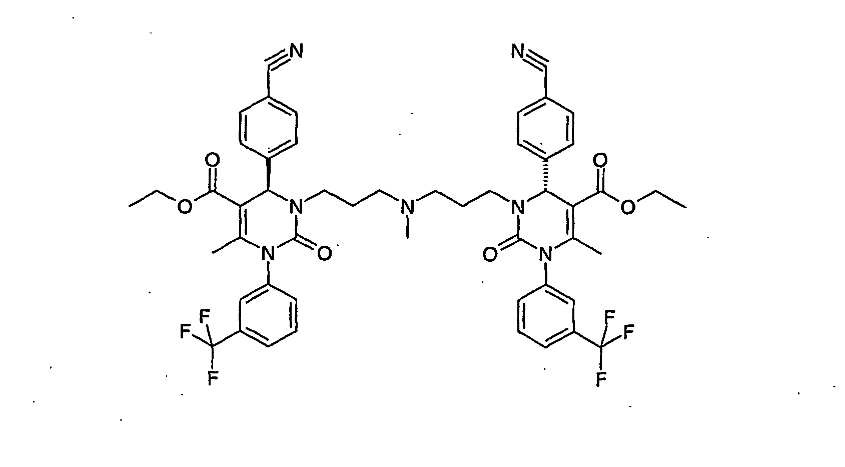

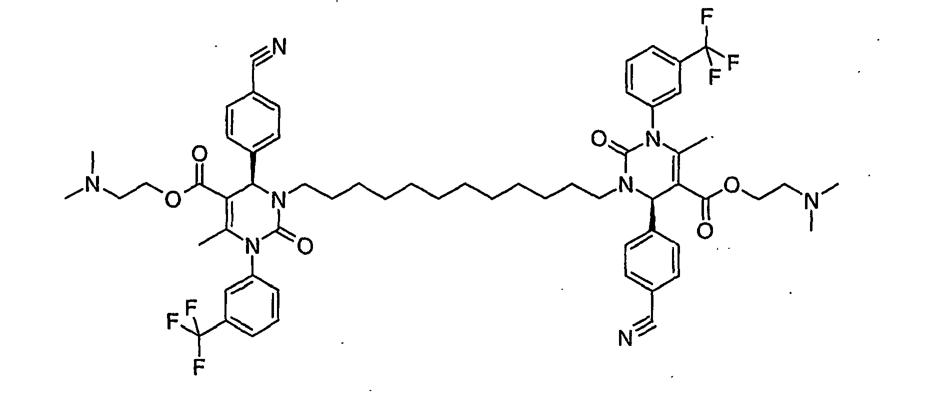

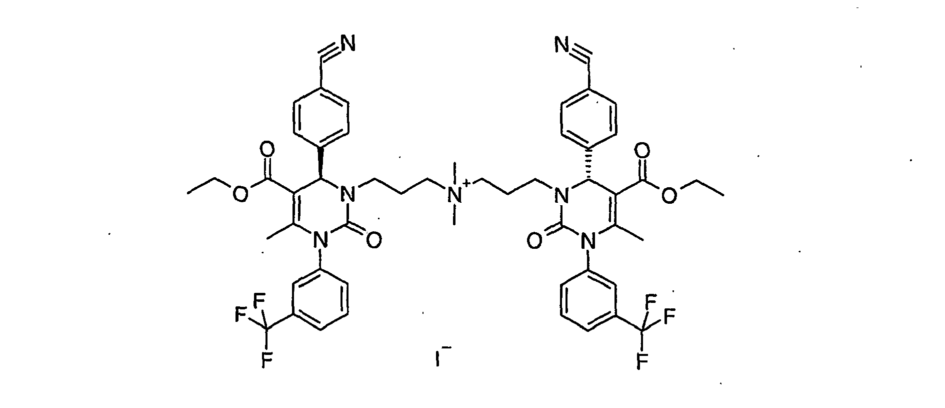

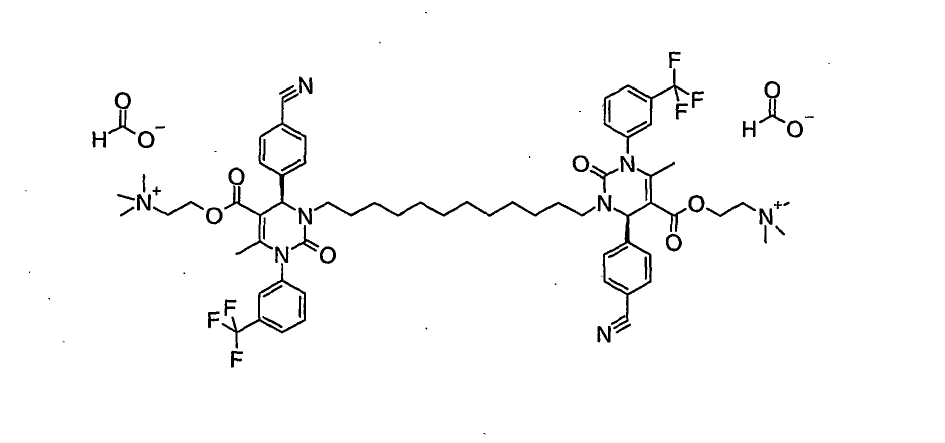

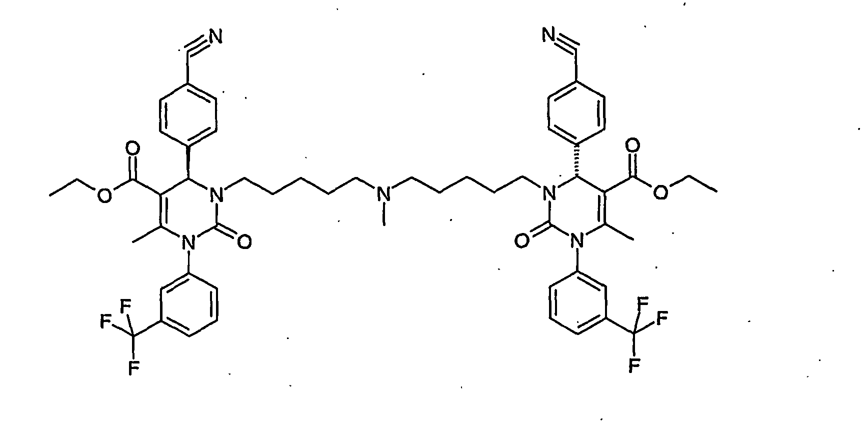

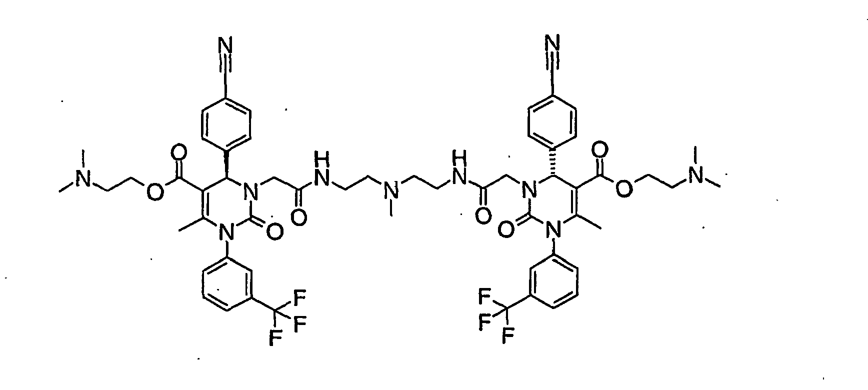

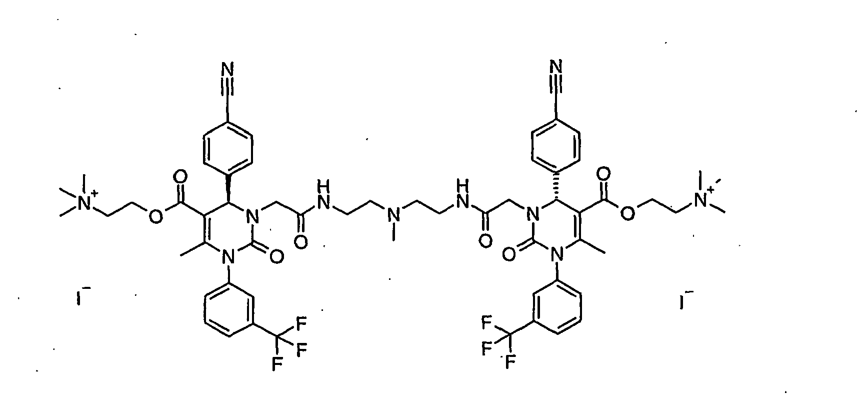

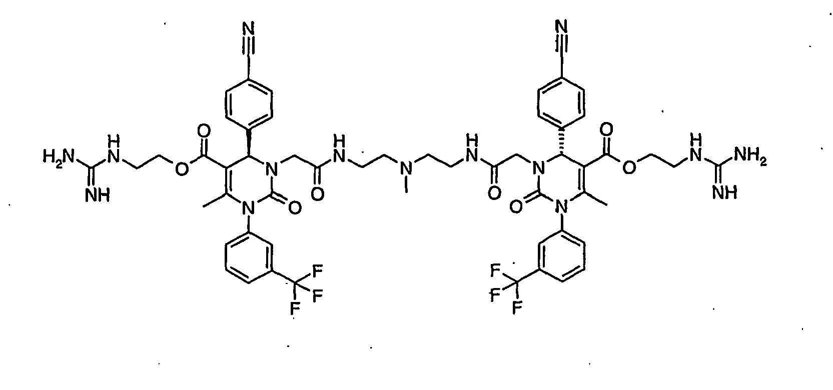

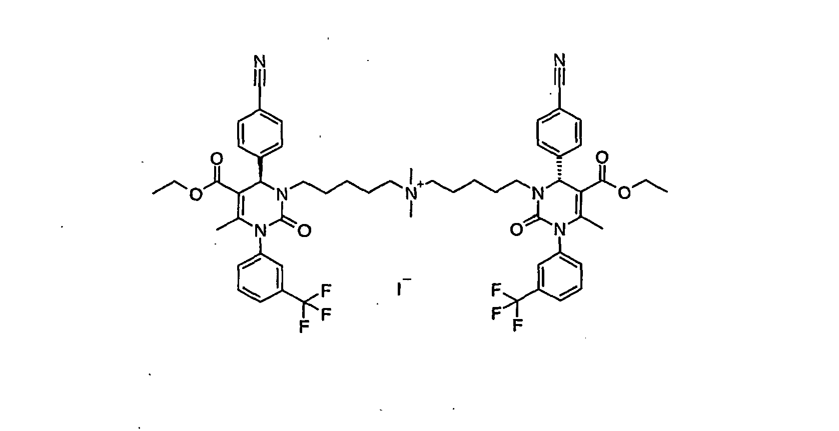

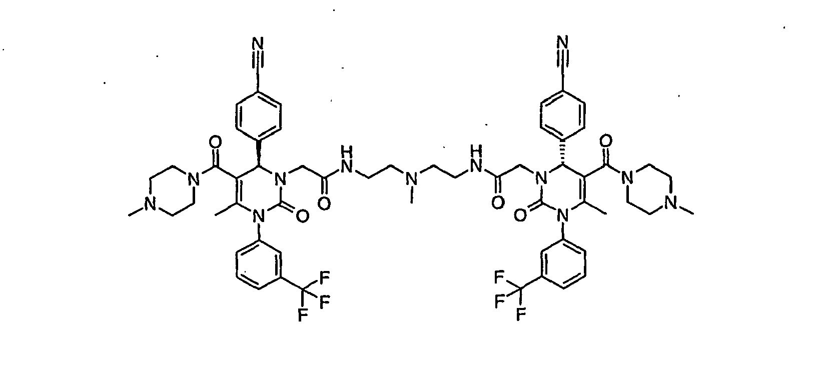

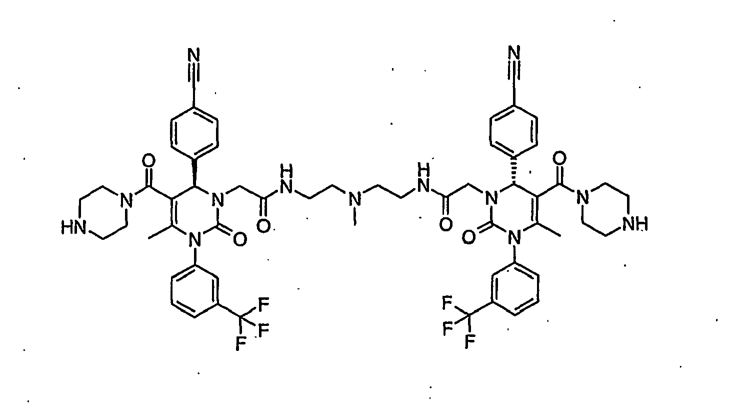

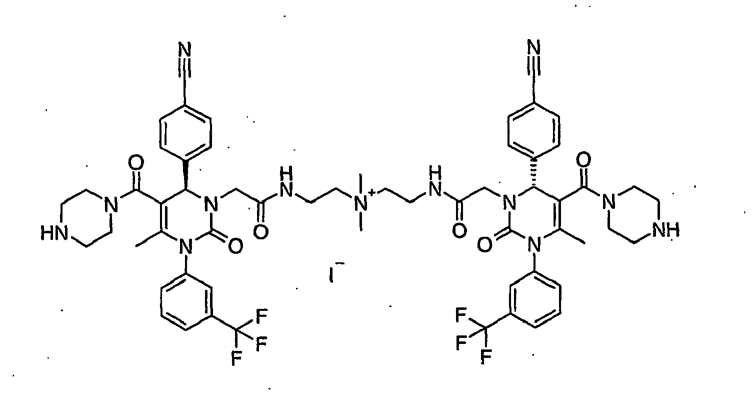

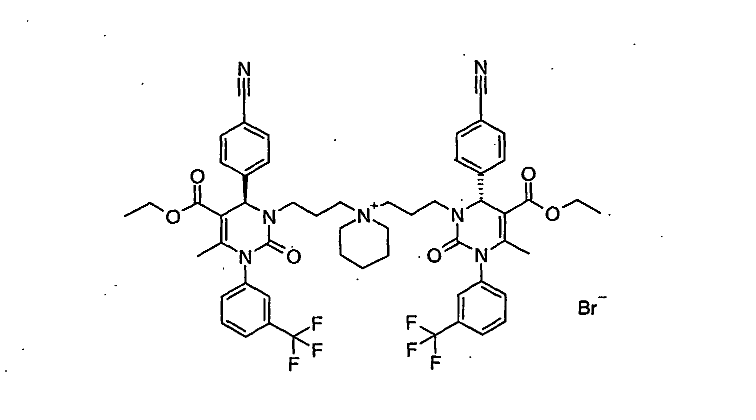

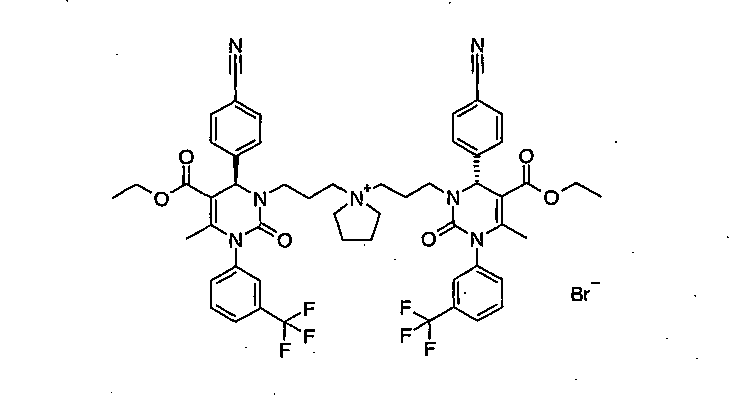

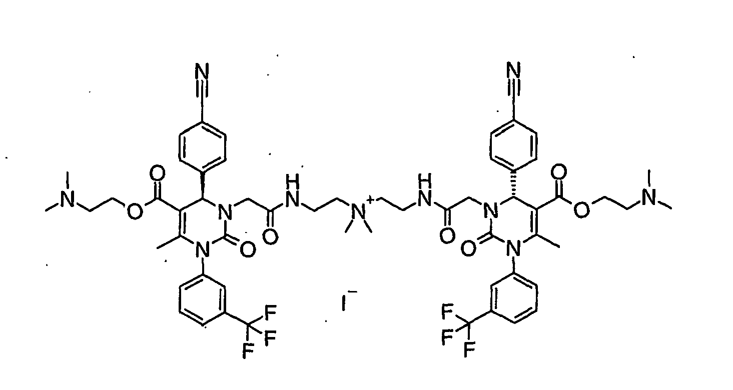

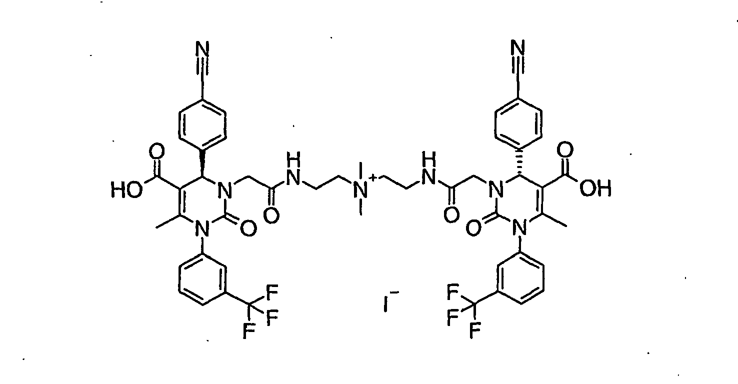

The therapeutic utility of the present compounds is pertinent to any disease that is known to be at least partially mediated by the action of human neutrophil elastase. For example, the present compounds may be beneficial in the treatment of chronic obstructive pulmonary disease (COPD), cystic fibrosis (CF), acute respiratory distress syndrome (ARDS), pulmonary emphysema, pneumonia and lung fibrosis.

The present invention is also concerned with pharmaceutical formulation comprising, as an active ingredient, a compound of the invention. Other compounds may be combined with compounds of this invention for the prevention and treatment of inflammatory diseases of the lung. Thus the present invention is also concerned with pharmaceutical compositions for preventing and treating inflammatory diseases of the lung comprising a therapeutically effective amount of a compound of the invention and one or more other therapeutic agents.

Suitable therapeutic agents for a combination therapy with compounds of the invention include: (1) a corticosteroid, for example fluticasone, cilomilast or budesonide; (2) a β2-adrenoreceptor agonist, for example salmeterol or formeterol; (3) a leukotriene modulator, for example montelukast or pranlukast; (4) muscarinic-3 (M3) receptor antagonists such as tiotropium bromide; (5) bronchodilators that possess both M3 receptor antagonism and β2-adrenoreceptor agonism in a single molecule (6) phosphodiesterase-IV (PDE-IV) inhibitors, for example roflumilast or cilomilast; (7) an antitussive agent, such as codeine or dextramorphan;(8) a non-steroidal antiinflammatory agent (NSAID), for example ibuprofen or ketoprofen; (9) kinase inhibitors such as p38 MAP kinase inhibitors, IKK2 inhibitors and (10) receptor antagonists for cytokines and chemokines for example IL-8, MCP-1, TNFα and IL-1β.

The weight ratio of the first and second active ingredients may be varied and will depend upon the effective dose of each ingredient Generally, an effective dose of each will be used.

The magnitude of prophylactic or therapeutic dose of a compound of the invention will, of course, vary with the nature of the severity of the condition to be treated and with the particular compound and its route of administration. It will also vary according to the age, weight and response of the individual patient. In general, the daily dose range will lie within the range of from about 0.001 mg to about 100 mg per kg body weight of a mammal, preferably 0.01 mg to about 50 mg per kg, and most preferably 0.1 to 10 mg per kg, in single or divided doses. On the other hand, it may be necessary to use dosages outside these limits in some cases.

Another aspect of the present invention provides pharmaceutical compositions which comprise a compound of the invention and a pharmaceutically acceptable carrier. The term "composition", as in pharmaceutical composition, is intended to encompass a product comprising the active ingredient(s), and the inert ingredient(s) (pharmaceutically acceptable excipients) that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by admixing a compound of the invention, additional active ingredient(s), and pharmaceutically acceptable excipients.

The pharmaceutical compositions of the present invention comprise a compound of the invention as an active ingredient or a pharmaceutically acceptable salt thereof, and may also contain a pharmaceutically acceptable carrier and optionally other therapeutic ingredients. The term "pharmaceutically acceptable salts" refers to salts prepared from pharmaceutically acceptable non-toxic bases or acids including inorganic bases or acids and organic bases or acids.

Any suitable route of administration may be employed for providing a mammal, especially a human, with an effective dosage of a compound of the present invention. In therapeutic use, the active compound may be administered by any convenient, suitable or effective route. Suitable routes of administration are known to those skilled in the art, and include oral, intravenous, rectal, parenteral, topical, ocular, nasal, buccal and pulmonary. Delivery by inhalation is preferred.

Compositions suitable for administration by inhalation are known, and may include carriers and/or diluents that are known for use in such compositions. The composition may contain 0.01-99% by weight of active compound. Preferably, a unit dose comprises the active compound in an amount of 1µg to 10 mg.

The most suitable dosage level may be determined by any suitable method known to one skilled in the art. It will be understood, however, that the specific amount for any particular patient will depend upon a variety of factors, including the activity of the specific compound that is used, the age, body weight, diet, general health and sex of the patient, time of administration, the route of administration, the rate of excretion, the use of any other drugs, and the severity of the disease undergoing treatment.

For delivery by inhalation, the active compound is preferably in the form of microparticles. They may be prepared by a variety of techniques, including spray-drying, freeze-drying and micronisation.

By way of example, a composition of the invention may be prepared as a suspension for delivery from a nebuliser or as an aerosol in a liquid propellant, for example for use in a pressurised metered dose inhaler (PMDI). Propellants suitable for use in a PMDI are known to the skilled person, and include CFC-12, HFA-134a, HFA-227, HCFC-22 (CCl2F2) and HFA-152 (CH4F2 and isobutane).

In a preferred embodiment of the invention, a composition of the invention is in dry powder form, for delivery using a dry powder inhaler (DPI). Many types of DPI are known.

Microparticles for delivery by administration may be formulated with excipients that aid delivery and release. For example, in a dry powder formulation, microparticles may be formulated with large carrier particles that aid flow from the DPI into the lung. Suitable carrier particles are known, and include lactose particles; they may have a mass median aerodynamic diameter of greater than 90 µm.

In the case of an aerosol-based formulation, a preferred composition is:

| | |
|---|---|
| Compound of the invention | 24 mg / canister |
| Lecithin, NF Liq. Conc. | 1.2 mg / canister |
| Trichlorofluoromethane, NF | 4.025 g / canister |
| Dichlorodifluoromethane, NF | 12.15 g / canister. |

Compounds of the invention may be used in combination with other drugs that are used in the treatment/prevention/suppression or amelioration of the diseases or conditions for which present compounds are useful. Such other drugs may be administered, by a route and in an amount commonly used therefore, contemporaneously or sequentially with a compound of the invention. When a compound of the invention is used contemporaneously with one or more other drugs, a pharmaceutical composition containing such other drugs in addition to the compound of the invention is preferred. Accordingly, the pharmaceutical compositions of the present invention include those that also contain one or more other active ingredients, in addition to a compound of the invention.

The agents of the invention may be administered in inhaled form. Aerosol generation can be carried out using, for example, pressure-driven jet atomizers or ultrasonic atomizers, preferably using propellant-driven metered aerosols or propellant-free administration of micronized active compounds from, for example, inhalation capsules or other "dry powder" delivery systems.

The active compounds may be dosed as described depending on the inhaler system used. In addition to the active compounds, the administration forms may additionally contain excipients, such as, for example, propellants (e.g. Frigen in the case of metered aerosols), surface-active substances, emulsifiers, stabilizers, preservatives, flavorings, fillers (e.g. lactose in the case of powder inhalers) or, if appropriate, further active compounds.

For the purposes of inhalation, a large number of systems are available with which aerosols of optimum particle size can be generated and administered, using an inhalation technique which is appropriate for the patient. In addition to the use of adaptors (spacers, expanders) and pear-shaped containers (e.g. Nebulator®, Volumatic®), and automatic devices emitting a puffer spray (Autohaler®), for metered aerosols, in particular in the case of powder inhalers, a number of technical solutions are available (e.g. Diskhaler®, Rotadisk®, Turbohaler® or the inhalers for example as described EP-A-0505321).

The compounds of the invention of the present invention can be prepared according to the procedures of the following schemes and examples, using appropriate materials, and are further exemplified by the following specific examples. Moreover, by utilising the procedures described with the disclosure contained herein, one of ordinary skill in the art can readily prepare additional compounds of the present invention claimed herein. The compounds illustrated in the examples are not, however, to be construed as forming the only genus that is considered as the invention. The examples further illustrate details for the preparation of the compounds of the present invention. Those skilled in the art will readily understand that known variations of the conditions and processes of the following preparative procedures can be used to prepare these compounds.

The compounds of the invention may be isolated in the form of their pharmaceutically acceptable salts, such as those described previously herein above. The free acid form corresponding to isolated salts can be generated by neutralisation with a suitable acid such as acetic acid and hydrochloric acid and extraction of the liberated free acid into an organic solvent followed by evaporation. The free acid form isolated in this manner can be further converted into another pharmaceutically acceptable salt by dissolution in an organic solvent followed by addition of the appropriate base and subsequent evaporation, precipitation, or crystallisation.

It may be necessary to protect reactive functional groups (e.g. hydroxy, amino, thio or carboxy) in intermediates used in the preparation of compounds of the invention to avoid their unwanted participation in a reaction leading to the formation of the compounds. Conventional protecting groups, for example those described by T. W. Greene and P. G. M. Wuts in "Protective groups in organic chemistry" John Wiley and Sons, 1999, may be used.

The following reaction schemes illustrate how compounds of the invention, in particular the Example compounds may be prepared. It will be understood that the processes detailed below are solely for the purpose of illustrating the invention and should not be construed as limiting. A process utilising similar or analogous reagents and/or conditions known to one skilled in the art may also be used to obtain a compound of the invention.

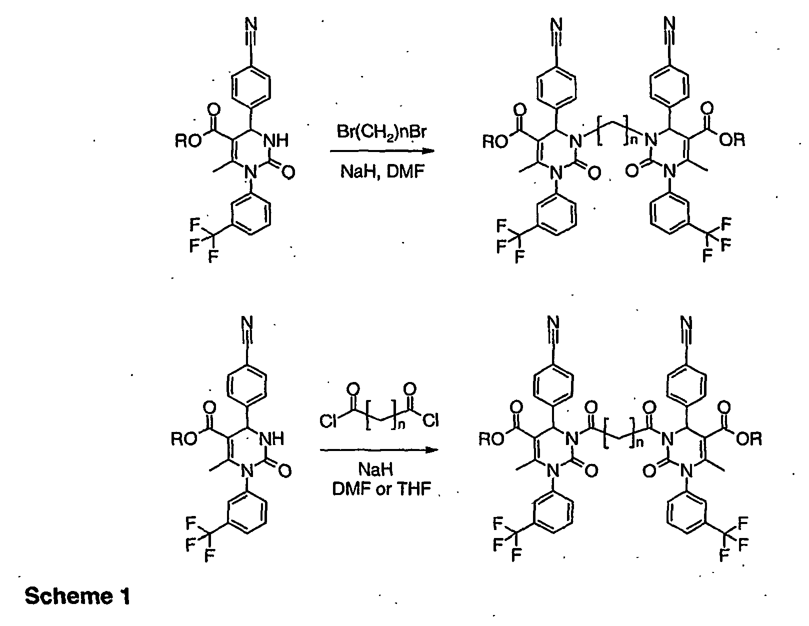

The monomers may be joined together by a range of standard chemistries. For example, alkylation with a suitable qω-difunctionalised alkane can be effected in the presence of a suitable base, for example a metal hydride and optional solvent. Suitable leaving groups include halogen and sulfonate. Similarly, monomers may be joined by bis-acylation with a bis acid halide under similar conditions, Scheme 1.

An alternative methodology involves attachment of a spacer group incorporating a second functional group, which subsequently allows attachment of a bidenetate linker group, as in Scheme 2. Alkylation of the monomer with a suitably substituted ω-halo acid (protected as an ester), ω-haloaldehyde (protected as an acetal), or qω-dihaloalkane, employing a base such as NaH in a suitable inert solvent such as DMF or THF, followed by deprotection as necessary leads to intermediates that can be reacted with suitable bidentate species to generate compounds of the invention, Scheme 2. 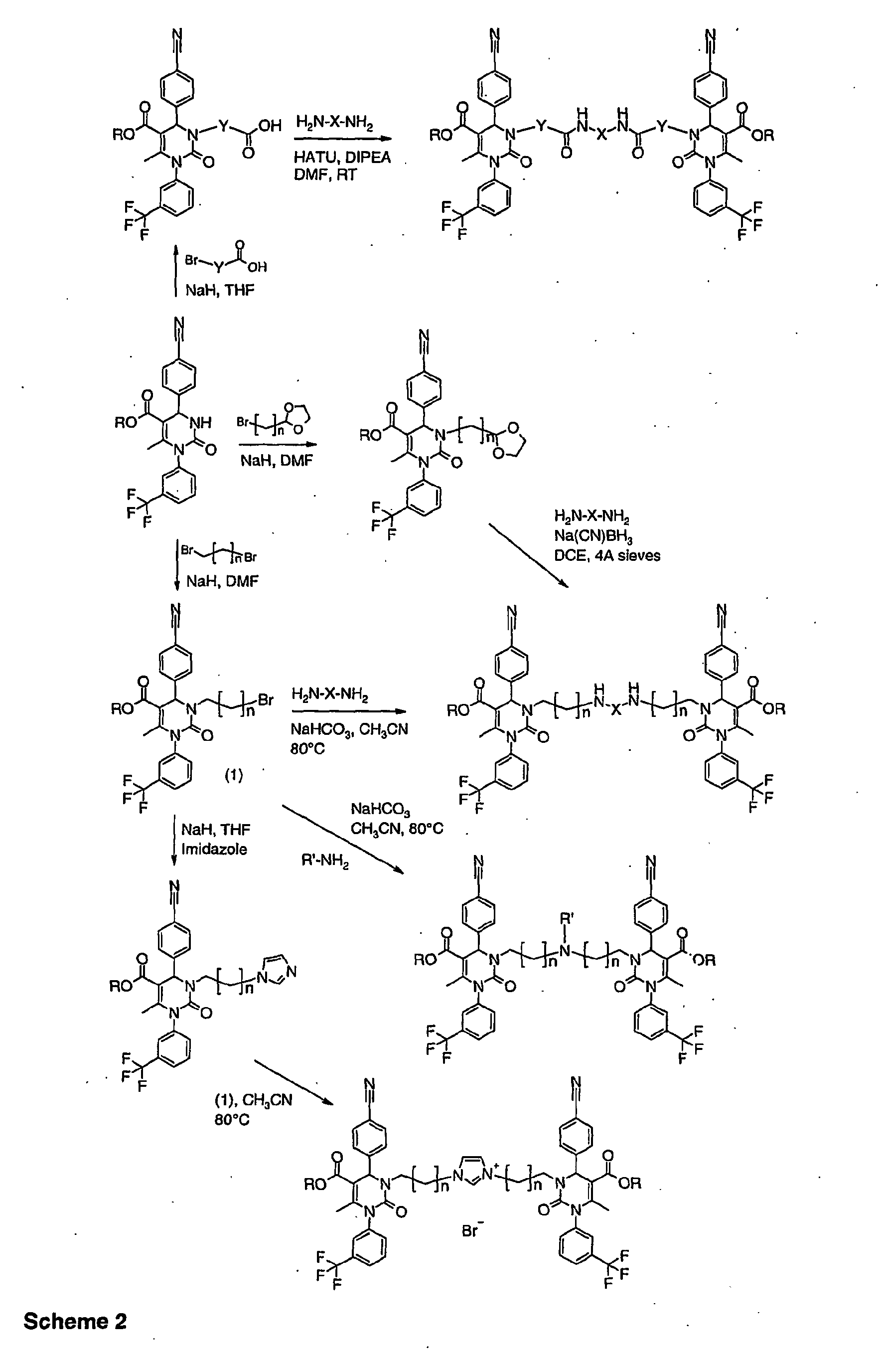 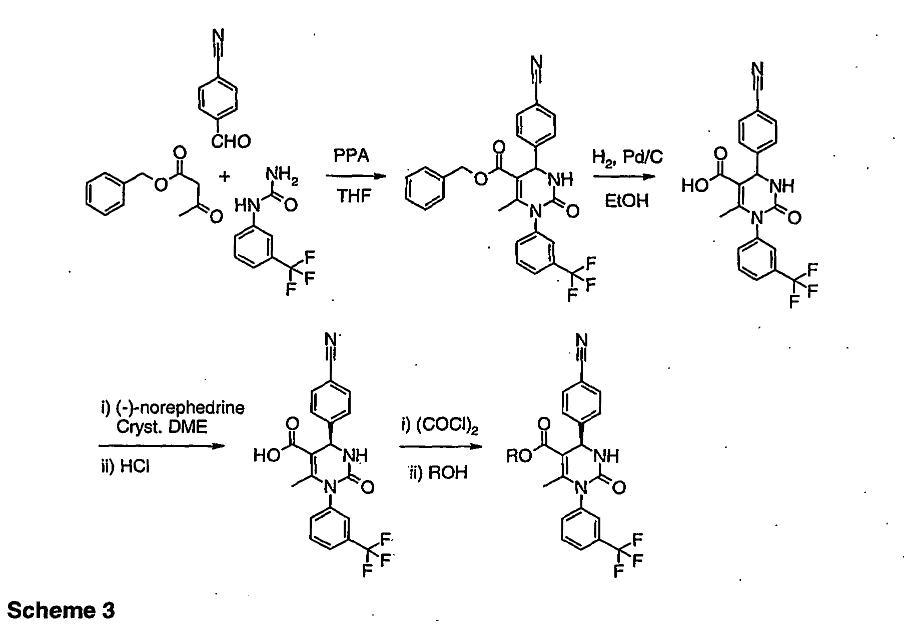 The monomers of Formula (II) may be prepared as racemates according to methods described in WO2004024700, WO2004024701, GB2392910, WO200508263 and WO2005082864. The monomers may be separated by chiral HPLC into their enantiomers.

Alternatively the monomer (as a carboxylic acid) may be resolved by formation of diastereomeric salts with a suitable chiral base, such as norephedrine, followed by fractional recrystallisation, Scheme 3.

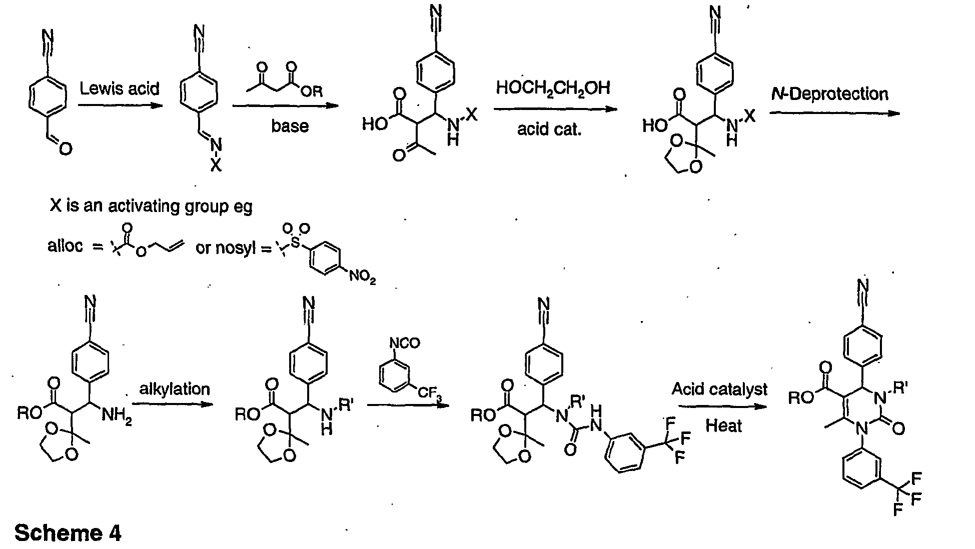

An alternative method for the preparation of compounds of Formula (II) is illustrated in Scheme 4.

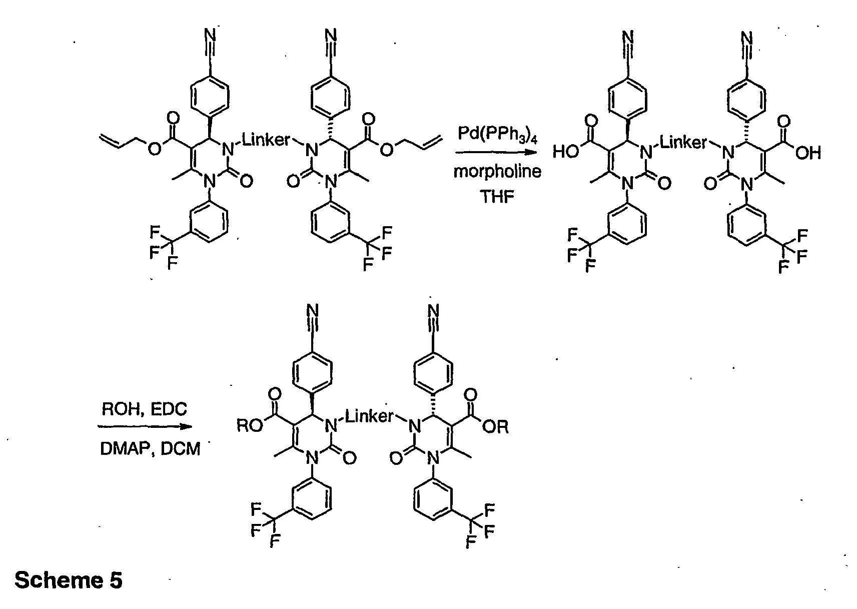

In some cases the ester corresponding to R⁴ of compounds of Formula (II) may be hydrolysed to a parent acid using suitable conditions, and then interconverted to other esters using standard ester coupling conditions. Additionally, the acid may be converted to amides and other compounds of the invention using standard methods familiar to those skilled in the art, Scheme 5.

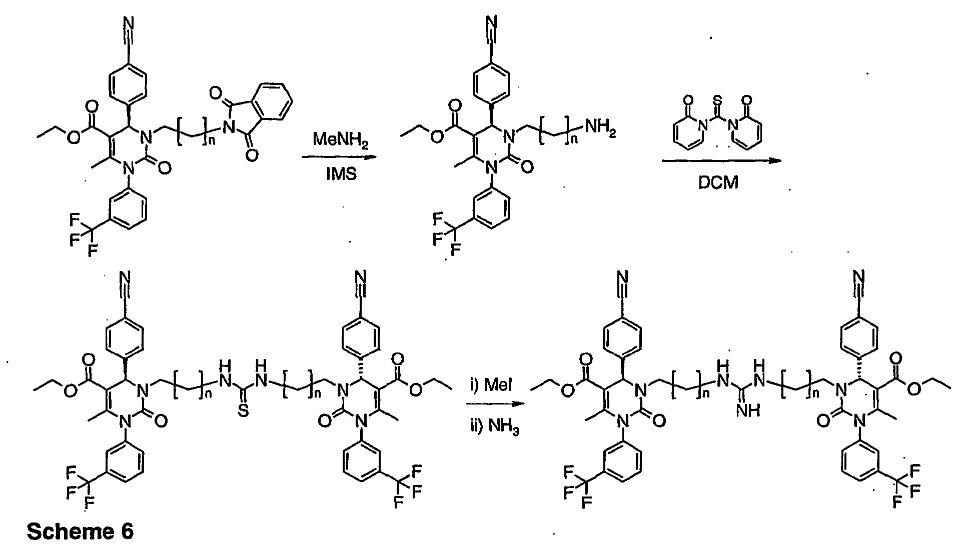

Compounds of Formula (II) containing a guanidine moiety in the linker group may be prepared according to the method outlined in Scheme 6.

Compounds that are quaternary ammonium salts generally have counter-ions that are derived from the alkylating agent used in the quaternisation, i.e reaction of a tertiary amine with methyl iodide leads to a quaternary ammonium iodide. This counter-ion can be exchanged to another counter-ion according to the method of Bach Nielsen, Frydenvang, Liljeforts, Buur & Larsen, Eur. J. Pharm. Sci, 24 (2005) 85-93. Compounds containing alternative counter-ions may be expected to have different

### General Experimental Details:

All reactions were carried out under an inert atmosphere of nitrogen unless specified otherwise.

Where products were purified by column chromatography on silica, 'silica' refers to silica gel for chromatography, 0.035 to 0.070 mm (220 to 440 mesh) (e.g. Fluka silica gel 60), and an applied pressure of nitrogen up to 10 p.s.i for accelerated column elution. All solvents and commercial reagents were used as received.

### Preparative HPLC conditions:

### HPLC system 1:

C18-reverse-phase column (100 × 22.5 mm i.d Genesis column with 7 µm particle size), eluting with a gradient of A: water + 0.1 % TFA; B: acetonitrile + 0.1 % TFA at a flow rate of 5 ml/min and gradient of 1 %/min increasing in B. UV detection at 230 nm.

### HPLC system 2:

Phenyl hexyl column (250 × 21.20 mm Luna column with 10 µm particle size), eluting with an isocratic mixture of A: water + 0.1% TFA; B: acetonitrile + 0.1 % TFA at a flow rate of 20 ml/min with UV detection at 254 nm.

### HPLC system 3:

C18-reverse-phase column (100 × 22.5 mm i.d Genesis column with 7 µm particle size), eluting with a gradient of A: water + 0.1% formic acid; B: acetonitrile + 0.1 % formic acid at a flow rate of 5 ml/min and gradient of 1 %/min increasing in B. UV detection at 230 nm.

### HPLC system 4:

C18-reverse-phase column (100 × 22.5 mm i.d Genesis column with 7 µm particle size), eluting with a gradient of A: water + 0.1 % formic acid; B: MeOH + 0.1 % formic acid at a flow rate of 5 ml/min and gradient of 1 %/min increasing in B. UV detection at 230 nm.

### HPLC system 5:

Phenyl hexyl column (250 × 21.20 mm Luna column with 10 µm particle size), eluting with an isocratic mixture of A: water + 0.1 % TFA; B: MeOH + 0.1 % TFA at a flow rate of 20 ml/min with UV detection at 254 nm.

After MPLC purification, fractions containing product were combined and freeze-dried to give the product as a white or off-white solid. In some cases, where the compound contained a basic centre, the product was obtained as the TFA or formate salt.

### LC/MS Systems

The Liquid Chromatography Mass Spectroscopy (LC/MS) systems used:

### LC-MS method 1

Micromass Platform LCT with a C18-reverse-phase column (100 × 3.0 mm Higgins Clipeus with 5 µm particle size), elution with A: water + 0.1% formic acid; B: acetonitrile + 0.1 % formic acid. Gradient:

| Gradient-Time | flow ml/min | %A | %B |
|---|---|---|---|
| 0.00 | 1.0 | 95 | 5 |
| 1.00 | 1.0 | 95 | 5 |
| 15.00 | 1.0 | 5 | 95 |
| 20.00 | 1.0 | 5 | 95 |
| 22.00 | 1.0 | 95 | 5 |
| 25.00 | 1.0 | 95 | 5 |

Detection - MS, ELS, UV (100 µl split to MS with in-line UV detector)
MS ionisation method - Electrospray (positive ion)

### LC-MS method 2

Micromass Platform LCT with a C18-reverse-phase column (30 × 4.6 mm Phenomenex Luna 3 µm particle size), elution with A: water + 0.1 % formic acid; B: acetonitrile + 0.1 % formic acid. Gradient:

| Gradient-Time | flow ml/min | %A | %B |
|---|---|---|---|
| 0.00 | 2.0 | 95 | 5 |
| 0.50 | 2.0 | 95 | 5 |
| 4.50 | 2.0 | 5 | 95 |
| 5.50 | 2.0 | 5 | 95 |
| 6.00 | 2.0 | 95 | 5 |

Detection - MS, ELS, UV (100 µl split to MS with in-line UV detector)
MS ionisation method - Electrospray (positive and negative ion)

### LC-MS method 3

Waters Micromass ZQ with a C18-reverse-phase column (30 × 4.6 mm Phenomenex Luna 3 µm particle size), elution with A: water + 0.1 % formic acid; B: acetonitrile + 0.1 % formic acid. Gradient:

| Gradient-Time | flow ml/min | %A | %B |
|---|---|---|---|
| 0.00 | 2.0 | 95 | 5 |
| 0.50 | 2.0 | 95 | 5 |
| 4.50 | 2.0 | 5 | 95 |
| 5.50 | 2.0 | 5 | 95 |
| 6.00 | 2.0 | 95 | 5 |

Detection - MS, ELS, UV (100 µl split to MS with in-line UV detector)
MS ionisation method - Electrospray (positive and negative ion)

### LC-MS method 4

Waters Micromass ZMD with a C18-reverse-phase column (30 × 4.6 mm Phenomenex Luna 3 µm particle size), elution with A: water + 0.1% formic acid; B: acetonitrile + 0.1 % formic acid. Gradient:

| Gradient - Time | flow ml/min | %A | %B |
|---|---|---|---|
| 0.00 | 2.0 | 95 | 5 |
| 0.50 | 2.0 | 95 | 5 |
| 4.50 | 2.0 | 5 | 95 |
| 5.50 | 2.0 | 5 | 95 |
| 6.00 | 2.0 | 95 | 5 |

Detection - MS, ELS, UV (100 µl split to MS with in-line UV detector)
MS ionisation method - Electrospray (positive and negative ion)

### NMR Spectrometers

NMRs were run on either a Varian Unity Inova 400 MHz spectrometer or a Bruker Avance DRX 400 MHz spectrometer.

### Abbreviations used in the experimental section:

DCM = dichloromethane
THF = tetrahydofuran
DMF = *N*,*N*-dimethylformamide
DIPEA = di-isopropylethylamine
RT = room temperature
HATU = *O*-(7-Azabenzotriazol-1-yl)-*N*,*N*,*N'*,*N'-*tetramethyluroniumhexafluorophosphate.
TFA = trifluoroacetic acid
Boc = tertiary-butyloxycarbonyl
Rt = retention time
EDC = 1-[3-(dimethylamino)propyl]-3-ethylcarbodiimide hydrochloride
DCE = 1,1-dichloroethane
IPA = isopropyl alcohol
IMS = industrial methylated spirits
EtOAc = ethyl acetate
MPLC = Medium pressure liquid chromatography
DMAP = 4-N,N-dimethylaminopyridine

### Intermediate 1

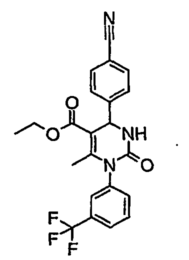

Intermediate 1 was prepared according to WO2004/024700.

### Intermediate 2

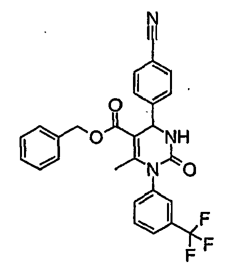

Polyphosphoric acid (350 g) was suspended in THF (1.9 I) and stirred, mechanically, whilst 3-(trifluoromethyl)phenylurea (129 g), 4-cyanobenzaldehyde (100 g) and benzyl acetoacetate (121.3 g) were added. The resulting mixture was heated at reflux overnight. The bulk of the solvent was evaporated under reduced pressure and the residue partitioned between water and EtOAc. The organic phase was washed with water, aqueous K₂CO₃ solution, water, brine, dried (Na₂SO₄) and evaporated. The residue was triturated with diethyl ether to give Intermediate 2 as a yellow solid.
Yield: 216.6g (70%)
¹H-NMR (400 MHz, DMSO-d₆): δ = 8.38 (1H, d), 7.84 (2H, m), 7.80-7.55 (6H m), 7.30 (3H, m), 7.15 (2H, m), 5.13 (1H, d), 5.06 (1H, d), 5.39 (1H, d), 2.08 (3H, s).

### Intermediate 3

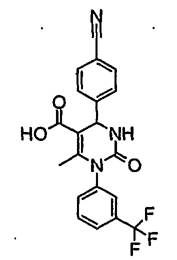

Intermediate 2 (215 g, 0.54 mol) was suspended in EtOAc (1.5 I) and IMS (500 ml) was added. The mixture was warmed slightly by immersion in a warm water bath (ca. 40°C) until homogeneous and then treated, under nitrogen, with Pd(OH)₂ on carbon (20 g, 20% w/w, 50% water wet). The flask was sealed and evacuated and then the mixture was stirred under an atmosphere of hydrogen whilst keeping warm by replenishment of the warm water bath. After ca. 4-6 h, the flask was evacuated and the mixture filtered through 'hyflo'. The filtrate was evaporated under vacuum and the residue triturated with diethyl ether to furnish Intermediate 3 as a solid which was used directly in the next step.

### Intermediate 4

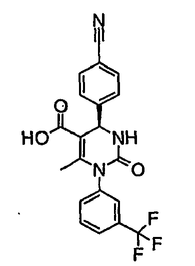

Intermediate 3 (156.2 g) was suspended in IPA (1500 ml) and treated with L-(-)-norephedrine. The mixture became homogeneous and then began to deposit a solid. After stirring for ca. 6 h the solid was filtered off and washed with IPA and sucked as dry as possible on the filter. The cake was removed and dissolved in a minimum amount of hot IPA (ca. 2.5 I) and allowed to cool, with stirring, overnight. The mixture was filtered and washed with IPA and dried to give a white solid. This was partitioned between EtOAc and 2M HCI until all solid had dissolved. The organic layer was washed with brine, dried (Na₂SO₄) and evaporated to give Intermediate 4 as a colourless foam (61.7 g).
Yield: 61.7 g (29%)
¹H-NMR (400 MHz, DMSO-d₆): δ =12.48 (1H, br s), 8.32 (1H, d), 7.88 (2H, m), 7.79-7.54 (6H, m), 5.36 (1H, d), 4.03 (2H, q), 2.05 (3H, s), 1.18 (3H, t).

### Intermediate 5

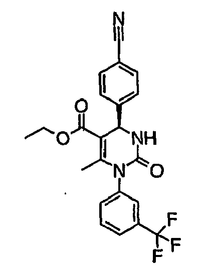

Intermediate 4 (47.6 g) was suspended in toluene and treated with oxalyl chloride (18 g; 12.5 ml). The mixture was stirred and treated with DMF (20 drops). The resulting mixture was stirred at room temperature until gas evolution ceased and the mixture was homogeneous (ca. 1h). The solution was cooled and treated with diisopropylethylamine (20 ml) followed by a mixture of EtOH (20 ml) and diisopropylethylamine (20 ml). After stirring at room temperature for ca 30 min, the solution was diluted with EtOAc and washed with 2M HCl, water, aqueous K₂CO₃, water, brine, dried (Na₂SO₄) and evaporated to a brown foam. Chromatography on silica gel, eluting with 0-20% EtOAc in DCM as eluant, gave Intermediate 5 as a pale yellow foam.
Yield: 40.3 g (79%)
¹H-NMR (400 MHz, CDCl₃): δ = 7.72-7.39 (8H, m), 5.95 (1H, d), 5.58 (1H, d), 4.15 (2H, q), 2.10 (3H, s), 1.21 (3H, t).

### Intermediate 6

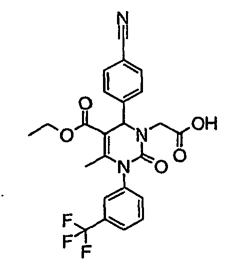

Intermediate 6 was prepared from Intermediate 1 according to WO2004/024700.

### Intermediate 7

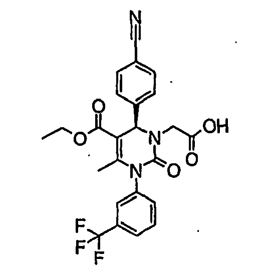

Intermediate 5 (1.69 g, 3.94 mmol) was dissolved in DMF (35 ml) and the solution was cooled to -10°C under argon. Sodium hydride (60% dispersion in mineral oil) (394 mg, 9.85 mmol) was added and the reaction was stirred for 10 min before the addition of bromoacetic acid. Stirring was continued for 1.5 h and then the reaction was quenched with sat. NH₄Cl (10 ml). The mixture was diluted with 1 M HCl (100 ml) and extracted with EtOAc (3 × 75 ml). The combined organic extracts were washed with water (100 ml) and brine (50 ml), dried (Na₂SO₄) and evaporated. The residue was taken up into acetonitrile (20 ml) and loaded onto an Isolute NH₂ cartridge (25 g). The cartridge was flushed with acetonitrile (250 ml) and then the product was eluted with 2% conc. aqueous HCI in acetonitrile (350 ml). The fractions containing the product were combined and the volume reduced. The residue was partitioned between EtOAc (150 ml) and 1 M aqueous HCI (75 ml). The organic layer was separated, washed with water (70 ml) and brine (50 ml), dried (Na₂SO₄) and evaporated. The product was obtained as a pale cream foam.
Yield: 1.63 g (85%)
LC-MS (Method 2): Rt 3.67 min, m/z 488 [MH⁺]

### Intermediate 8

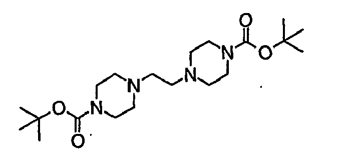

Boc-Piperazine (0.5 g, 2.69 mmol), 1,2-dibromoethane (253 mg, 1.34 mmol) and NaHCO₃ (564 mg, 6.72 mmol) in acetonitrile (20 ml) were heated at 90°C for 17h. After cooling to RT, the solvent was removed and the residue was dissolved in EtOAc (80 ml). The organic solution was washed with water (50 ml) and brine (20 ml), dried (Na₂SO₄) and evaporated to give Intermediate 8 as a white solid.
Yield: 426 mg (80%)
LC-MS (Method 2): Rt 0.34/2.05 min, m/z 399 [MH⁺]

### Intermediate 9

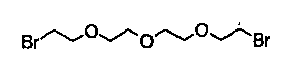

Prepared according to: Mills, O. S.; Mooney, N. J.; Robinson, P. M.; Watt, C. I. F.; Box, B. G. J. Chem. Soc., Perkin Trans. 2: (1995) 4, 697-706.

### Intermediate 10

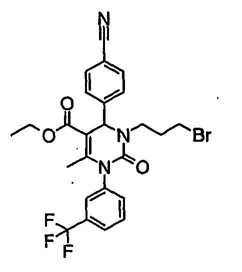

Intermediate 1 (2.00 g, 4.66 mmol) was dissolved in anhydrous DMF (40 ml) and the solution was cooled to 0°C. Sodium hydride (60% dispersion in mineral oil) (280 mg, 7.00 mmol) was added and the reaction mixture was stirred until the effervescence had ceased. 1,3-Dibromopropane (2.5 ml) was added and stirring was continued at 0°C for a further 1 h, after which time 1 M HCI (60 ml) and diethyl ether (60 ml) were added. The organic layer was separated and the aqueous solution was further extracted with diethyl ether (2 × 50 ml). The organic extracts were combined and washed with water (50 ml) and brine (30 ml), dried (Na₂SO₄) and evaporated. The residue was purified using MPLC on silica gel eluting with 20% EtOAc in pentane.
Yield: 1.05 g (41 %)
LC-MS (Method 3): Rt 4.12 min, m/z 550/552 [MH⁺]

### Intermediate 11

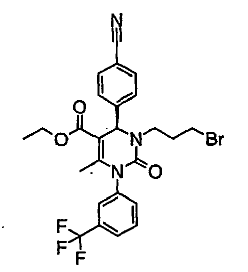

Intermediate 11 was prepared in a similar manner from Intermediate 5. The product was purified on silica eluting with DCM.
Yield: 65%
LC-MS (Method 3): Rt 4.10 min, m/z 550/552 [MH⁺]

### Intermediate 12

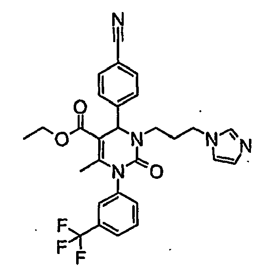

Imidazole (12 mg, 0.182 mmol) was dissolved in anhydrous THF (10 ml) and sodium hydride (60% dispersion in mineral oil) (7 mg, 0.175 mmol) was added. The reaction mixture was stirred until the effervescence had ceased and Intermediate 10 (200 mg, 0.364 mmol) was added. The reaction mixture was heated at 80°C for 40 h, after which time it was allowed to cool and the solvent was evaporated. EtOAc (40 ml) and water (40 ml) were added and the organic layer was separated. The aqueous solution was further extracted with EtOAc (2 × 50 ml) and the organic extracts were combined, washed with water (50 ml) and brine (30 ml), dried (Na₂SO₄) and evaporated. The residue was purified using an Isolute Si(II) cartridge (10 g) eluting with 0-20% EtOAc in pentane.
Yield: 70 mg (36%)
LC-MS (Method 2): Rt 2.68 min, m/z 538 [MH⁺]

### Intermediate 13

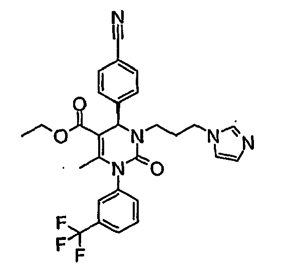

Intermediate 11 (150 mg, 0.27 mmol) was dissolved in acetonitrile (10 ml) and imidazole (111 mg, 1.636 mmol) and K₂CO₃ (226 mg, 1.636 mmol) were added. The reaction was heated at 70°C for 7 h and then, after allowing to cool, the solvent was evaporated. The residue was dissolved in EtOAc (100 ml) and the solution was washed with water (50 ml) and brine (40 ml), dried (Na₂SO₄) and evaporated. The product was purified by chromatography on an Isolute Si(II) cartridge (20 g) eluting with 0-20% MeOH in EtOAc. LC-MS (Method 2): Rt 2.68 min, m/z 538 [MH⁺]

### Intermediate 14

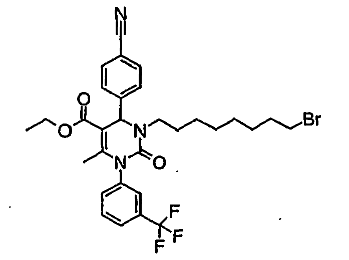

Intermediate 14 was prepared from Intermediate 1 and 1,8-dibromooctane by a similar procedure to that used in the synthesis of Intermediate 10.
Yield: 45%
LC-MS (Method 3): Rt 470/4.79 min, m/z 620/622 [MH]⁺

### Intermediate 15

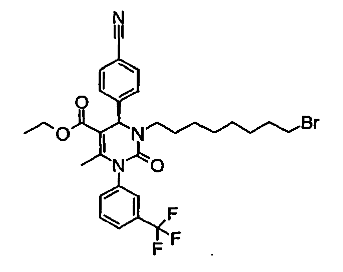

Intermediate 15 was prepared in a similar manner from Intermediate 5 and purified on an Isolute Si II cartridge eluting with 0-25% EtOAc in cyclohexane.
Yield: 57%
LC-MS (Method 2): Rt 4.89 min, m/z 620/622 [MH⁺]

### Intermediate 16

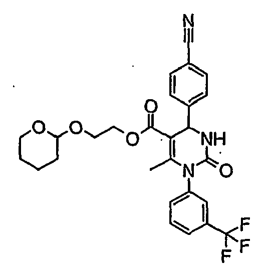

Intermediate 3 (7.0g, 15.4 mmol) was dissolved in DCM (75 ml). EDC (5.02 g, 26.2 mmol) and DMAP (2.34 g,19.2 mmol) were added and the solution was stirred for 5 min before the addition of 2-(tetrahydro-2H-pyran-2-yloxy)ethanol (3.19 g, 21.9 mmol). The mixture was allowed to stand at RT for 24 h and then washed with 1 M aqueous Na₂CO₃ and brine, dried (MgSO₄) and evaporated. The resulting oil was chromatographed on silica gel using 0-10% EtOAc in DCM as eluent, giving Intermediate 16 as a pale yellow viscous oil.
Yield: 7.49 g (81 %)
LC-MS (Method 2): Rt 3.82 min, m/z 530 [MH⁺]

### Intermediate 17

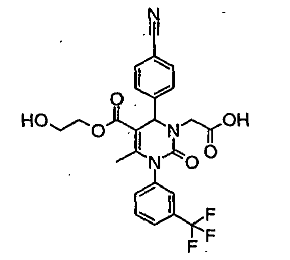

Intermediate 16 (2.00 g, 3.78 mmol) was dissolved in DMF (50 ml) and the solution was cooled to 0°C under nitrogen. Sodium hydride (60% dispersion in mineral oil) (378 mg, 9.45 mmol) was added portion wise and the reaction was stirred for 15 min. The temperature was reduced to -10°C and bromoacetic acid was added (526 mg, 3.78 mmol). The reaction was stirred whilst being allowed to warm to RT over 2 h, before being quenched by the addition of sat. aqueous NH₄Cl. The product was extracted into EtOAc (3x) and the combined organic extracts were washed with water then brine and finally dried (Na₂SO₄). Evaporation gave a brown oil which was loaded onto an Isolute NH₂ cartridge (25 g). The cartridge was flushed with acetonitrile and then the product was eluted with 2% conc. HCl in acetonitrile. The solvent was reduced and the residue was re-dissolved in MeOH (100 ml). Conc. HCl (3 ml) was added and after 30. min the solvent was reduced. The residue was treated with 1M NaOH (30 ml) in MeOH (100 ml) and the solution was acidified by the addition of 1 M HCl. The product was extracted into EtOAc (3x) and the combined organics were washed with water and brine, dried (Na₂SO₄), and evaporated. The residue was purified using HPLC system 2 and Intermediate 17 was obtained as a white solid.
Yield: 450 mg (24%)
LC-MS (Method 2): Rt 3.16 min, m/z 504 [MH]⁺

### Intermediate 18

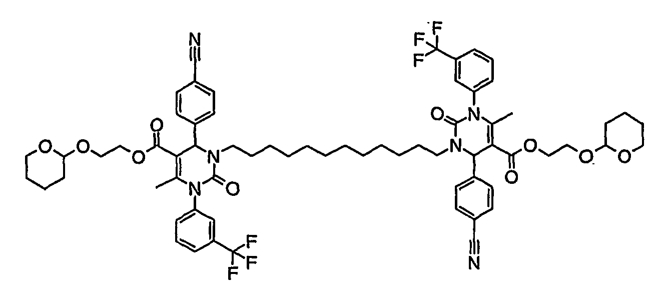

Intermediate 18 was prepared from Intermediate 16 and 0.5 equivalents of 1,12-dibromododecane by a similar procedure to that used in the synthesis of Intermediate 10.
LC-MS (Method 2): Rt 5.56 min, m/z 1057 [MH-2xTHP]⁺

### Intermediate 19

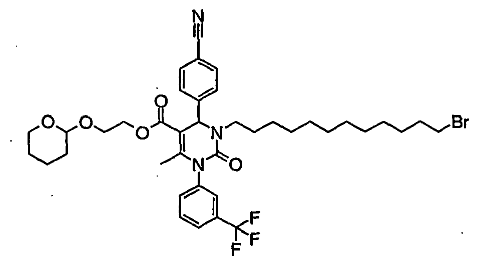

Intermediate 19 was obtained as a by-product in the synthesis of Intermediate 18. LC-MS (Method 2): Rt 5.43 min.

### Intermediate 20

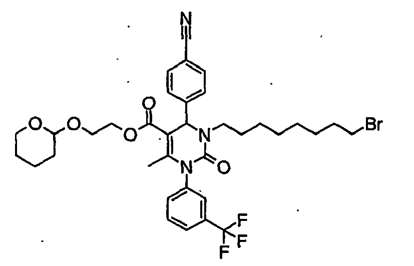

Intermediate 20 was prepared from Intermediate 16 and 1,8-dibromooctane by a similar procedure to that used in the synthesis of Intermediate 10. The product was purified on an Isolute Si II cartridge eluting with 20% EtOAc in cyclohexane, then by HPLC system 4.
Yield: 17%
LC-MS (Method 2): Rt 4.92 min, m/z 720/722 [MH⁺]

### Intermediate 21

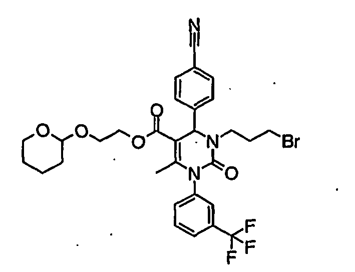

Intermediate 21 was prepared from Intermediate 16 and 1,3-dibromopropane by a similar procedure to that used in the synthesis of Intermediate 10. The product was purified on an Isolute Si II cartridge eluting with 20% EtOAc in cyclohexane.
Yield: 65%
LC-MS (Method 2): Rt 4.40 min, m/z 650 [MH⁺]

### Intermediate 22

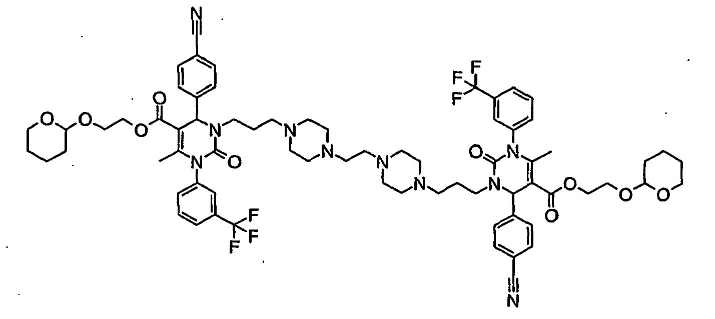

Intermediate 8 (129 mg, 0.32 mmol) was dissolved in DCM (1 ml) and treated with TFA (1 ml) for 30 min. The volatiles were evaporated and the residue was dissolved in acetonitrile (15 ml). NaHCO₃ (485 mg, 5.77 mmol) and Intermediate 21 (420 mg, 0.65 mmol) were added and the reaction mixture was heated at 80°C for 24 h. The solvent was evaporated and the residue treated with DCM and the mixture filtered. After removal of the solvent the product was purified on an Isolute Si II cartridge (2 g) eluting with 0-10% MeOH in DCM.
Yield: 15%
LC-MS (Method 2): Rt 2.84 min, m/z 1337 [MH⁺]

### Intermediate 23

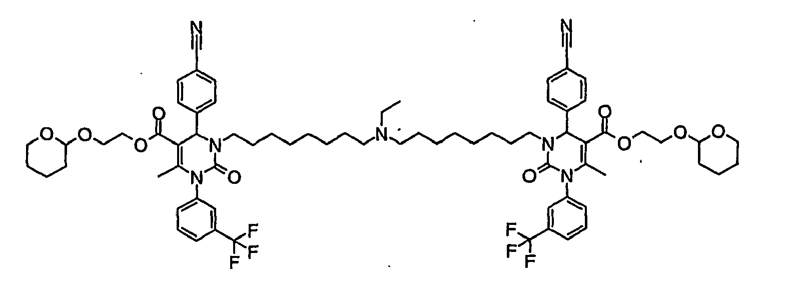

To a mixture of Intermediate 20 (110 mg, 0.153 mmol) and NaHCO₃ (40 mg, 0.478 mmol) in acetonitrile (5 ml) was added 2M ethylamine in THF (77 µl, 0.153 mmol). The reaction mixture was heated at 80°C for 24 h. The solvent was removed and the residue was purified on an Isolute Si II cartridge (2 g) eluting with 0-1% NH₃ in EtOAc as eluent.
Yield: 66 mg (65%)
LC-MS (Method 2): Rt 3.79 min, 1324 m/z [MH⁺]

### Intermediate 24

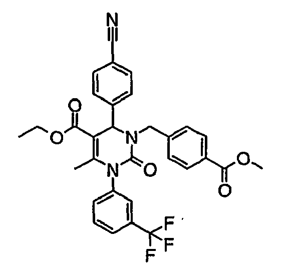

Intermediate 24 was prepared from_{.} Intermediate 1 and 1.2 equivalents of methyl 4-(bromomethyl)benzoate using a similar procedure to that used for the synthesis of Intermediate 10.
Yield: 80%
LC-MS (Method 2): Rt 4.29 min, m/z 578 [MH]⁺

### Intermediate 25

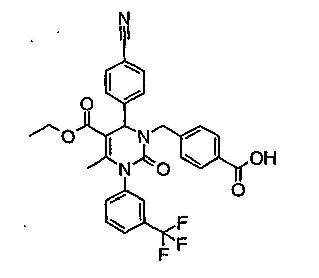

Intermediate 24 (320 mg, 0.55 mmol) was dissolved in THF (15 ml) and 1 M NaOH (5 ml) was added. MeOH (1 ml) was added to aid dissolution and the reaction was stirred at RT for 2 h. The reaction mixture was acidified with 1 M HCI and extracted with EtOAc. The organic layer was separated, washed with water and brine, dried (Na₂S0₄) and evaporated. The product was purified using HPLC system 1 and obtained as a white foam. '
Yield: 128 mg (41%)
LC-MS (Method 2): Rt 3.86 min, m/z 564 [MH]⁺

### Intermediate 26

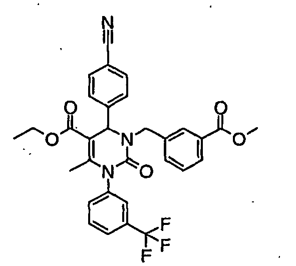

Intermediate 26 was prepared from Intermediate 1 and 1.2 equivalents of methyl 3-(bromomethyl)benzoate using a similar procedure to that used for the synthesis of Intermediate 10.
Yield: 83%
LC-MS (Method 2): Rt 4.28 min, m/z 578 [MH]⁺

### Intermediate 27

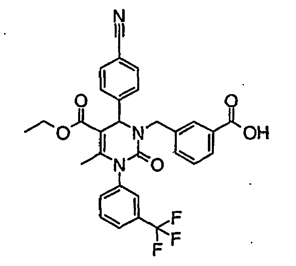

Intermediate 27 was prepared by a similar method to Intermediate 25 from Intermediate 26.
Yield: 29%
LC-MS (Method 2): Rt 3.92 min, m/z 564 [MH]⁺

### Intermediate 28

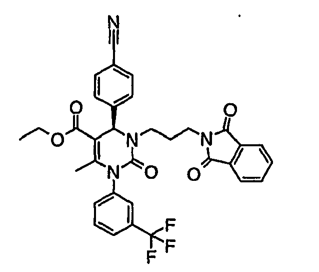

Intermediate 28 was prepared from Intermediate 5 and 1 equivalent of *N*-(3-bromopropyl)phthalimide using a similar procedure to that used to prepare Intermediate 10.
Yield: 69%
LC-MS (Method 4): Rt 4.19 min, m/z 617 [MH]⁺

### Intermediate 29

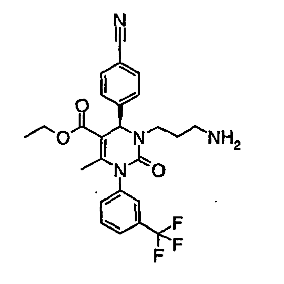

Intermediate 28 (882 mg,1.43 mmol) was dissolved in IMS (45 ml) and 2M methylamine in THF (8.6 ml,17.2 mmol) was added. The solution was stirred at 50°C for 24 h and the volatiles were evaporated. The residue was dissolved in MeOH and loaded onto an Isolute SCX-2 cartridge. After flushing with MeOH, the product was eluted with 2M NH₃ in MeOH. Evaporation gave the desired product as a cream foam.
Yield: 422 mg (61 %)
LC-MS (Method 3): Rt 2.46 min, m/z 487 [MH]⁺

### Intermediate 30

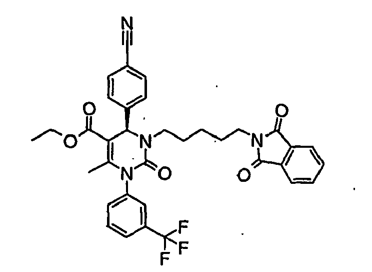

Intermediate 30 was prepared from Intermediate 5 and 1 equivalent of *N*-(5-bromopentyl)phthalimide using a similar procedure to that used to prepare Intermediate 10.
Yield: 39%
LC-MS (Method 3): Rt 4.28 min, m/z 645 [MH]⁺

### Intermediate 31

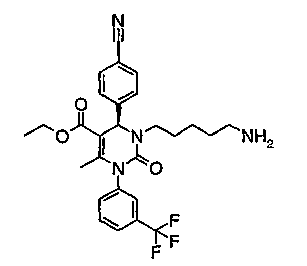

Intermediate 31 was prepared from Intermediate 30 a using a similar procedure to that used to prepare Intermediate 29.
Yield: 74%
LC-MS (Method 3): Rt 2.50 min, m/z 515 [MH]⁺

### Intermediate 32

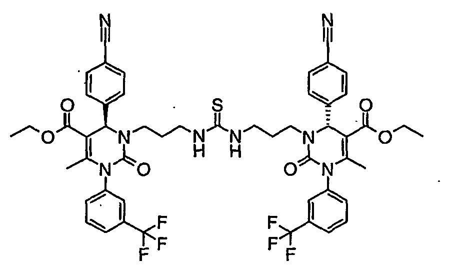

Intermediate 29 (372 mg, 0.77 mmol) was dissolved in DCM (15 ml) and 1,1'-thiocarbonyldipyridone (89 mg, 0.38 mmol) was added. The solution was allowed to stand at RT for 48 h. The solvent was removed and the residue was chromatographed on an Isolute Si II cartridge (10g) eluting with 50-80% EtOAc in pentane. The desired product was obtained as a colourless gum.
Yield: 263 mg (68%)
LC-MS (Method 2): Rt 4.66 min, m/z 1015 [MH]⁺

### Intermediate 33

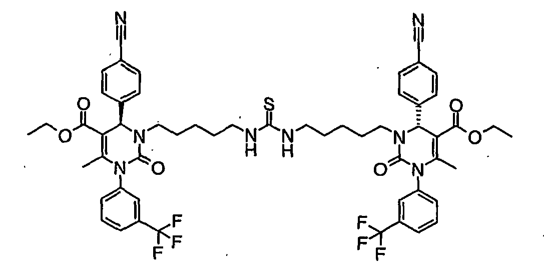

Intermediate 33 was prepared from Intermediate 31 by a method similar to that for the synthesis of Intermediate 32.
Yield: 63%
LC-MS (Method 3): Rt 4.63 min, m/z 1071 [MH]⁺

### Intermediate 34

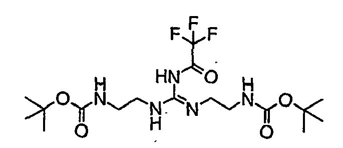

Prepared according to Bartoli, S.; Jensen, K. B.; Kilburn, J. D. J. Org. Chem.: (2003) 68, 9416-9422.

### Intermediate 35

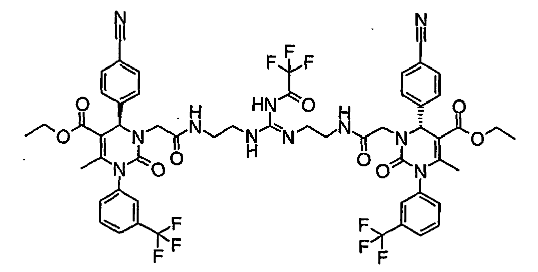

Intermediate 34 (181 mg, 0.41 mmol) was dissolved in 20% TFA in DCM (20 ml) and the solution was allowed to stand at RT for 3 h. Toluene was added, the solvents were evaporated and the residue was re-dissolved in DMF (20 ml). Intermediate 7 (400 mg, 0.821 mmol) and DIPEA (1 ml) were added, followed by HATU (343 mg, 0.903 mmol). The solution was allowed to stand at RT until LC-MS indicated complete reaction (30 min) and the solvent was evaporated. The residue was dissolved in EtOAc (150 ml) and the organic solution was washed with sat. aqueous NaHCO₃ (100 ml), water (50 ml) and brine (50 ml). After drying over Na₂SO₄, the solvent was removed to give a residue which was purified by chromatography on an Isolute Si 11 cartridge (10 g) eluting with 60% EtOAc in pentane, 100% EtOAc then 10% MeOH in EtOAc.
Yield: 279 mg (57%)
LC-MS (Method 3): Rt 4.30 min, m/z 1180 [MH]⁺

### Intermediate 36

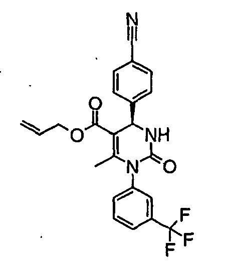

Intermediate 36 was prepared from Intermediate 4 and allyl alcohol using a similar method to that used for the preparation of Intermediate 5.
Yield: 57%
LC-MS (Method 3): Rt 3.65 min, m/z 442 [MH⁺]

### Intermediate 37

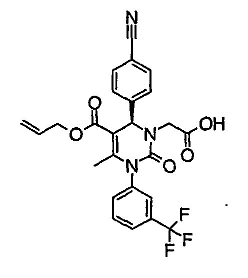

Intermediate 37 was prepared from Intermediate 36 by a similar method to that used for the preparation of Intermediate 7.
Yield: 84%
LC-MS (Method 2): Rt 3.77 min, m/z 500 [MH⁺]

### Intermediate 38

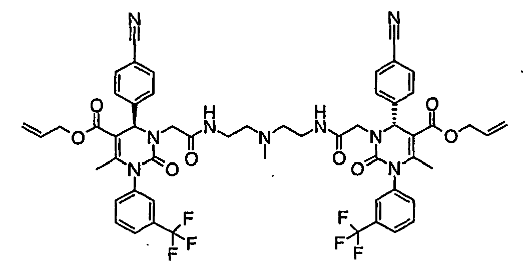

Intermediate 37 (1.14 g, 2.28 mmol), *N*-methyl-2,2'-diaminoethylamine (133 mg, 1.14 mmol) and DIPEA (2 ml) were dissolved in DMF (60 ml). HATU (953 mg, 2.51 mmol) was added and the solution was allowed to stand at RT until the reaction was complete by LC-MS (30 min). The DMF was evaporated and the residue was dissolved in EtOAc (200 ml). The organic solution was washed with aqueous NaHCO₃ (100 ml), water (50 ml) and brine (50 ml). After drying (Na₂SO₄), the solvent was removed to give a brown oil which was purified by chromatography on an Isolute Si II cartridge (25 g) eluting with 0-7% MeOH in EtOAc.
Yield: 800 mg (65%)
LC-MS (Method 3): Rt 3.05 min, m/z 1080 [MH⁺]

### Intermediate 39

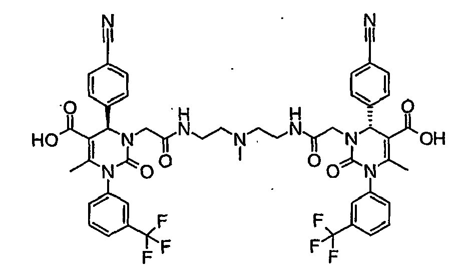

Intermediate 38 (800 mg, 0.74 mmol) was dissolved in THF (8 ml) and morpholine (648 mg, 7.45 mmol) and tetrakis(triphenylphosphine)palladium(0) (30 mg, 0.026 mmol) were added. The mixture was stirred at RT. After 3 h the mixture was diluted with EtOAc and the solution was washed with 10% aqueous HCl and brine. After drying (Na₂SO₄), the solvent was evaporated to give a solid which was triturated with diethyl ether. The di-acid was obtained as a yellow solid.
Yield: 742 mg (100%)
LC-MS (Method 3): Rt 2.70 min, m/z 1000 [MH⁺]

### Intermediate 40

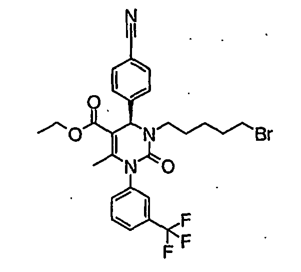

Intermediate 40 was prepared from Intermediate 5 and 1,5-dibromopentane by a similar method to that used for the synthesis of Intermediate 10.
Yield: 58%
LC-MS (Method 4): Rt 4.63 min, m/z 578/580 [MH⁺]

### Intermediate 41

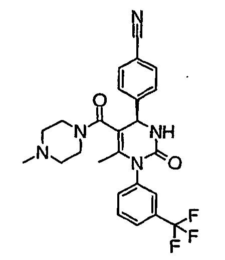

Intermediate 41 was prepared from Intermediate 4 and 1 equivalent of *N-*methylpiperazine by a similar method to that used in the synthesis of Intermediate 38. The product was purified by SCX chromatography.
Yield: 93%
LC-MS (Method 4): Rt 2.10 min, m/z 484 [MH⁺]

### Intermediate 42

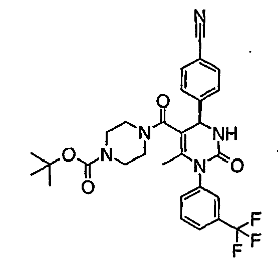

Intermediate 42 was prepared from Intermediate 4 and 1 equivalent of *N*-Boc piperazine by a similar method to that used in the synthesis of Intermediate 38. The product was used without chromatographic purification.
Yield: 88%
LC-MS (Method 4): Rt 3.56 min, m/z 568 [M-]

### Intermediate 43

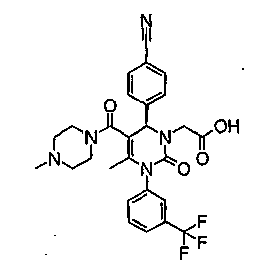

Intermediate 43 was prepared from Intermediate 41 by a similar method to that used for the preparation of Intermediate 7.
Yield: 29%
LC-MS (Method 4): Rt 2.22 min, m/z 542 [MH⁺]

### Intermediate 44

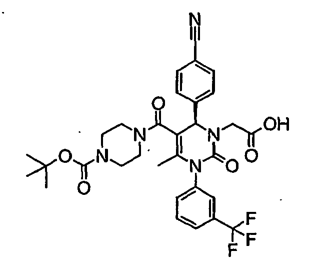

Intermediate 44 was prepared from Intermediate 42 by a similar method to that used for the preparation of Intermediate 7.
Yield: 93%
LC-MS (Method 4): Rt 3.61 min, m/z 628 [MH⁺]

### Intermediate 45

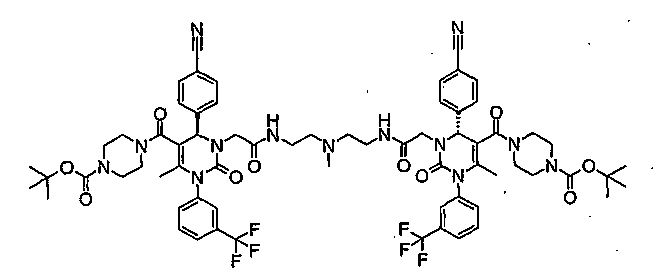

Intermediate 45 was prepared from Intermediate 44 and 0.5 equivalents of *N*-methyl-2,2'-diaminoethylamine by a similar method to that used in the synthesis of Intermediate 38. The product was purified by SCX chromatography.
Yield: 64%
LC-MS (Method 4): Rt 3.18 min, m/z 1336 [MH⁺]

### Intermediate 46

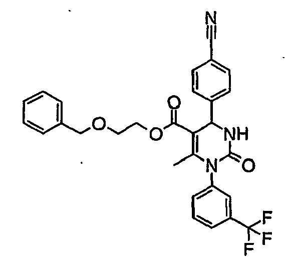

EDC (5.38 g, 28.1 mmol) and DMAP (2.51 g, 20.6 mmol) were added to a solution of Intermediate 3 (7.5 g, 18.7 mmol) in DCM (75 ml). 2-Benzyloxyethariol (3.32 ml, 23.4 mmol) was added to the mixture and the reaction was left to stir at room temperature overnight. The organics were washed with 1 M aqueous Na₂CO₃ and brine. The combined aqueous layers were extracted with DCM, and the combined organic layers were washed with 1 M aqueous HCl and brine. The organic layer was dried (MgSO₄) and the solvent removed to give a foam. Purification was achieved *via* column chromatography on silica gel eluting the product with 10% EtOAc in DCM. The main fraction (7.59 g) was a 1:1 mixture of the desired product and 2-benzyloxyethanol, but 0.26 g of pure product was also obtained. The 7.59 g batch was distilled to remove the alcohol (135°C, -15 mmHg). Purification was repeated to give 5.2 g of product contaminated with 10% of 2-benzyloxyethanol.
Yield: 55%
LC-MS (Method 3): Rt 3.82 min, m/z 536 [MH⁺]

### Intermediate 47

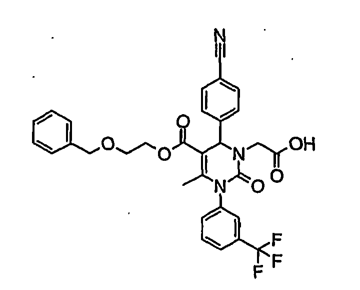

Intermediate 47 was prepared from Intermediate 46 using a method similar to that used to prepare Intermediate 7.
LC-MS (Method 3): Rt 3.75 min, m/z 594 [MH⁺]

### Intermediate 48

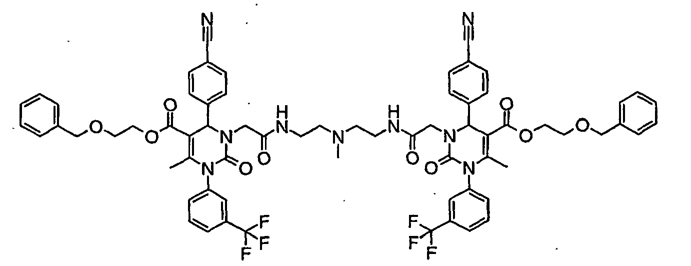

Intermediate 48 was prepared from Intermediate 47 and N-methyl-2,2'-diaminoethylamine by a method similar to the used in the synthesis of Intermediate 38.
LC-MS (Method 2): Rt 3.44 min, m/z 1268 [MH⁺]

### Intermediate 49

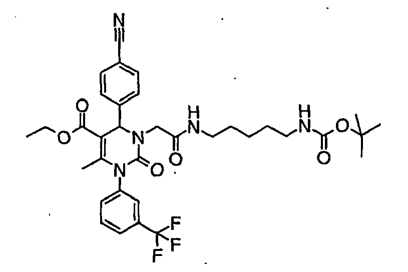

Intermediate 49 was prepared from Intermediate 6 and 1.1 equivalents of *N*-Boc-penta-1,5-diamine by a similar method to that used in the synthesis of Intermediate 38. The product was used without chromatographic purification.
Yield: 64%
LC-MS (Method 3): Rt 3.85 min, m/z 672 [MH⁺]

### Intermediate 50

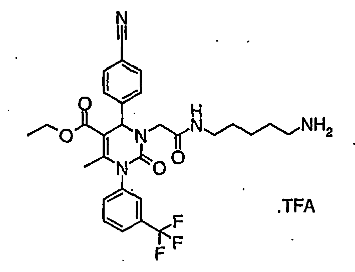

Intermediate 49 (100 mg, 0.149 mmol) was dissolved in DCM (10 ml) and treated with TFA (3 ml). After 2 h the volatiles were evaporated and the product was used without further purification.
Yield: 100%
LC-MS (Method 2): Rt 2.61 min, m/z 572 [MH⁺]

### Intermediate 51

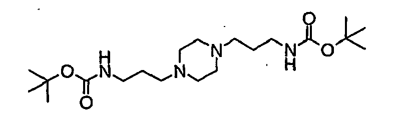

Piperazine (144 mg,1.68 mmol), 3-(Boc-amino)propyl bromide (800 mg, 3.36 mmol) and NaHCO₃ (722 mg, 8.4 mmol) were stirred in acetonitrile (20 ml) at 90°C for 18 h. The solvent was removed and water (30 ml) and EtOAc (60 ml) were added then the layers separated. The organic layer was dried (MgSO₄) and evaporated.
Yield: 588 mg (88%)
LC-MS (Method 2): Rt 0.35/1.84 min, 401 m/z [MH⁺]

### Intermediate 52

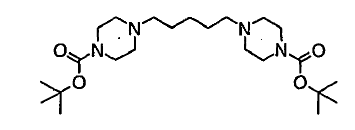

*N*-Boc piperazine (2.0 g, 10.75 mmol), glutaraldehyde (50% aqueous solution, 1.08 ml, 5.37 mmol) and sodium triacetoxyborohydride (3.68 g, 17.2 mmol) in DCE (30 ml) were stirred at RT under nitrogen for 3 h. The reaction mixture was quenched using sat. aqueous NaHCO₃, extracted with EtOAc, dried (MgSO₄) and evaporated to give a colourless oil which solidified on standing.
Yield: 2.00 g (42%)
LC-MS (Method 3): Rt 0.26/1.5 min, 441 m/z [MH⁺]

### Intermediate 53

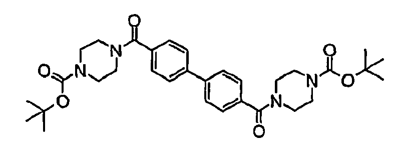

4,4-Biphenyldicarboxylic acid (2.0 g, 8.3 mmol), *N*-Boc-piperazine (3.38 g, 18.2 mmol), HATU (6.55 g,18.2 mmol) and DIPEA (9.4 ml, 55 mmol) were suspended in DMF (30 ml) and stirred at RT for 30 min. The mixture was divided into three portions and each was irradiated in the microwave at 100°C for 5 min before the samples were re-combined, diluted with diethyl ether and filtered. The solid was washed with diethyl ether and suction dried to an off-white powder.
Yield: 3.04 g (63%)
LC-MS (Method 2): Rt 3.79 min, 579 m/z [MH⁺]

The following compounds were prepared from a di-acid and *N*-Boc-piperazine by a procedure similar to that used for the synthesis of Intermediate 53:

| **Compound** | **Structure** | **Di-acid** | **Reacti on time** | **Yield (%)** | **Rt (min) (Method 2)** | **Mass [MH]⁺** |
|---|---|---|---|---|---|---|
| Intermediate 54 | 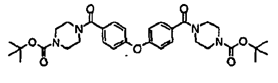 | 4,4-Oxybisbenzoic | 30 min | 49 | See NMR data | |
| Intermediate 55 | 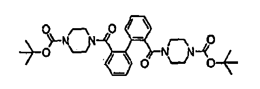 | Diphenic | 10 min (100°C µwave) | 83 | 3.88 | 579 |
| Intermediate 56 | 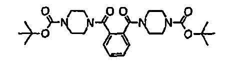 | Phthalic | 24 h | 83 | 3.40 | 525 [M+Na⁺] |
| Intermediate 57 | 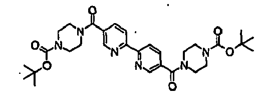 | 2,2'-Bipyridine-5,5'-dicarboxylic- | 17 h | 74 | 3.19 | 581 |
| Intermediate 58 | 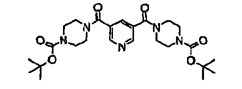 | 3,5-Pyridinedicarboxylic | 17 h | 96 | 2.90 | 504 |

¹H-NMR (400 MHz, MeOD): δ = 7.47 (1H, d); 7.11 (1H, d); 3.32-3.60 (16h, br s); 1.41 (18H, s)

### Intermediate 59

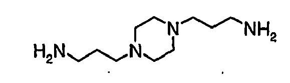

Intermediate 51 (550 mg, 1.375 mmol) was stirred in 1.25 M HCl in MeOH (10 ml) at RT for 1h then evaporated.
Yield: 370 mg (99%)
LC-MS (Method 2): Rt 0.25 min, 201 m/z [MH⁺]

### Intermediate 60

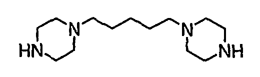

Intermediate 52 (1.0 g, 2.27 mmol) was dissolved in DCM (4 ml) and TFA (4 ml) was added. The mixture was stirred at RT for 30 min. The solvent was removed and the residue was taken up into 1:1 DCM/ MeOH and applied to an SCX-2 cartridge. After washing with 1:1 DCM/ MeOH then MeOH, the product was eluted with 2M NH₃ in MeOH. The solvent was removed.
Yield: 676 mg (100% (contains solvent))
LC-MS (Method 2): Rt 0.26 min, 241 m/z [MH⁺]

The following compounds were prepared using a procedure similar to that used for the synthesis of Intermediate 60:

| **Compound No.** | **Structure** | **Starting materials** | **Reaction time (min)** | **Yield (%)** | **Rt (min) (Method 1)** | **Mass [MH]⁺** |
|---|---|---|---|---|---|---|
| Intermediate 61 | 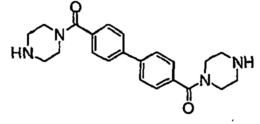 | Intermediate 53 | 30 | 100 | 0.33 | 379 |
| Intermediate 62 | 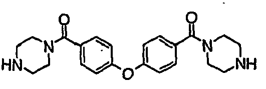 | Intermediate 54 | 30 | 70 | 0.35 | 395 |
| Intermediate 63 | 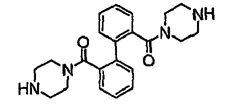 | Intermediate 55 | 30 | 100 | 0.33 | 379 |
| Intermediate 64 | 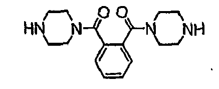 | Intermediate 56 | 30 | 91 | 0.28 | 303 |
| Intermediate 65 | 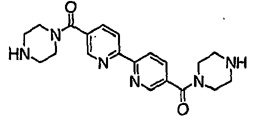 | Intermediate 57 | 30 | | 0.23 | 381 |
| Intermediate 66 | 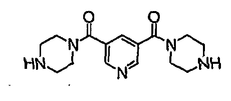 | Intermediate 58 | 30 | 100 | 0.19 | 304/607 |

### Intermediate 67

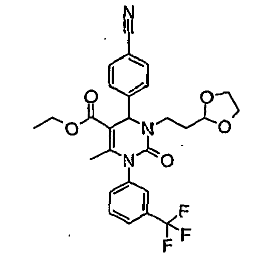

Intermediate 67 was prepared from Intermediate 1 and 6 equivalents of 2-(2-bromoethyl)-1,3-dioxolane using a method similar to that used for the preparation of Intermediate 10. After removal of the DMF, the product was purified directly on neutral alumina using 0-5% EtOH in DCM.
Yield: 77%
LC-MS (Method 2): Rt 4.03 min, 530 m/z [MH⁺]

### Intermediate 68

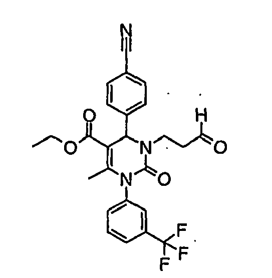

A solution of Intermediate 67 (3.4 g, 6.43 mmol) was dissolved in THF (20 ml), 1 M HCl (50 ml) was added and the reaction was stirred at 80°C overnight. The solvent was reduced and DCM (50 ml) and water (30 ml) were added. The phases were separated before the organic layer was washed with brine and dried (MgSO₄). The crude material was purified by chromatography using 0-2% MeOH in DCM.
Yield: 2.38 g (76%)
LC-MS (Method 3): Rt 3.20/ 3.67 min, 486 m/z [MH⁺]

### Intermediate 69

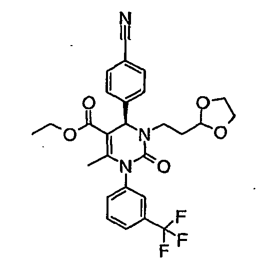

Intermediate 69 was prepared from Intermediate 5 by a similar procedure to that used for the synthesis of Intermediate 10. The product was purified by addition of cyclohexane, decanting off the liquid and then repeating.
Yield: 5.18 g (70%)
LC-MS (Method 3): Rt 3.78 min, 530 m/z [MH⁺]

### Intermediate 70

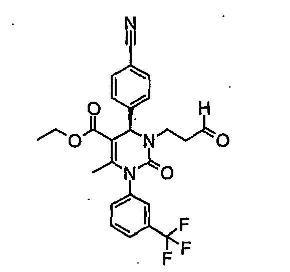

Intermediate 70 was prepared from Intermediate 69 by the method described for the synthesis of Intermediate 68.
Yield: 1.82 g (38%)
LC-MS (Method 3): Rt 3.67 min, 486 m/z [MH⁺]

### Intermediate 71

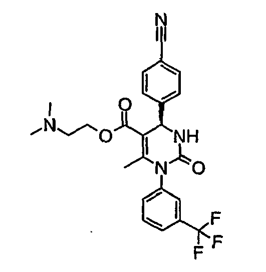

Intermediate 71 was prepared from Intermediate 4 and *N,N*-dimethylethanolamine by a procedure similar to that used for the synthesis of Intermediate 5. The crude material was purified by column chromatography using 0% then 10% MeOH in DCM. The material obtained was further purified by loading onto a PE-AX cartridge and washing with acetonitrile then evaporation of the solvent.
Yield: 311 mg (44%)
LC-MS (Method 2): Rt 2.38/2.47 min, 473 m/z [MH⁺]

### Intermediate 72

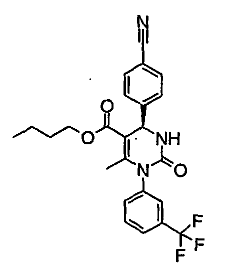

Intermediate 72 was prepared from Intermediate 4 and n-butanol by a procedure similar to that used for the synthesis of Intermediate 5.
Yield: 54%
LC-MS (Method 2): Rt 4,14 min, 458/915 m/z [MH⁺]

### Intermediate 73

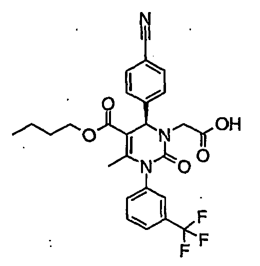

Intermediate 73 was prepared from Intermediate 72 by a procedure similar to that used for the synthesis of Intermediate 7, but the product was used without purification on an NH₂ cartridge.
Yield: 49%
LC-MS (Method 3): Rt 3.77 min, 514 m/z [M-H⁺]

### Intermediate 74

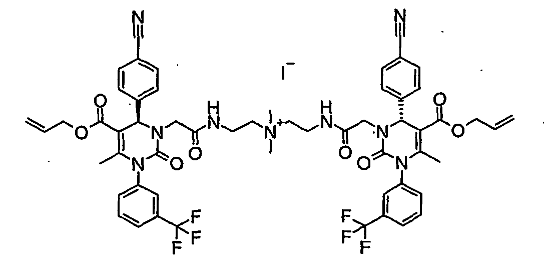

Intermediate 38 (450 mg, 0.417 mmol) was dissolved in acetonitrile (10 ml) and NaHCO₃ (105 mg, 1.25 mmol) and iodomethane (1 ml) were added. The solution was stirred at RT for 3.7 h, filtered and evaporated.
Yield: 496 mg (97%)
LC-MS (Method 4): Rt 3.28 min, 1094 m/z [MH⁺]

### Intermediate 75

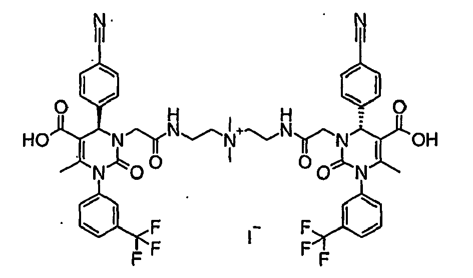

Intermediate 75 was prepared from Intermediate 74 according to method described for Intermediate 39.
Yield: 98%
LC-MS (Method 4): Rt 2.80min, 1015 m/z [MH⁺]

### Intermediate 76

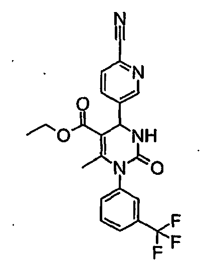

Intermediate 76 was prepared by a procedure analogous to that used for the synthesis of Intermediate 2. The product was purified by column chromatography using 0-10% EtOAc in DCM.
Yield: 34%
LC-MS (Method 1): Rt 10.62 min, 430 m/z [MH⁺]

### Intermediate 77

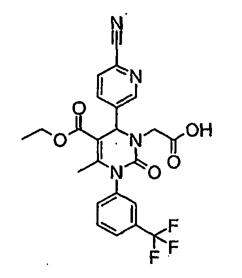

Intermediate 77 was prepared from Intermediate 76 by a procedure similar to that used for the synthesis of Intermediate 7, but the product was used without purification. Yield: 55%

LC-MS (Method 3): Rt 3.27 min, 489 m/z [MH⁺]

### Intermediate 78

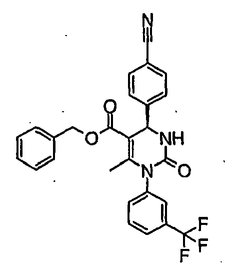

Intermediate 4 (2.00g, 4.98 mmol), benzyl bromide (600 µl, 4.98 mmol) and K₂CO₃ (1.38 g, 1.00 mmol) in DMF (50 ml) were stirred at RT for 6.5 h. Sat. aqueous NH₄Cl solution (50 ml) was added and the organic layer was separated. Evaporation gave a straw coloured glass.
Yield: 2.43 g (99%)
LC-MS (Method 2): Rt 4.09 min, 492 m/z [MH⁺]

### Intermediate 79

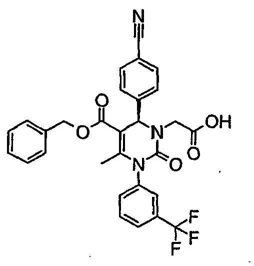

Intermediate 79 was prepared from Intermediate 78 using a similar procedure to that used for Intermediate 7.
Yield: 29%
LC-MS (Method 2): Rt 3.99 min, 550 m/z [MH⁺]

### Intermediate 80

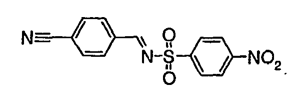

4-Cyanobenzaldehyde (3.24 g, 24.75 mmol) and 4-nitrophenylsulphonamide (5.00 g, 24.75 mmol) were stirred in DCM (100 ml) whilst BF₃ etherate was added. The reaction was stirred for a further 4 h after which time the solid was filtered off and washed with diethyl ether.
Yield: 4.40 g, 56%

### Intermediate 81

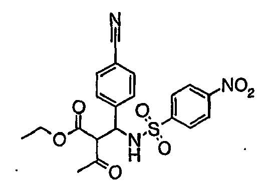

Intermediate 80 (2.90 g, 9.21 mmol) was suspended in THF (60 ml) and triethylamine (3.8 ml, 27.6 mmo) was added, followed by ethyl acetoacetate (1.32 g, 10.13 mmol). The reaction was stirred at RT for 1.5 h. The mixture was filtered and the solvent was evaporated. The residue was dissolved in a minimum amount of EtOAc and diethyl ether (150 ml) was added with rapid stirring. After 15 min, the white solid was filtered off, washed with diethyl ether and dried.
Yield: 2.88 g (70%)
LC-MS (Method 2): Rt 3.47 min, m/z 444 [M-H⁺]

### Intermediate 82

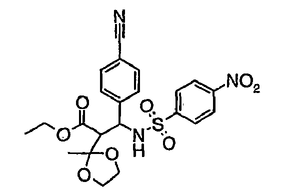

Intermediate 81 (1.189 g, 2.67 mmol), ethylene glycol (446 µl, 8.01 mmol) and 4-toluenesulphonic acid (51 mg, 0.27 mmol) in toluene were heated at reflux under Dean-Stark conditions (a slight vacuum was applied to the apparatus). After 6 h the reaction mixture was allowed to cool and the toluene was removed by evaporation. The residue was partitioned between DCM (200 ml) and sat. aqueous sodium hydrogen carbonate (150 ml). The organic layer was separated, washed with brine (50 ml), dried (Na₂SO₄) and evaporated. The resulting cream solid was triturated with diethyl ether and the product was obtained as a white solid.
Yield 665 mg (51 %)
LC-MS (Method 2): Rt 3.49 and 3.57 min, m/z 488 [M-H] diasteromeric mixture.

### Intermediate 83

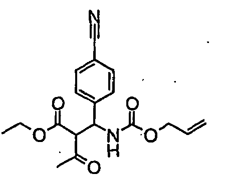

To a solution of allyl carbamate (100 mg,1.0 mmol) and 4-cyanobenzaldehyde (131 mg, 1.0 mmol) in dry DCM (3 ml) at -78°C was added BF₃ etherate (246 µl, 2.0 mmol) and the mixture warmed to RT and stirred for 20 min. This solution was added dropwise to a stirring suspension of (-)-cinchonidine (29 mg, 0.1 mmol) and ethyl acetoacetate (127 µl, 1.0 mmol) in dry DCM (3 ml) at -35°C, which had previously been stirred together for 5 mins. The reaction mixture was stirred at -35°C for 17h then the reaction mixture was filtered through a pad of silica, washing with EtOAc, then evaporated. The crude material was purified by chromatography using 10-25% EtOAc in cyclohexane to give Intermediate 83.
Yield: 98 mg (28%)
LC-MS (Method 3): Rt 3.12 min, 367 m/z [M+Na⁺]

### Intermediate 84

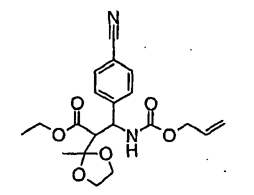

Intermediate 84 was prepared from Intermediate 83 by a similar method to that used in the synthesis of Intermediate 82. The product was triturated with ether to afford Intermediate 84, which was used without further purification.
-Yield: 845 mg (76%)
LC-MS (Method 3): Rt 3.24 min, 411 m/z [MNa⁺]

### Intermediate 85

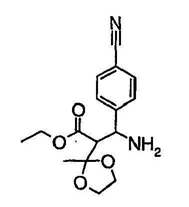

### Method A: From Intermediate 82

Octanethiol (597 mg, 4.090 mmol) was dissolved in DMF (10 ml) and sodium hydride (60% dispersion in mineral oil) was added. The reaction was stirred at RT and after 15 min, Intermediate 82 (500 mg, 1.022 mmol) was added. The solution was stirred for 3 h and then treated with 2 equivalents of acetic acid. The mixture was loaded directly onto an Isolute SCX-2 cartridge (20 g). After flushing with MeOH the product was eluted with 2M NH₃ in MeOH. The fractions containing UV-active material were combined and evaporated.
Yield:-343 mg (100%)

### Method B: From Intermediate 84

A flask was charged with Pd(PPh₃)₄ (8 mg, 0.0072 mmol) and 1,3-dimethylbarbituric acid (56 mg, 0.361 mmol) and Intermediate 84 (140 mg, 0.361 mmol) was added as a solution in dry DCM (2 ml). The reaction mixture was stirred under argon for 1 h and then filtered through a pad of silica and evaporated. The crude material was applied to a MeOH preconditioned SCX-2 cartridge, washed with MeOH and the product eluted using 2N NH₃ in MeOH. Evaporation afforded Intermediate 85.
Yield: 57 mg (52%)
LC-MS (Method 2): Rt 0.34 min, 305 m/z [MH⁺]

### Intermediate 86

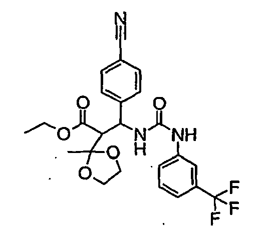

To a solution of Intermediate 85 (303 mg, 0.81 mmol) in DCM (6 ml) was added 3-(trifluoromethyl)phenyl isocyanate (111 µl, 0.806 mmol) and the reaction mixture was stirred for 1 h. The solvent was evaporated to afford the product, which wars used without further purification.
Yield: 430 mg (95%)
LC-MS (Method 2): Rt 4.27min, 560 [MH+]; 582 m/z [MNa+]

### Intermediate 87

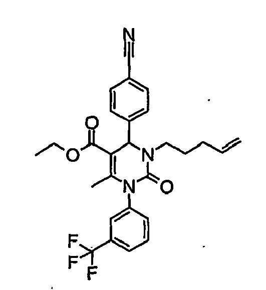

### Method A

Intermediate 1 (195 mg, 0.349 mmol) was dissolved in IMS (2 ml) and conc. aqueous HCI (100 µl) was added. The solution was heated in the microwave at 120°C for 10 min. the solvent was evaporated and the residue was partitioned between DCM (10 ml) and sat. aqueous sodium hydrogen carbonate (10 ml). The organic phase was separated and evaporated. The residue was chromatographed on an Isolute SCX-2 cartridge (10 g) eluting with 0-30% ethyl actate in pentane. The product was obtained as a colourless gum.
Yield: 43 mg (25%)
LC-MS (Method 3): Rt 4.32 min, m/z 498 [MH⁺]

### Method B

From Intermediate 1 and 2 equivalents of 5-bromopent-1-ene using a similar procedure to that used in the synthesis of Intermediate 10.
Yield: 65%
LC-MS (Method 3): Rt 4.32 min, m/z 498 [MH⁺]

### Example 1

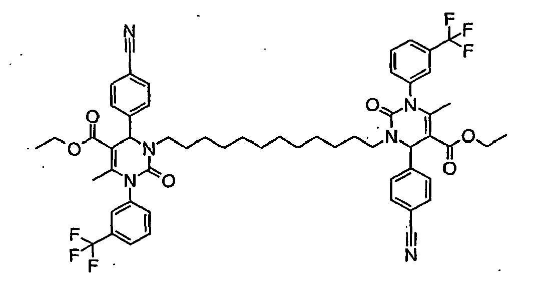

Example 1 was prepared from Intermediate 1 and 0.5 equivalents of 1,12-dibromododecane using a method similar to that used for the preparation of Intermediate 10. The product was purified by HPLC system 1.
Yield: 104 mg (30%)
LC-MS (Method 1): Rt 17.53, m/z 1025.12 [MH]⁺

### Examples 2

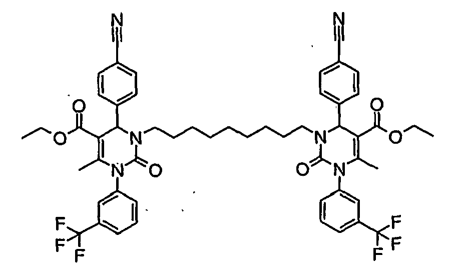

Example 2 was prepared from Intermediate 1 and 0.5 equivalents of 1,9-dibromononane using a method similar to that used for the preparation of Intermediate 10. The product was purified by HPLC system 1.
Yield: 22%
LC-MS (Method 1): Rt 16.50 min, m/z 983.09 [MH⁺]

### Example 3

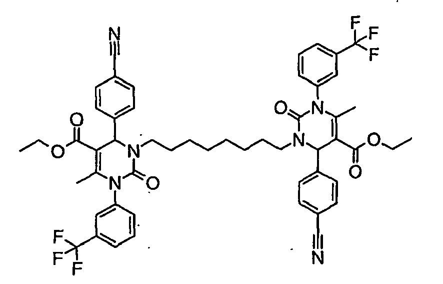

Example 3 was prepared from Intermediate 1 and 0.5 equivalents of 1,8-dibromooctane using a method similar to that used for the preparation of Intermediate 10. The product was purified by HPLC system 1.
Yield: 6%
LC-MS (Method 1): Rt 16.24 min, m/z 968.92 [MH⁺]

### Example 4

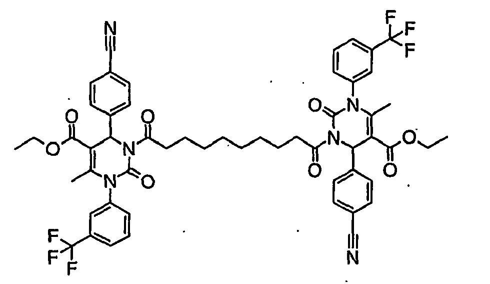

Example 4 was prepared from Intermediate 1 and 0.5 equivalents of sebacoyl chloride using a method similar to that used for the preparation of Intermediate 10, but using THF as the reaction solvent. The product was purified by MPLC on silica gel eluting with 25% EtOAc in pentane.
Yield: 94 mg (32%)
LC-MS (Method 1): Rt 16.42 min, m/z 1025.10 [MH⁺]

### Example 5

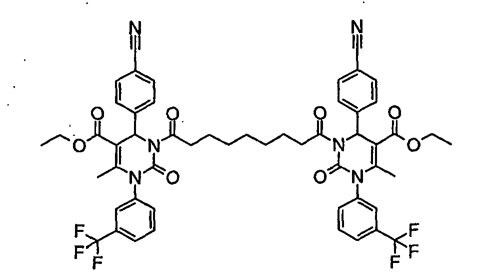

Example 5 was prepared from Intermediate 1 and 0.5 equivalents of azelaoyl chloride using a method similar to that used for the preparation of Intermediate 10 but using THF as the reaction solvent. Purification was carried out on an Isolute Si II column eluting with 0-15% EtOAc in cyclohexane, followed by HPLC (System I).
Yield: 44%
LC-MS (Method 1): Rt 16.10 min, m/z 1010.91 [MH⁺]

### Example 6

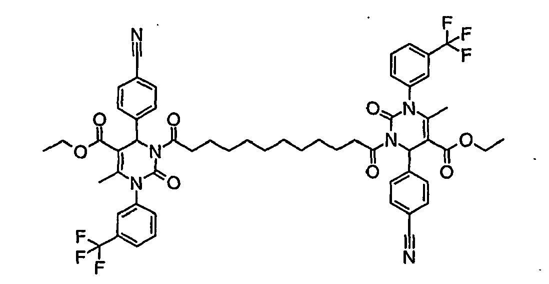

Example 6 was prepared from Intermediate 1 and 0.5 equivalents of dodecanedioyl dichloride using a method similar to that used for the preparation of Intermediate 10, but using THF as the reaction solvent. This compound was purified by HPLC system 1.
Yield: 50%
LC-MS (Method 1): Rt 16.93 min, m/z 1053.06 [MH⁺]

### Example 7

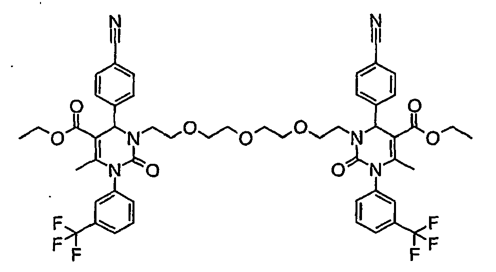

Example 7 was prepared from Intermediate 1 and 0.5 equivalents of Intermediate 9 using a method similar to that used for the preparation of Intermediate 10. This compound was purified twice by HPLC System 1 and then on an Isolute Si II cartridge (10 g) eluting with 0-30% EtOAc in pentane.
Yield: 30 mg (4%)
LC-MS (Method 1): Rt 15.15 min, m/z 1017.08 [MH⁺]

### Examples 8

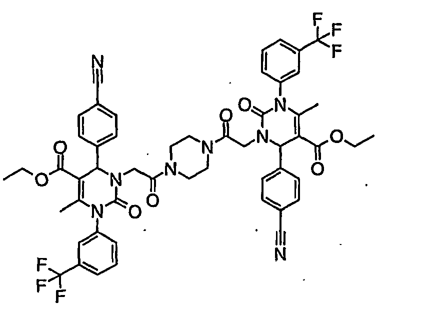

Example 8 was prepared from Intermediate 6 and 0.5 equivalents of piperazine using a similar procedure to that used for the synthesis of Intermediate 38. Purification was achieved using HPLC system 1.
Yield:: 19%
LC-MS (Method 1): Rt 13.64 min, m/z 1025.10 [MH⁺]

### Example 9

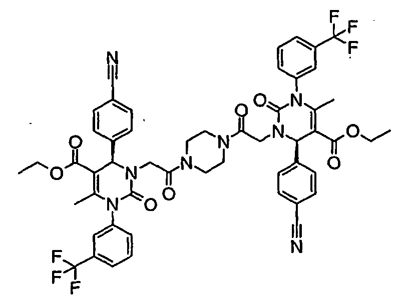

Example 9 was prepared in a similar manner from Intermediate 7 and purified using HPLC system 3.
Yield: 48%
LC-MS (Method 1): Rt 13.40 min, m/z 1025.22 [MH⁺]

### Example 10

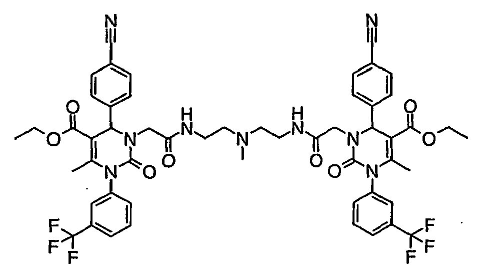

Example 10 was prepared from Intermediate 6 and 0.5 equivalents of *N*-methyl-2,2'-diaminoethylamine using a similar procedure to that used for the synthesis of Intermediate 38. Purification was achieved using HPLC system 1.
Yield: 11 %
LC-MS (Method 1): Rt 10.44 min, m/z 1056.17 [MH⁺]

### Example 11

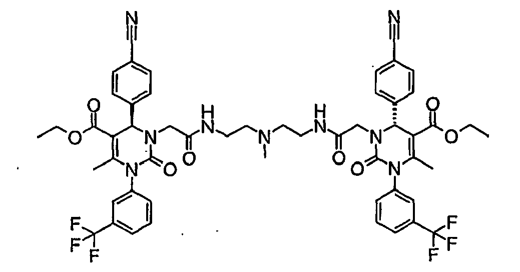

Example 11 was prepared in a similar manner from Intermediate 7 and purified using HPLC system 3...
Yield: 50%
LC-MS (Method 1): Rt 10.10 min, m/z 1056.21 [MH⁺]

### Example 12

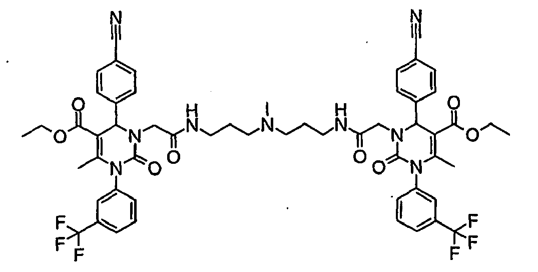

Example 12 was prepared from Intermediate 6 and 0.5 equivalents of 3,3'-diamino-*N-*methyldipropylamine using a similar procedure to that used for the synthesis of Intermediate 38. Purification was achieved using HPLC system 1.
Yield: 6% .
LC-MS (Method 1):Rt 10.38 min, m/z 1084.30 [MH⁺]

### Example 13

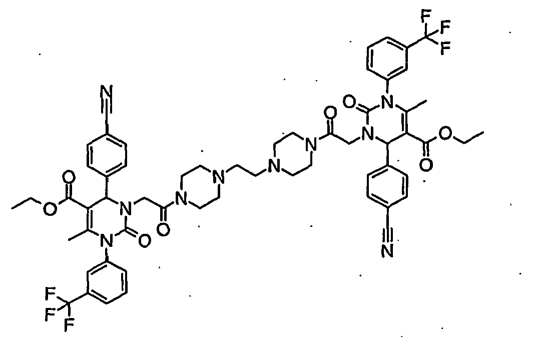

Intermediate 8 (61 mg, 0.154 mmol) was dissolved in 25% TFA in DCM (15 ml) and the solution was allowed to stand at RT for 1.5 h. The solvents were evaporated and the residue was re-dissolved in DMF (5 ml). Intermediate 6 (150 mg, 0.308 mmol) and DIPEA (161 µl, 0.924 mmol) were added, followed by HATU (176 mg, 0.462 mmol). The solution was allowed to stand at RT for 17 h and the solvent was evaporated. The residue was dissolved in EtOAc (60 ml) and the organic solution was washed with aqueous NaHCO₃ (30 ml), water (30 ml) and brine (20 ml). After drying (Na₂SO₄), the solvent was removed to give a residue which was purified by HPLC System 1.
Yield: 21%
LC-MS (Method 1): Rt 10.35 min, m/z 1136.97 [MH⁺]

### Example 14

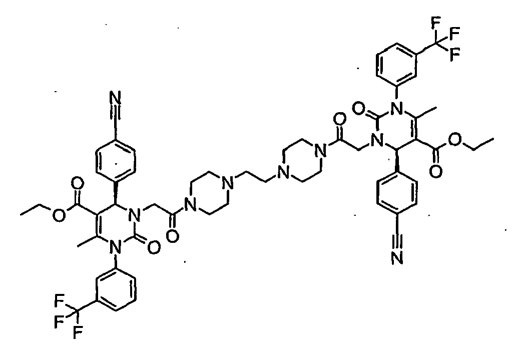

Example 14 was prepared in a similar manner from Intermediate 7 and purified using HPLC system 3.
Yield: 32%
LC-MS (Method 1): Rt 10.14 min, m/z 1137.29 [MH⁺]

### Example 15

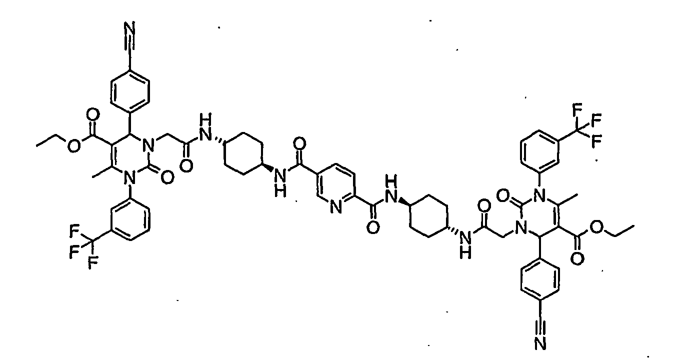

Example 15 was prepared by a similar procedure from Intermediate 6. The product was purified by HPLC system 1 and then HPLC system 5.
Yield: 5%
LC-MS (Method 1): Rt 13.42 min, m/z 1298.26 [MH⁺]

### Example 16

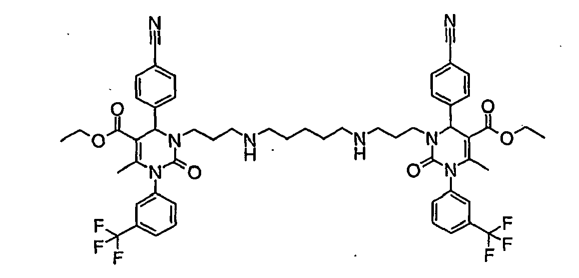

Intermediate 10 (98 mg, 0.178 mmol), 1,5-dibromopentane (8 mg, 0.080 mmol) and NaHCO₃ (60 mg, 0.712 mmol) in acetonitrile (5 ml) were heated at 80°C for 17 h. After cooling to RT the solvent was reduced and the mixture was purified by HPLC system 1.
Yield: 22 mg (22%)
LC-MS (Method 1): Rt 9.28 min, m/z 1041.13 [MH⁺]

### Example 17

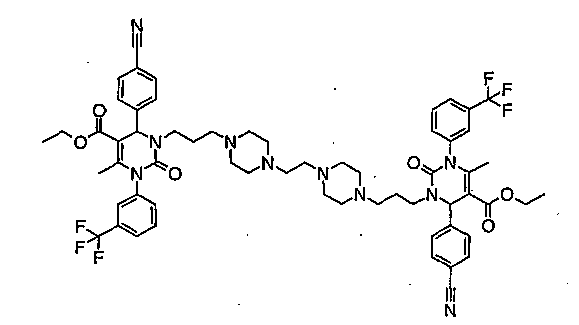

Intermediate 8 (72 mg, 0.181 mmol) was dissolved in 25% TFA in DCM (30 ml) and the solution was allowed to stand at RT for 1 h. The solvents were evaporated and the residue was re-dissolved in acetonitrile (10 ml). Intermediate 10 (200 mg, 0.364 mmol), and NaHCO₃ (276 mg, 3.286 mmol) were added and the mixture was heated at 80°C for 17 h. After cooling to RT and evaporation of the solvent, the residue was purified by HPLC system 1.
Yield: 65 mg (29%)
LC-MS (Method 1): Rt 8.91 min, m/z 1137.32 [MH⁺]

### Example 18

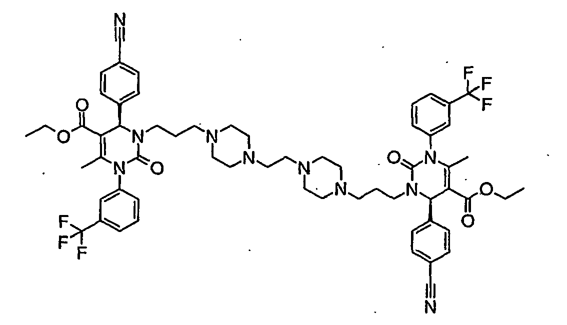

Example 18 was prepared in a similar manner from Intermediate 11 and purified using HPLC system 1.
Yield: 8%
LC-MS (Method 1): Rt 8.69 min, m/z 1137.44 [MH⁺]

### Example 19

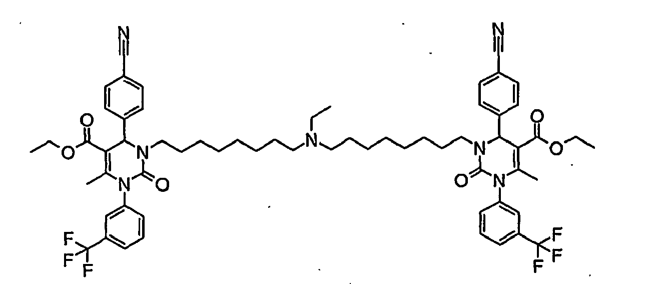

To a mixture of Intermediate 14 (33 mg, 0.0532 mmol) and NaHCO₃ (13 mg, 0,160 mmol) in acetonitrile (2 ml) was added 2M ethylamine in THF (53 µl, 0.106 mmol). The reaction mixture was heated at 80°C for 41 h. The solvent was removed and the residue was purified using HPLC system 1.
Yield: 14 mg (21 %)
LC-MS (Method 1): Rt 12.89 min, 1124.30 m/z [MH⁺]

### Example 20

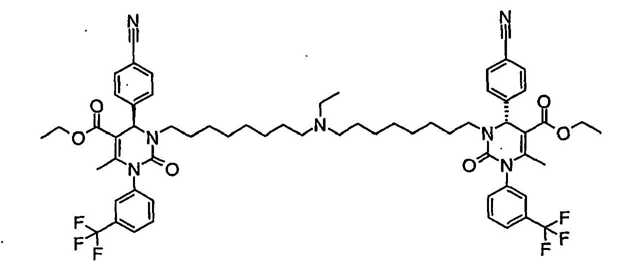

To a solution of Intermediate 15 (248 mg, 0.40 mmol) in acetonitrile (15 ml) was added NaHCO₃ (101 mg,1.2 mmol) and 2M ethylamine in THF (400 µl, 0.8 mmol). The mixture was heated at 80°C for 17 h and the volatiles were evaporated. The residue was re-dissolved in acetonitrile (15 ml) and heating was continued for 24 h. The mixture was filtered and concentrated. Purification was achieved using HPLC system 3.
Yield: 23%
LC-MS (Method 1): Rt 12.09 min, m/z 1124.30 [MH⁺]

### Example 21

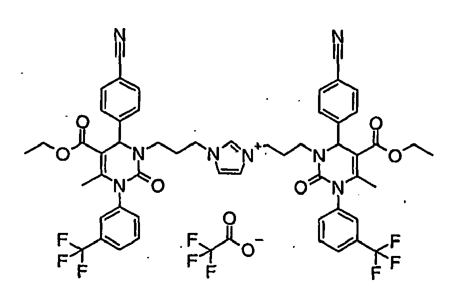

Intermediate 10 (70 mg, 0.130 mmol) and Intermediate 12 (72 mg, 0.130 mmol) were dissolved in acetonitrile (5 ml) and the solution was heated at 80°C for 40h. Evaporation of the solvent gave a residue that was purified by HPLC system 1.
Yield: 49 mg (34%)
LC-MS (Method 1): Rt 11.03 min, 1007.06 m/z [M⁺]

### Example 22

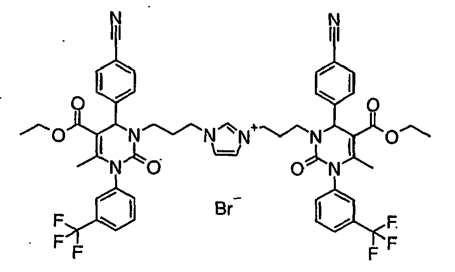

Example 22 was prepared in a similar manner from Intermediate 13 and Intermediate 11 and purified using HPLC system 3.
Yield: 8%
LC-MS (Method 1): Rt 10.85 min, m/z 1007.22 [MH⁺]

### Example 23

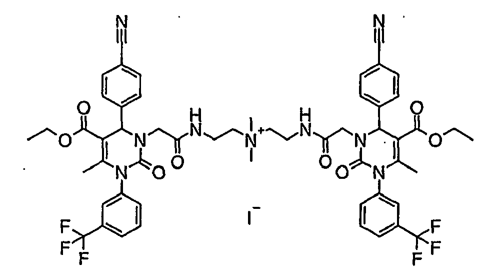

Example 10 (38 mg, 0.035 mmol) and iodomethane (1 ml) were dissolved in acetonitrile (5 ml). NaHCO₃ (18 mg, 0.214 mmol) was added and the mixture was heated at 80°C for 25 h. A further portion of iodomethane (1 ml) was added and heating was continued for a further 17 h, after which time the reaction mixture was allowed to cool and the solvent and excess iodomethane were evaporated. The residue was purified using HPLC system 1.
Yield: 11 mg (26%)
LC-MS (Method 1): Rt 10.40 min, 1070.07 m/z [M⁺]

### Example 24

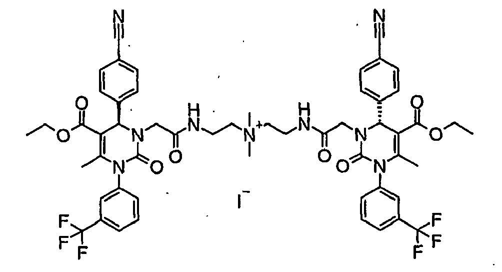

Example 24 was prepared in a similar manner from Example 11 and purified using HPLC system 3.
Yield: 67%
LC-MS (Method 1): Rt 10.15 min, m/z 1070.26 [M⁺]

### Example 25

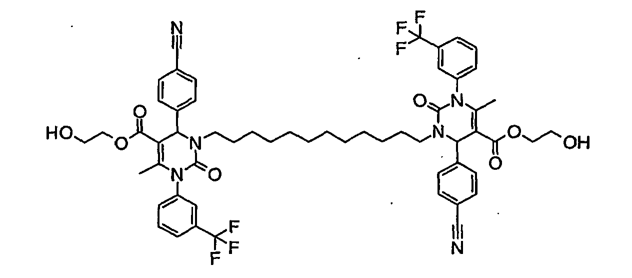

Intermediate 18 (100 mg, 0.095 mmol) was dissolved in acetonitrile (3 ml) and 1M aqueous HCI was added until the mixture became opaque. Acetonitrile was added dropwise until the solution became clear: After 1 h the product was extracted into EtOAc and the solution was washed with brine and dried (Na₂SO₄). Evaporation gave a residue which was purified using HPLC system 4.
Yield: 6.5 mg (6%)
LC-MS (Method 1): Rt 14.97 min, m/z 1057.30 [MH⁺]

### Example 26

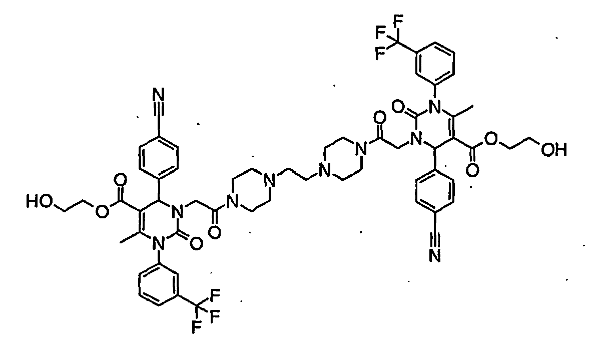

Example 26 was prepared from Intermediate 8 and Intermediate 17 by a method similar to that used for the synthesis of Example 13 The product was purified using HPLC system 4.
Yield: 17%
LC-MS (Method 1): Rt 9.34 min, m/z 1169.24 [MH⁺]

### Example 27

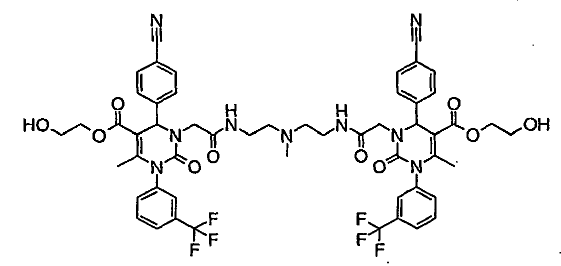

Example 27 was prepared from Intermediate 17 and *N*-methyl-2,2'-diaminoethylamine by a method similar to that used for the synthesis of Intermediate 38. The product was purified using HPLC system 4.
Yield:14%
LC-MS (Method 1): Rt 9.42 min, m/z 1088.18 [MH⁺]

### Example 28

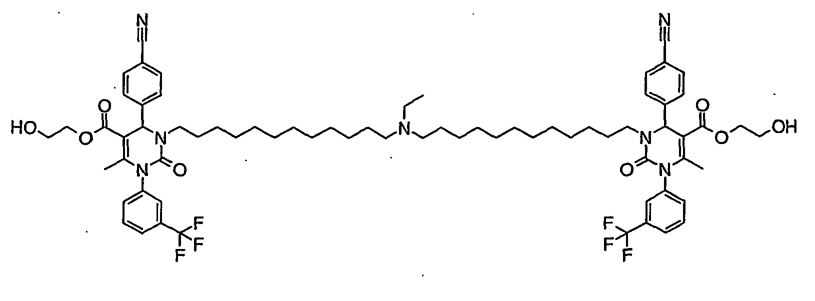

Intermediate 19 (40 mg, 0.058 mmol) was dissolved in acetonitrile (4 ml) and 1 M aqueous HCI (-2 ml) was added until the mixture went opaque. Acetonitrile was added drop wise until the solution was clear. After 1 h diethyl ether was added. The organic layer was separated, washed with water and brine, and dried (Na₂SO₄). The solvent was evaporated and the residue was re-dissolved in acetonitrile (4 ml). NaHCO₃ (15 mg, 0.17 mmol) and 2M ethylamine in THF (58 ml, 0.12 mmol) were added and the mixture was heated at 80°C for 24 h. After filtering, the solvent was removed and the product was purified using HPLC system 3.
Yield: 10 mg (27%)
LC-MS (Method 1): Rt 12.20 min, m/z 1268.41 [MH⁺]

### Example 29

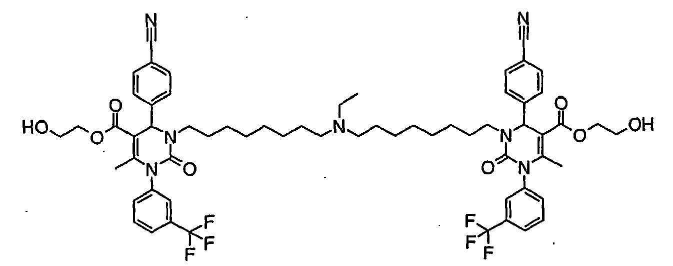

Example 29 was prepared from Intermediate 23 by a method similar to that used for the synthesis of Example 28. The product was purified using HPLC system 3.
Yield: 69%
LC-MS (Method 1): Rt 10.70 min, m/z 1156.38 [MH⁺]

### Example 30

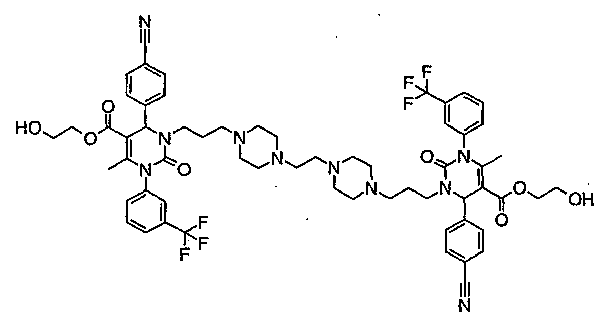

Example 30 was prepared from Intermediate 22 by a similar method to that used in the synthesis of Example 25. The product was purified by HPLC system 3.
Yield: 36%
LC-MS (Method 1): Rt 8.10 min, m/z 1169.38 [MH⁺]

### Example 31

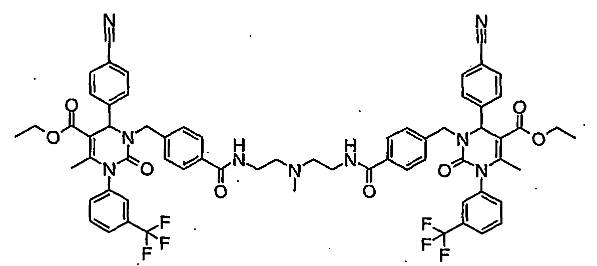

Example 31 was prepared from Intermediate 25 and *N*-methyl-2,2'-diaminoethylamine by a method similar to that used for the synthesis of Intermediate 38. The product was purified using HPLC system 1.
Yield: 61%
LC-MS (Method 1): Rt 10.76 min, m/z 1208.18 [MH⁺]

### Example 32

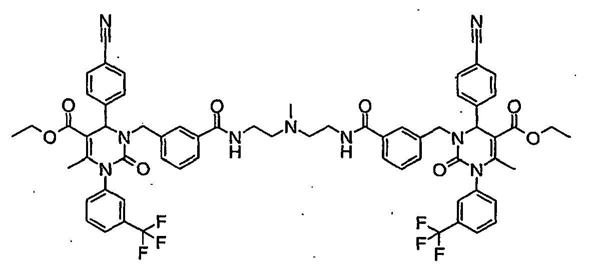

Example 32 was prepared from Intermediate 27 and *N*-methyl-2,2'-diaminoethylamine by a method similar to that used for the synthesis of Intermediate 38. The product was purified using HPLC system 1.
Yield: 53%
LC-MS (Method 1): Rt 10.93 min, m/z 1208.20 [MH⁺]

### Example 33

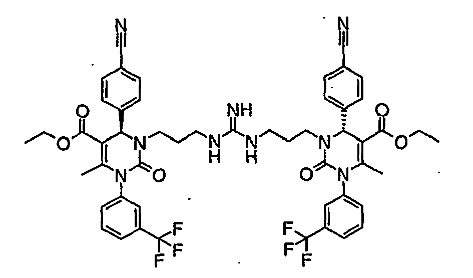

Intermediate 32 (263 mg, 0.27 mmol) was dissolved in IMS (10 ml) and iodomethane (379 ml, 2.67 mmol) was added. The solution was allowed to stand for 24 h and the volatiles were evaporated. The residue was dissolved in 2M NH₃ in EtOH (10 ml) and heated at 50°C in a sealed tube for 50 h. The solvent was removed and the product was purified by HPLC system 3.
Yield: 24 mg (9%)
LC-MS (Method 1): Rt 10.84 min, m/z 998.19 [MH⁺]

### Example 34

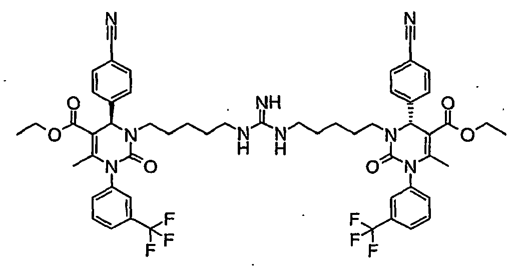

Example 34 was prepared from Intermediate 33 by a method similar to that used for the synthesis of Example 33. The product was purified using HPLC system 1.
Yield: 44%
LC-MS (Method 1): Rt 11.28 min, m/z 1054.26 [MH⁺]

### Example 35

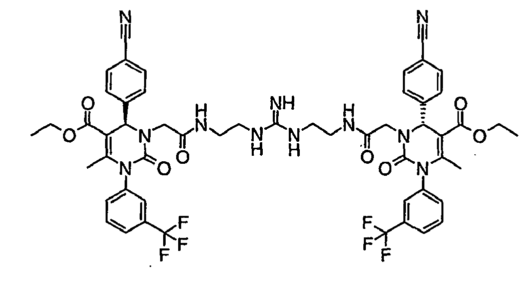

Intermediate 35 (150 mg, 0.127 mmol) was dissolved in MeOH (10 ml) and a solution of K₂CO₃ (235 mg, 1.71 mmol) in water (4 ml) was added. The mixture was stirred at RT for 30 min and then diluted with EtOAc (60 ml) and water (50 ml). The organic layer was separate and washed with brine. After drying (Na₂SO₄) the solvent was evaporated and the residue was purified using HPLC system 3.
Yield: 50 mg (36%)
LC-MS (Method 1): Rt 10.20 min, m/z 1084.23 [MH⁺]

### Example 36

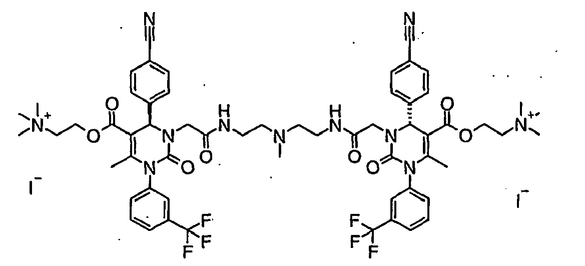

Intermediate 39 (150 mg, 0.15 mmol) was dissolved in DCM (4 ml) and hydroxyethyltrimethyl-ammonium iodide (173 mg, 0.75 mmol), EDC (86 mg, 0.45 mmol) and DMAP (55 mg, 0.45 mmol) were added. The mixture was stirred at RT for 7 h and water (4 ml) was added. The aqueous layer was separated and evaporated. Purification was achieved using HPLC system 3.
Yield: 35 mg (16%)
LC-MS (Method 1): Rt 6.78 min, m/z 585.80 [M²⁺]/2

### Example 37

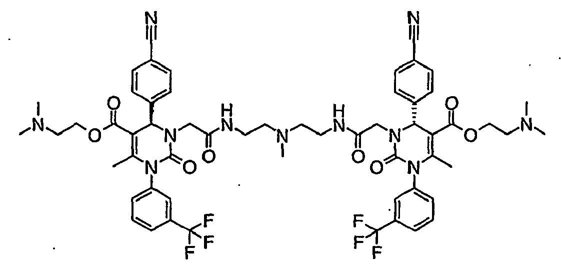

Intermediate 39 (150 mg, 0.15 mmol) was dissolved in DCM (4 ml) and *N*,*N-*dimethylethanolamine (67 mg, 0.75 mmol), EDC (86 mg, 0.45 mmol) and DMAP (55 mg, 0.45 mmol) were added. The mixture was stirred at RT for 50 h and water (4 ml) was added. After shaking, the organic layer was separated and evaporated. Purification was achieved using HPLC system 3.
Yield: 36 mg (21%)
LC-MS (Method 1): Rt 6.84 min, m/z 1142.37 [MH⁺]

### Example 38

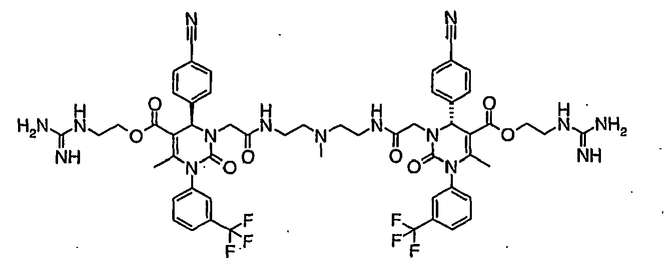

Intermediate 39 (150 mg, 0.15 mmol) was dissolved in DCM (4 ml) and 1,3-di-^{t}Boc-2-(2-hydroxyethyl)-guanidine (227 mg, 0.75 mmol), EDC (86 mg, 0.45 mmol) and DMAP (55 mg, 0.45 mmol) were added. The mixture was stirred at RT for 18 h the solution was diluted with DCM (8 ml) and washed with water (4 ml). TFA (2 ml) was added and the solution was allowed to stand at RT for 5 h after which time it was washed with sat. aqueous NaHCO₃ (5 ml) and evaporated. The residue, and any residue which did not dissolve in either DCM or water, was dissolved in 20% acetonitrile in water and purified by HPLC system 3.
Yield: 45 mg (26%)
LC-MS (Method 1): Rt 6.74 min, m/z 585.87 [MH₂²⁺]/2

### Example 39

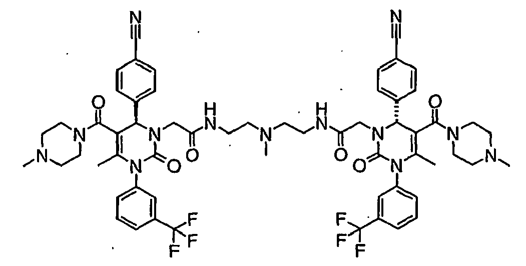

Example 39 was prepared from Intermediate 43 and *N*-methyl-2,2'-diaminoethylamine by a method similar to that used for the synthesis of Intermediate 38. The product was purified using HPLC system 3.
Yield: 37%
LC-MS (Method 1): Rt 5.72 min, m/z 1164.17 [MH⁺]

### Example 40

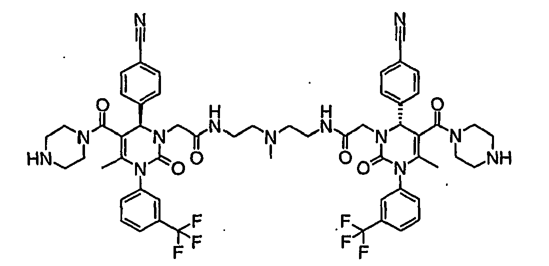

Intermediate 45 (191 mg, 0.143 mmol) was dissolved in DCM (15 ml) and TFA (5 ml) was added. The reaction was allowed to stand at RT for 1.5 h before evaporation of the volatiles. The product was purified using HPLC system 3.
Yield: 67 mg (41%)
LC-MS (Method 1): Rt 5.64 min, m/z 1136.14 [MH⁺]

### Example 41

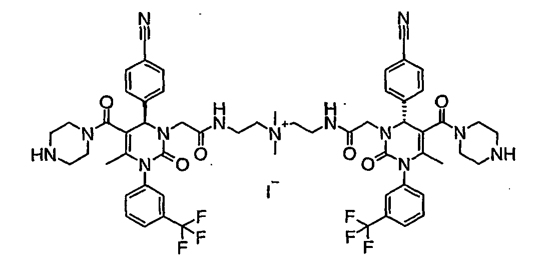

Intermediate 45 (191 mg, 0.143 mmol) was dissolved in DCM (20 ml) and iodomethane (5 ml) was added. The solution was allowed to stand at RT for 48 h. The volatiles were evaporated and the residue dissolved in 25% TFA in DCM (20 ml). After 1 h, evaporation gave a residue which was purified by HPLC system 3.
Yield: 35 mg (19%)
LC-MS (Method 1): Rt 5.65 min, m/z 1150.15 [M⁺]

### Example 42

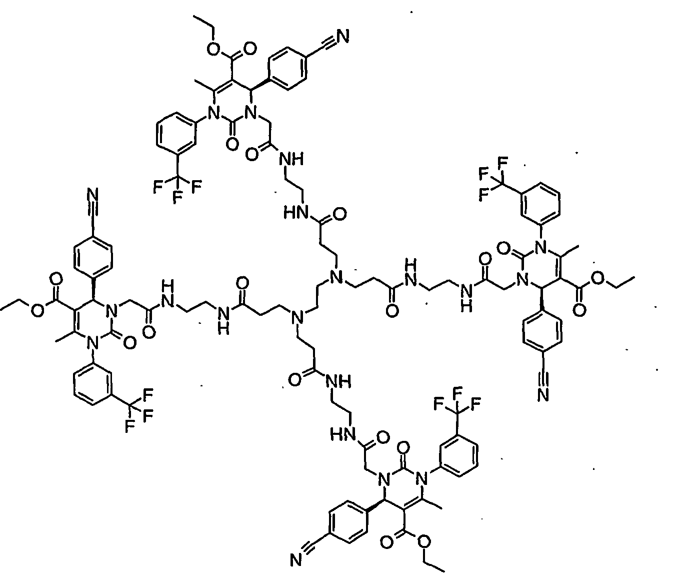

Example 42 was prepared from Intermediate 7 and 0.25 equivalents of Go PAMAM dendrimer using a similar procedure to that used in the synthesis of Intermediate 38. The product was purified using HPLC system 3.
Yield: 19%
LC-MS (Method 1): Rt 12.32 min, m/z 2394.49 [MH⁺]

### Example 43

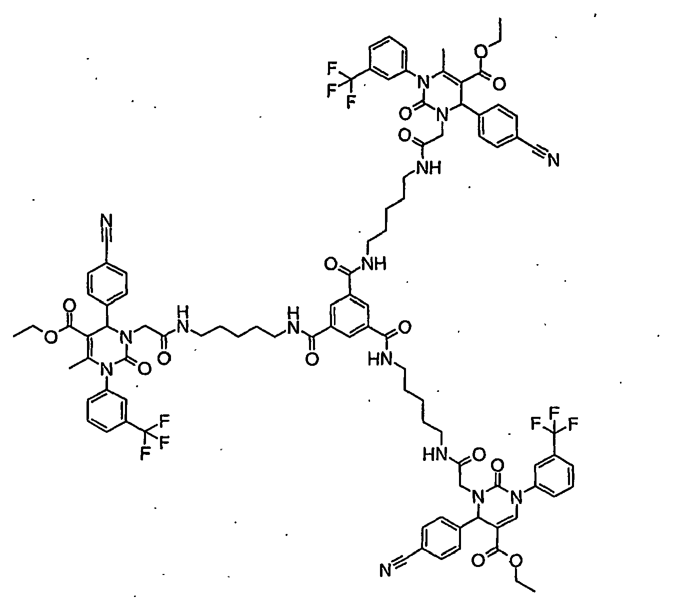

Intermediate 50 (168 mg, 0.295 mmol), benzenetricarbonyl trichloride (13 mg, 0.05 mmol) and DIPEA (0.2 ml) were dissolved in DCM (10 ml) and the mixture was stirred at RT for 40 min. A further quantity of benzenetricarbonyl trichloride (5 mg, 0.019 mmol) was added and the reaction was stirred for a further 48 h. The solvent was evaporated and the residue partially solidified. The product was isolated using HPLC system 2.
Yield: 90 mg (49%)
LC-MS (Method 1): Rt 14.45 min, m/z 1871.31 [MH⁺]

### Example 44

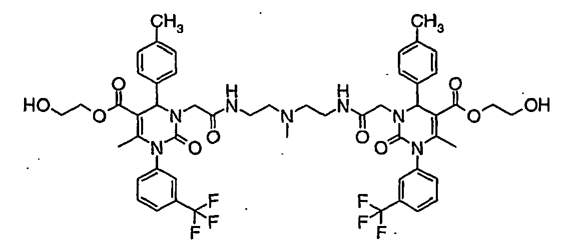

Intermediate 48 (150 mg, 0.118 mmol) was dissolved in EtOAc (15 ml) and acetic acid (2 drops) was added, followed by a spatula of Pd(OH)₂ on carbon. The reaction mixture was stirred under an atmosphere of hydrogen for 17 h. A further spatula of catalyst was added and stirring was continued for a further 5 h. The mixture was filtered and the solvent was evaporated. The product was purified by HPLC system 2.
Yield: 32 mg (25%)
LC-MS (Method 1): Rt 9.77 min, m/z 1066.22 [MH⁺]

### Example 45

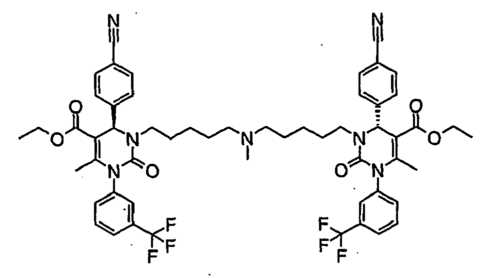

Example 45 was prepared from Intermediate 40 and 2M methylamine in THF by a method similar to that used for the synthesis of Example 19. The product was purified using HPLC system 3.
Yield: 22%
LC-MS (Method 1): Rt 11.08 min, m/z 1026.16 [MH⁺]

### Example 46

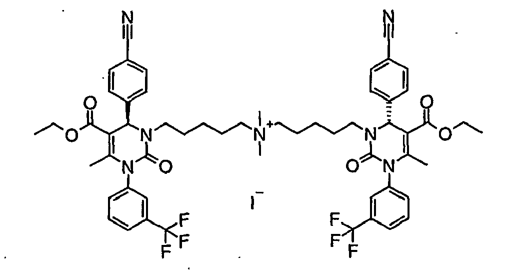

Example 45 (50 mg, 0.049 mmol) was dissolved in DCM (5 ml) and treated with iodomethane (1.5 ml). The solution was allowed to stand for 24 h. The volatiles were evaporated and the product was purified using HPLC system 3.
Yield: 24 mg (42%)
LC-MS (Method 1): Rt 11.27 min, m/z 1040.27 [M⁺]

### Example 47

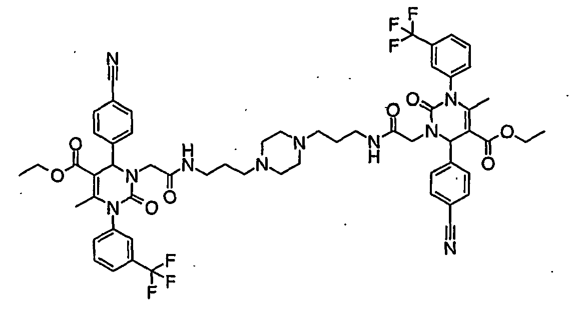

Example 47 was prepared from Intermediate 6 and Intermediate 59 by a procedure similar to that used for the synthesis of Intermediate 38. The product was purified by column chromatography using 0-5% EtOH in DCM. The material obtained was evaporated and purified further by preparative HPLC system 3.
Yield: 23%
LC-MS (Method 1): Rt 9.64 min, 1139.22 m/z [MH⁺]

### Example 48

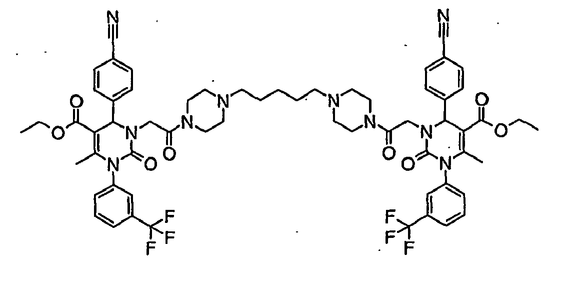

Intermediate 48 was prepared from Intermediate 6 and Intermediate 60 by a procedure similar to that used for the synthesis of Intermediate 38.
Yield: 51%
LC-MS (Method 1): Rt 8.61 min, 1179.37 m/z [MH⁺]

### Example 49

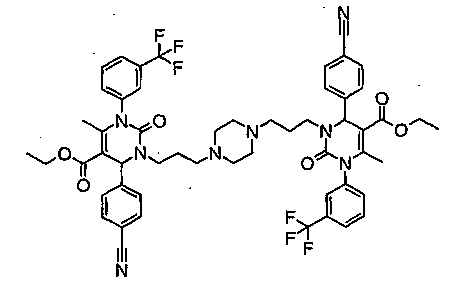

To a solution of Intermediate 68 (200 mg, 0.412 mmol) in DCE (8 ml) was added piperazine (17 mg, 0.206 mmol), powdered molecular sieves and sodium triacetoxyborohydride (98 mg, 0.464 mmol). The reaction mixture was stirred overnight then diluted with DCM and filtered before the solvent was removed. The crude material was purified by preparative HPLC system 3.
Yield: 35 mg (17%)
LC-MS (Method 1): Rt 10.69 min, 1025.30 m/z [MH⁺]

In analogy to the procedure for Example 49, the following compounds were prepared from Intermediate 67 and 0.5 equivalents of an amine or diamine (respectively Intermediates 60 to 66, homopiperazine, *N*-(2-aminoethyl)-*N*-methylethane diamine and ethylamine).

| **Example** | **Structure** | **Reaction time (h) (%)** | **Yield** | **Rt (min) (Method 1)** | **Mass [MH]⁺** |
|---|---|---|---|---|---|
| Example 50 | 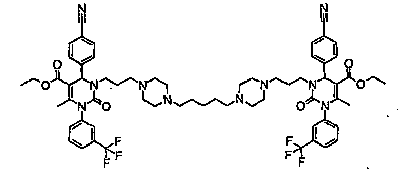 | 18 | 20 | 8.57 | 1179.42 |
| Example 51 | 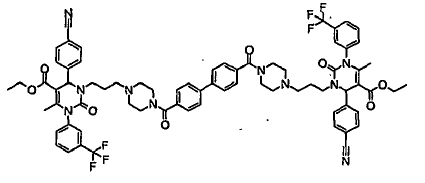 | 18 | 11* | 9.02 | 1317.19 |
| Example 52 | 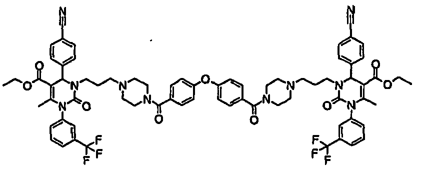 | 18 | 16* | 9.01 | 1333.28 |
| Example 53 | 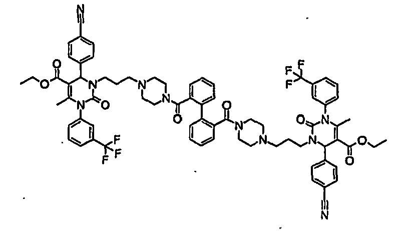 | 18 | 10* | 9.12 | 1317.22 |
| Example 54 | 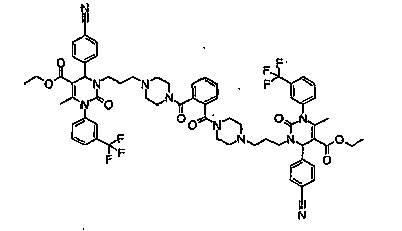 | 18 | 28* | 8.80 | 1241.33 |
| Example 55 | 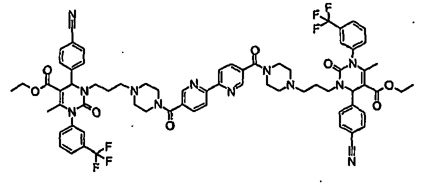 | 18 | 25* | 8.77 | 1319.32 |
| Example 56 | 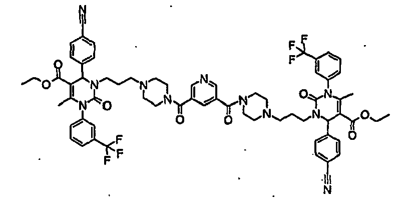 | 18 | 18* | 8.65 | 1242.15 |
| Example 57 | 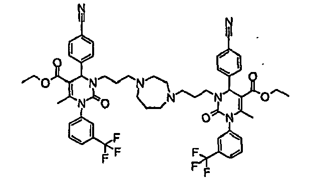 | 18 | 25* | 9.38 | 1039.27 |
| Example 58 | 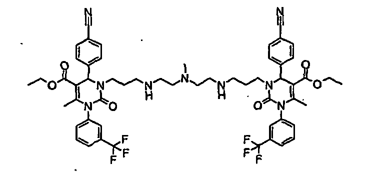 | 18 | ND | 8.84 | 1056.23 |
| Example 59 | 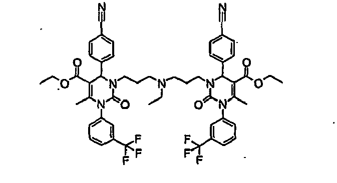 | 18 | 35* | 10.75 | 984.21 |

| | | | | | |
|---|---|---|---|---|---|
| *yield based on purification of half of the crude mixture | | | | | |

### Example 60

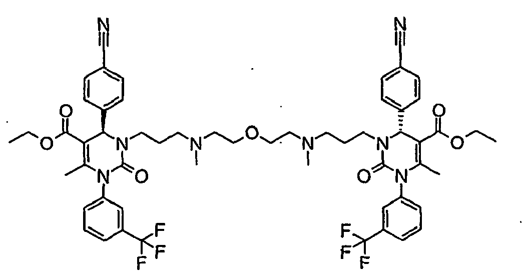

To a solution of Intermediate 70 (200 mg, 0.412 mmol) in THF (4 ml) under nitrogen was added 1,5-bis(methylamino)-3-oxapentane (22 mg, 0.166 mmol) and PS-Triacetoxyborohydride (358 mg, 0.8 mmol). The reaction mixture was shaken overnight then filtered and the solvent removed. The crude material was purified by HPLC system 3.
Yield: 45 mg (25%)
LC-MS (Method 1): Rt 8.94 min, 1071.28 m/z [MH⁺]

In analogy to the procedure for Example 60 the following compounds were prepared from Intermediate 70 and 0.5 equivalents of a diamine:

| **Example** | **Structure** | **Diamine** | **Reaction time (h)** | **Yield (%)** | **Rt (min) (Method 1)** | **Mass [MH]⁺** |
|---|---|---|---|---|---|---|
| Example 61 | 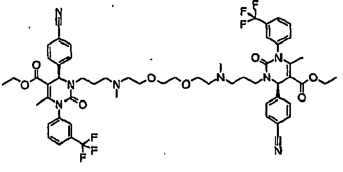 | 1,8-bis(methylamino)-3,6-dioxaoctane | 18 | 24 | 8.92 | 1115.29 |
| Example 62 | 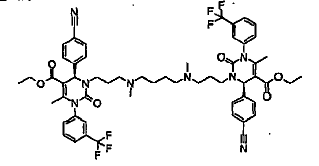 | *N*,*N*-dimethylbutane-1,4-diamine | 18 | 29 | 8.85 | 1055.28 |
| Example 63 | 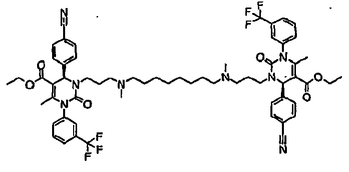 | *N*,*N*'-dimethyloctane-1,8-diamine | 18 | 33 | 9.07 | 1111.33 |

### Example 64

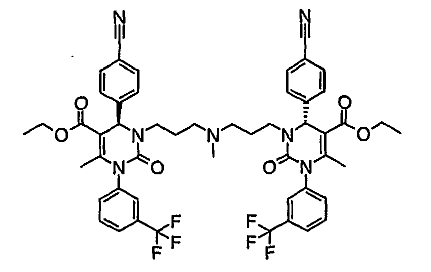

To a solution of Intermediate 11 (250 mg, 0.455 mmol) in THF (3 ml) was added NaHCO₃ (115 mg, 1.37 mmol) and 2M methylamine in THF (250 µl, 0.455 mmol). The reaction mixture was heated at 80°C for 5 h. A further amount of methylamine (500 µl) was added and heating continued overnight. The volatiles were evaporated and THF (3 ml) and NaHCO₃ (115 mg) were added. Heating at 80°C was resumed for 18 h. Evaporation gave crude material which was purified by HPLC system 3.
Yield: 55 mg (35%)
LC-MS (Method 1): Rt 10.73 min, 970.18 m/z [MH⁺]

### Example 65

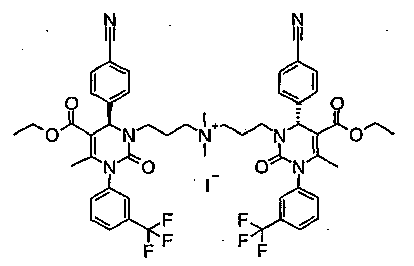

Example 65 was prepared from Example 64 using a similar procedure to that used for the synthesis of Example 46. The crude material was purified by HPLC system 3.
Yield: 69%
LC-MS (Method 1): Rt 10.73 min, 985.08 m/z [M⁺]

### Example 66

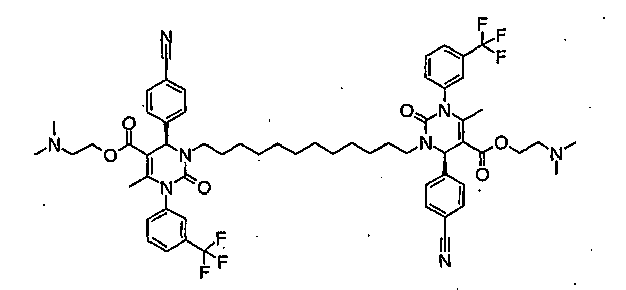

Example 66 was prepared from Intermediate 71 and 0.5 equivalents of 1,9-dibromododecane using a method similar to that used for the preparation of Intermediate 10. The product was purified by HPLC system 3.
Yield: 25%
LC-MS (Method 1): Rt 9.04 min, 1111.39 m/z [MH⁺]

### Example 67

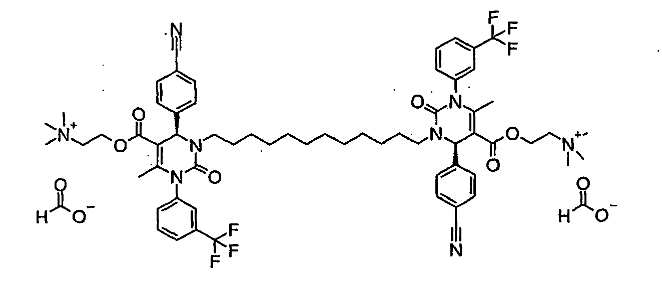

Example 66 (43 mg, 0.039 mmol) was dissolved in DCM (5 ml) and iodomethane (1ml) was added. The solution was stirred for 17 h then the solvent was removed and the crude material was purified by HPLC system 3.
LC-MS (Method 1): Rt 9.22 min, 570.80 m/z [M²⁺]/2

### Example 68

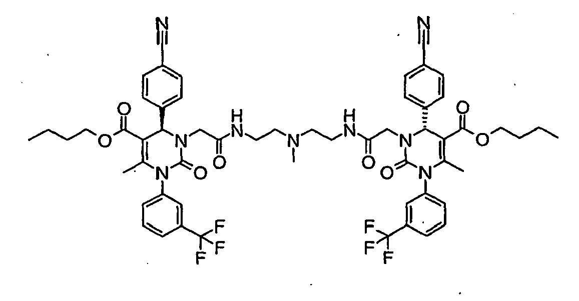

Example 68 was prepared from Intermediate 72 and 0.5 equivalents of *N*-methyl-2,2'-diaminoethylamine by a method similar to that used for the synthesis of Intermediate 38.
Yield: 42%
LC-MS (Method 1): Rt 11.36 min, 1112.28 m/z [MH⁺]

### Example 69

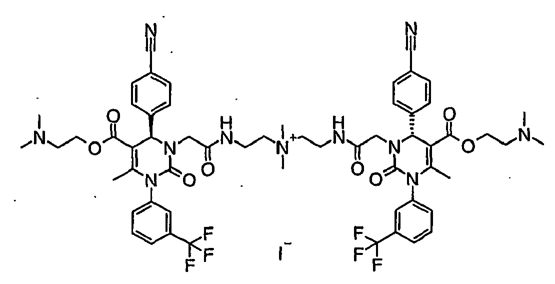

Example 69 was prepared from Intermediate 75 and dimethylaminoethanol by a similar procedure to that used in the synthesis of Example 36. The product was purified by chromatography on an Isolute Si II cartridge (10 g) using EtOAc; 5% of 2M NH₃ in MeOH in EtOAc; 100% of 2M NH₃ in MeOH then 5% conc. HCl (37% aqueous solution) in MeOH. The material isolated was taken up in acetonitrile and water then filtered and the filtrate evaporated then purified using HPLC system 3.
Yield: 52 mg (9%)
LC-MS (Method 1): Rt 6.57min, 1156.26 m/z [M⁺]

### Example 70

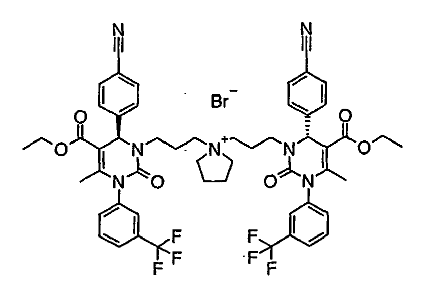

Intermediate 11 (100 mg, 0.182 mmol) was dissolved in acetonitrile (3 ml) and NaHCO₃ (76 mg, 0.91 mmol) and pyrrolidine (45 µl, 0.545 mmol) were added. The reaction was stirred for 18 h then filtered and evaporated. The residue was re-dissolved in DCM and washed with water and the solvent removed to afford monosubstituted pyrrolidine. This material was dissolved in acetonitrile (3 ml) then Intermediate 11 (100 mg, 0.182 mmol) was added, followed by NaHCO₃ (31 mg, 0.364 mmol) and the mixture stirred at RT for 30 h then at 40°C for 6 h. A further amount of Intermediate 11 (145 mg, 0.264 mmol) was added and the reaction heated at 80°C for 30 h. The crude material was filtered then evaporated and purified using HPLC system 3.
Yield: 103 mg (52%)
LC-MS (Method 1): Rt 10.83 min, 1010.09 m/z [M⁺]

### Example 71

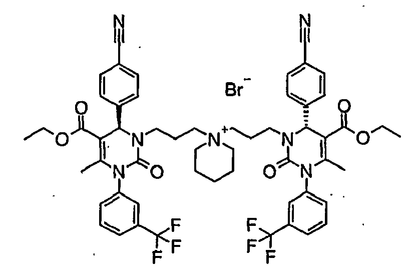

Example 71 was prepared from Intermediate 11 and piperidine using a similar method to that used in the synthesis of Example 70, except that the reaction was heated at 80°C for 48 h in second step of procedure.
Yield: 34%
LC-MS (Method 1): Rt 10.91 min, 1024.22 m/z [M⁺]

### Example 72

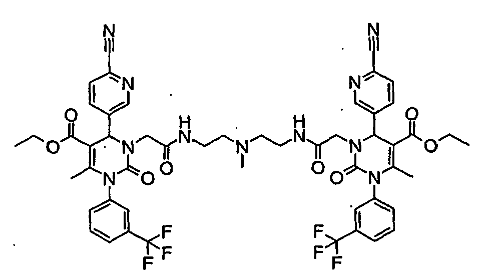

Example 72 was prepared from Intermediate 77 and 0.5 equivalents of *N*-methyl-2,2'-diaminoethylamine according to a method similar to that described for described for Intermediate 38.
Yield: 39%
LC-MS (Method 1): Rt 9.82 min, 1058.19 m/z [MH⁺]

### Example 73

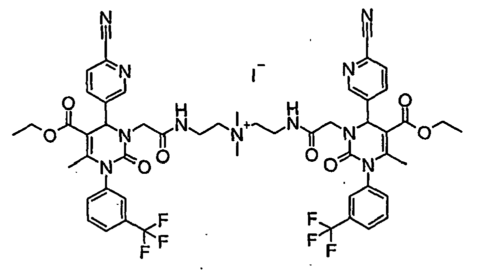

Example 72 (25 mg, 0.024 mmol) was dissolved in acetonitrile (3 ml), iodomethane (1 ml) was added and the reaction stirred at 80°C overnight. The solvent was removed and the crude material purified using HPLC system 3.
Yield: 5 mg (20%)
LC-MS (Method 1): Rt 9.77 min, 1072 [M⁺]

### Example 74

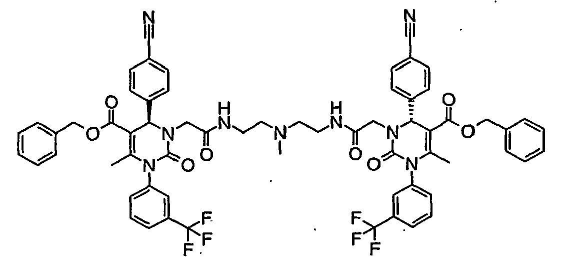

Example 74 was prepared from Intermediate 79 and 0.5 equivalents of *N-*methyl-2,2'-diaminoethylamine according to a method similar to that described for described for Intermediate 38. Purification was achieved using HPLC system 3.
Yield: 29%
LC-MS (Method 1): Rt 11.25 min, 1180.10 [MH⁺]

### Example 75

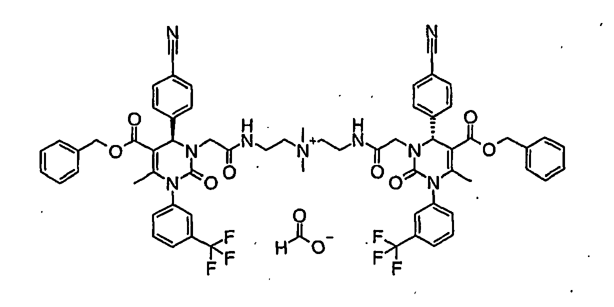

Example 74 (49 mg, 0.0415 mmol) was dissolved in acetonitrile (5 ml) and NaHCO₃ (7 mg, 0.083 mmol) and iodomethane (3 drops) added. The mixture was stirred at RT for 4 h then a further amount of iodomethane (3 drops) was added and the mixture stirred for 17 h. The crude material was filtered then evaporated then purified by using HPLC system 3.
Yield: 27 mg (54%)
LC-MS (Method 1): Rt 11.20min, 1194.23 m/z [M⁺]

### Example 76

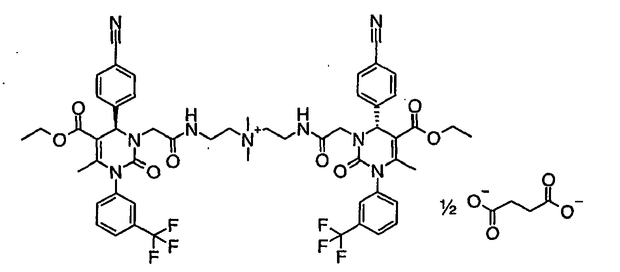

Succinic acid (4.9 mg, 0.042 mmol) was added to a stirred suspension of silver (I) oxide (9.67 mg, 0.042 mmol) in de-ionised water (2 ml). The mixture was stored at RT for 3 h with occasional warming. The cloudy solution was treated with Example 24 (100 mg, 0.083 mmol) and the resulting mixture was stirred at RT for 16 h. After this time the mixture was centrifuged (15°C, 13000 r.p.m.) for 1 h and the supernatant was removed and freeze-dried to give the hemi-succinate salt as a beige solid.
Yield: 64 mg (64%)
LC-MS (Method 1): Rt 10.25 min, 1070.11 m/z [M+]
Melting point: 138-143°C

### Elastase inhibition assays

Various compounds of the invention were tested for their inhibitory activity towards HNE.

### Fluorescent peptide substrate

Assays were performed in 96-well plates at a total assay volume of 100µl. The final concentration of the enzyme (human leukocyte elastase, Sigma E8140) was 0.00036 units/well. A peptide substrate (MeO-Suc-Ala-Ala-Pro-ValAMC, Calbiochem #324745) was used, at the final concentration of 100µM. The final concentration of DMSO was 1% in the assay buffer (0.05M Tris.HCl, pH 7.5, 0.1M NaCl; 0.1M CaCl2; 0.0005% brij-35).

The enzymatic reaction was started by adding the enzyme. The enzymatic reaction was performed at RT and after 30mins stopped by adding 50µl soybean trypsin inhibitor (Sigma T-9003) at a final concentration of 50µg/well. Fluorescence was read on the FLEXstation (Molecular Devices) using 380 nm excitation and 460 nm emission filters. The potency of the compounds was determined from a concentration series of 10 concentrations in range from 1000 nM to 0.051 nM. The results are means of two independent experiments, each performed in duplicate.

The compounds tested were shown to have IC₅₀ values for HNE in the range 1-1000nM.

### Using Fluorescently labelled elastin

Assays were performed in 96-well plate at a total assay volume of 100µl. The final concentration of the enzyme (human leukocyte elastase, Sigma E8140) was 0.002 units/well. Fluorescently labelled, solubilised elastin from bovine neck ligament (Molecular Probes, E-12056) was used at the final concentration of 15µg/ml. The final concentration of DMSO was 2.5% in the assay buffer (0.1 M Tris-HCL,pH8.0, containing 0.2mM sodium azide).

The enzymatic reaction was started by adding the enzyme. The enzymatic reaction was performed at RT and read after 120 minutes. Fluorescence was read on the FLEXstation (Molecular Devices) using 485 nm excitation and 530 nm emission filters. The potency of the compounds was determined from a concentration series of 10 concentrations in range from 25000nM to 1nM. The results are means of two independent experiments, each performed in duplicate.

The compounds tested were shown to have IC₅₀ values for HNE in the range 1-1000nM.

### Elastase Selectivity Assays

Selectivity for elastase inhibition was determined by testing the compounds against a panel of 6 proteases: plasmin, thrombin, cathepsin G, proteinase 3, trypsin, chymotrypsin (all sourced from Sigma, cat. No. P1867, T1063, C4428, P0615, T6424, C8949 respectively). Assays were performed in 96-well plate at a total assay volume of 100µl. A common, generic substrate was used for all proteases: fluorescently labelled casein (Molecular Probes, E-6639), at the final concentration of 20µg/ml (Cathepsin G and Chymotrypsin), 10µg/ml (Plasmin and Thrombin) or 5µg/ml (Proteinase 3 and Trypsin). The final concentration of the substrate was close to the respective Kₘ values as determined for this substrate. The final concentration of DMSO was 5% in the assay buffer (0.05M Tris.HCl, pH 7.5, 0.1M NaCl; 0.1M CaCl2; 0.0005% brij-35). The enzymatic reaction was started by adding the enzyme. The enzymatic reaction was performed at RT for 60min. Fluorescence was read on the FLEXstation (Molecular Devices) using 589 nm excitation and 617 nm emission filters. The potency of the compounds was determined from a concentration series of 8 concentrations in range from 500µM to 0.2µM. The results are means of two independent experiments, each performed in duplicate.

The compounds tested showed selectivities for a range of proteases from1 to >300 fold.

### Membrane Bound Elastase

Blood was collected from healthy human volunteers. PMNs were isolated by density centrifugation on ficol and red blood cells lysed hypotonically Cells were fixed with paraformaldehyde / gluteraldehyde and washed by centrifugation.

Compounds were made up in HBSS containing and incubated for 5 minutes at 37°C with cells. Fluorogenic AAPV substrate (Calbiochem #324745) was added to each well to make 100µl final volume and the plate read using a Spectramax Gemini Ex 380nm Em 460 for 30minutes at 37°C.

Compounds of the invention examined in this assay were found to exhibit IC₅₀ values of less than 100nM, preferably less than 20nM.

### Intracellular Elastase (controlled with lysed cell elastase)

PMNs were isolated as described previously. PMNs were added to 96-well polypropylene plates and DMSO or compound added to each well to give 150µl volume. The plate was incubated at 37°C for 30minutes. Cells were washed by centrifugation and lysed with HBSS containing 0.04% triton. Cell debris was pelleted and the supernatant transferred to a fresh pate, with compounds or DMSO. Fluorogenic AAPV substrate was added to all wells and the plate was read using a Spectramax Gemini Ex 380nm Em 460 for 30minutes at 37°C.

### Neutrophil Released Elastase Activity Assay (Human, Mouse, Guinea Pig) Generation of Released Neutrophil Elastase, from Guinea Pigs

Guinea pigs were treated with an LPS aerosol. Animals were left for 4 hours, euthanized and the lungs lavaged to recover PMN. Bronchoalveolar lavage fluid (BAL) was spun at 400g for 10minutes and the cells resuspended in HBSS. 10µM cytocholasin B was added to the cell suspension and incubated at 370C for 5 minutes after which 1 µM fMLP was added for a further 5 minutes. Cells were centrifuged at 400g for 10 minutes. 'Elastase rich supernatant' was transferred to a fresh tube.

### Generation of Released Neutrophil Elastase, from Mice

Mice were anaesthetised and treated with LPS i.n. Animals were left for 4 hours, euthanized and the lungs lavaged to recover PMN. Bronchoalveolar lavage fluid (BAL) was centrifuged at 400g for 10minutes and the cells resuspended in 1 ml of HBSS. 10µM cytocholasin B was added to the cell suspension and incubated at 370C for 5 minutes after which 1µM fMLP was added for a further 5 minutes. Cells were centrifuged at 400g for 10 minutes. Elastase rich supernatant' was transferred to a fresh tube.

### Generation of Human Released Neutrophil Elastase, from Humans

Human PMN were isolated as described previously. 10µM cytocholasin B was added to the cell suspension and incubated at 370C for 5 minutes after which 1µM fMLP was added for a further 5 minutes. Cells were centrifuged at 400g for 10 minutes. 'Elastase rich supernatant' was transferred to a fresh tube.

To a clear bottomed 96-well plate compounds were added and incubated for 5 minutes at 37)C with 'elastase rich' supernatant. Fluorogenic AAPV substrate was added to all wells and the plate read using a Spectramax Gemini Ex 380nm Em 460 for 30 minutes at 37°C. For comparison, an activity matched control of human elastase was also run.

### HNE induced lung haemorrhage in the rat

Instillation of human neutrophil elastase (HNE) into rat lung causes acute lung damage. The extent of this injury can be assessed by measuring lung haemorrhage. Male Sprague Dawley rats (175-220g) were obtained from Harlan UK Ltd., full barrier-bred and certified free from specified micro-organisms on receipt. Animals were weighed and randomly assigned to treatment groups (7-12 animals per group).

The vehicle used was 1 % DMSO/Saline. Inhibitors were dissolved in 1 % DMSO before the addition of 0.9% saline.

Animals in each study used to determine the efficacy of the elastase inhibitors delivered locally to the lung by a variety of routes. Rats were anaesthetised with the inhaled anaesthetic Isoflurane (4%) when the dose was given from 30 minutes to 6h prior to human neutrophil elastase (HNE) administration or terminally anaesthetised with hypnorm:hypnovel:water (1.5:1:2 at 2.7ml/kg) when the predose was given at less than 30 minutes prior to HNE administration and dosed either intratracheally (i.t.) by transoral administration using a Penn Century microsprayer or intranasally (i.n.) by dropping the fluid on to the nares. Animals either received vehicle or compound at a dose volume of 0.5ml/kg.

Animals that had been allowed to recover after dosing were terminally anaesthetised with hypnorm:hypnovel:water (1.5:1:2 at 2.7ml/kg). Once sufficiently anaesthetised, HNE (600units/ml) or sterile saline was administered by transoral tracheal instillation at a volume of 100µl using a Penn Century microsprayer. Animals were kept warm in a temperature controlled box and given top up doses of anaesthetic as required to ensure continuous anaesthesia until termination.

Animals were sacrificed (0.5ml to 1 ml sodium pentobarbitone) one hour post HNE challenge. The trachea was exposed and a small incision made between two tracheal rings allowing a cannula (10gauge, O.D. 2-10mm, Portex Ltd.) to be inserted approximately 2cm into the trachea towards the lung. This was secured into place with a cotton ligature. The lungs were then lavaged (BAL) three times with fresh 4ml aliquots of heparinised (10units/ml) phosphate buffered saline (PBS). The resultant BALF was kept on ice until it was centrifuged.

The BALF was centrifuged at 1000 r.p.m. for 10 minutes in a centrifuge cooled to between 4 and 10oC. The supernatant was discarded and the cell pellet resuspended in 1ml 0.1 % CETAB/PBS to lyse the cells. Cell lysates were frozen until spectrophotometric analysis for blood content could be made. Standards were prepared by making solutions of whole rat blood in 0.1% CETAB/PBS.

Once defrosted 100µl of each lysed cell suspension was placed into a separate well of a 96 well flat bottomed plate. All samples were tested in duplicate and 100µl 0.1% CETAB/PBS was included on the plate as a blank. The OD of the contents of each well was measured at 415nm using a spectramax 250 (Molecular devices).

A standard curve was constructed by measuring the OD (at 415nm) of different concentrations of blood in 0.1% CETAB/PBS (30, 10, 7, 3, 1, 0.3, 0.1µl/ml).

The amount of blood in each experimental sample was calculated by comparison to the standard curve. Data were then analysed as below:
1) The mean OD for duplicates was calculated
2) The value for the blank was subtracted from the value for all other samples
3) Data were assessed to evaluate the normality of distribution.

The compounds were shown to have desirable HNE inhibitory activity.

## Claims

1. A compound of formula (I)
M-L-M (I)
wherein L is a linker and each M is independently a group of formula (II): 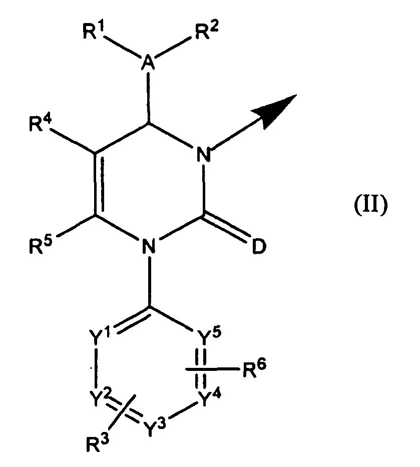 wherein A is aryl or heteroaryl;
D is oxygen or sulphur;
Y¹, Y², Y³, Y⁴ and Y⁵ are independently each CH, CR³, CR⁶ or N, with the proviso that one of them is CR³, one is CR⁶ and not more than two are N;
R¹, R² and R³ are independently each hydrogen, halogen, nitro, cyano, C₁-C₆-alkyl, hydroxy or C₁-C₆-alkoxy, wherein alkyl and alkoxy are each optionally substituted with one to three radicals independently selected from halogen, hydroxy and C₁-C₄-alkoxy;
R⁴ is trifluoromethylcarbonyl, C₁-C₆-alkylcarbonyl, C₁-C₆-alkoxycarbonyl, C₁-C₆-alkenoxycarbonyl, hydroxycarbonyl, aminocarbonyl, mono- or di-C₁-C₄-alkylaminocarbonyl, C₆-C₁₀-arylaminocarbonyl, phenylcarbonyl, heteroarylcarbonyl, heteroaryl or cyano, wherein alkylcarbonyl, alkoxycarbonyl and alkylaminocarbonyl are each optionally substituted with one to three radicals independently selected from C₃-C₈-cycloalkyl, hydroxy, C₁-C₄-alkoxy, C₁-C₄-alkoxycarbonyl, hydroxycarbonyl, aminocarbonyl, mono- and di-C₁-C₄-alkylaminocarbonyl, C₁-C₄-alkylcarbonylamino, (C₁-C₄-alkylcarbonyl)-C₁-C₄-alkylamino, cyano, amino, mono- and di-C₁-C₄-alkylamino, heteroaryl, heterocyclyl and tri-(C₁-C₆-alkyl)-silyl, and wherein heteroarylcarbonyl, and heteroaryl can be further substituted with C₁-C₄-alkyl;
or R⁴ represents a group of Formula (VIII) 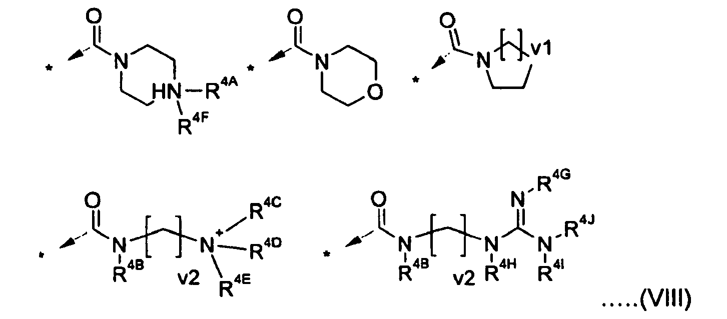 wherein
R^{4A}, R^{4B}, R^{4G}, R^{4H}, R^{4I} and R^{4J} are independently hydrogen or alkyl, or R^{4H} and R^{4I} may may be joined together with the nitrogen atom to which they are attached to form a ring;
R^{4F} is a lone pair or R^{4F} is alkyl and the nitrogen atom to which it is attached is quaternary and carries a positive charge;
R^{4C}, R^{4D} and R^{4E} are alkyl, or any two of R^{4C}, R^{4D} or R^{4E} may be joined together with the nitrogen atom to which they are attached to form a ring, optionally containing a further heteroatom selected from oxygen and nitrogen;
v1 is 1-3;
v2 is 1-6;
R⁵ is C₁-C₄-alkyl or amino, and alkyl is optionally substituted with one to three radicals independently selected from halogen, hydroxy, C₁-C₆-alkoxy, C₁-C₆-alkenoxy, C₁-C₆-alkylthio, amino, mono- and di- C₁-C₆-alkylamino, phenylamino, hydroxycarbonyl, C₁-C₆-alkoxycarbonyl and the radical -O-(C₁-C₄-alkyl)-O-(C₁-C₄-alkyl); and
R⁶ is halogen, nitro, cyano, C₁-C₆-alkyl, hydroxy or C₁-C₆-alkoxy, wherein alkyl and alkoxy are optionally substituted with one to three radicals independently selected from halogen, hydroxy and C₁-C₄-alkoxy;
wherein heteroaryl is selected from benzimidazolyl, benzoxazolyl, benzothiazolyl, benzofuranyl, benzothienyl, furyl, imidazolyl, indolyl, indolizinyl, isoxazolyl, isoquinolinyl, isothiazolyl, oxazolyl, oxadiazolyl, pyrazinyl, pyridazinyl, pyrazolyl, pyridyl, pyrimidinyl, pyrrolyl, quinazolinyl, quinolinyl, tetrazolyl, 1,3,4-thiadiazolyl, thiazolyl, thienyl and triazolyl;
or a pharmaceutically acceptable salt, solvate or N-oxide thereof.

2. A compound according to claim 1, wherein A is phenyl.

3. A compound according to claim 1 or claim 2, wherein R¹ is hydrogen.

4. A compound according to any preceding claim, wherein R² is methyl or -CN.

5. A compound according to any preceding claim, wherein R³ is hydrogen.

6. A compound according to any preceding claim, wherein R⁴ is -C(O)OCH₂CH₃ or -C(O)OCH₂CH₂OH.

7. A compound according to any preceding claim, wherein R⁵ is methyl.

8. A compound according to any preceding claim, wherein R⁶ is trifluoromethyl.

9. A compound according to any preceding claim, wherein none of Y¹, Y², Y³, Y⁴ and Y⁵ is N.

10. A compound according to any preceding claim, wherein D is oxygen.

11. A compound according to any preceding claim, wherein L is a group of formula (III)
-L^{a}-R⁷-L^{b}-W-L^{b}-R⁷-L^{a}- (III)
wherein L^{a} is a bond or -C(O)-;
L^{b} is a bond or -C(O)-;
R⁷ is alkylene or cycloalkylene;
W is a bond, N(R^{9B})(R^{9C}) or is selected from the following divalent radicals
-(O-R^{8A})ₘ₁-O-
-N(R^{9A})-(O-R^{8A})ₘ₁-R^{8A}-N(R^{9A})-
-N(R^{9A})-R^{8B}-N(R^{9B})(R^{9C})-R^{8B}-N(R^{9A})-
-N(R^{9A})-R^{8B}-N(R^{10B})C(=NR^{10A})(NR^{10C})-R^{8B}-N(R^{9A})-
-N(R^{9A})-R^{8B}-N(R^{9A})- 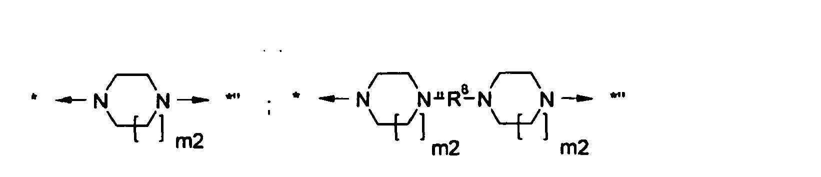 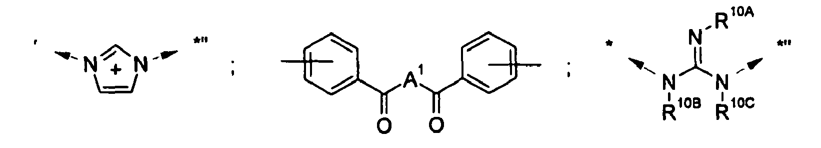
wherein
m1 is 1-4;
R^{8A} is alkylene or cycloalkylene;
R^{8B} is an alkylene or cycloalkylene group, or a group of Formula A²;
R^{9A} is hydrogen or lower alkyl;
one of R^{9B} or R^{9C} is a lone pair and the other is hydrogen or C₁₋₁₂ alkyl, or R^{9B} and R^{9C} are both C₁₋₁₂ alkyl, in which case the nitrogen to which they are attached is quaternary and carries a positive charge, or NR^{9B}R^{9C} is a ring;
R^{10A} is hydrogen or C₁₋₁₂ alkyl;
R^{10B} and R^{10C} are independently hydrogen or C₁₋₁₂ alkyl, or R^{10B} and R^{10C} are joined together to form a ring;
m2 is 1-3;
A¹ is selected from -N(R^{9A})-R⁸-N(R^{9B})(R^{9C})-R⁸-N(R^{9A})- and -N(R^{9A})-R⁸-N(R^{10B})C(=NR^{10A})(NR^{10C})-R⁸-N(R^{9A})-; and
A² is selected from 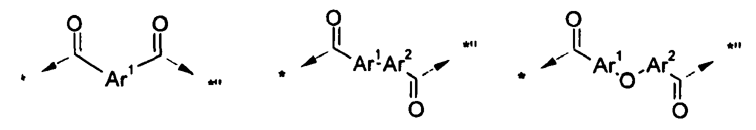 wherein Ar¹ and Ar² are each independently phenyl or heteroaryl as defined in claim 1.

12. A compound according to claim 11, wherein each L^{a} is independently -C(O)- or a covalent bond.

13. A compound according to claim 12, wherein L^{a} is a bond.

14. A compound according to claims 11 to 13, wherein R⁷ is alkylene.

15. A compound according to any of claims 11 to 14, wherein W is -N(R^{9A})-R^{8B}-N(R^{9B})(R^{9C})-R^{8B}-N(R^{9A})-.

16. A compound according to any of claims 11 to 14, wherein W is -N(R^{9A})-R^{8B}-N(R^{10B})C(=NR^{10A})(NR^{10C})-R^{8B}-N(R^{9A}).

17. A compound according to any of claims 11 to 14, wherein W is

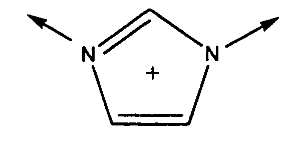

18. A compound according to any of claims 11 to 14, wherein W is

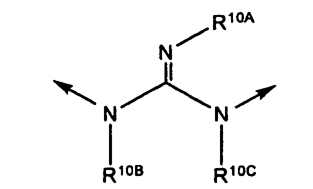

19. A compound according to any of claims 11 to 14, wherein W is -N(R^{9B})(R^{9C})-.

20. A compound according to any preceding claim, wherein A is phenyl or heteroaryl; and
R⁴ is trifluoromethylcarbonyl, C₁-C₆-alkylcarbonyl, C₁-C₆-alkoxycarbonyl, C₁-C₆-alkenoxycarbonyl, hydroxycarbonyl, aminocarbonyl, mono- or di-C₁-C₄-alkylaminocarbonyl, C₆-C₁₀-arylaminocarbonyl, phenylcarbonyl, heteroarylcarbonyl, heteroaryl or cyano, wherein C₁-C₆-alkylcarbonyl, C₁-C₆-alkoxycarbonyl, mono- and di-C₁-C₄-alkylaminocarbonyl can be further substituted with one to three identical or different radicals selected from the group consisting of C₃-C₈-cycloalkyl, hydroxy, C₁-C₄-alkoxy, C₁-C₄-alkoxycarbonyl, hydroxycarbonyl, aminocarbonyl, mono- and di-C₁-C₄-alkylaminocarbonyl, C₁-C₄-alkylcarbonylamino, (C₁-C₄-alkylcarbonyl)-C₁-C₄-alkylamino, cyano, amino, mono- and di-C₁-C₄-alkylamino, heteroaryl, heterocyclyl and tri-(C₁-C₆-alkyl)-silyl, and wherein heteroarylcarbonyl and heteroaryl can be further substituted with C₁-C₄-alkyl.

21. A compound according to claim 1, which is any of

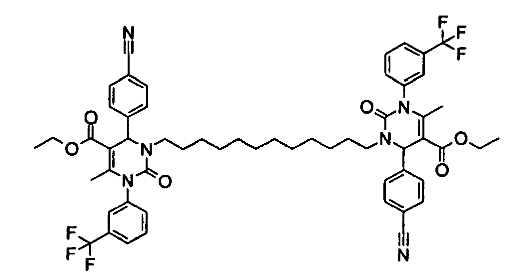

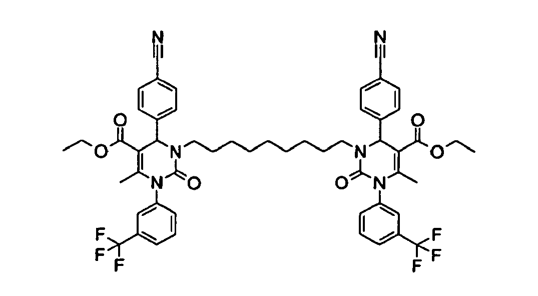

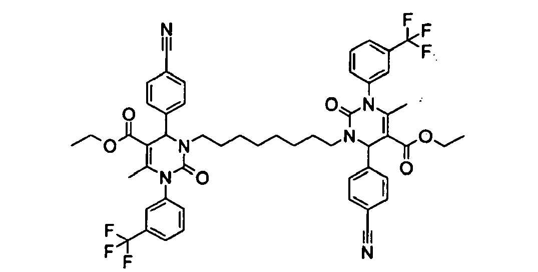

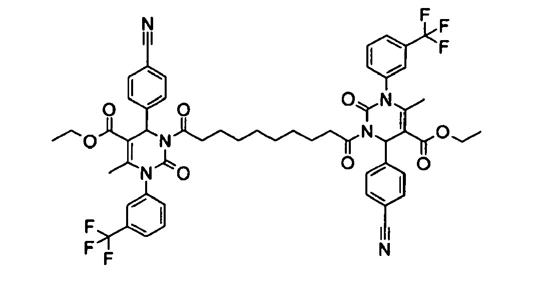

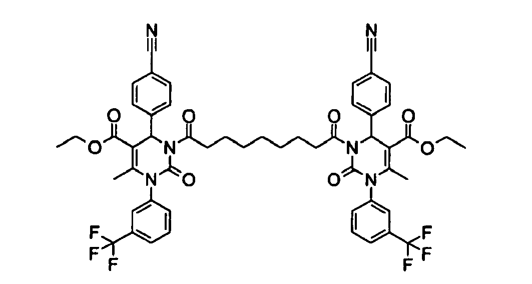

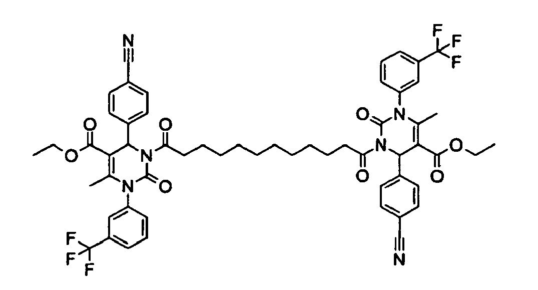

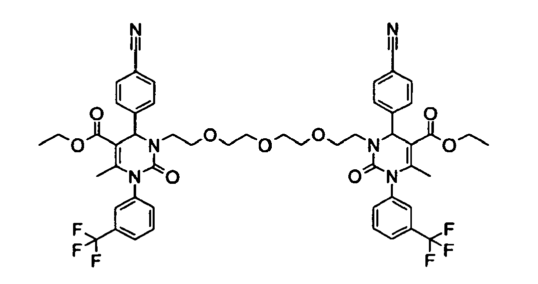

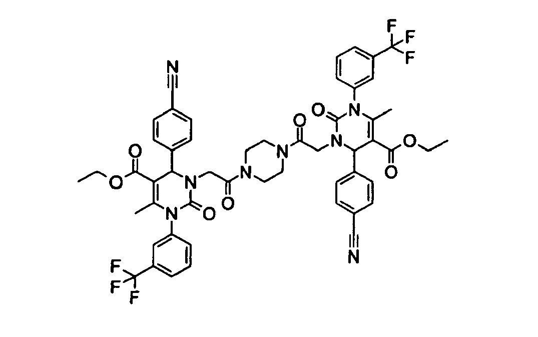

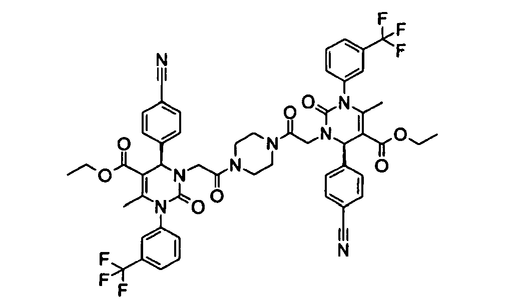

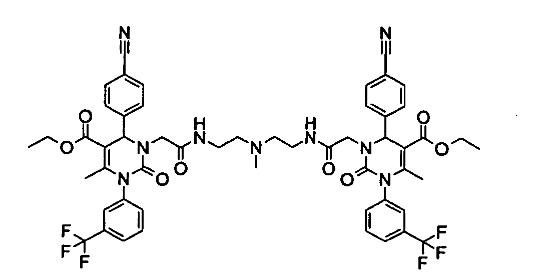

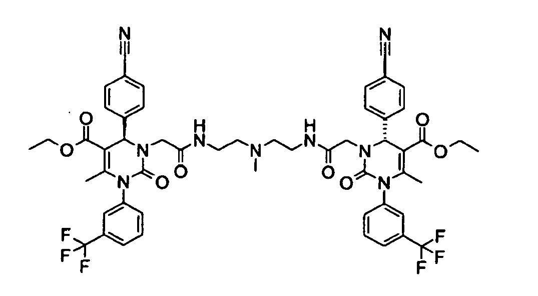

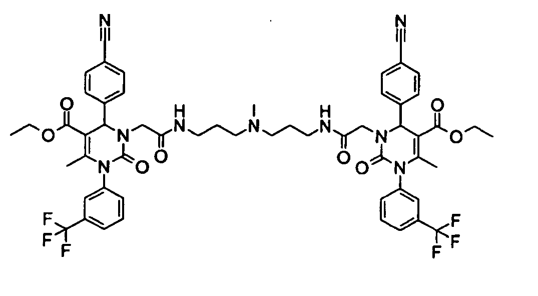

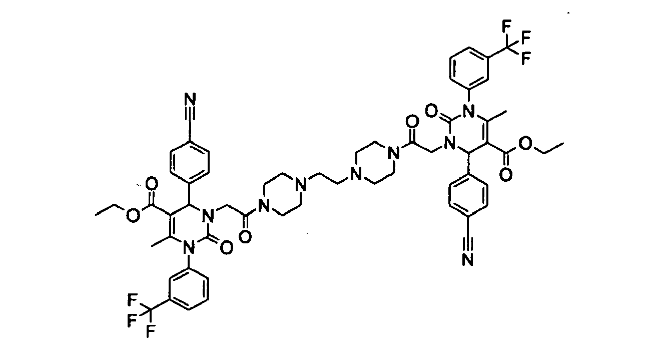

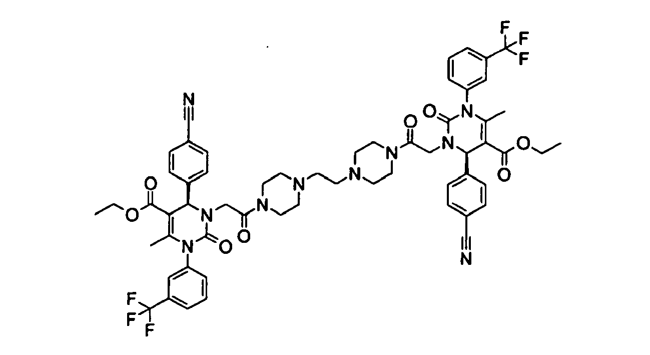

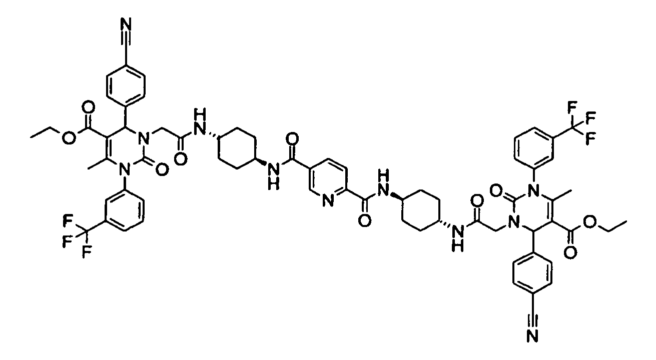

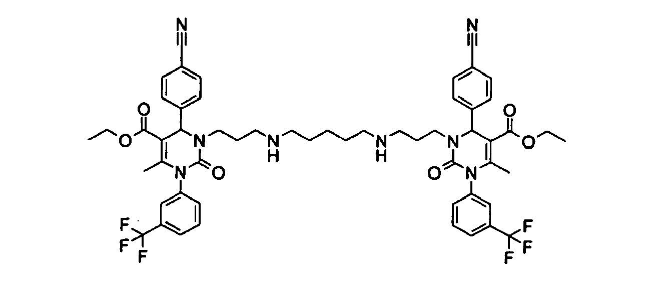

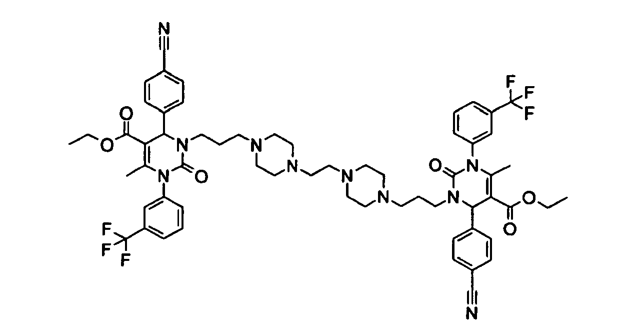

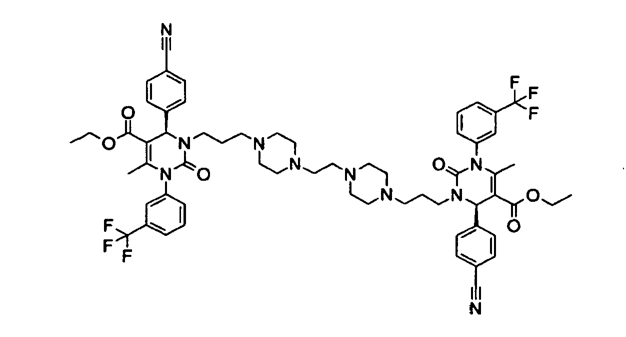

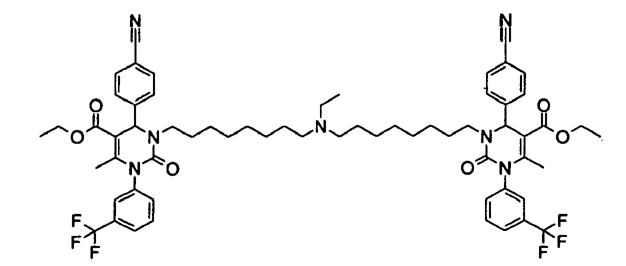

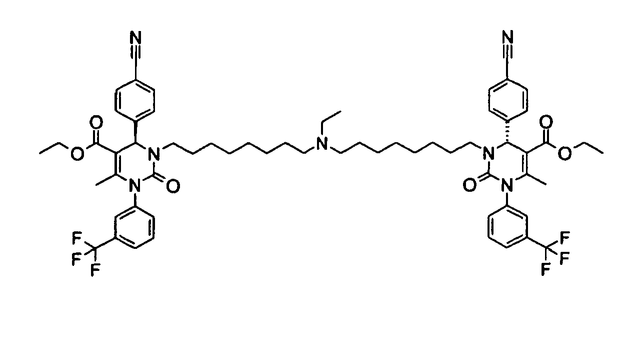

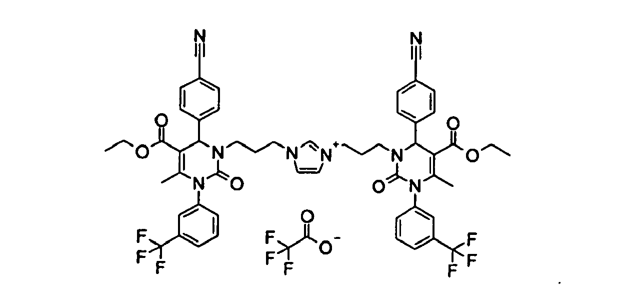

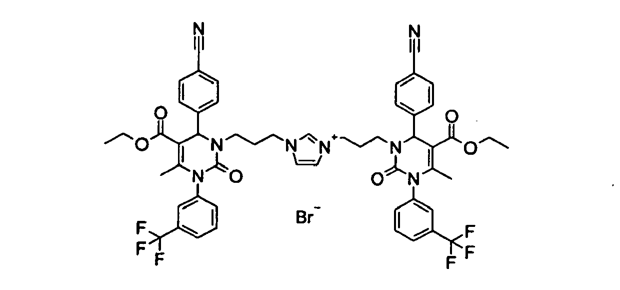

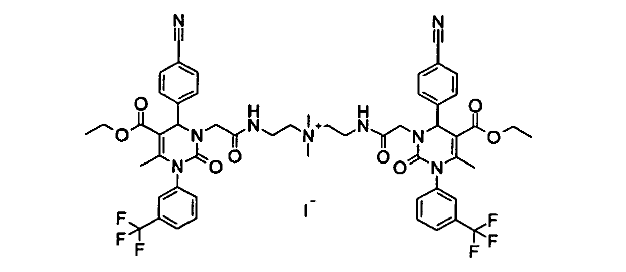

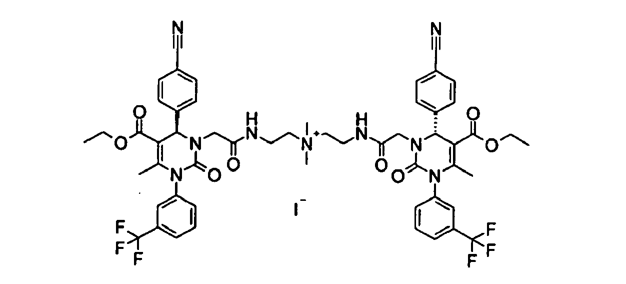

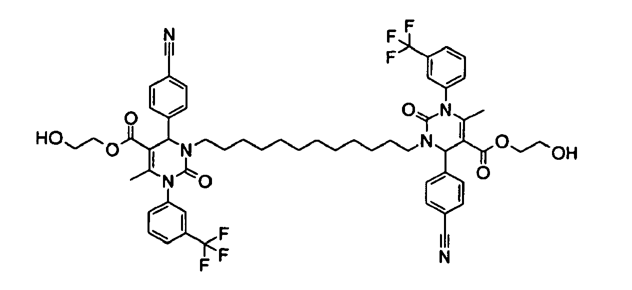

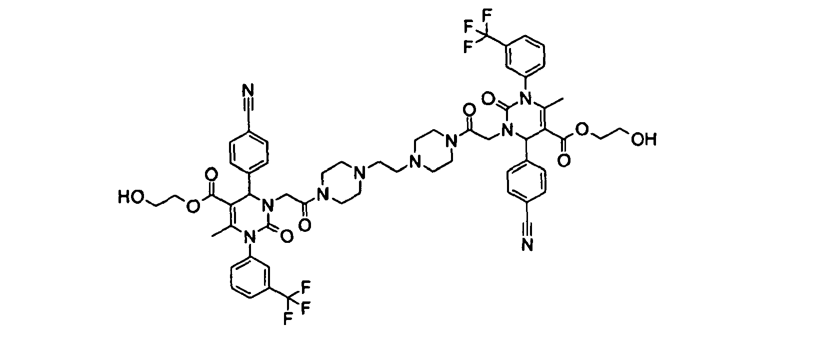

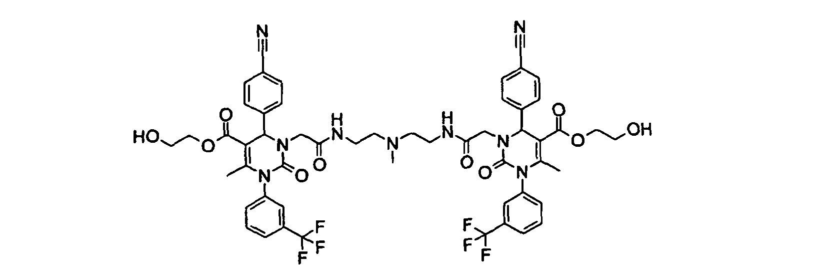

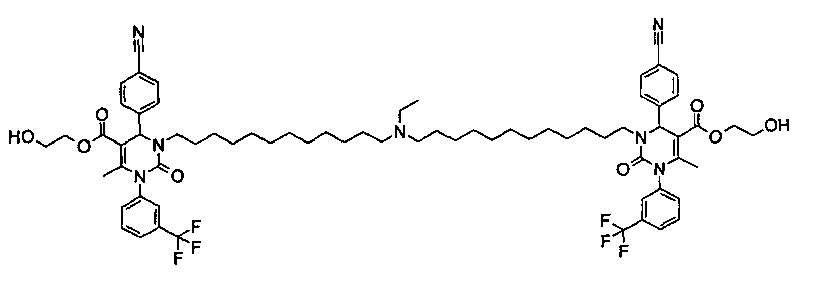

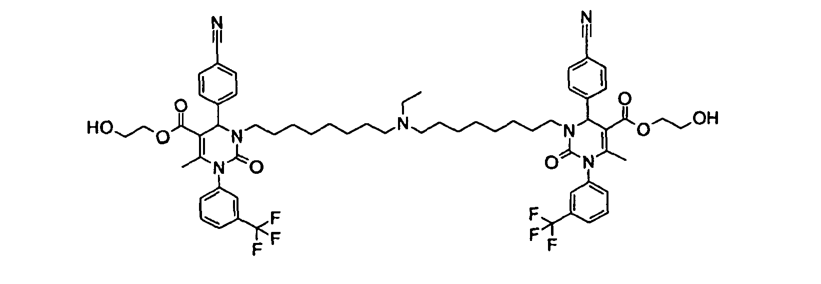

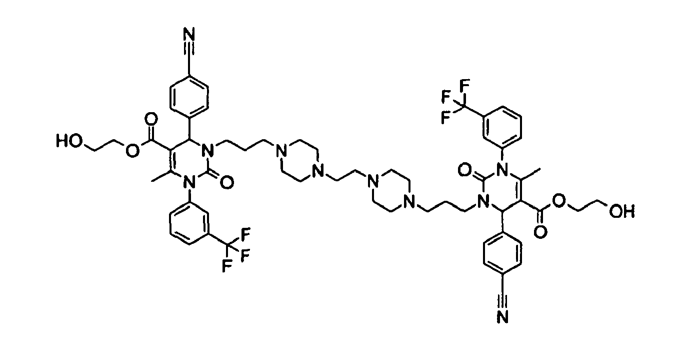

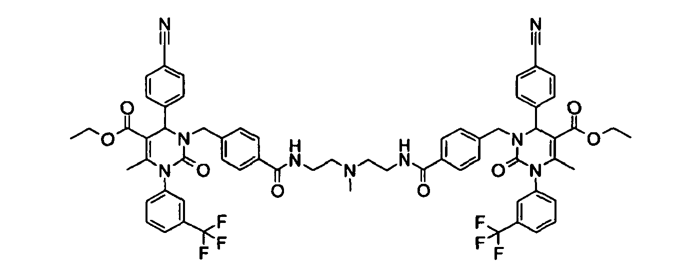

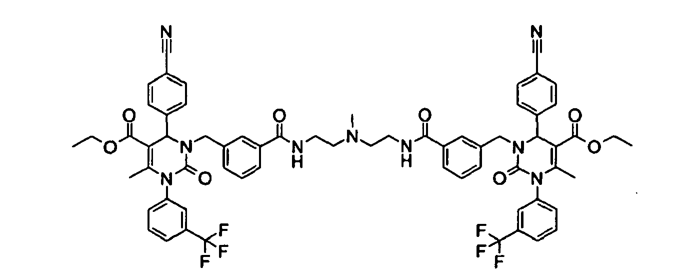

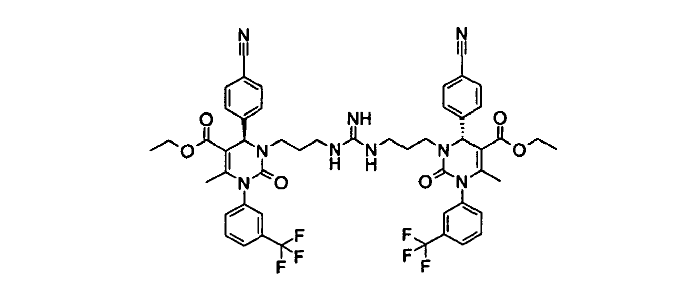

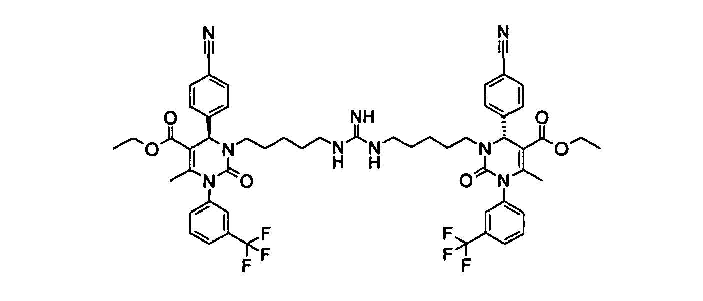

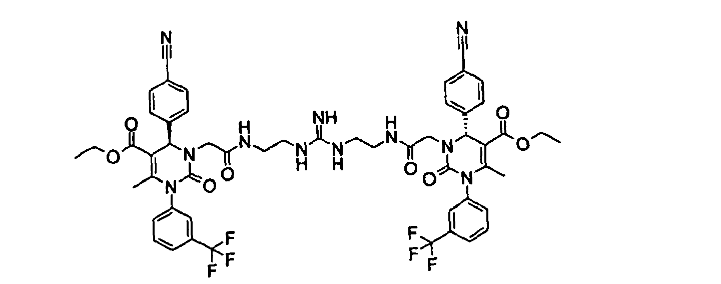

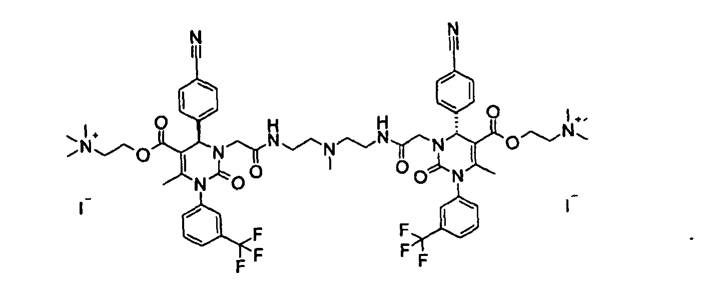

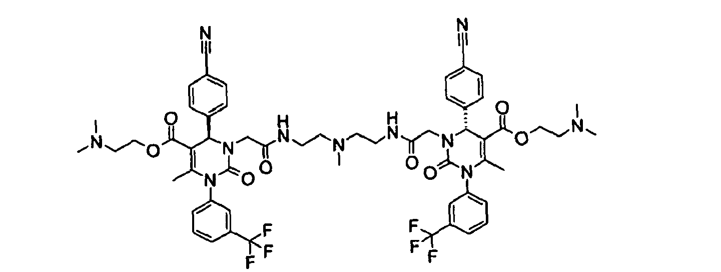

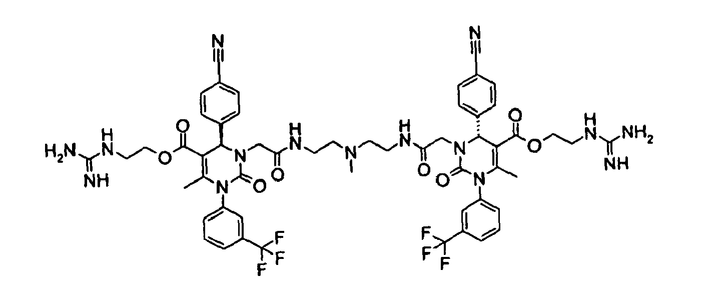

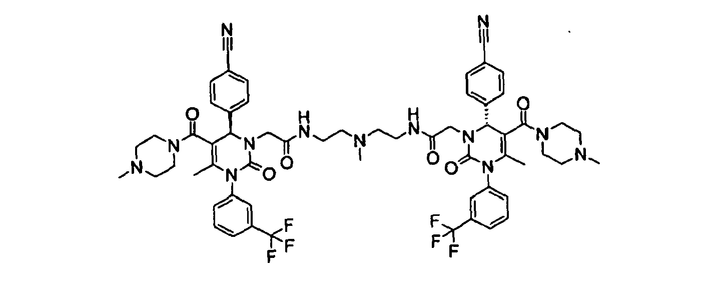

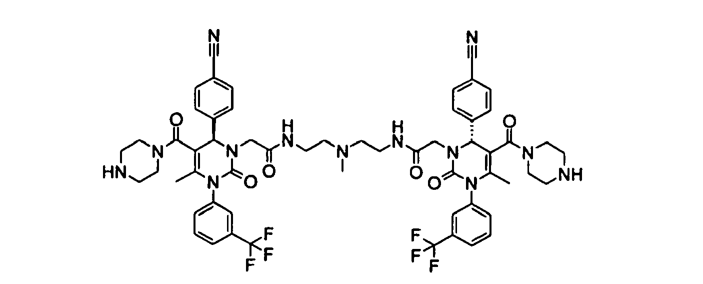

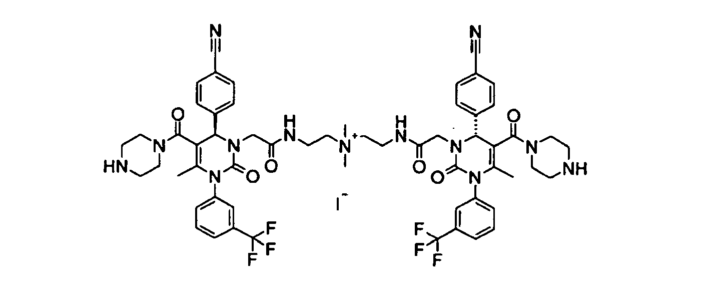

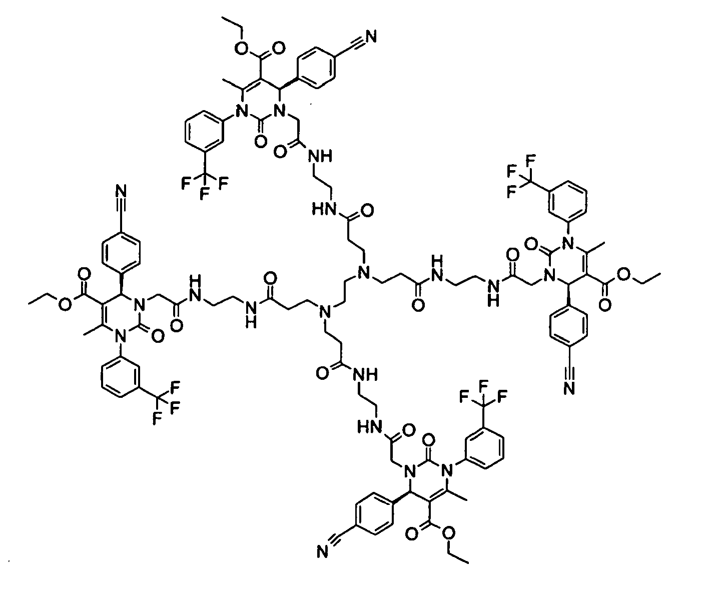

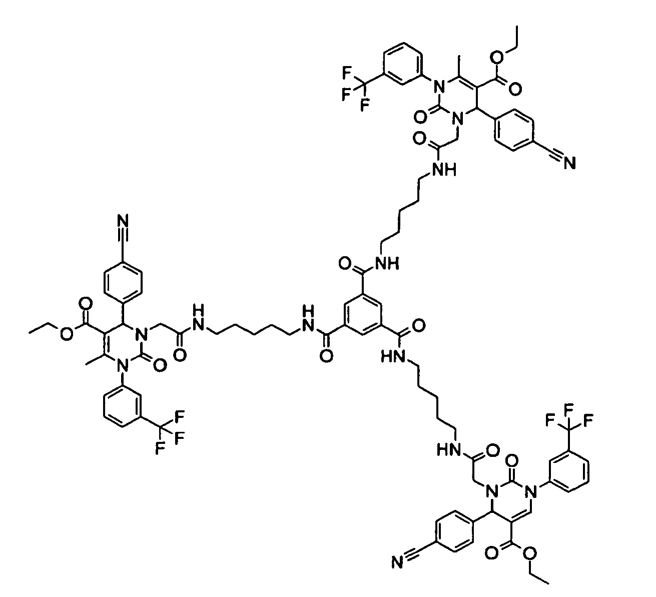

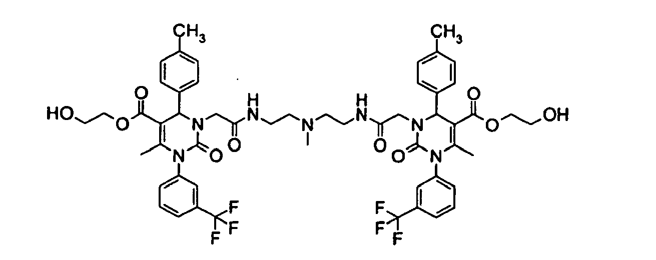

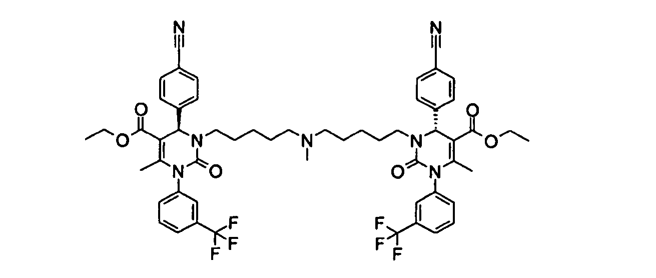

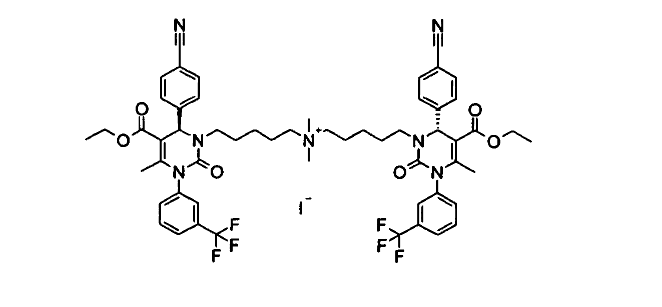

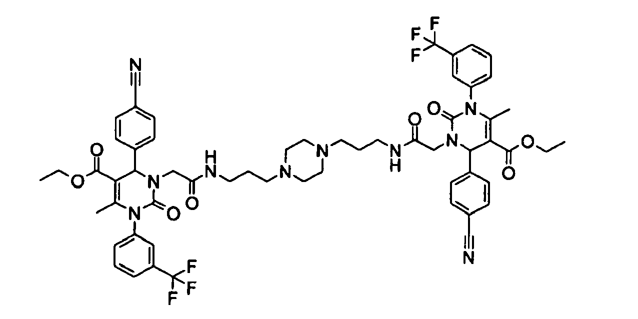

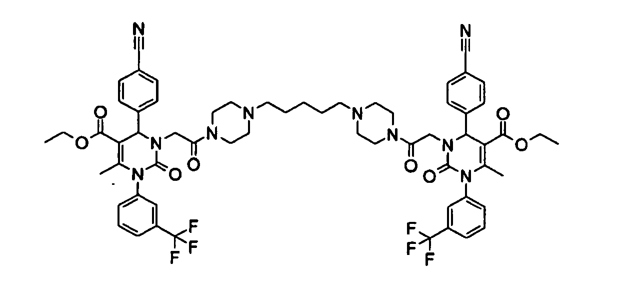

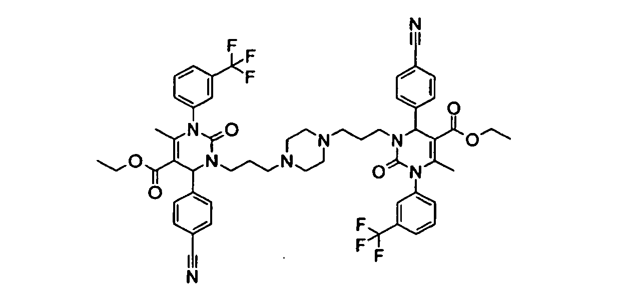

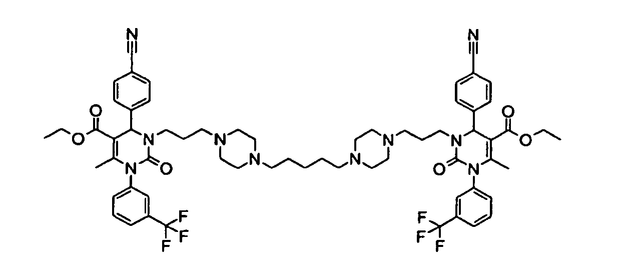

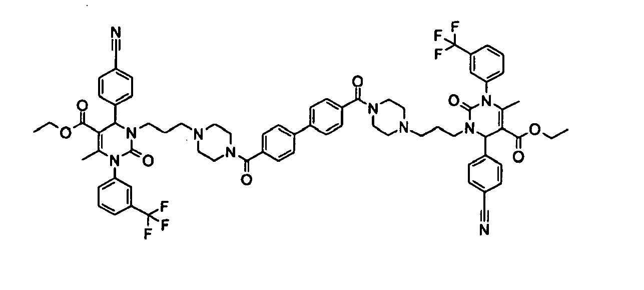

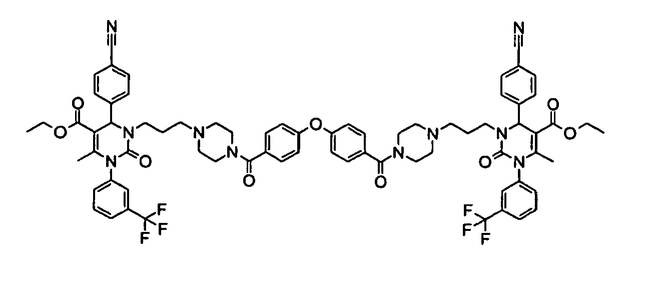

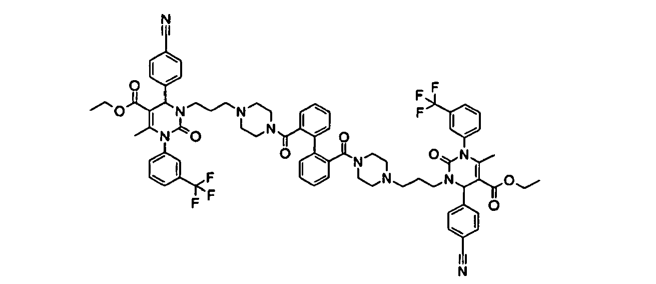

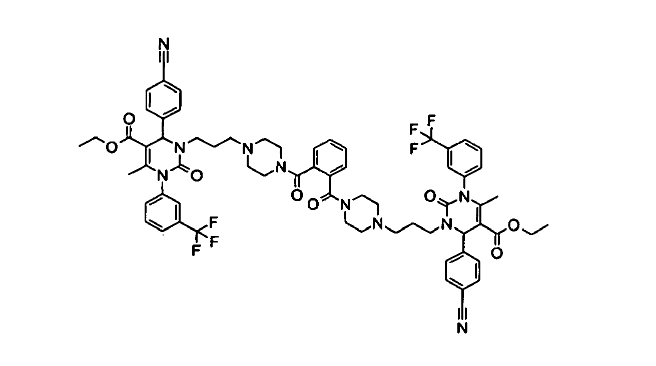

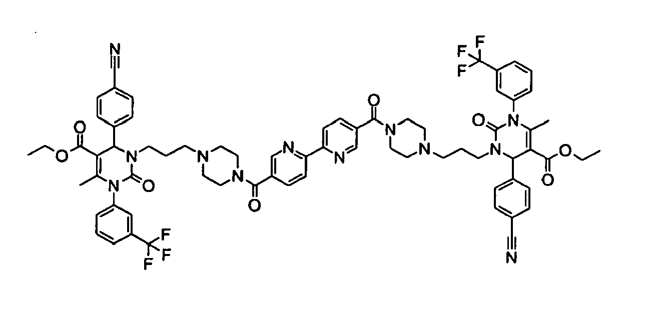

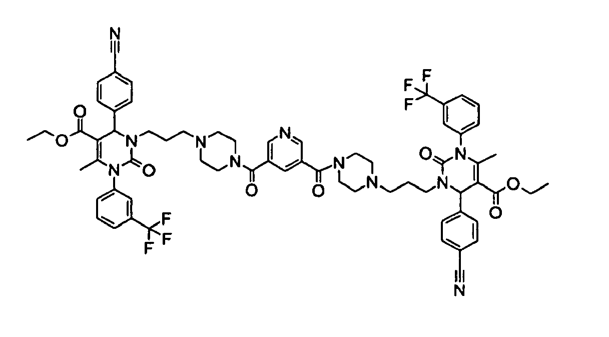

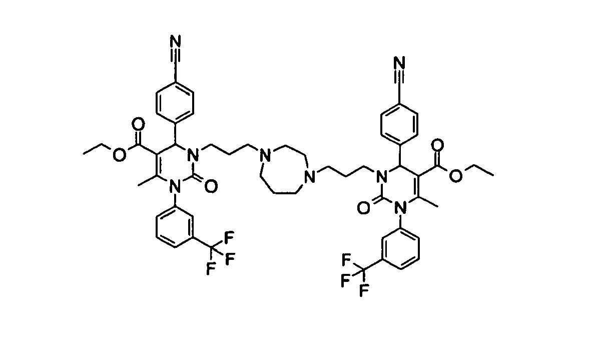

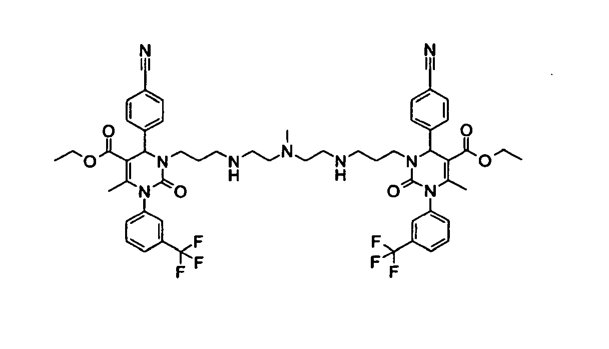

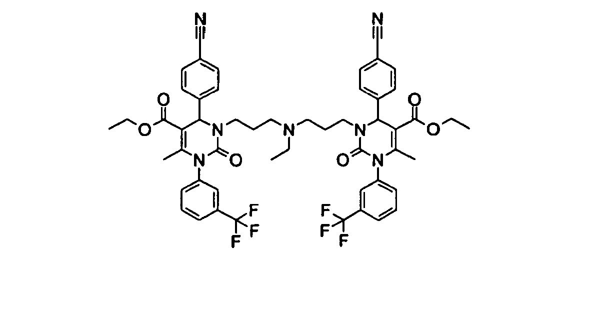

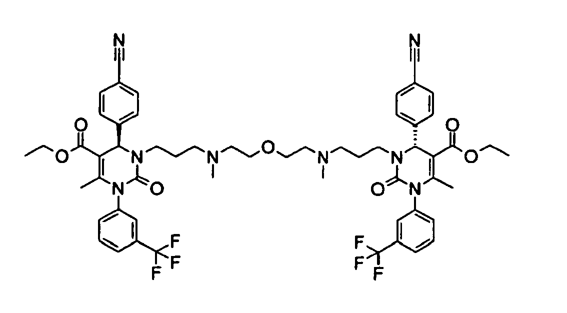

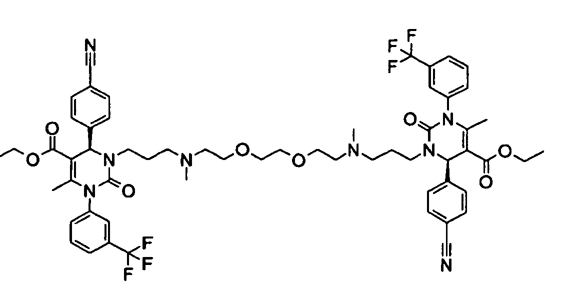

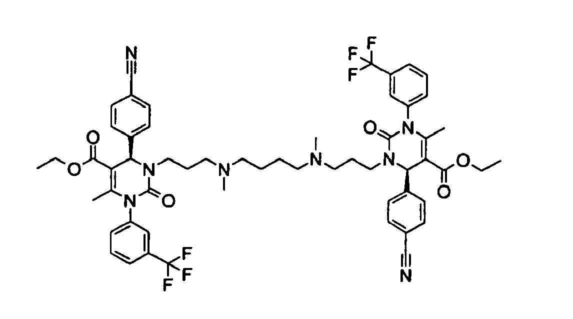

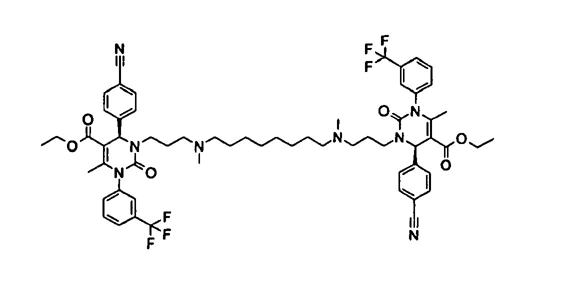

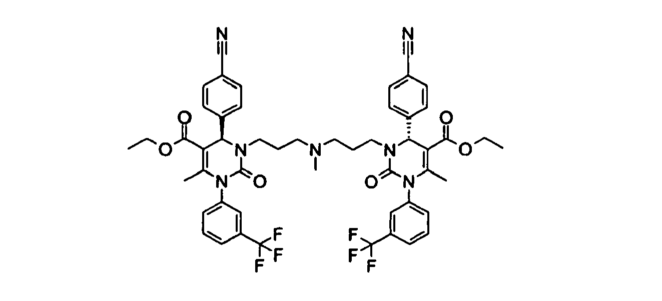

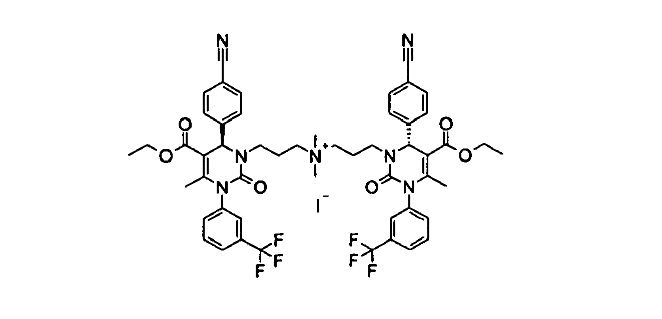

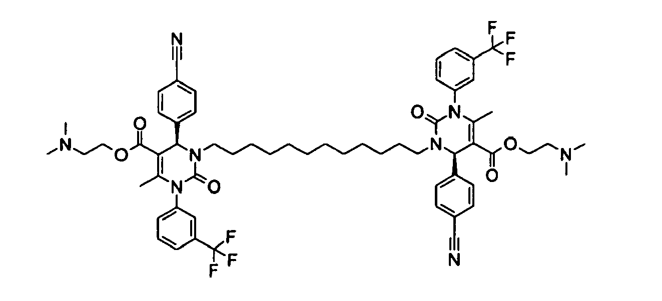

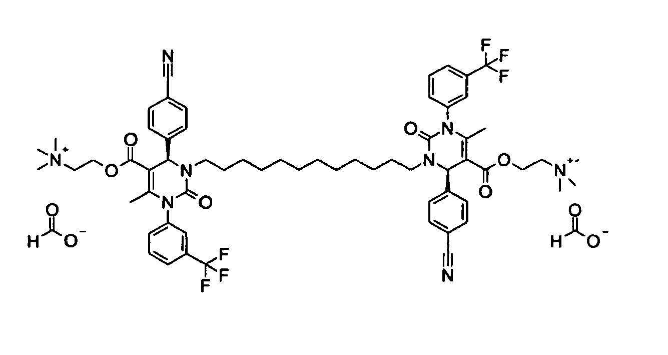

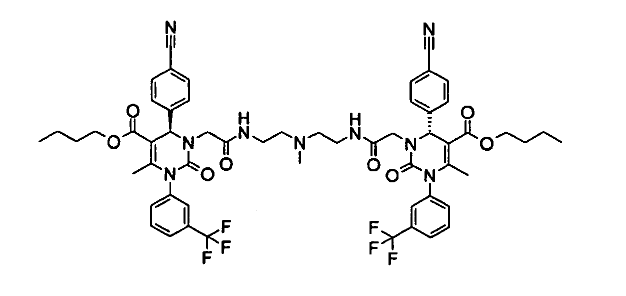

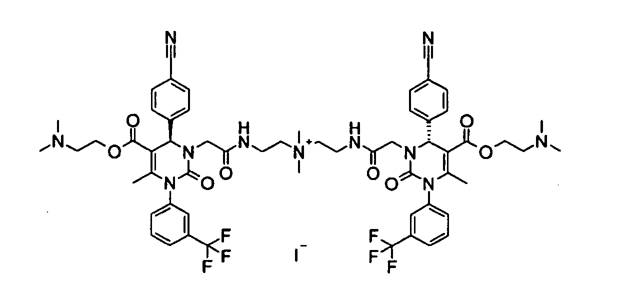

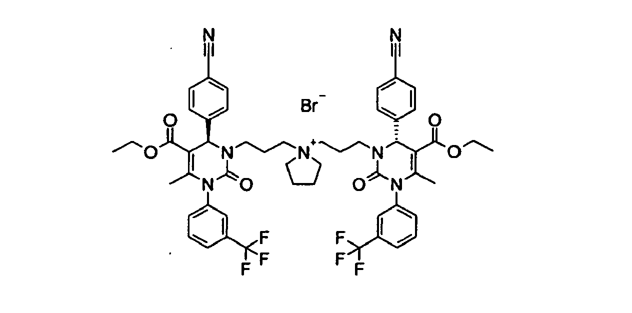

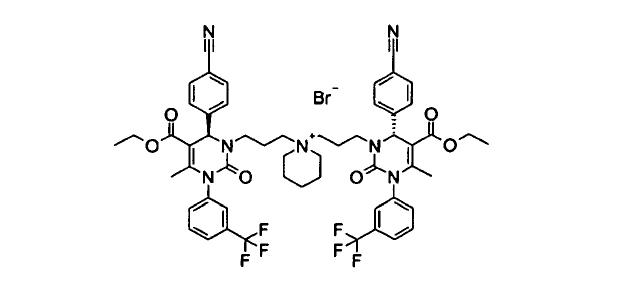

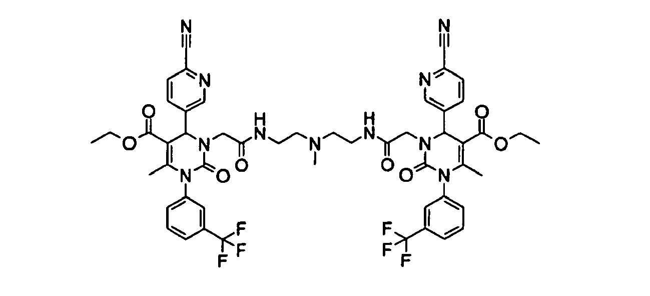

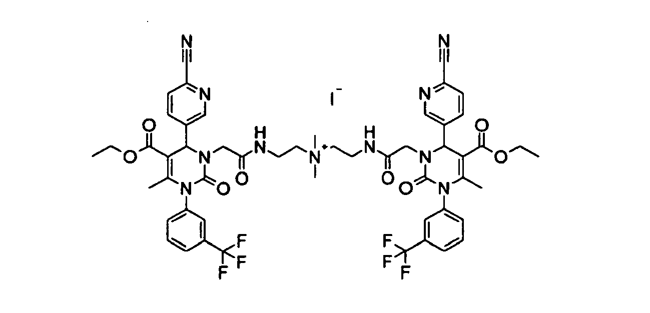

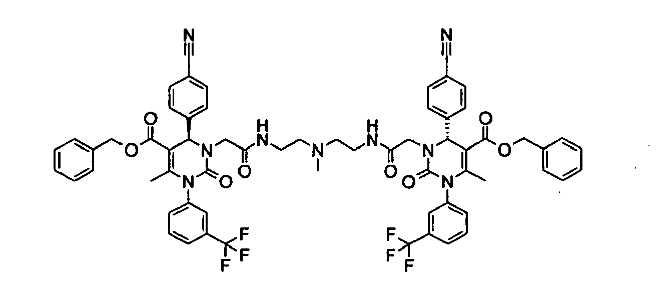

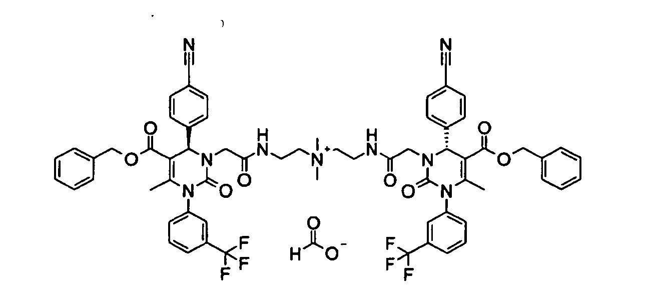

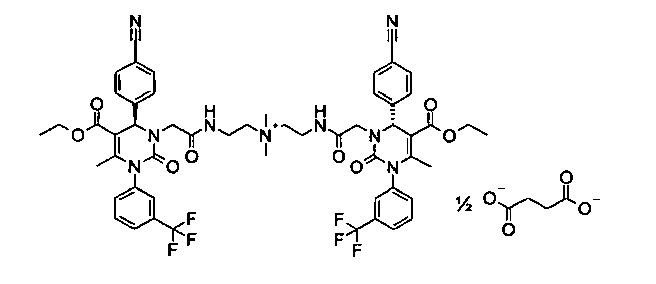

22. A compound according to claim 1, which is any of the first 17 compounds defined in claim 21.

23. A compound according to claim 1, which is any of the 22nd, 24th, 33rd, 35th, 36th, 38th, 41st, 46th, 65th, 69th, 70th, 71st and 76th compounds defined in claim 21.

24. A compound according to any preceding claim, for use in therapy.

25. A pharmaceutical composition comprising a compound of any of claims 1 to 23 and a pharmaceutically acceptable carrier or excipient.

26. Use of a compound according to any of claims 1 to 23, for the manufacture of a medicament for use in therapy of a condition selected from asthma, inflammatory bowel diseases, chronic obstructive pulmonary disease (COPD), chronic bronchitis, lung fibrosis, pneumonia, acute respiratory distress syndrome (ARDS), pulmonary emphysema, smoking-induced emphysema, sarcoidosis, bronchiectasis or cystic fibrosis (CF).

27. Use according to claim 26, wherein the condition is COPD.

28. Use according to claim 26, wherein the condition is CF.

29. Use according to claim 26, wherein the condition is ulcerative colitis or Crohn's disease.

30. Use according to any of claims 26 to 29, wherein the condition is respiratory and the medicament is to be administered via the inhaled route.

## Patentansprüche

1. Verbindung der Formel (I)
M-L-M (I)
wobei L ein Verknüpfer und jedes M unabhängig eine Gruppe der Formel (II) 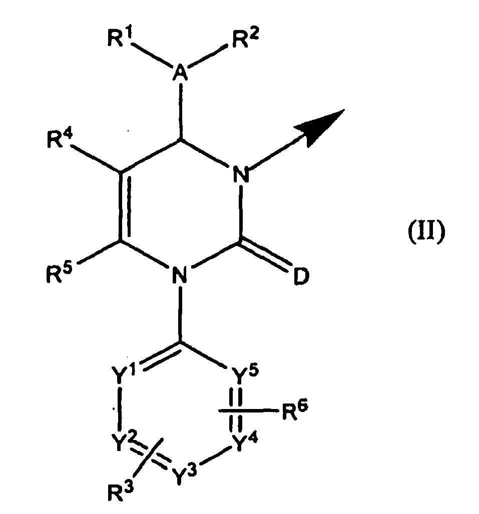 ist, wobei A Aryl oder Heteroaryl ist;
D Sauerstoff oder Schwefel ist;
Y¹, Y², Y³, Y⁴ und Y⁵ unabhängig jeweils CH, CR³, CR⁶ oder N sind, mit der Maßgabe, dass eines davon CR³ ist, eines CR⁶ ist und nicht mehr als zwei N sind;
R¹, R² und R³ unabhängig jeweils Wasserstoff, Halogen, Nitro, Cyano, C₁-C₆-Alkyl, Hydroxy oder C₁-C₆-Alkoxy sind, wobei Alkyl und Alkoxy jeweils wahlweise mit einem bis drei Radikalen substituiert sind, die unabhängig ausgewählt sind unter Halogen, Hydroxy und C₁-C₄-Alkoxy;
R⁴ Trifluormethycarbonyl, C₁-C₆-Alkylcarbonyl, C₁-C₆-Alkoxycarbonyl, C₁-C₆-Alkenoxycarbonyl, Hydroxycarbonyl, Aminocarbonyl, Mono- oder Di-C₁-C₄-Alkylaminocarbonyl, C₆-C₁₀-Arylaminocarbonyl, Phenylcarbonyl, Heteroarylcarbonyl, Heteroaryl oder Cyano ist, wobei Alkylcarbonyl, Alkoxycarbonyl und Alkylaminocarbonyl jeweils wahlweise mit einem bis drei Radikalen substituiert sind, die unabhängig ausgewählt sind unter C₃-C₈-Cycloalkyl, Hydroxyy, C₁-C₄-Alkoxy, C₁-C₄-Alkoxycarbonyl, Hydroxycarbonyl, Aminocarbonyl, Mono- und Di-C₁-C₄-Alkylaminocarbonyl, C₁-C₄-Alkylcarbonylamino, (C₁-C₄-Alkylcarbonyl)-C₁-C₄-Alkylamino, Cyano, Amino, Mono- und Di-C₁-C₄-Alkylamino, Heteroaryl, Heterocyclyl und Tri-(C₁-C₆-alkyl)-silyl und wobei Heteroarylcarbonyl und Heteroaryl noch weiter mit C₁-C₄-Alkyl substituiert sein können; oder R⁴ eine Gruppe der Formel (VIII) 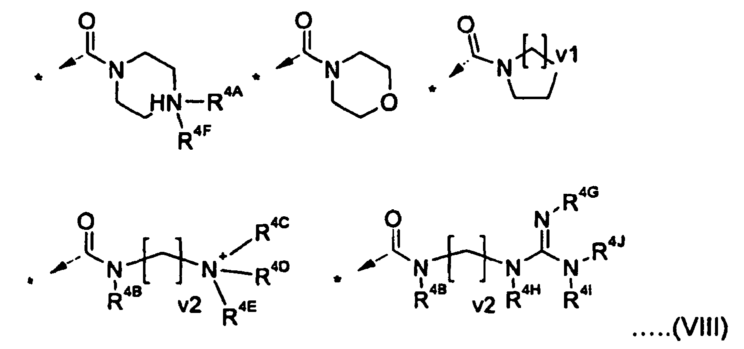 darstellt, wobei
R^{4A} R^{4B}, R^{4G}, R^{4H} R^{4I} und R^{4J} unabhängig Wasserstoff oder Alkyl sind oder R^{4H} und R^{4I} mit dem Stickstoffatom verbunden sein können, an das sie angelagert sind, um einen Ring zu bilden;
R^{4F} ein einziges Paar ist oder R^{4F} Alkyl ist und das Stickstoffatom, an das es angelagert ist, quaternär ist und eine positive Ladung trägt;
R^{4C}, R^{4D} und R^{4E} Alkyl sind oder irgendwelche zwei von R^{4C}, R^{4D} oder R^{4E} mit dem Stickstoffatom verbunden sein können, an das sie angelagert sind, um einen Ring zu bilden, der wahlweise ein weiteres Heteroatom enthält, das unter Sauerstoff und Stickstoff ausgewählt ist;
v1 1 - 3 beträgt;
v2 1 - 6 beträgt;
R⁵ C₁-C₄-Alkyl oder Amino ist und Alkyl wahlweise mit einem bis drei Radikalen substituiert ist, die unabhängig ausgewählt sind unter Halogen, Hydroxy, C₁-C₆-Alkoxy, C₁-C₆-Alkenoxy, C₁-C₆-Alkylthio, Amino, Mono- und Di-C₁-C₆-Alkylamino, Phenylamino, Hydroxycarbonyl, C₁-C₆-Alkoxycarbonyl und dem Radikal -O-( C₁-C₄-Alkyl)-O-( C₁-C₄-Alkyl); und
R⁶ Halogen, Nitro, Cyano, C₁-C₆-Alkyl, Hydroxy oder C₁-C₆-Alkoxy ist, wobei Alkyl und Alkoxy wahlweise mit einem bis drei Radikalen substituiert sind, die unabhängig ausgewählt sind unter Halogen, Hydroxy und C₁-C₄-Alkoxy;
wobei Heteroaryl unter Benzimidazolyl, Benzoxazolyl, Benzothiazolyl, Benzofuranyl, Benzothienyl, Furyl, Imidazolyl, Indolyl, Indolizinyl, Isoxazolyl, Isochinolyl, Isothiazolyl, Oxazolyl, Oxadiazolyl, Pyrazinyl, Pyridazinyl, Pyrazolyl, Pyridyl, Pyrimidinyl, Pyrrolyl, Chinazolyl, Chinolinyl, Tetrazolyl, 1,3,4-Thiadiazolyl, Thiazolyl, Thienyl und Triazolyl ausgewählt wird;
oder pharmazeutisch akzeptables Salz, Solvat oder N-Oxid derselben.

2. Verbindung nach Anspruch 1, wobei A Phenyl ist.

3. Verbindung nach Anspruch 1 oder Anspruch 2, wobei R¹ Wasserstoff ist.

4. Verbindung nach einem der vorhergehenden Ansprüche, wobei R² Methyl oder -CN ist.

5. Verbindung nach einem der vorhergehenden Ansprüche, wobei R³ Wasserstoff ist.

6. Verbindung nach einem der vorhergehenden Ansprüche, wobei R⁴ -C(O)OCH₂CH₃ oder -C(O)OCH₂CH₂OH ist.

7. Verbindung nach einem der vorhergehenden Ansprüche, wobei R⁵ Methyl ist.

8. Verbindung nach einem der vorhergehenden Ansprüche, wobei R⁶ Trifluormethyl ist.

9. Verbindung nach einem der vorhergehenden Ansprüche, wobei keines von Y¹, Y², Y³, Y⁴ und Y⁵ N ist.

10. Verbindung nach einem der vorhergehenden Ansprüche, wobei D Sauerstoff ist.

11. Verbindung nach einem der vorhergehenden Ansprüche, wobei L eine Gruppe der Formel (III)
-L^{a}-R⁷-L^{b}-W-L^{b}-R⁷-L^{a}- (III)
ist, wobei L^{a} eine Bindung oder -C(O)- ist;
L^{b} eine Bindung oder -C(O)- ist;
R⁷ Alkylen oder Cycloalkylen ist;
W eine Bindung, N(R⁹⁸)(R^{9C}) oder unter folgenden zweiwertigen Radikalen ausgewählt ist:
-(O-R^{8A})ₘ₁-O-
-N(R^{9A})-(O-R^{8A})ₘ₁-R^{8A}-N(R^{9A})-
-N(R^{9A})-R^{8B}-N(R^{9B})(R^{9C})-R^{8B}-N(R^{9A})-
-N(R^{9A})-R^{8B}-N(R^{10B})C(=NR^{10A})(NR^{10C})-R^{8B}-N(R^{9A})-
-N(R^{9A})-R^{8B}-N(R^{9A})- 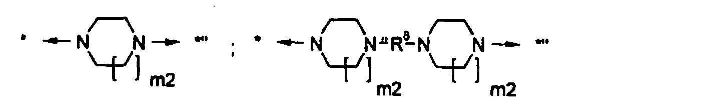 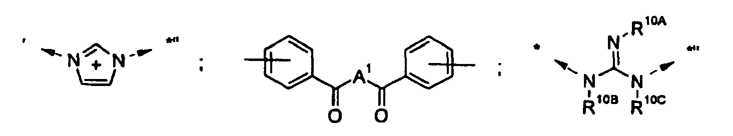
wobei
m1 1 - 4 beträgt;
R^{8A} Alkylen oder Cycloalkylen ist;
R^{8B} eine Alkylen- oder Cycloalkylengruppe oder eine Gruppe der Formel A² ist;
R^{9A} Wasserstoff oder niederes Alkyl ist;
eines von R^{9B} oder R^{9C} ein einziges Paar ist und das andere Wasserstoff oder C₁₋₁₂-Alkyl ist oder R^{9B} und R^{9C} beide C₁₋₁₂-Alkyl sind, in welchem Falle der Stickstoff, an den sie angelagert sind quaternär ist und eine positive Ladung trägt oder NR^{9B}R^{9C} ein Ring ist;
R^{10A} Wasserstoff oder C₁₋₁₂-Alkyl ist;
R^{10B} und R^{10C} unabhängig Wasserstoff oder C₁₋₁₂-Alkyl sind oder R^{10B} und R^{10C} mit einander verbunden sind unter Bildung eines Rings;
m2 1 - 3 beträgt;
A¹ ausgewählt ist unter -N(R^{9A})-R⁸-N(R^{9B})(R^{9C})-R⁸-N(R^{9A})- und
-N(R^{9A})-R⁸-N(R^{10B})C(=NR^{10A})(NR^{10c})-R⁸-N(R^{9A})-; und
A² ausgewählt ist unter 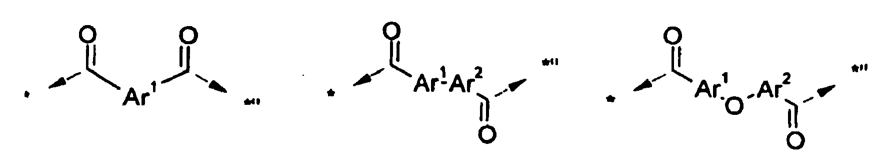 wobei Ar¹ und Ar² jeweils unabhängig Phenyl oder Heteroaryl, wie in Anspruch 1 definiert, sind.

12. Verbindung nach Anspruch 11, wobei jedes L^{a} unabhängig -C(O)- oder eine kovalente Bindung ist.

13. Verbindung nach Anspruch 12, wobei L^{a} eine Bindung ist.

14. Verbindung nach den Ansprüchen 11 bis 13, wobei R⁷ Alkylen ist.

15. Verbindung nach einem der Ansprüche 11 bis 14, wobei W N(R^{9A})-R^{8B}-N(R^{9B})(R^{9C})-R^{8B}-N(R^{9A})- ist.

16. Verbindung nach einem der Ansprüche 11 bis 14, wobei W N(R^{9A})-R^{8B}-N(R^{10B})C(=NR^{10A})(NR^{10C})-R^{8B}-N(R^{9A}) ist.

17. Verbindung nach einem der Ansprüche 11 bis 14, wobei W 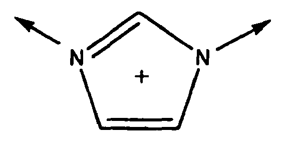 ist.

18. Verbindung nach einem der Ansprüche 11 bis 14, wobei W 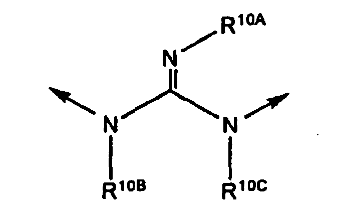 ist.

19. Verbindung nach einem der Ansprüche 11 bis 14, wobei W -N(R^{9B})(-R^{9C})- ist.

20. Verbindung nach einem der vorhergehenden Ansprüche, wobei A Phenyl oder Heteroaryl ist; und
R⁴ Trifluormethycarbonyl, C₁-C₆-Alkylcarbonyl, C₁-C₆-Alkoxycarbonyl, C₁-C₆-Alkenoxycarbonyl, Hydroxycarbonyl, Aminocarbonyl, Mono- oder Di-C₁-C₄-Alkylaminocarbonyl, C₆-C₁₀-Arylaminocarbonyl, Phenylcarbonyl, Heteroarylcarbonyl, Heteroaryl oder Cyano ist, wobei C₁-C₆-Alkylcarbonyl, C₁-C₆-Alkoxycarbonyl, Mono- und Di-C₁-C₄-Alkylaminocarbonyl noch weiter mit einem bis drei identischen oder verschiedenen Radikalen substituiert sein können, die aus der Gruppe ausgewählt sind bestehend aus C₃-C₈-Cycloalkyl, Hydroxyy, C₁-C₄-Alkoxy, C₁-C₄-Alkoxycarbonyl, Hydroxycarbonyl, Aminocarbonyl, Mono- und Di-C₁-C₄-Alkylaminocarbonyl, C₁-C₄- Alkylcarbonylamino, (C₁-C₄-Alkylcarbonyl)-C₁-C₄-Alkylamino, Cyano, Amino, Mono- und Di-C₁-C₄-Alkylamino, Heteroaryl, Heterocyclyl und Tri(C₁-C₆-alkyl)-silyl und wobei Heteroarylcarbonyl und Heteroaryl noch weiter mit C₁-C₄-Alkyl substituiert sein können.

21. Verbindung nach Anspruch 1, das irgendeines von 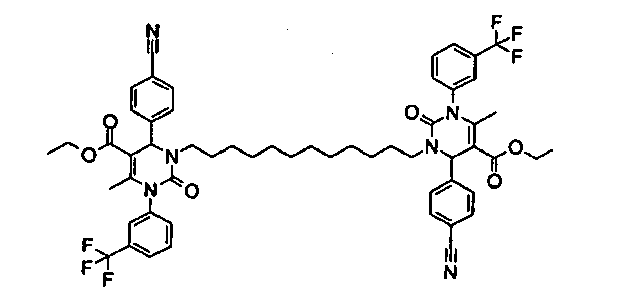 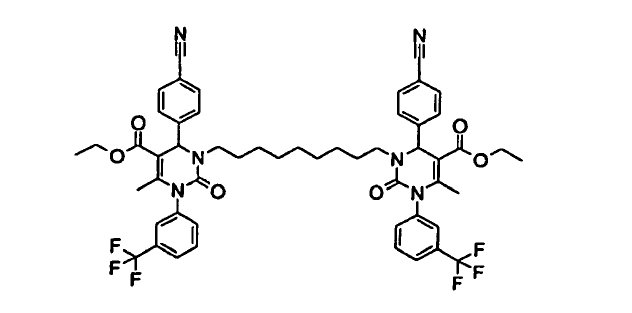 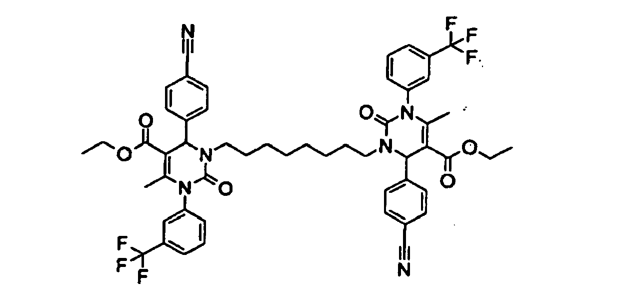 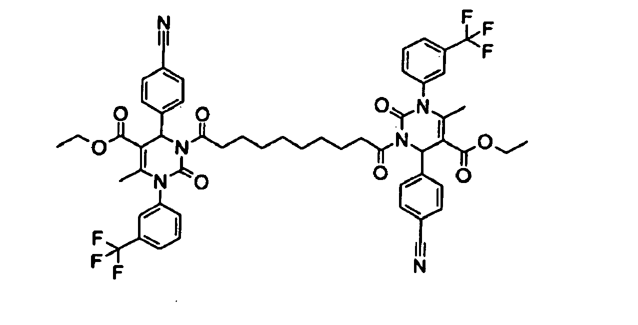 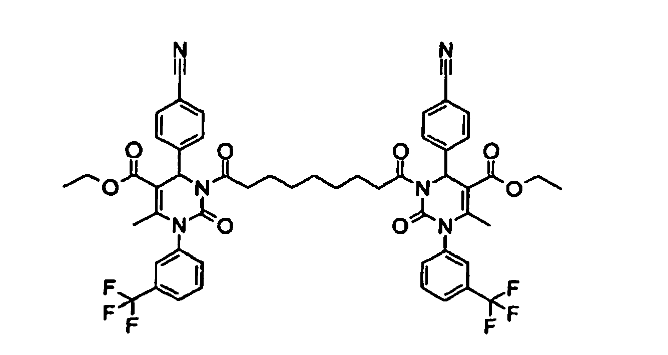 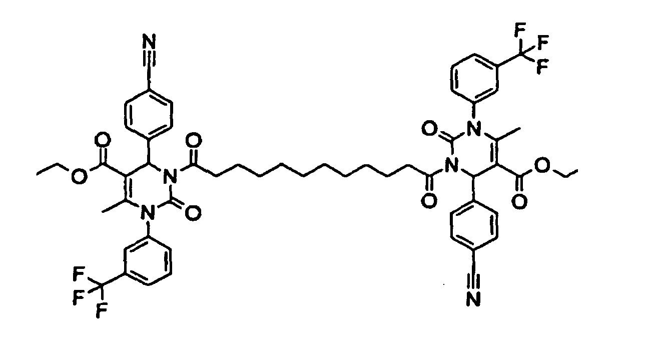 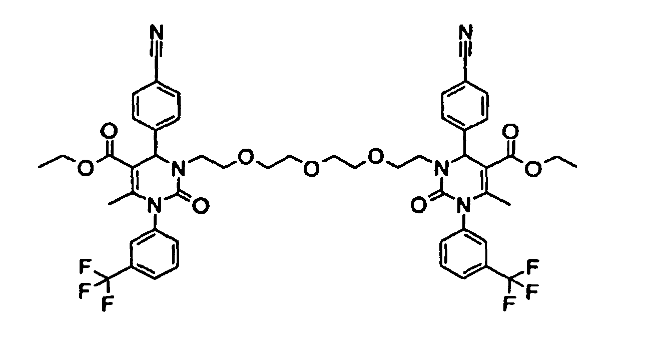 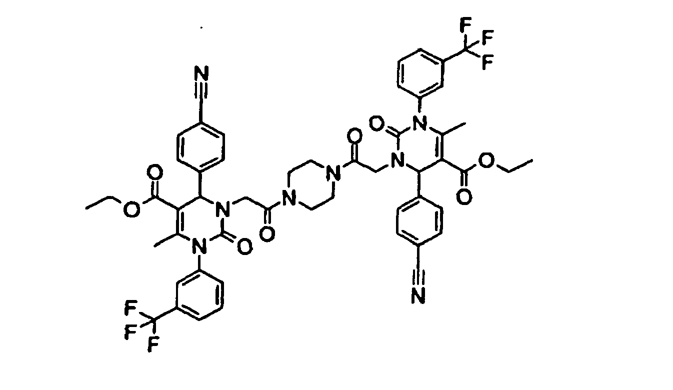 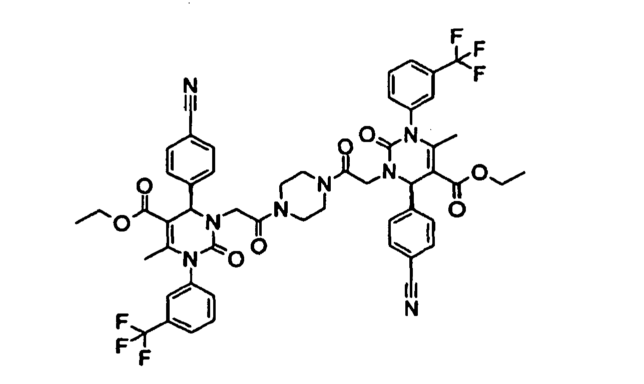 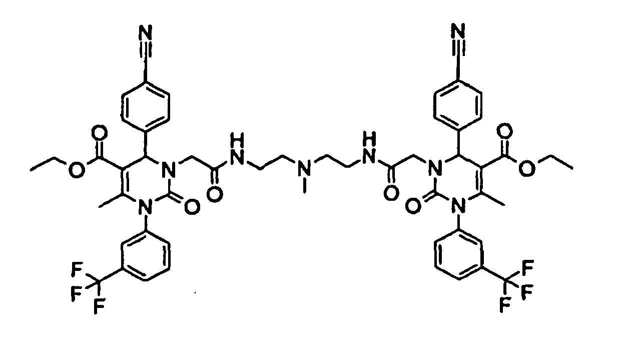 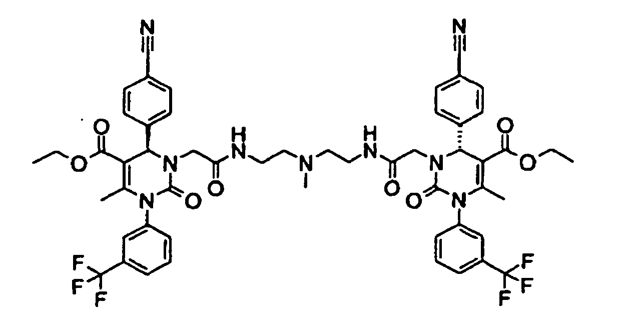 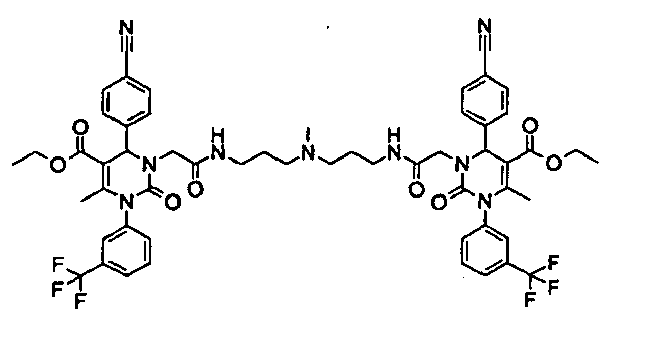 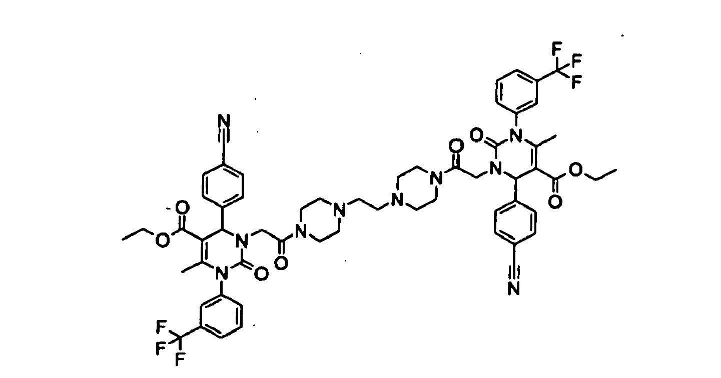 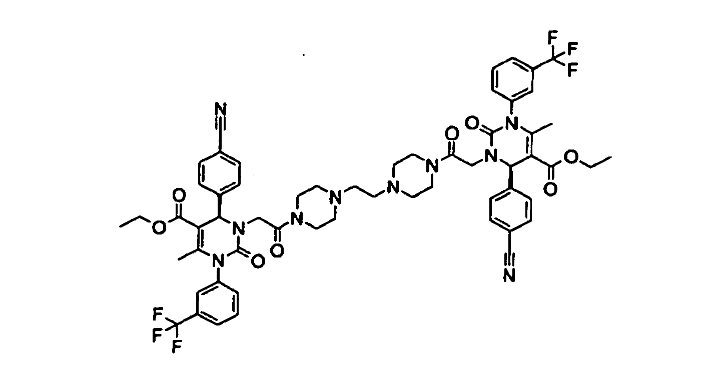 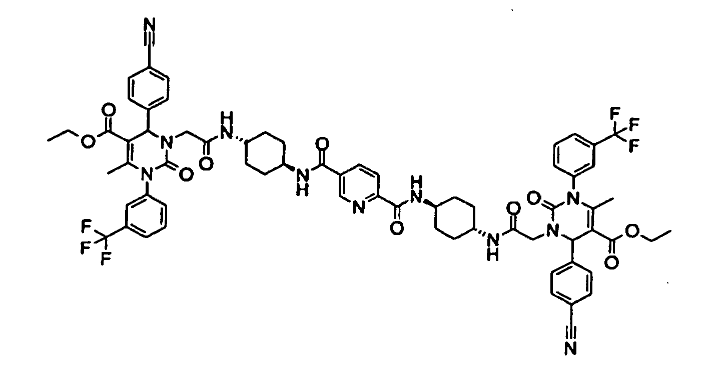 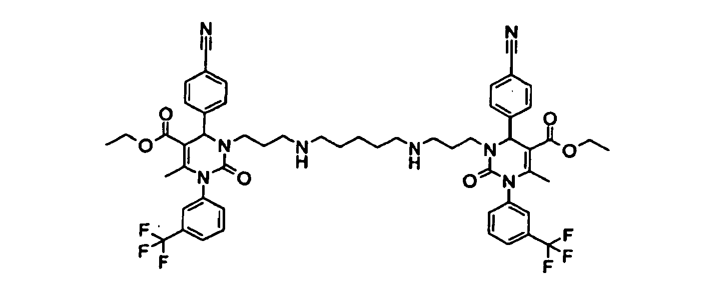 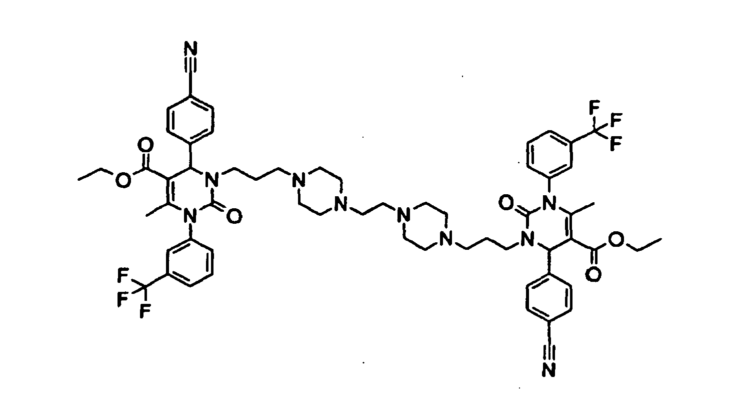 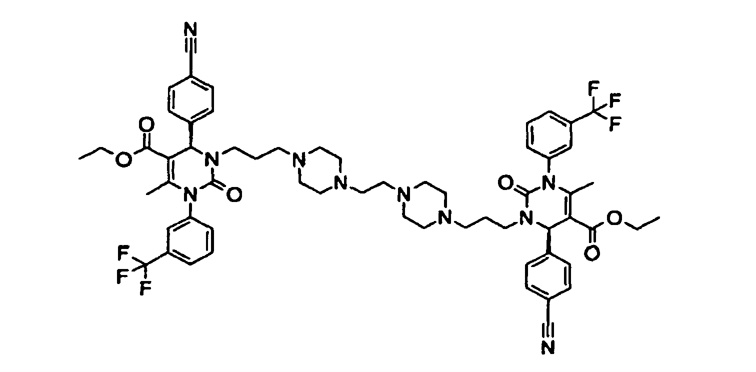 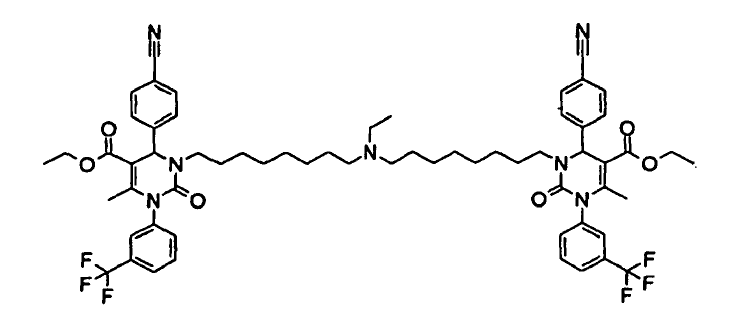 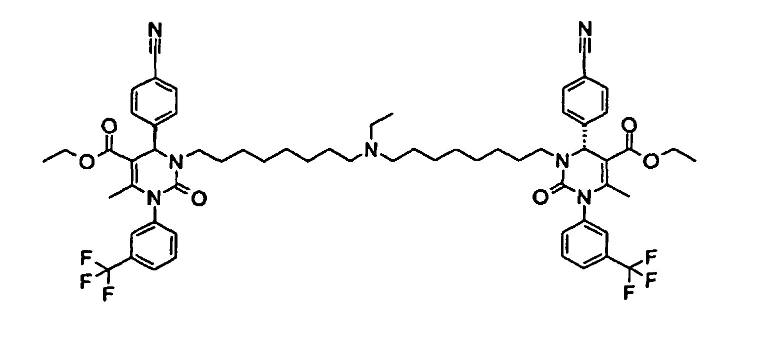 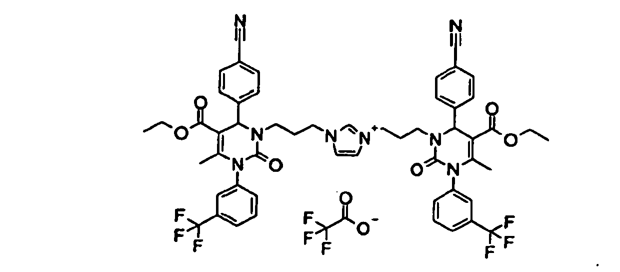 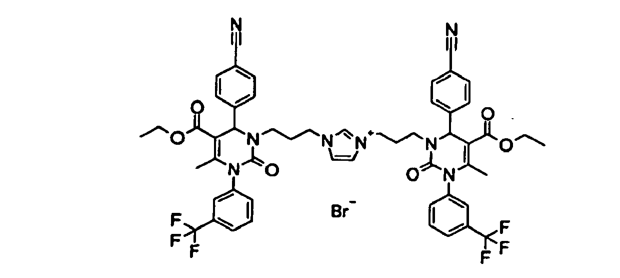 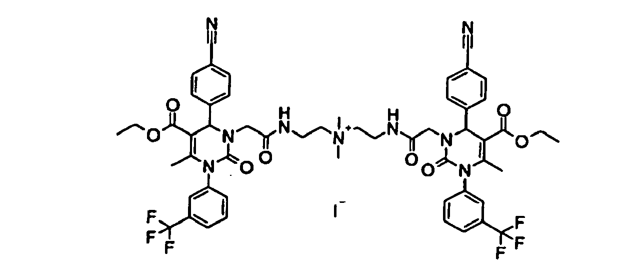 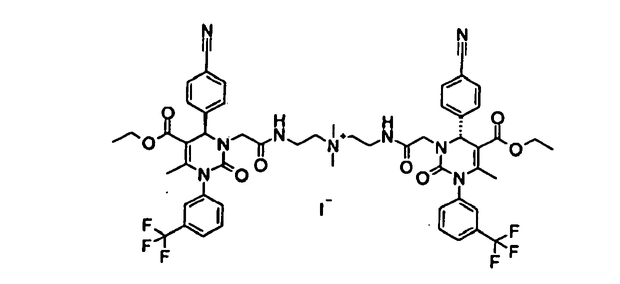 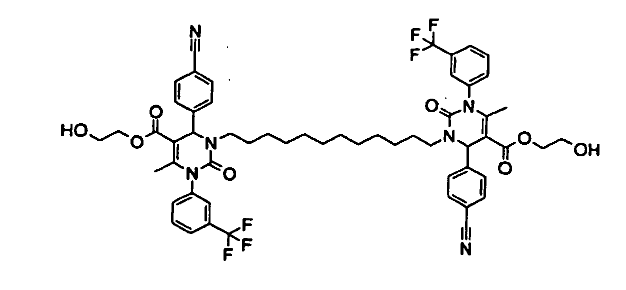 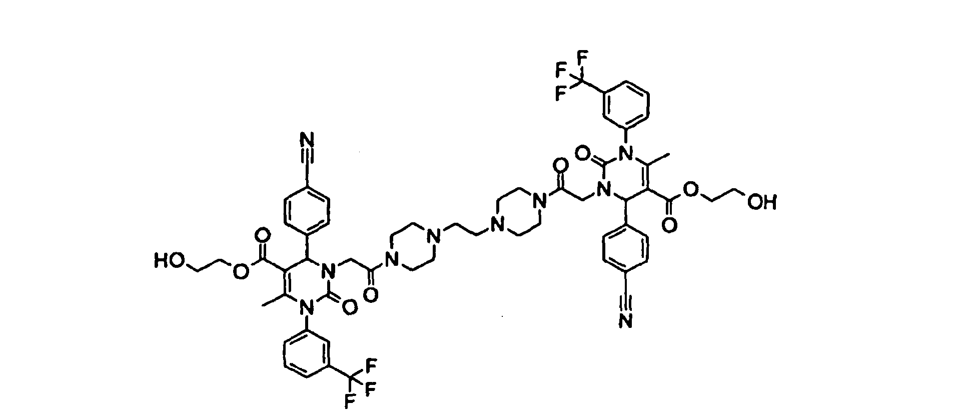 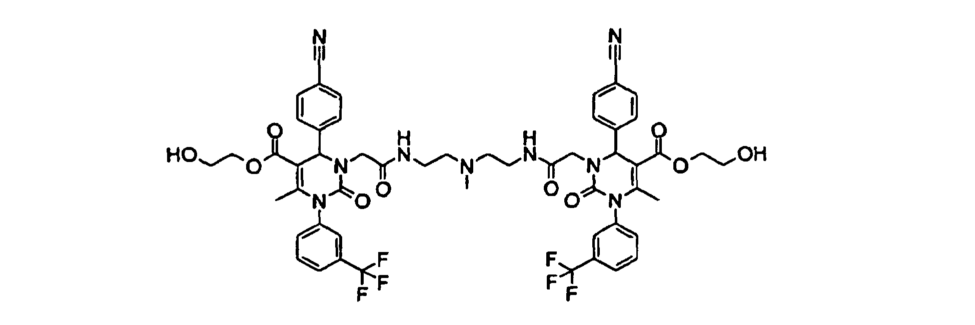 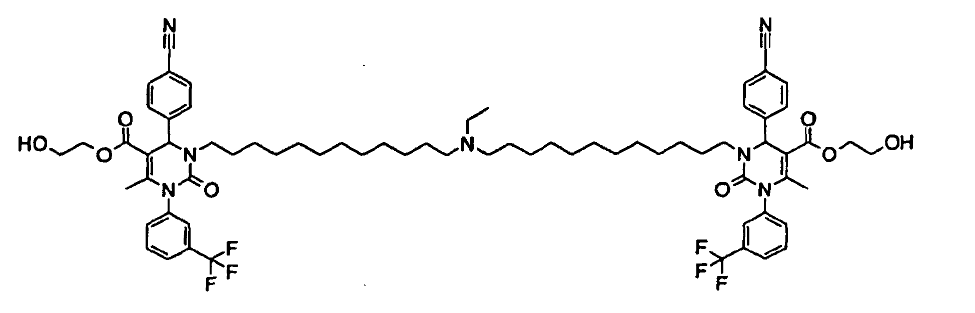 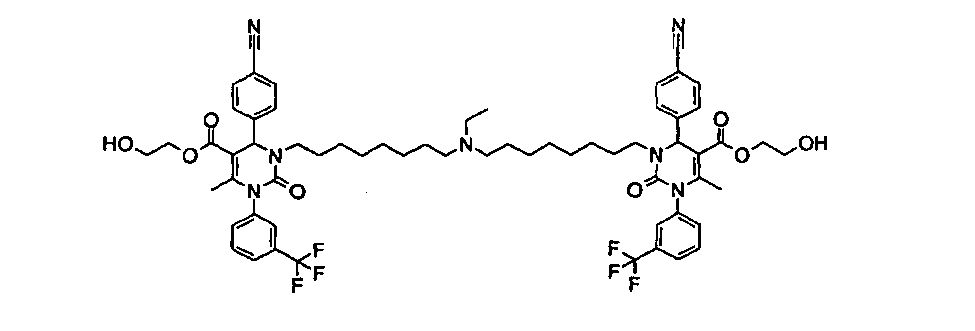 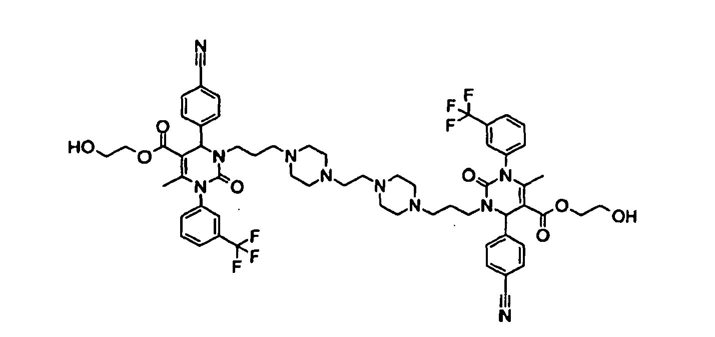 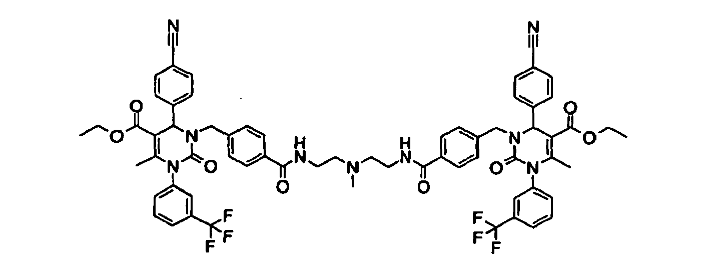 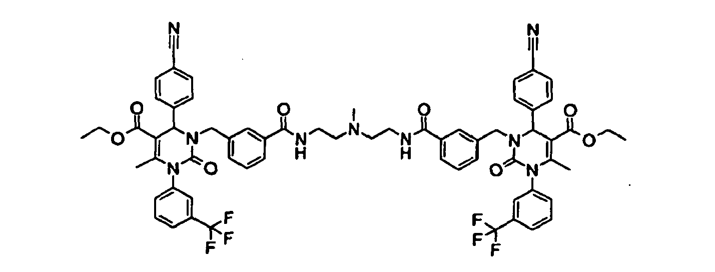 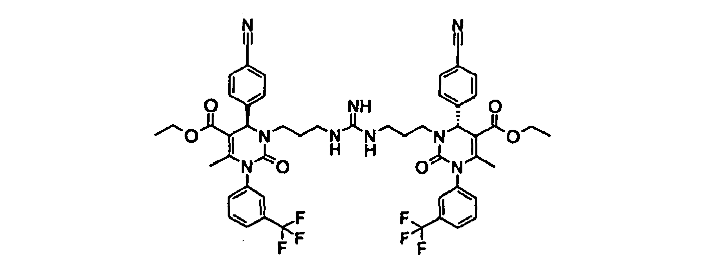 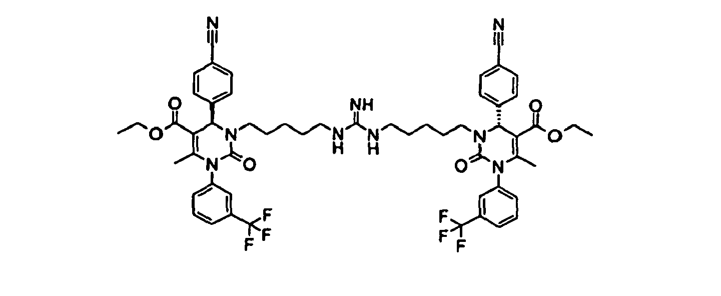 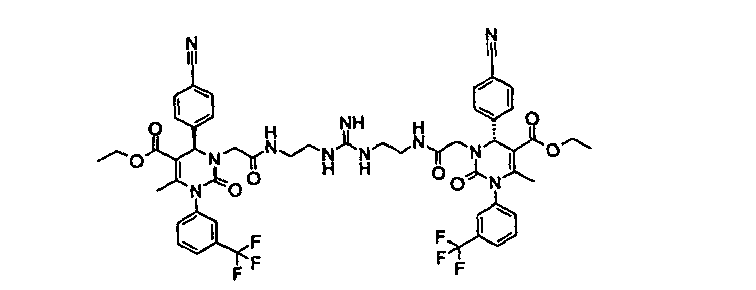 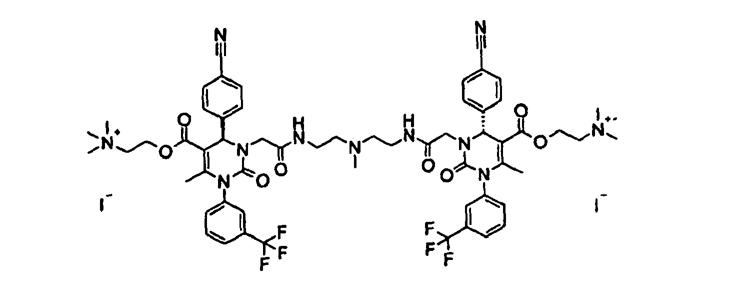 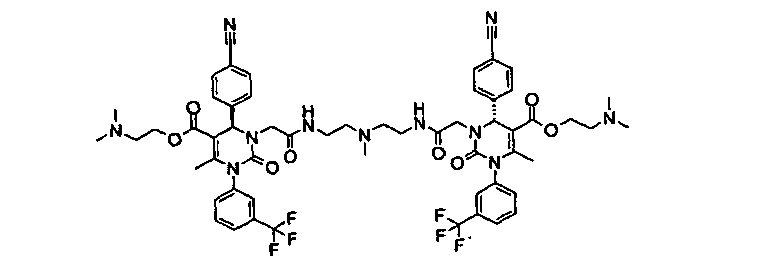 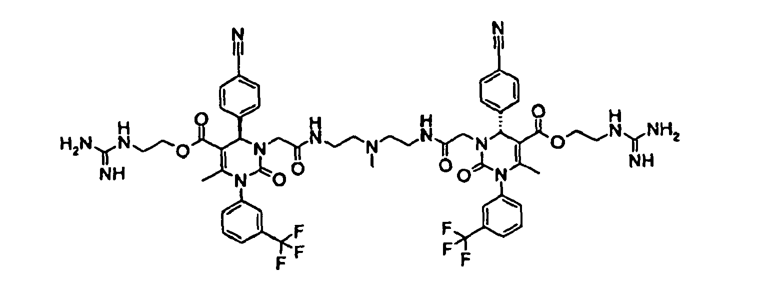 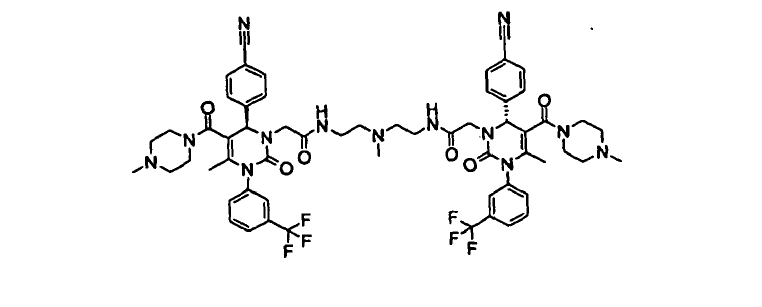 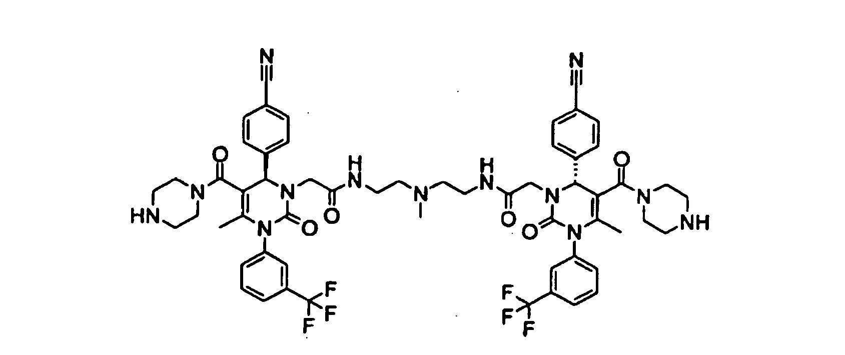 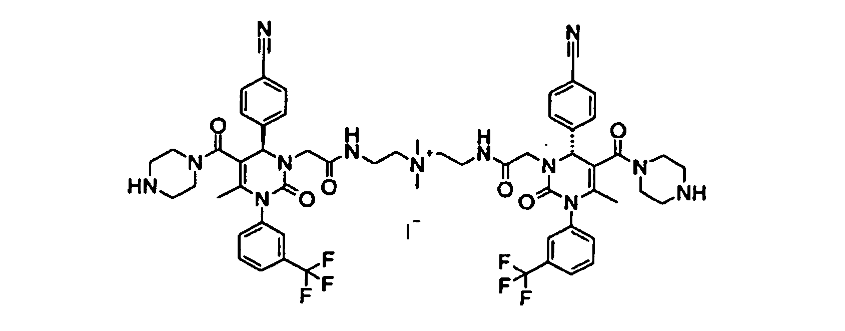 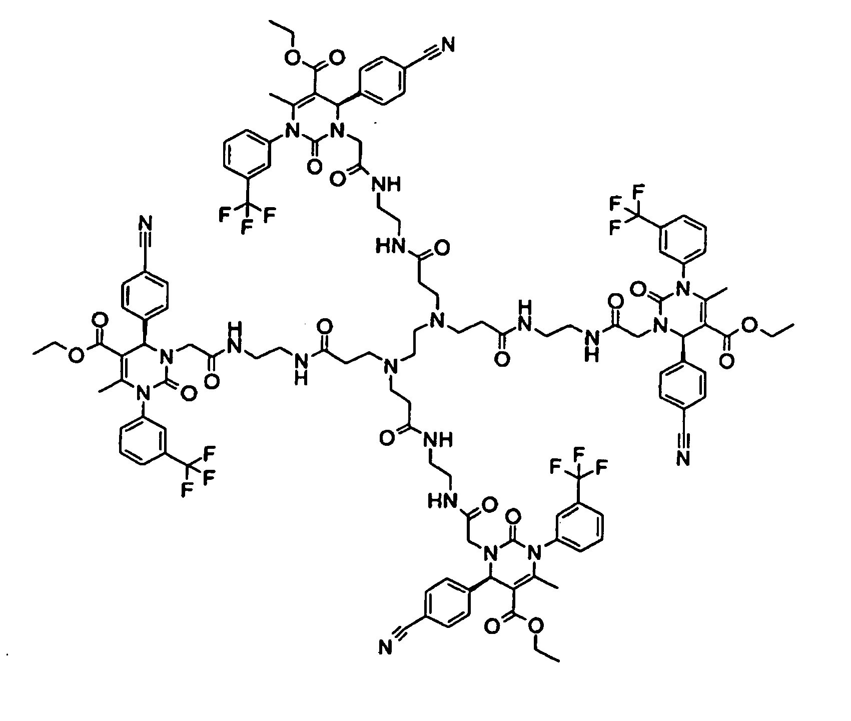 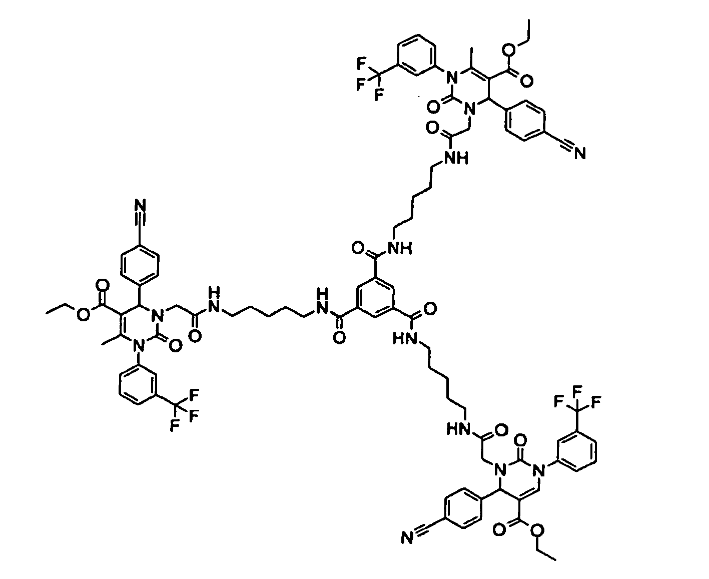 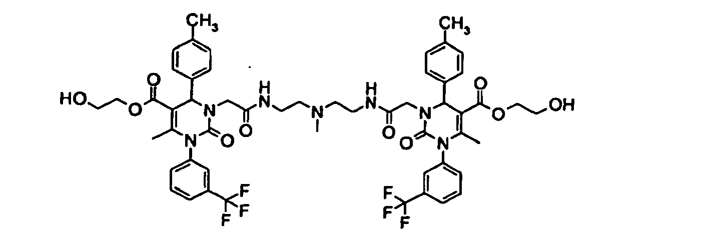 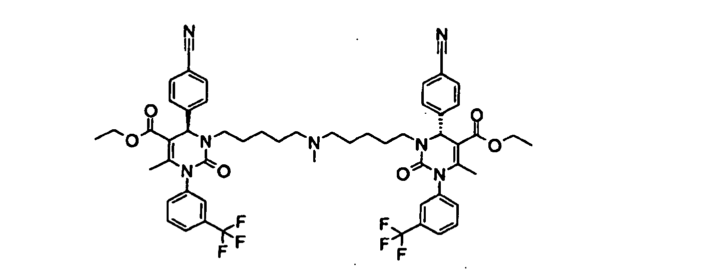 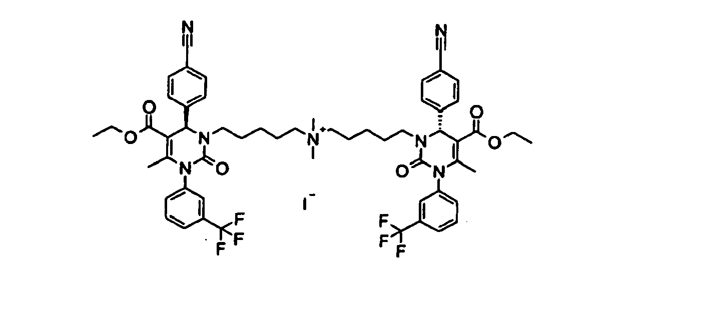 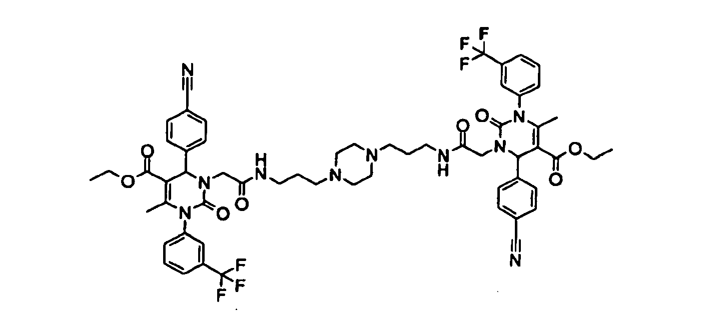 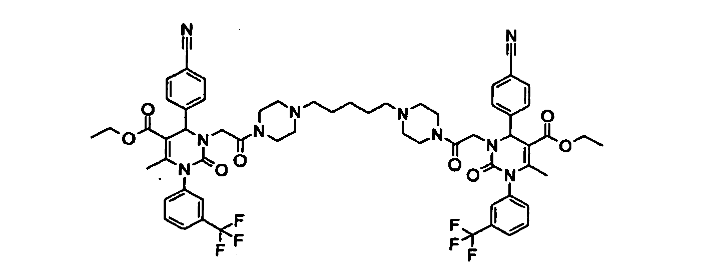 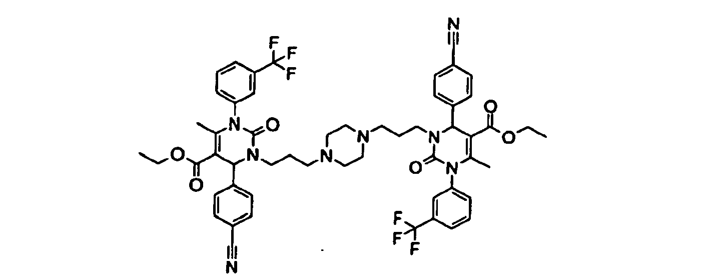 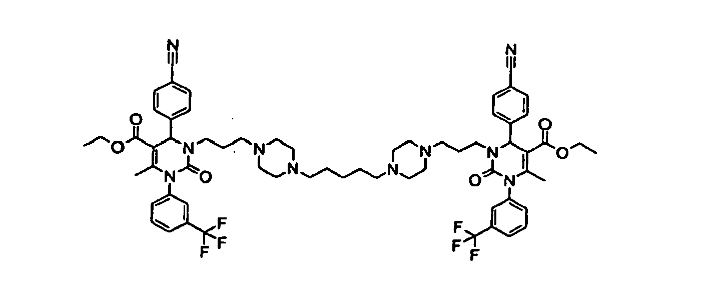 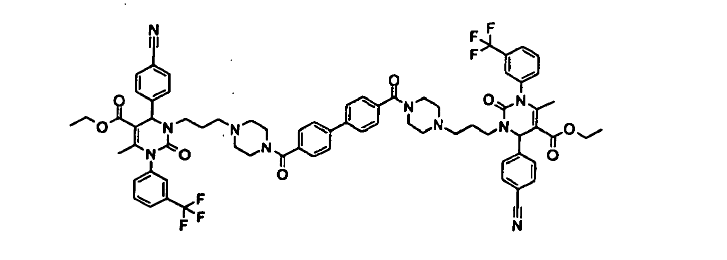 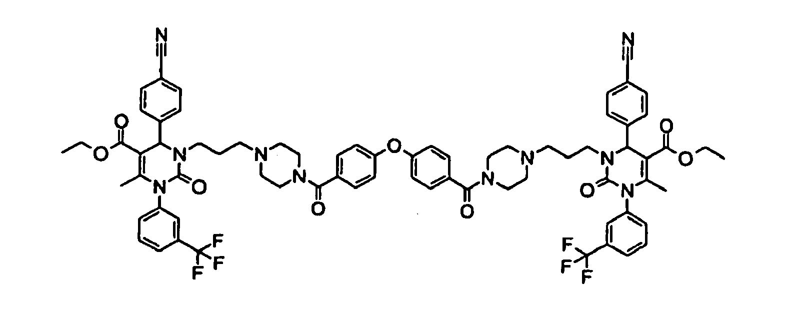 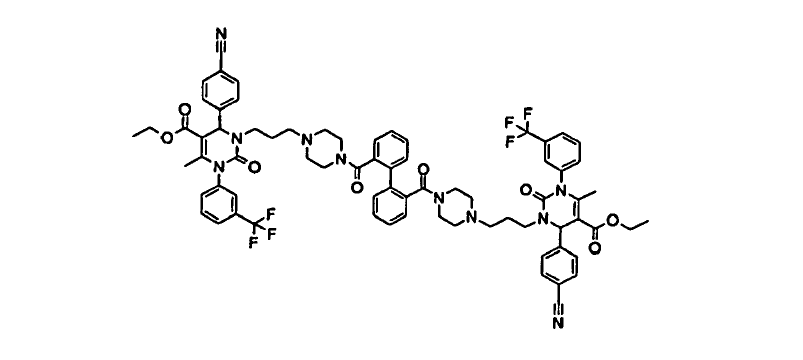 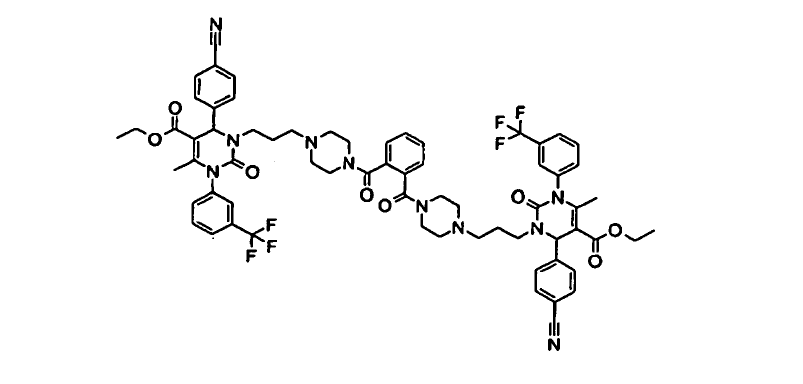 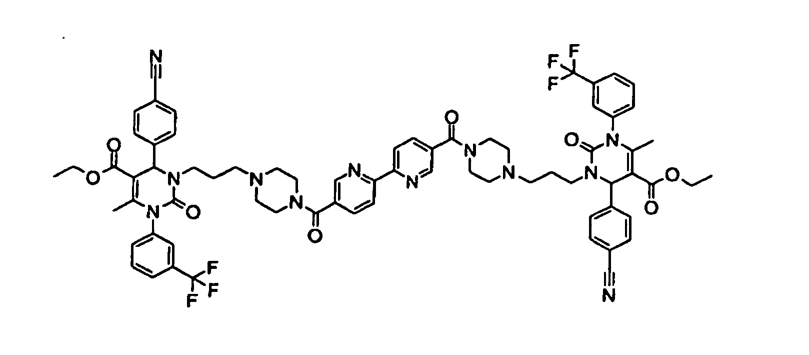 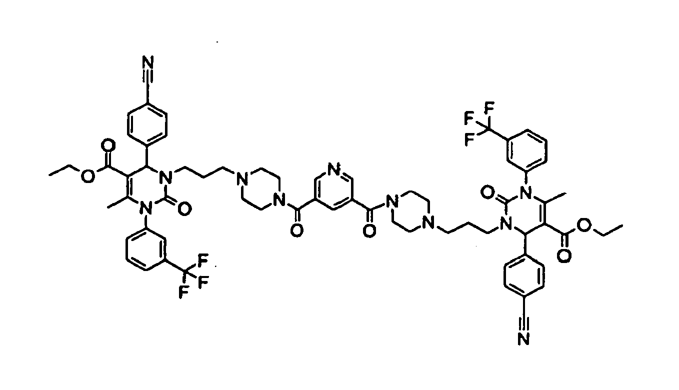 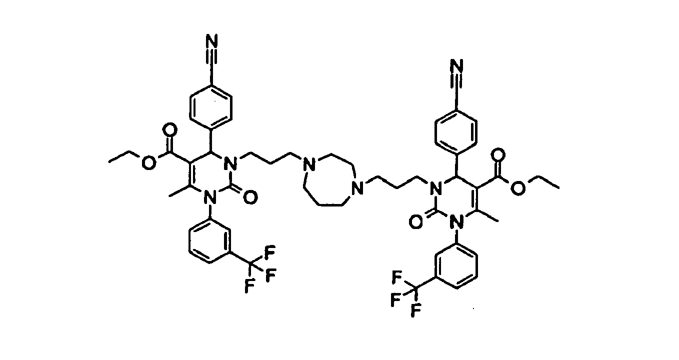 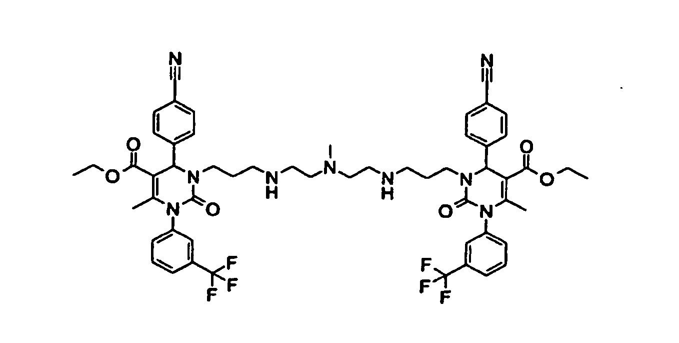 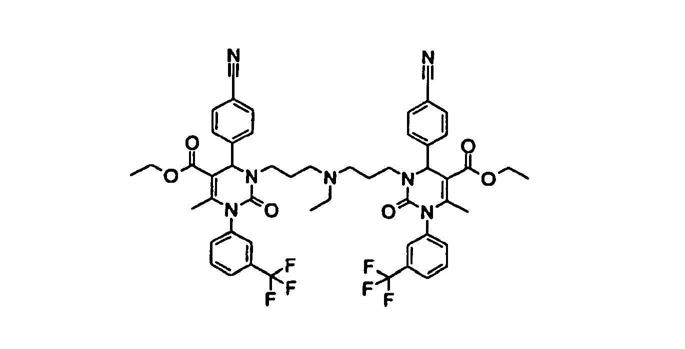 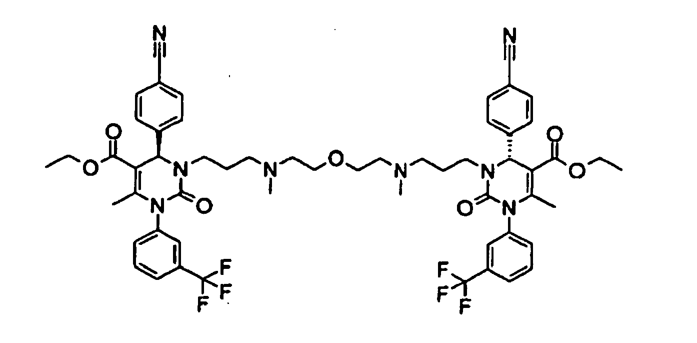 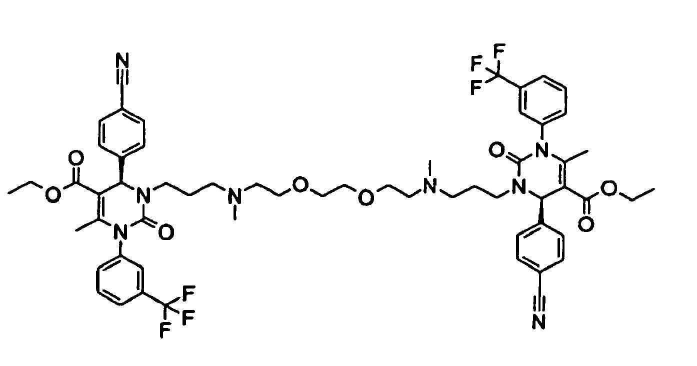 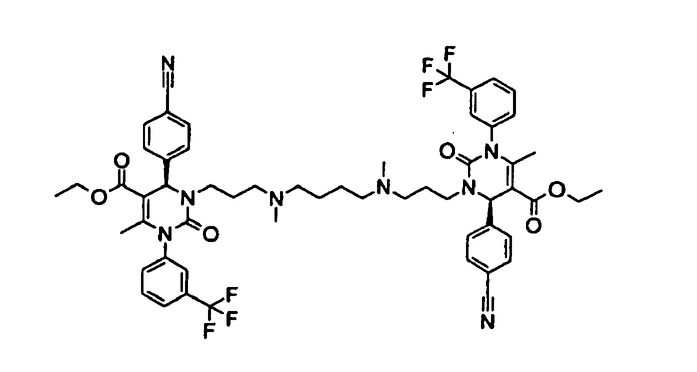 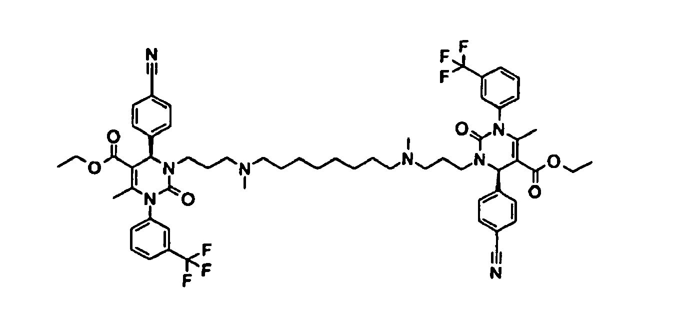 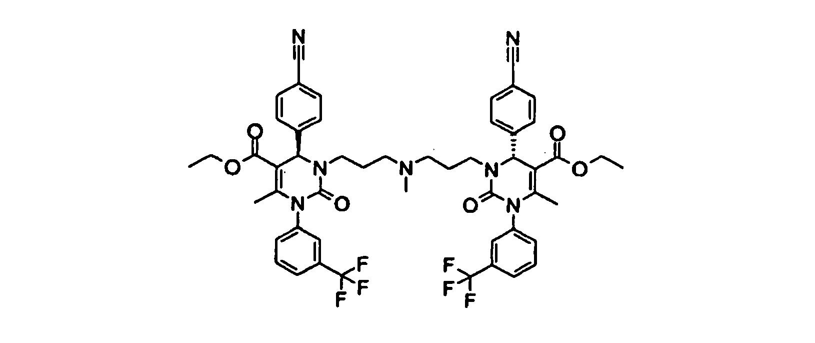 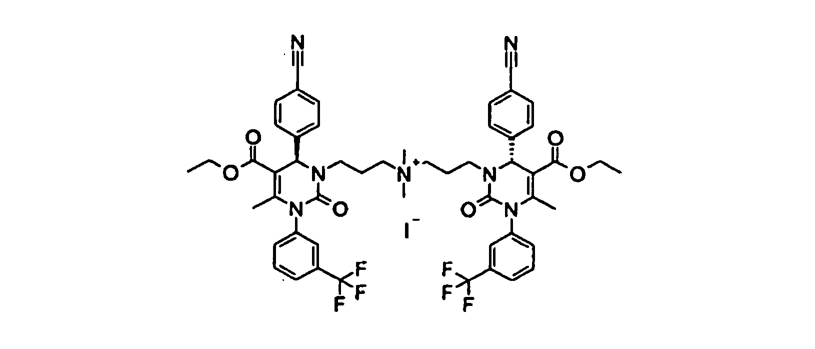 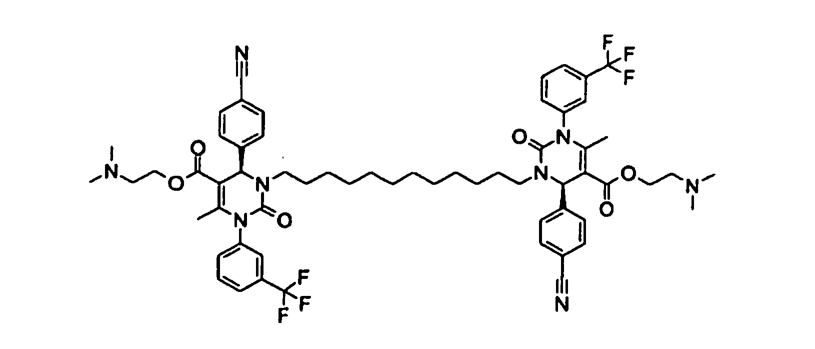 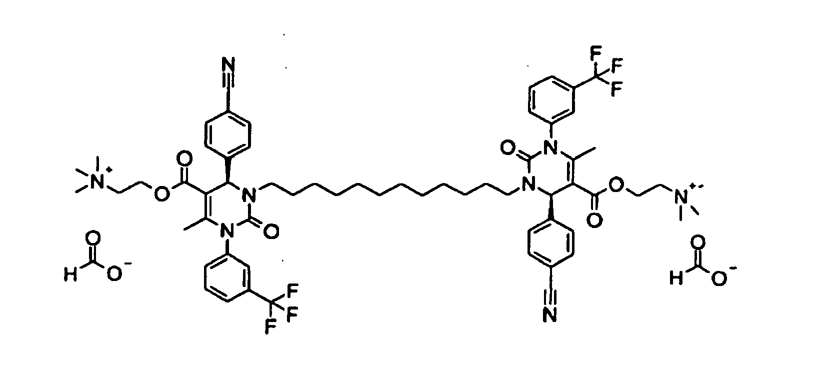 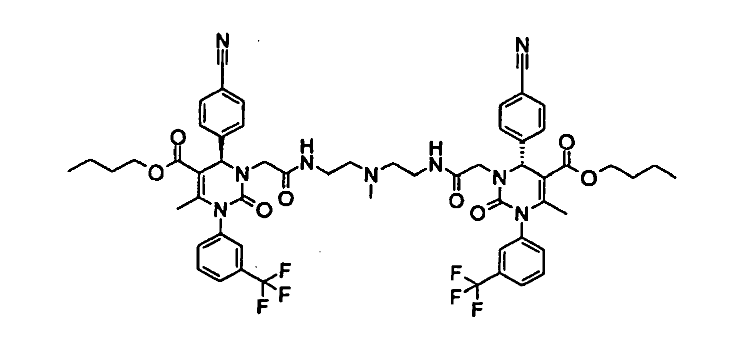 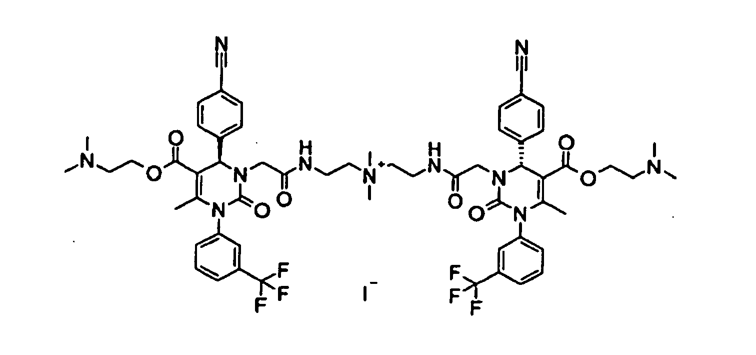 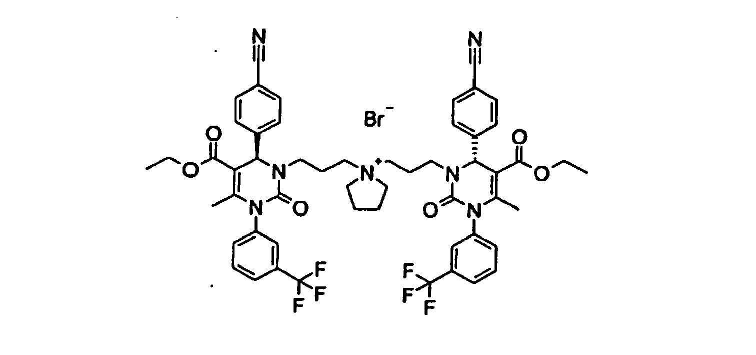 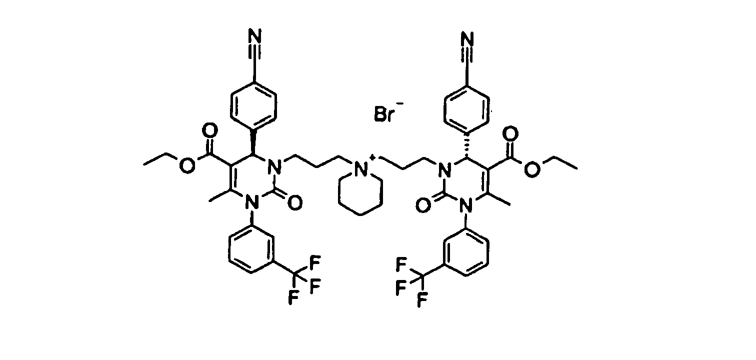 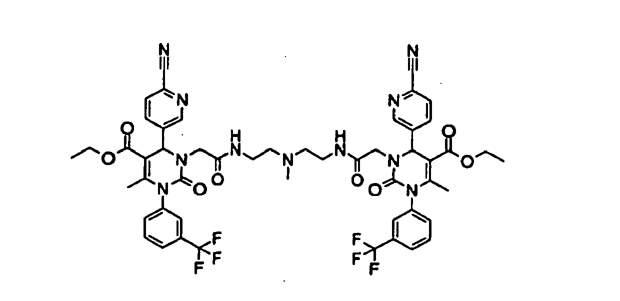 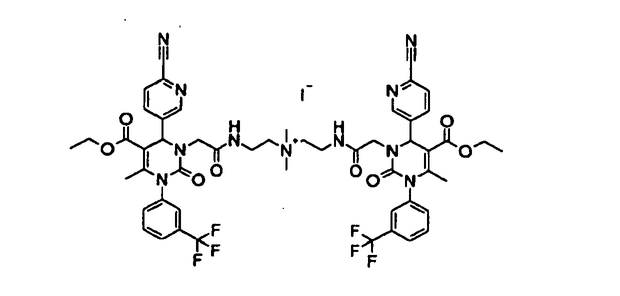 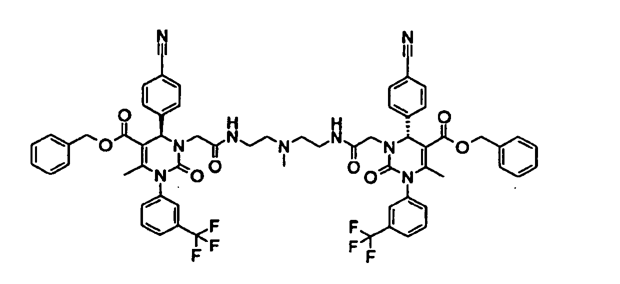 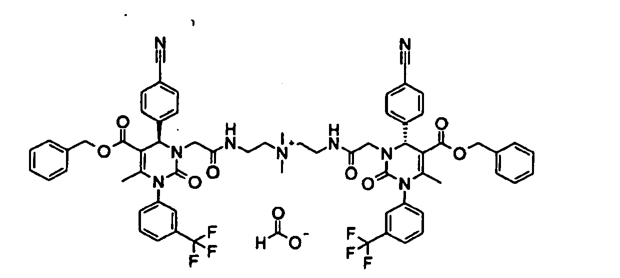 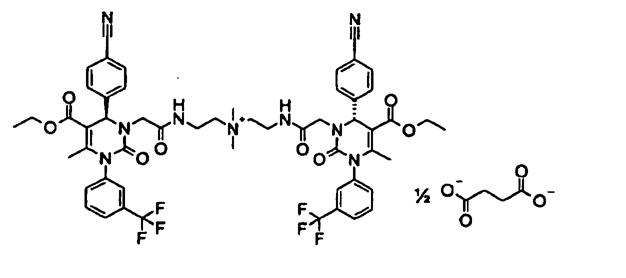 ist.

22. Verbindung nach Anspruch 1, die irgendeine der ersten 17 in Anspruch 21 definierten Verbindungen ist.

23. Verbindung nach Anspruch 1, die irgendeine von der 22., 24., 33., 35., 36., 38., 41., 46., 65., 69., 70., 71. und 76. in Anspruch 21 definierten ist.

24. Verbindung nach einem der vorhergehenden Ansprüche zur Verwendung bei der Therapie.

25. Pharmazeutische Zusammensetzung umfassend eine Verbindung nach einem der Ansprüche 1 bis 23 und einen pharmazeutisch akzeptablen Träger oder ein pharmazeutisch akzeptables Vehikel.

26. Verwendung einer Verbindung nach einem der Ansprüche 1 bis 23 für die Herstellung eines Medikaments zur Verwendung bei der Therapie eines Zustands ausgewählt unter Asthma, entzündlicher Darmerkrankung, chronisch-obstruktiver Lungenerkrankung (COPD), chronischer Bronchitis, Lungenfibrose, Lungenentzündung, akutem Atemnotsyndrom (ARDS), Lungenemphysem, durch Rauchen induziertem Emphysem, Sarkoidose, Bronchiektase oder zystischer Fibrose (ZF).

27. Verwendung nach Anspruch 26, wobei der Zustand COPD ist.

28. Verwendung nach Anspruch 26, wobei der Zustand CF ist.

29. Verwendung nach Anspruch 26, wobei der Zustand Colitis ulcerativa oder Crohn-Krankheit ist.

30. Verwendung nach einem der Ansprüche 26 bis 29, wobei der Zustand mit der Atmung zusammenhängt und das Medikament im Inhalationsweg verabreicht werden soll.

## Revendications

1. Composé de la formule (I)
M-L-M (I)
dans lequel L est un bras d'accrochage et chaque M est indépendamment un groupe de la formule (II) : 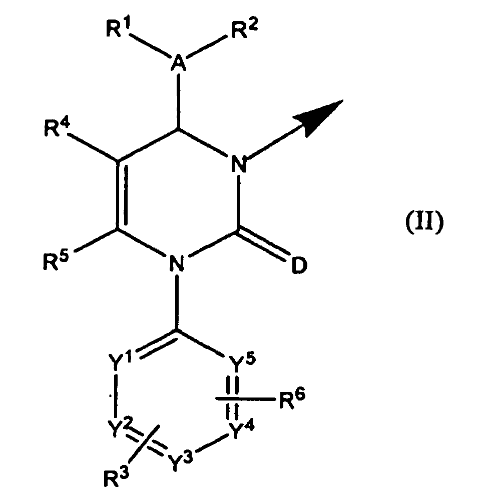 dans lequel A est aryle ou hétéroaryle ;
D est oxygène ou soufre ;
Y¹, Y², Y³, Y⁴ et Y⁵ sont indépendamment chacun CH, CR³, CR⁶ ou N, à condition que l'un d'eux soit CR³, l'un soit CR⁶ et que pas plus de deux soient N ;
R¹, R² et R³ sont indépendamment chacun hydrogène, halogène, nitro, cyano, C₁C₆-alkyle, hydroxy ou C₁-C₆-alkoxy, dans lequel alkyle et alkoxy sont chacun optionnellement substitués par un à trois radicaux sélectionnés indépendamment parmi halogène, hydroxy et C₁-C₄-alkoxy ;
R4 est trifluorométhylcarbonyle, C₁-C₆-alkylcarbonyle, C₁-C₆-alkoxycarbonyle, C₁-C₆-alkenoxycarbonyle, hydroxycarbonyle, aminocarbonyle, mono- ou di-C₁-C₄-alkylaminocarbonyle, C₆-C₁₀-arylaminocarbonyle, phénylcarbonyle, hétéroarylcarbonyle hétéroaryle ou cyano, dans lequel alkylcarbonyle, alkoxycarbonyle et alkylaminocarbonyle sont chacun optionnellement substitué par un à trois radicaux sélectionnés indépendamment parmi : C₃-C₈-cycloalkyle, hydroxy, C₁-C₄-alkoxy, C₁C₄-alkoxycarbonyle, hydroxycarbonyle, aminocarbonyle, mono- et di-C₁-C₄-alkylaminocarbonyle, C₁-C₄-alkylcarbonylamino, (C₁-C₄-alkylcarbonyl)-C₁C₄-alkylamino, cyano, amino, mono- et di-C₁-C₄-alkylamino, hétéroaryle, hétérocyclyle et tri-(C₁-C₆-alkyl)-silyle, et dans lequel hétéroarylcarbonyle et hétéroaryle peuvent être en outre substitués par C₁-C₄-alkyle ;
ou R⁴ représente un groupe de formule (VIII) 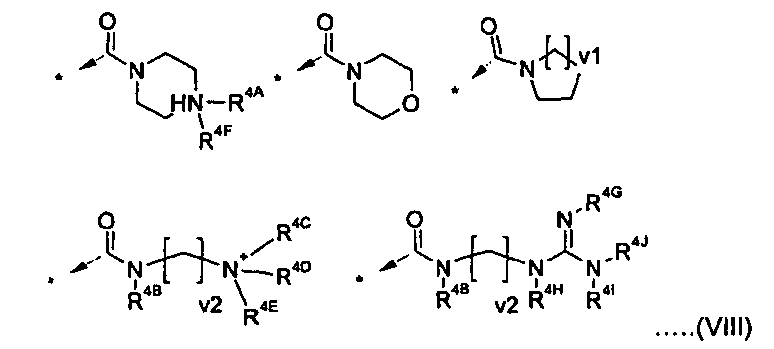 dans lequel
R^{4A}, R^{4B}, R^{4G}, R^{4H} R^{4I} et R^{4J} sont indépendamment hydrogène ou alkyle, ou R^{4H} et R^{4I} peuvent être unis avec l'atome d'azote auquel ils sont attachés pour former un noyau ;
R^{4F} est une paire célibataire ou R^{4F} est alkyle et l'atome d'azote auquel il est attaché est quaternaire et porte une charge positive ;
R^{4C}, R^{4D} et R^{4E} sont alkyle, ou deux quelconques parmi R^{4C}, R^{4D} ou R^{4E} peuvent être unis avec l'atome d'azote auxquels ils sont attachés pour former un noyau, contenant optionnellement un autre hétéroatome sélectionné parmi oxygène et nitrogène ;
v1 est 1-3;
v2 est 1-6;
R⁵ est C₁-C₄-alkyle ou amino, et alkyle est optionnellement substitué par un à trois radicaux indépendamment sélectionnés parmi : halogène, hydroxy, C₁-C₆-alkoxy, C₁-C₆-alkenoxy, C₁-C₆-alkylthio, amino, mono- et di-C₁-C₆-alkylamino, phénylamino, hydroxycarbonyle, C₁-C₆-alkoxycarbonyle et le radical -O-(C₁-C₄-alky)-O-(C₁-C₄-alkyl) ; et
R⁶ est halogène, nitro, cyano, C₁-C₆-alkyle, hydroxy ou C₁-C₆-alkoxy, dans lequel alkyle et alkoxy sont optionnellement substitués par un à trois radicaux sélectionnés indépendamment parmi halogène, hydroxy et C₁-C₄-alkoxy ;
dans lequel hétéroaryle est sélectionné parmi : benzimidazolyl, benzoxazolyle, benzothiazolyle, benzofuranyle, benzothiényle, furyle, imidazolyle, indolyle, indolizinyle, isoxazolyle, isoquinolinyle, isothiazolyle, oxazolyle, oxadiazolyle, pyrazinyle, pyridazinyle, pyrazolyle, pyridyle, pyrimidinyle, pyrrolyle, quinazolinyle, quinolinyle, tétrazolyle, 1,3,4-thiadiazolyle, thiazolyle, thiényle et triazolyle ;
ou bien un sel de qualité pharmaceutique, solvat ou N-oxyde de celui-ci.

2. Composé selon la revendication 1, dans lequel A est phényle.

3. Composé selon la revendication 1 ou la revendication 2, dans lequel R¹ est hydrogène.

4. Composé selon l'une quelconque des revendications précédentes, dans lequel R² est méthyle ou -CN.

5. Composé selon l'une quelconque des revendications précédentes, dans lequel R³ est hydrogène.

6. Composé selon l'une quelconque des revendications précédentes, dans lequel R⁴ est -C(O)OCH₂CH₃ ou -C(O)OCH₂CH₂OH.

7. Composé selon l'une quelconque des revendications précédentes, dans lequel R⁵ est méthyle.

8. Composé selon l'une quelconque des revendications précédentes, dans lequel R⁶ est trifluorométhyle.

9. Composé selon l'une quelconque des revendications précédentes, dans lequel aucun parmi Y¹, Y², Y³, Y⁴ et Y⁵ n'est N.

10. Composé selon l'une quelconque des revendications précédentes, dans lequel D est oxygène.

11. Composé selon l'une quelconque des revendications précédentes, dans lequel L est un groupe de formule (III)
-L^{a}-R⁷-L^{b}-W-L^{b}-R⁷-L^{a}-(III)
dans lequel La est une liaison ou -C(O)- ;
L^{b} est une liaison ou -C(O)-;
R⁷ est alkylène ou cycloalkylène ;
W est une liaison, N(R^{9B})(R^{9C}) ou bien est sélectionné parmi les radicaux divalents suivants :
-(O-R^{8A})ₘ₁-O-
-N(R^{9A})-(O-R^{8A})ₘ₁-R^{8A}-N(R^{9A})-
-N(R^{9A})-R^{8B}-N(R^{9B})(R^{9C})-R^{8B}-N(R^{9A})-
-N(R^{9A})-R^{8B}-N(R^{10B})C(=NR^{10A})(NR^{10c})-R^{8B}-N(R^{9A})-
-N(R^{9A})-R^{8B}-N(R^{9A})- 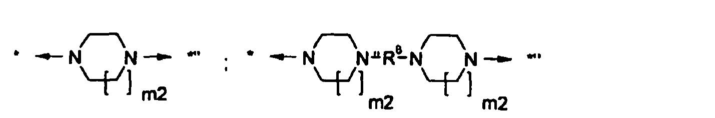 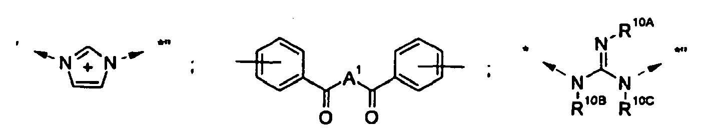
dans lequel
m1 est 1-4 ;
R^{8A} est alkylène ou cycloalkylène ;
R^{8B} est un groupe alkylène ou cycloalkylène, ou bien un groupe de la Formule A² ; R^{9A} est hydrogène ou alkyle inférieur ;
l'un de R^{9B} ou R^{9C} est une paire célibataire tandis que l'autre est hydrogène ou C₁₋₁₂ alkyle, ou bien R^{9B} et R^{9C} sont tous deux C₁₋₁₂ alkyle, dans lequel l'azote auquel ils sont attachés est quaternaire et porte une charge positive, ou bien NR^{9B}R^{9C} est un noyau ;
R^{10A} est hydrogène ou C₁₋₁₂ alkyle ;
R^{10B} et R^{10C} sont indépendamment hydrogène ou C₁₋₁₂ alkyle, ou R^{10B} et R^{10C} sont unis pour former un noyau ;
m2 est 1-3 ;
A¹ est sélectionné parmi -N(R^{9A})-R⁸-N(R^{9B})(R^{9C})-R⁸-N(R^{9A})- et -N(R^{9A})-R⁸-N(R¹⁰B)C(=NR^{10A})(NR^{10C})-R⁸-N(R⁹A)- ; et
A² est sélectionné parmi 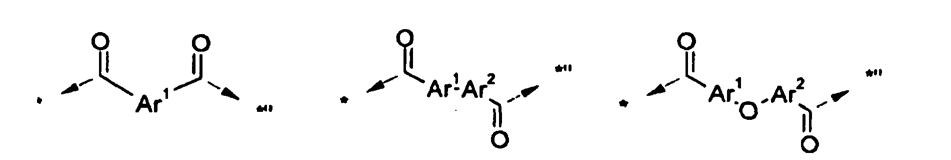 dans lequel Ar¹ et Ar² sont indépendamment chacun phényle ou hétéroaryle selon la revendication 1.

12. Composé selon la revendication 11, dans lequel chaque L^{a} est indépendamment - C(O)- ou une liaison covalente.

13. Composé selon la revendication 12, dans lequel L^{a} est une liaison.

14. Composé selon les revendications 11 à 13, dans lequel R⁷ est alkylène.

15. Composé selon l'une quelconque des revendications 11 à 14, dans lequel W est - N(R^{9A})-R^{8B}-N(R^{9B})(R^{9C})-R^{8B}-N(R^{9A})-.

16. Composé selon l'une quelconque des revendications 11 à 14, dans lequel W -N(R^{9A})-R^{8B}-N(R^{10B})C(=NR^{10A})(NR^{10C})-R^{8B}-N(R^{9A}).

17. Composé selon l'une quelconque des revendications 11 à 14, dans lequel W est

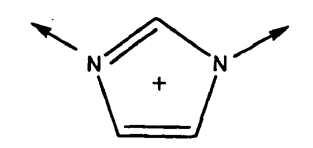

18. Composé selon l'une quelconque des revendications 11 à 14, dans lequel W est

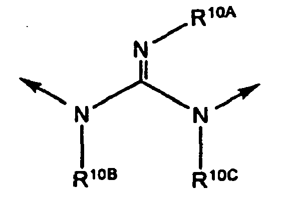

19. Composé selon l'une quelconque des revendications 11 à 14, dans lequel W est - N(R^{9B})(R^{9C})-.

20. Composé selon l'une quelconque des revendications précédentes, dans lequel A est phényle ou hétéroaryle ; et
R⁴ est trifluorométhylcarbonyle, C₁-C₆-alkylcarbonyle, C₁-C₆-alkoxycarbonyle, C₁-C₆-alkenoxycarbonyle, hydroxycarbonyle, aminocarbonyle, mono- ou di-C₁-C₄-alkylaminocarbonyle, C₆-C₁₀-arylaminocarbonyle, phénylcarbonyle, hétéroarylcarbonyle, hétéroaryle ou cyano, dans lequel C₁-C₆-alkylcarbonyle, C₁-C₆-alkoxycarbonyle, mono- et di-C₁-C₄-alkylaminocarbonyle peuvent être substitués en outre par un à trois radicaux identiques ou différents sélectionnés parmi le groupe constitué de : C₃-C₈-cycloalkyle, hydroxy, C₁-C₄-alkoxy, C₁-C₄-alkoxycarbonyle, hydroxycarbonyle, aminocarbonyle, mono- et di-C₁-C₄-alkylaminocarbonyle, C₁-C₄-alkylcarbonylamino, (C₁-C₄ alkylcarbonyl)-C₁-C₄-alkylamino, cyano, amino, mono- et di-C₁-C₄-alkylamino, hétéroaryle, hétérocyclyle et tri-(C₁-C₆-alkyl)-silyle, et dans lequel hétéroarylcarbonyle et hétéroaryle peuvent être en outre substitués par C₁-C₄-alkyle.

21. Composé selon la revendication 1, qui est l'un quelconque parmi :

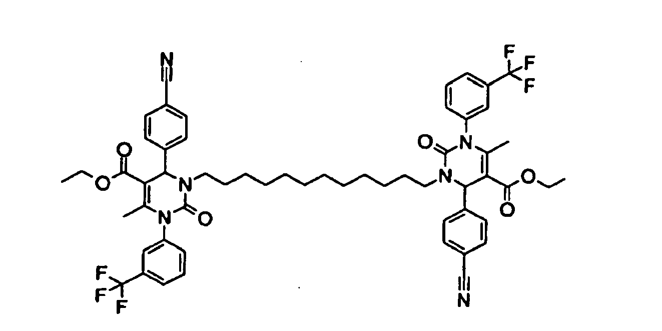

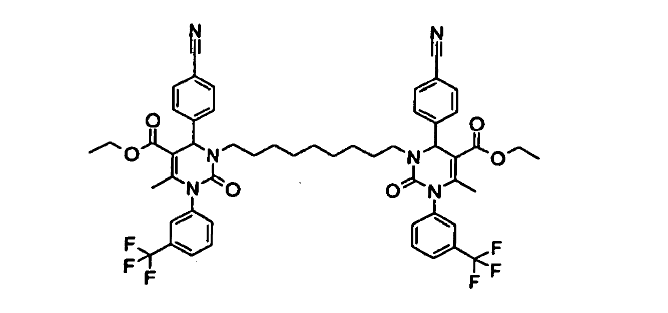

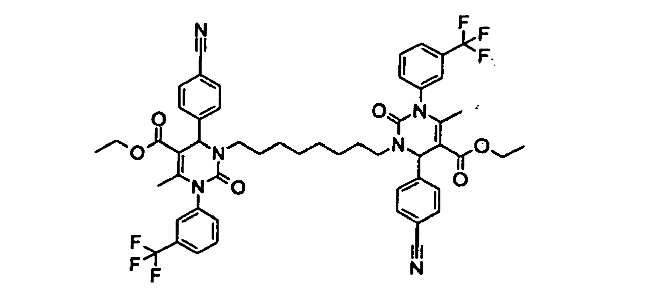

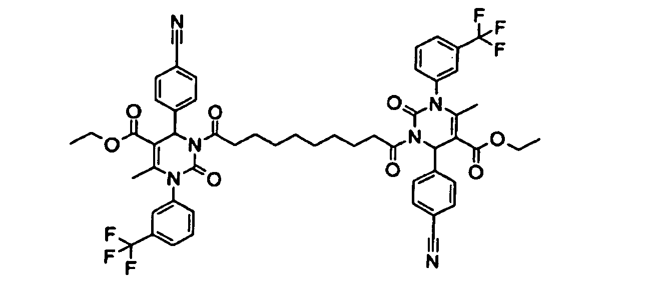

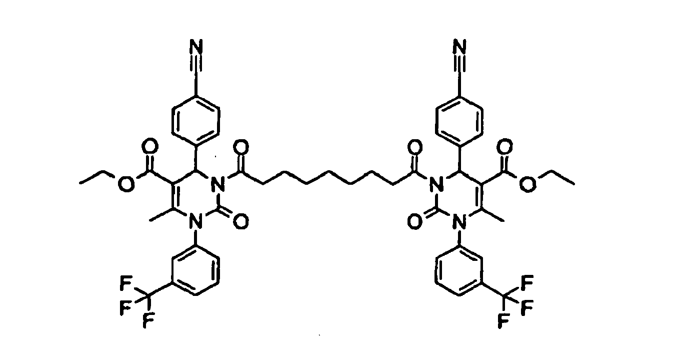

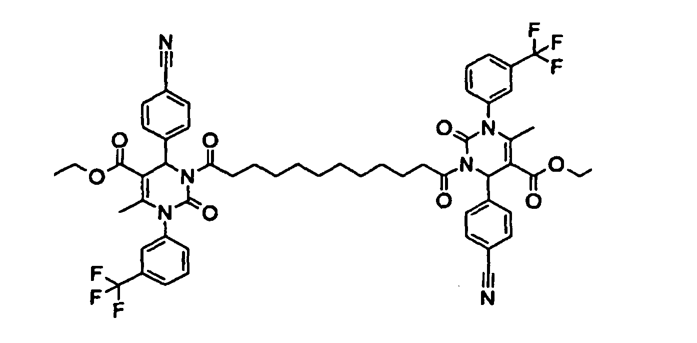

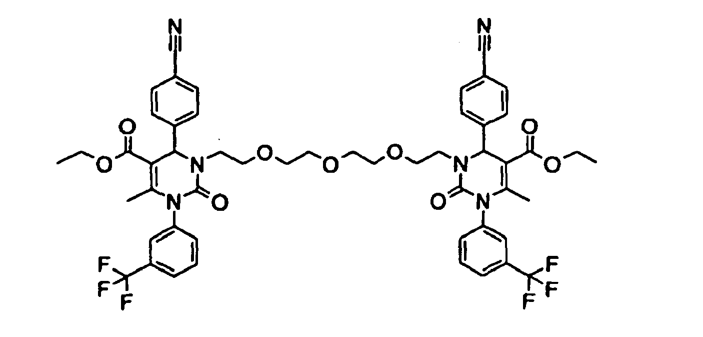

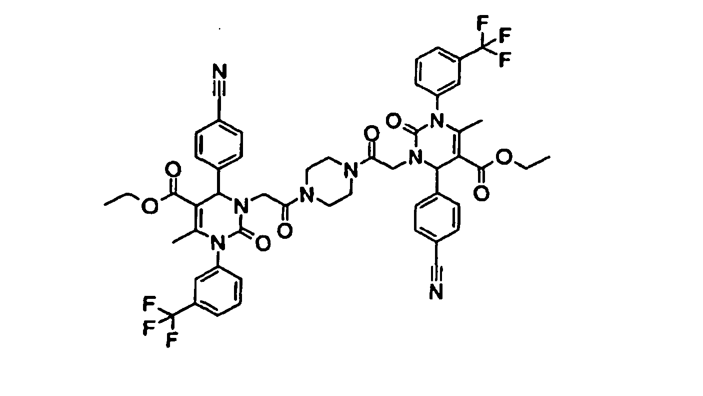

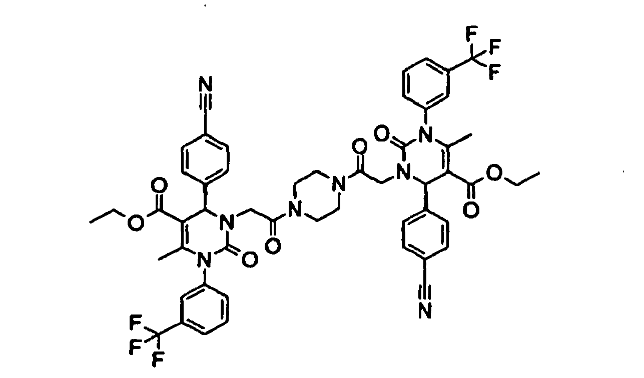

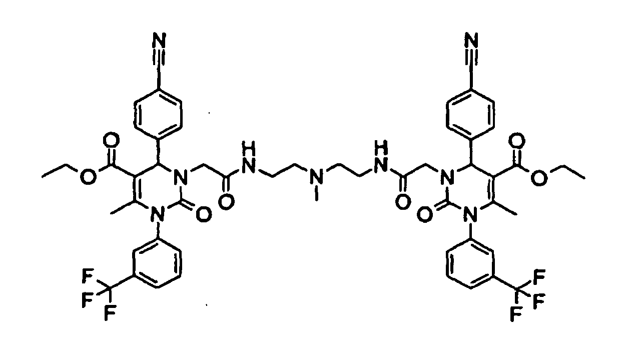

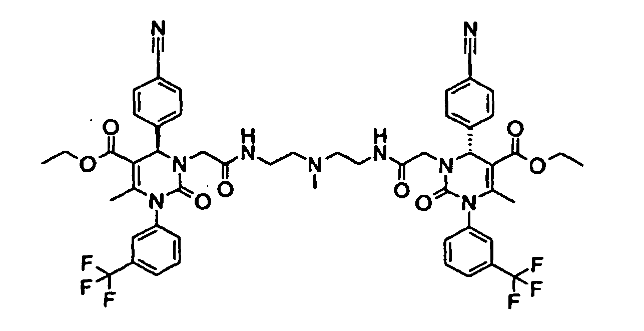

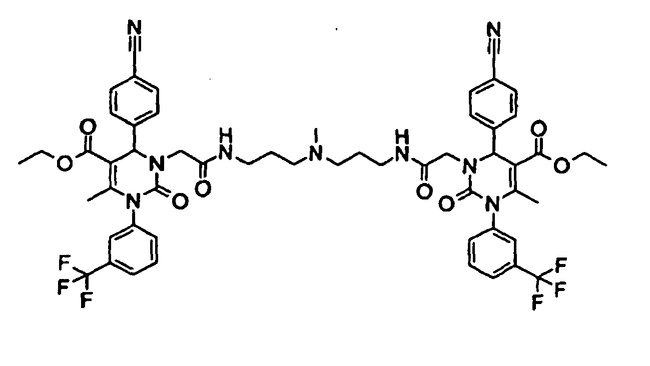

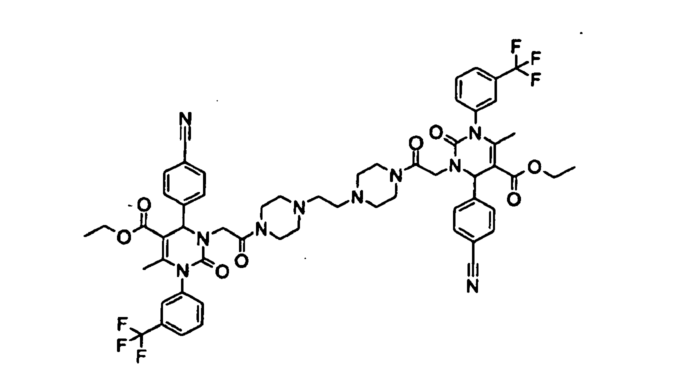

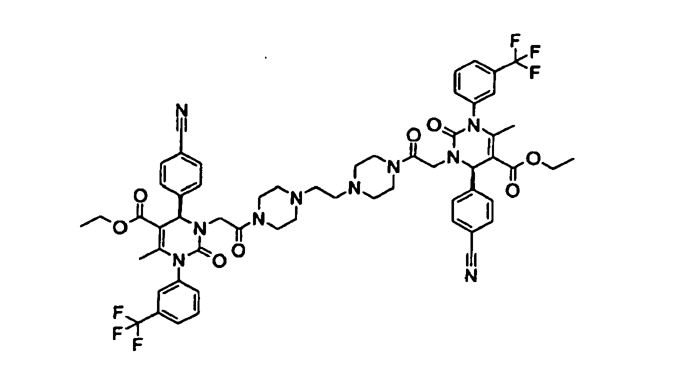

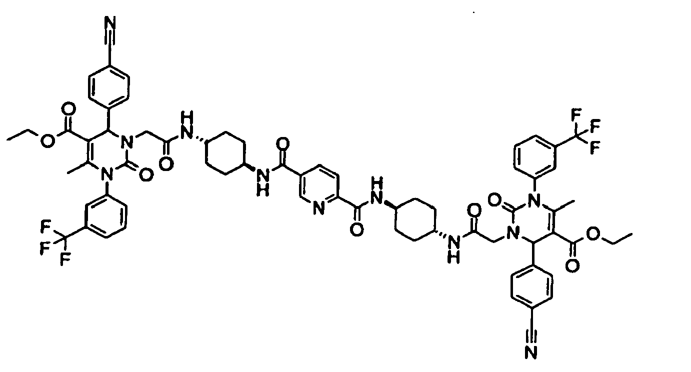

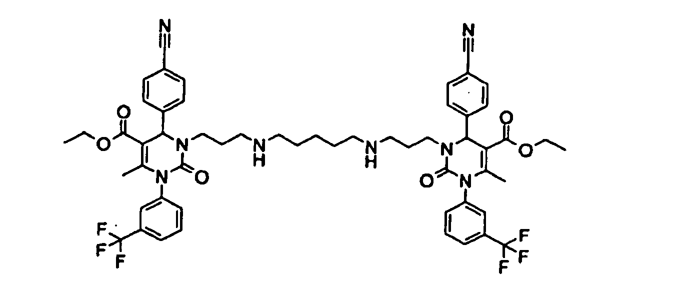

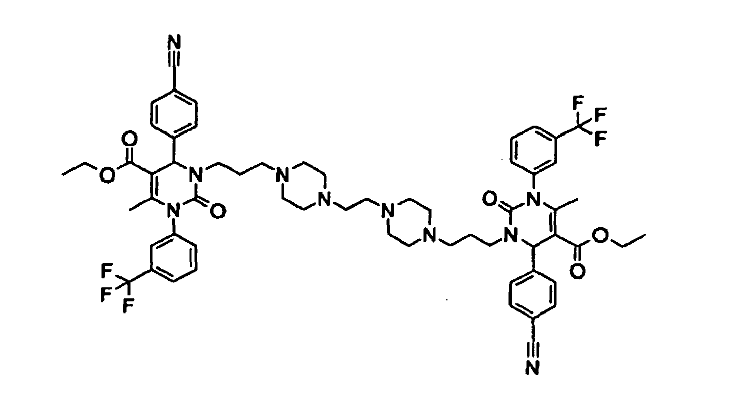

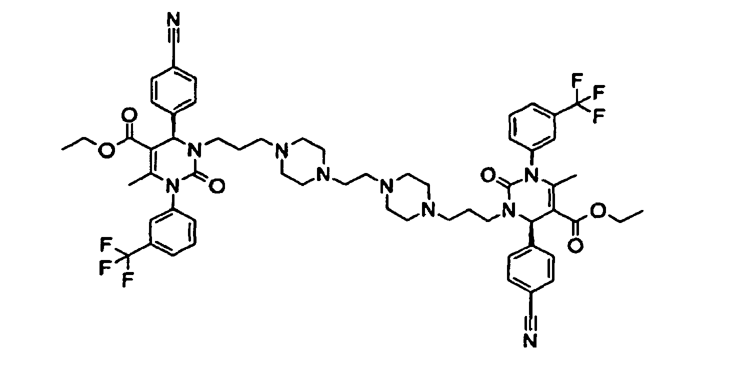

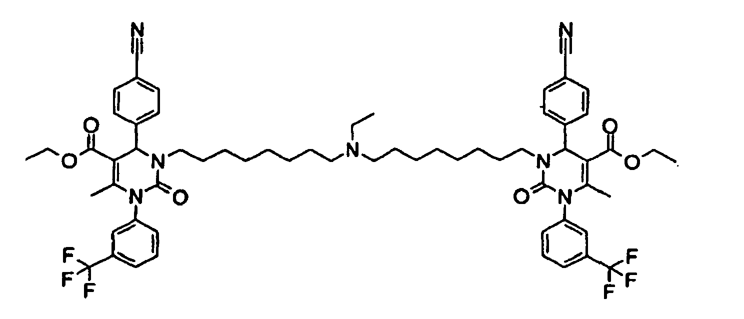

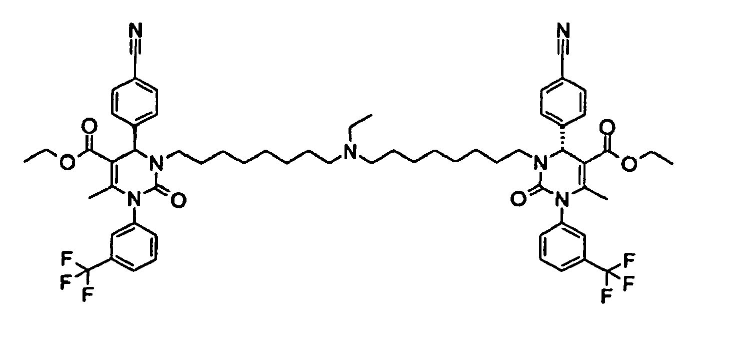

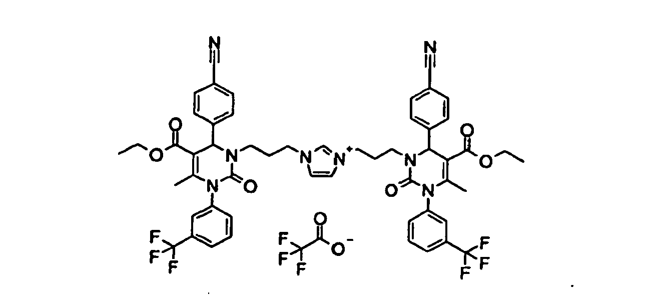

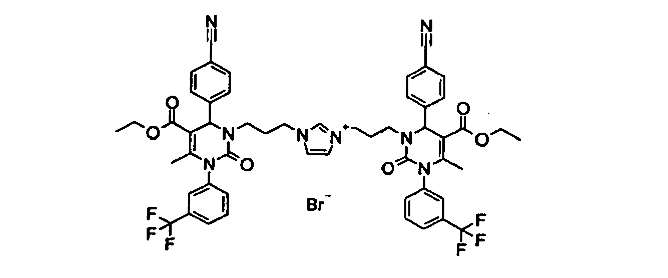

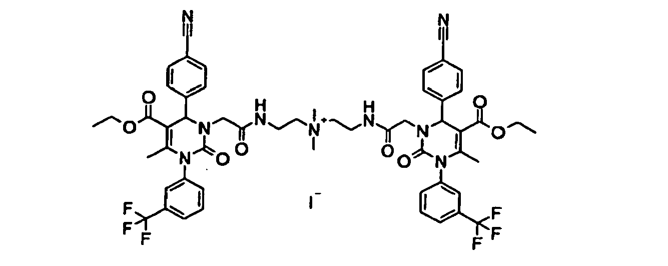

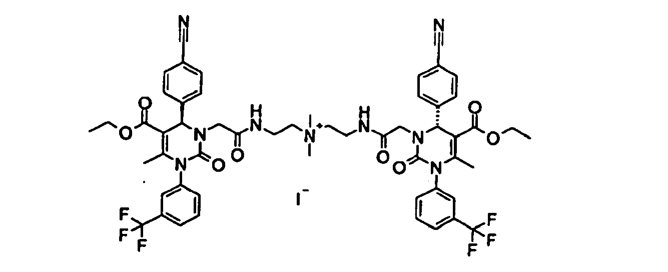

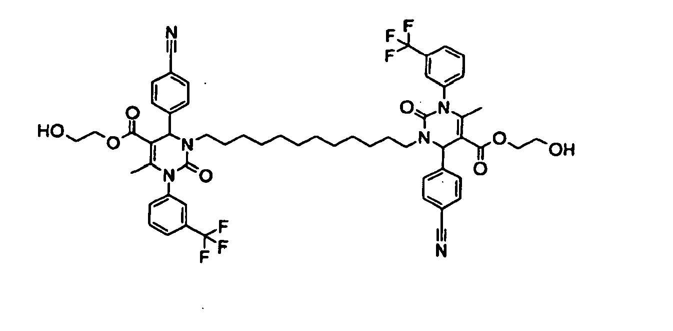

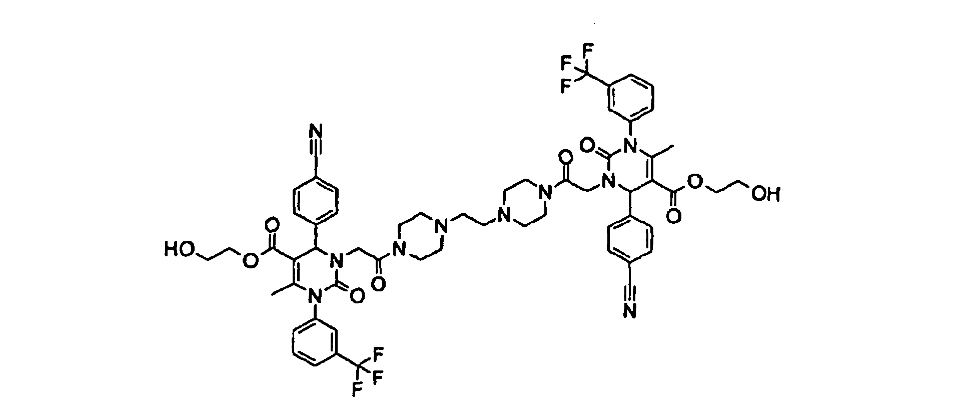

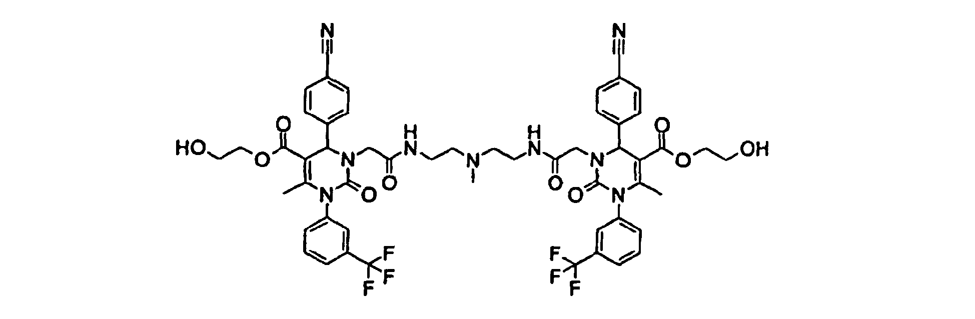

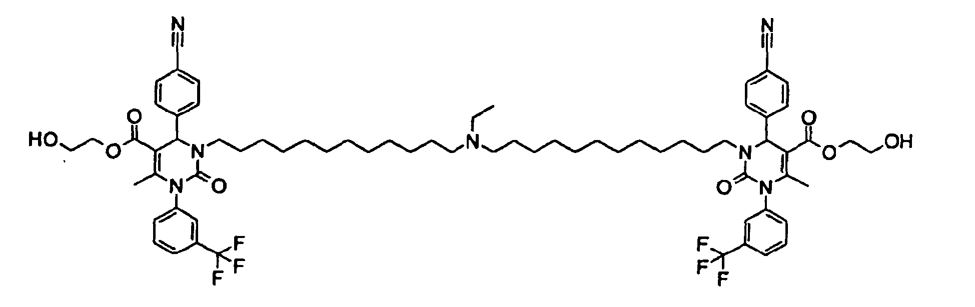

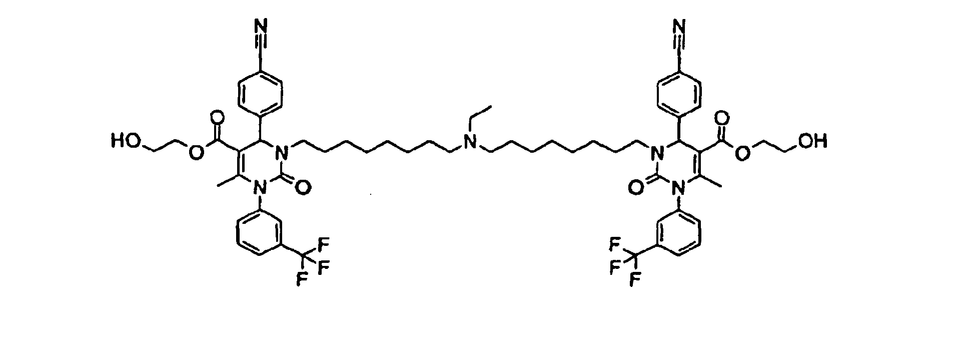

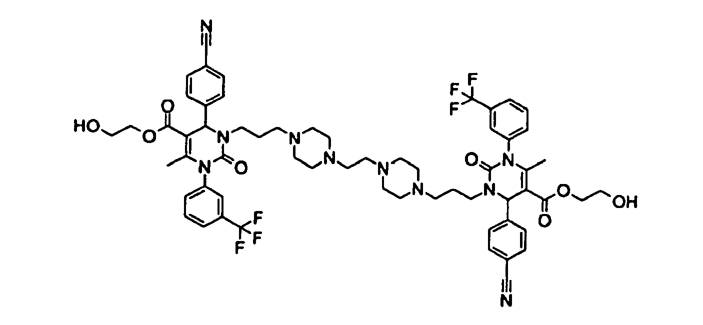

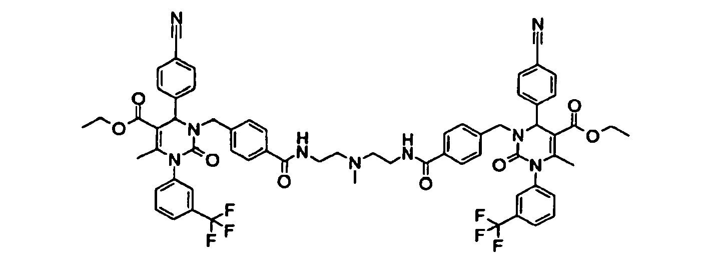

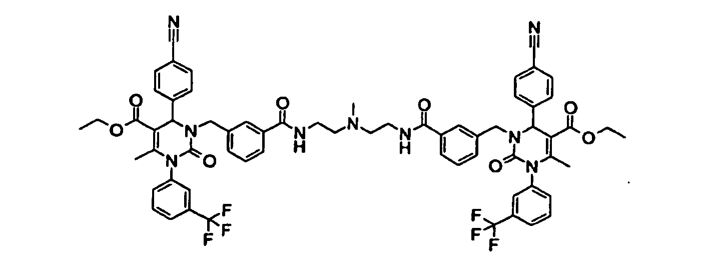

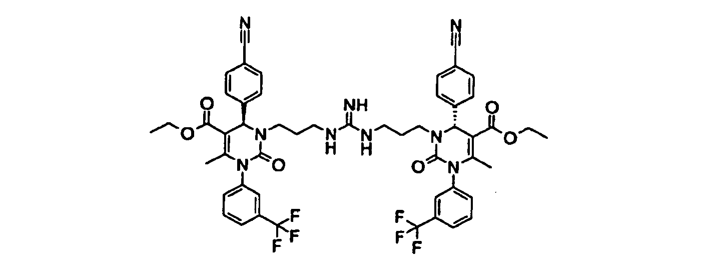

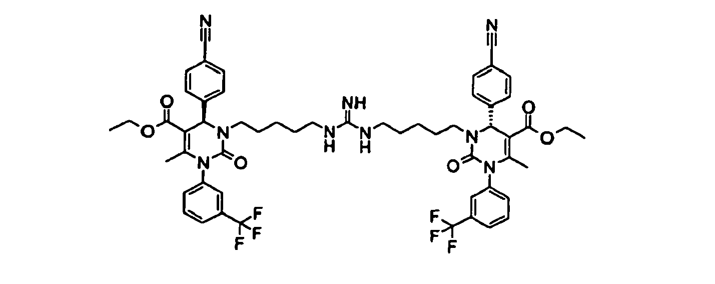

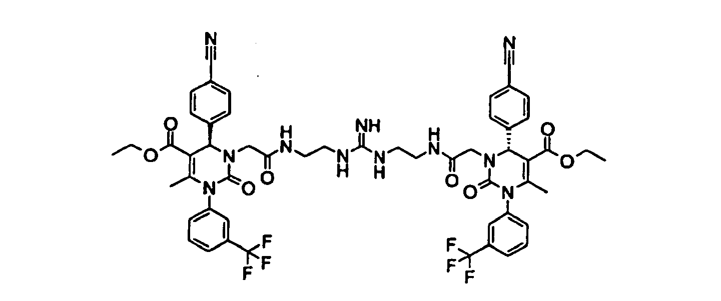

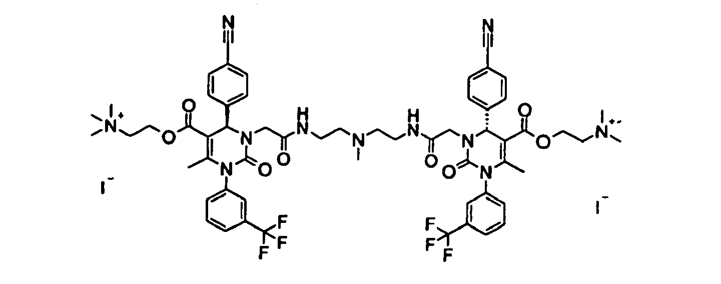

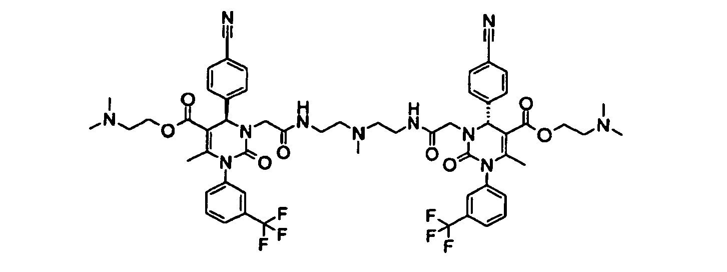

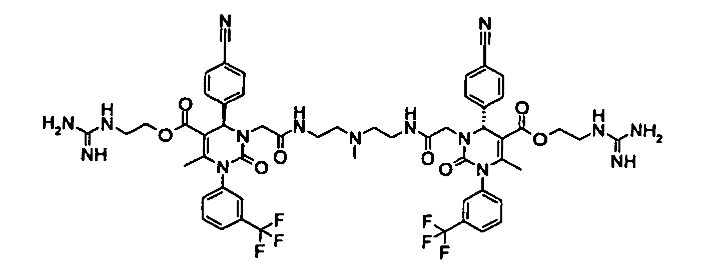

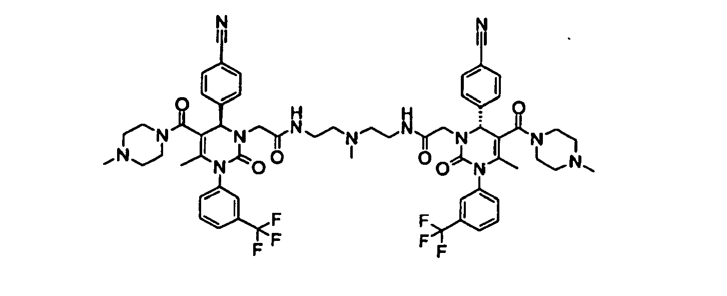

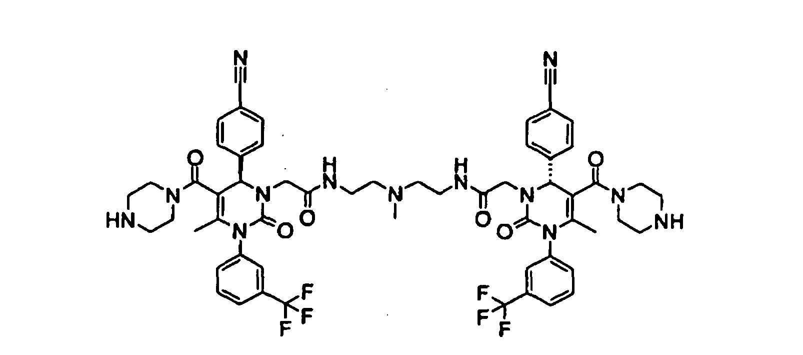

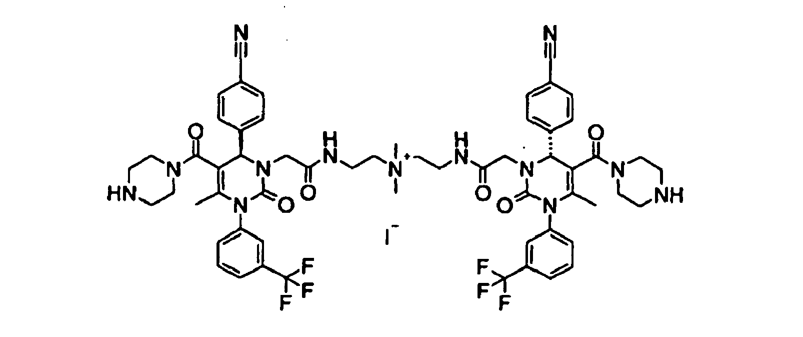

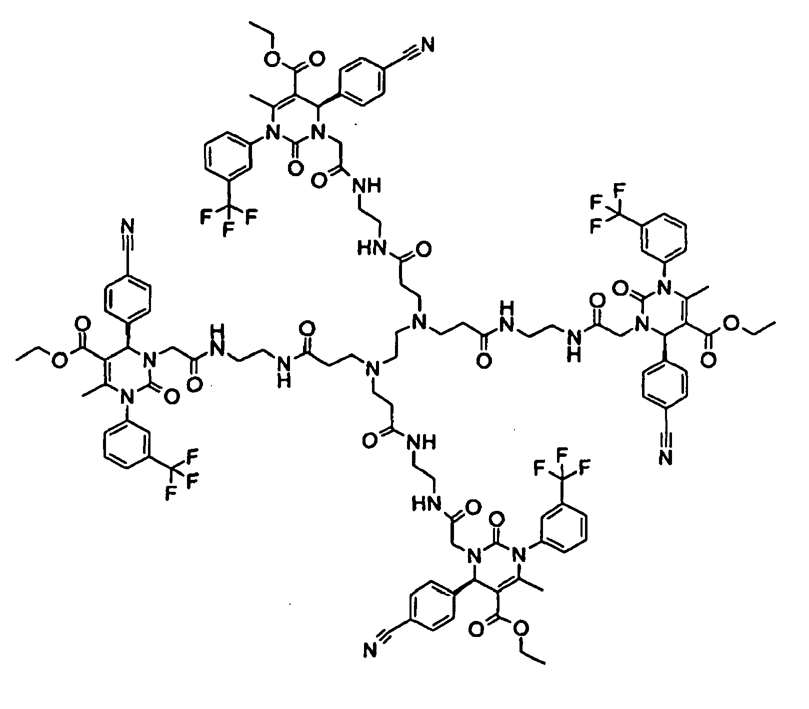

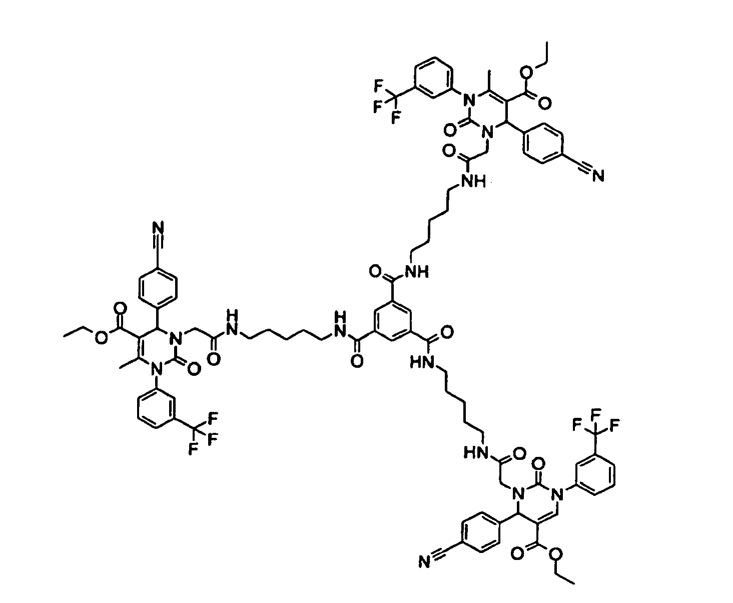

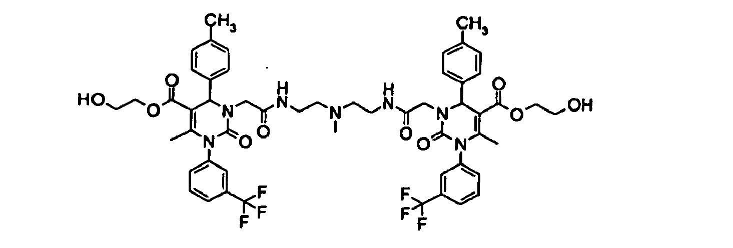

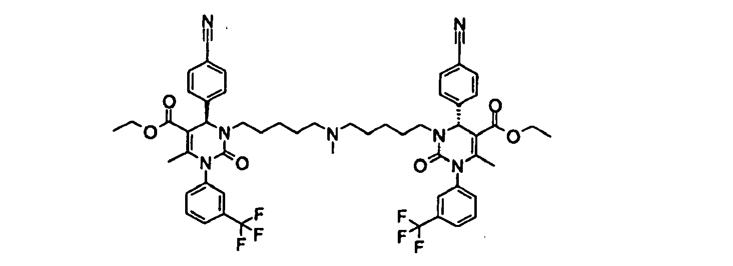

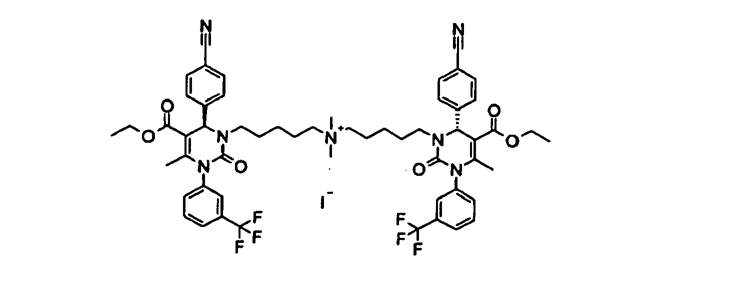

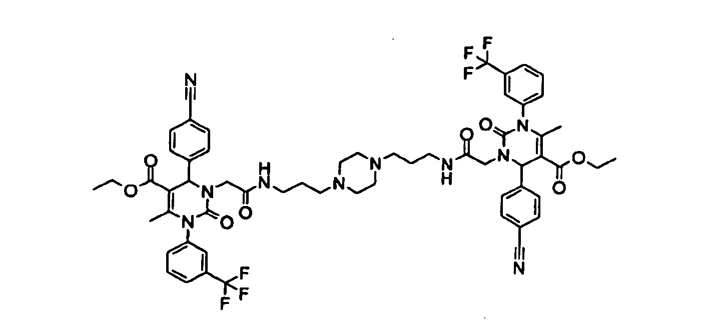

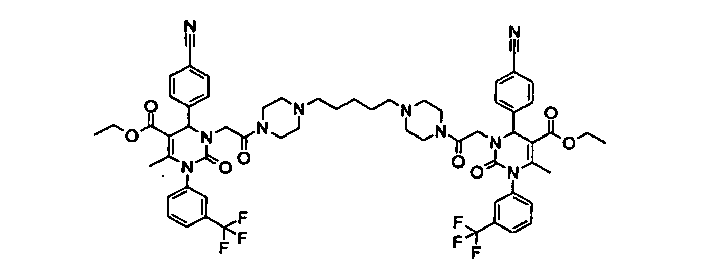

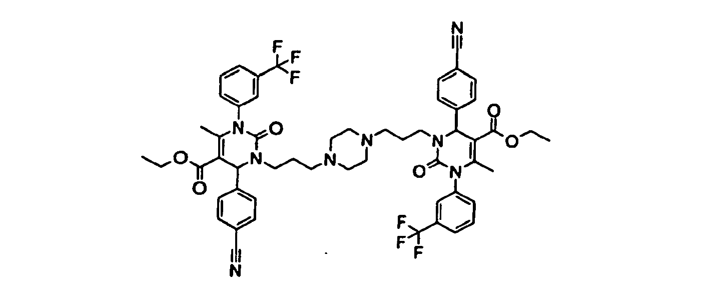

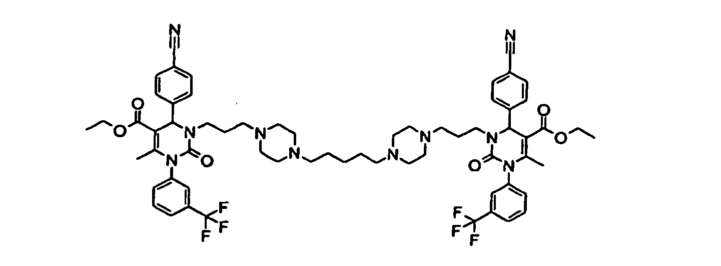

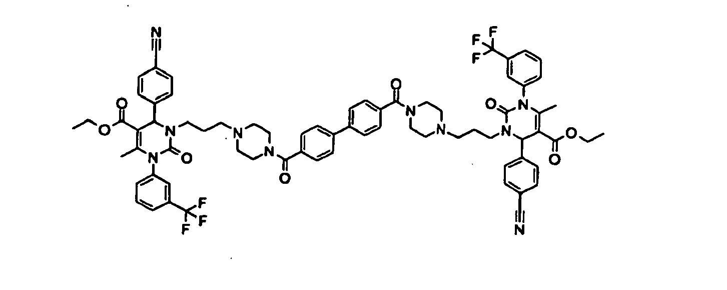

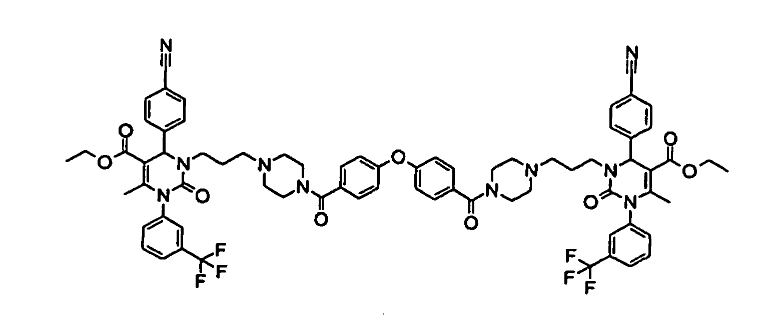

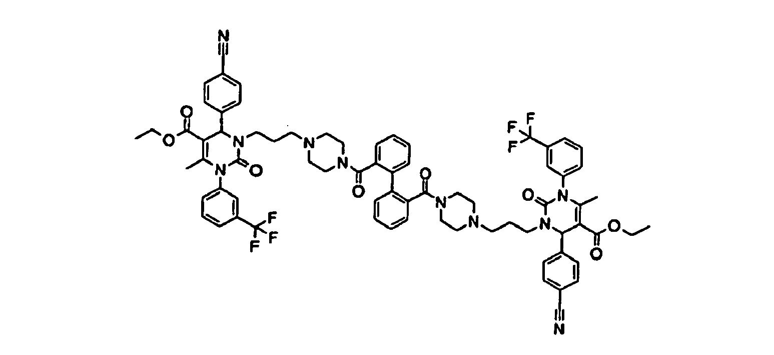

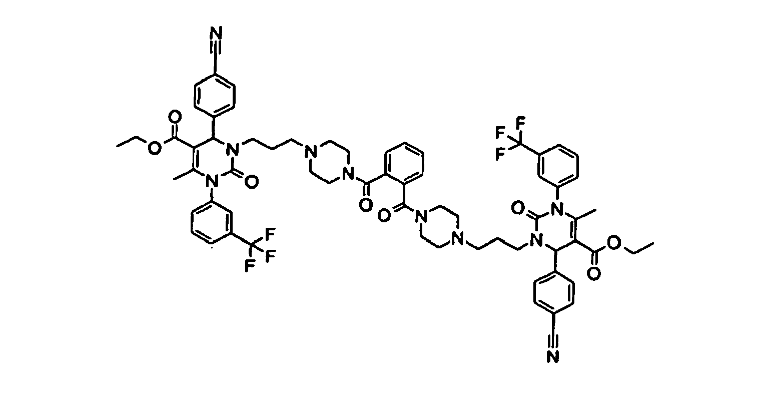

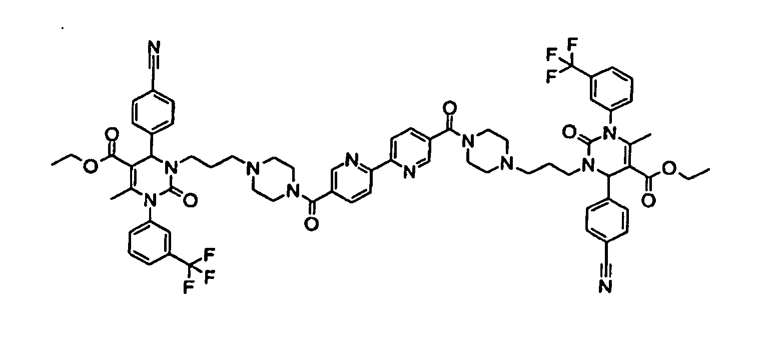

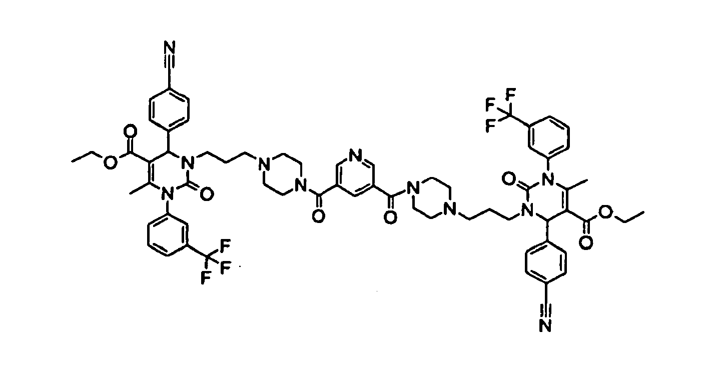

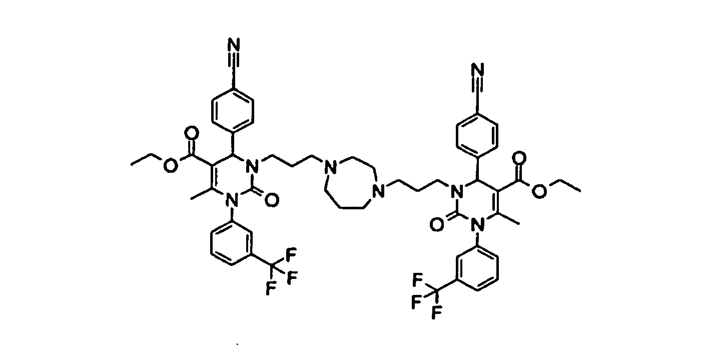

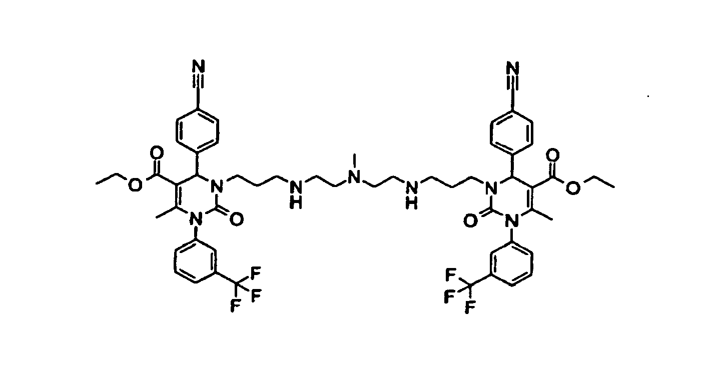

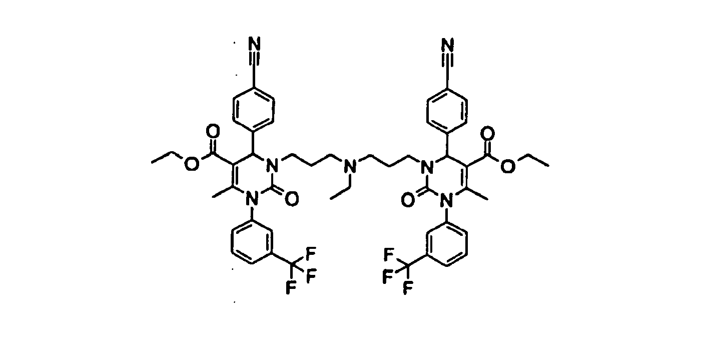

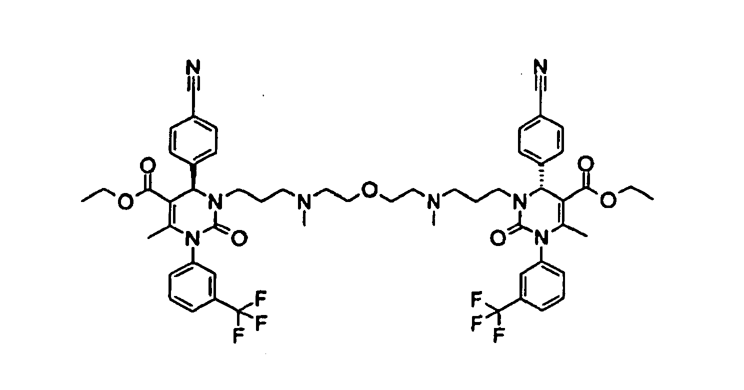

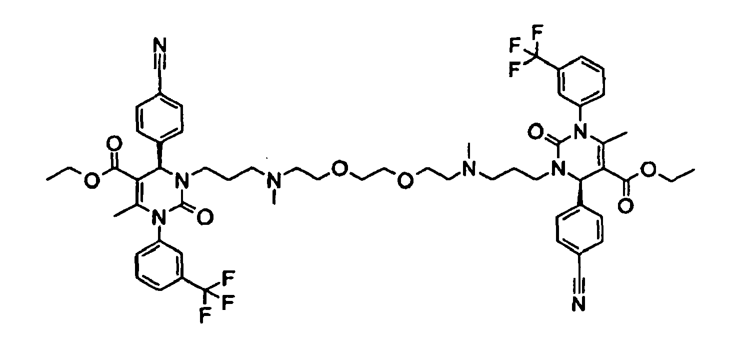

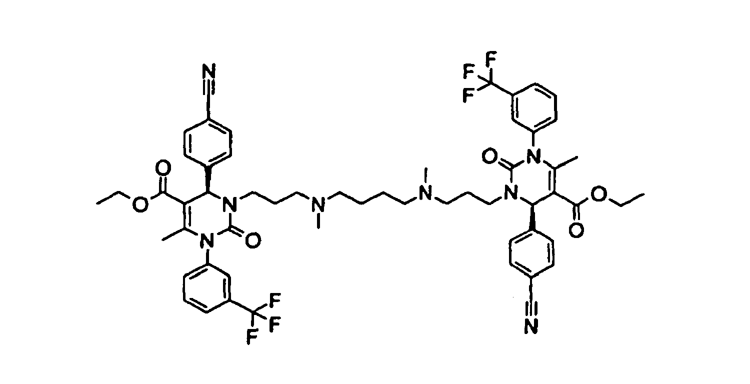

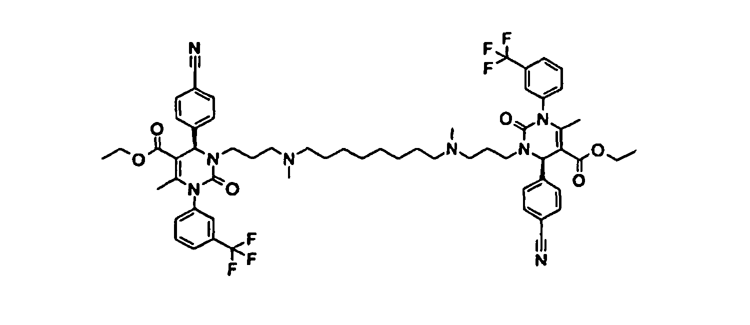

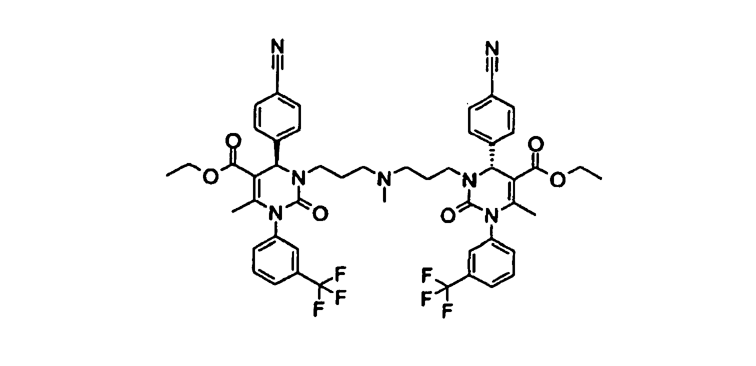

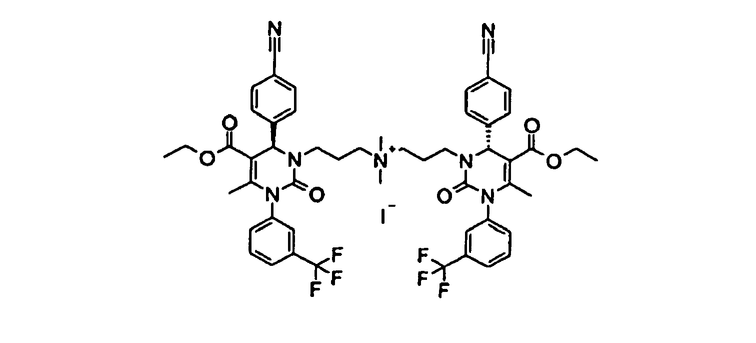

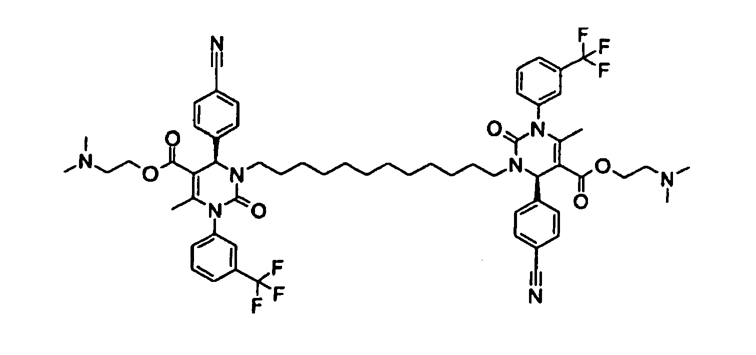

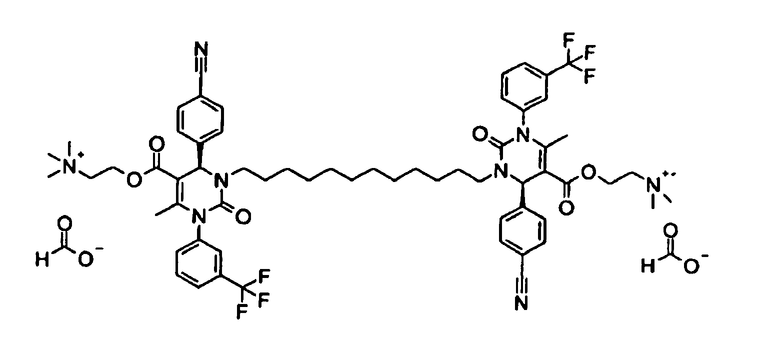

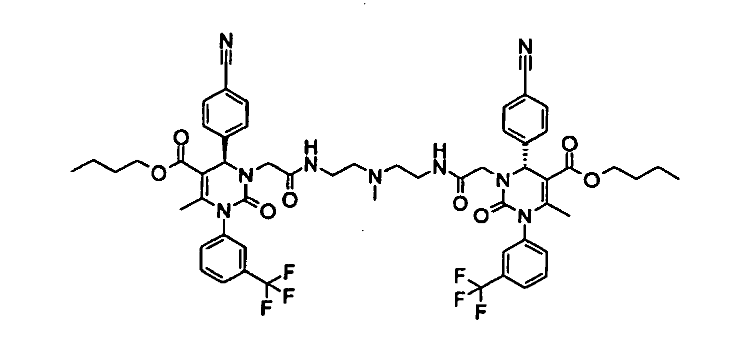

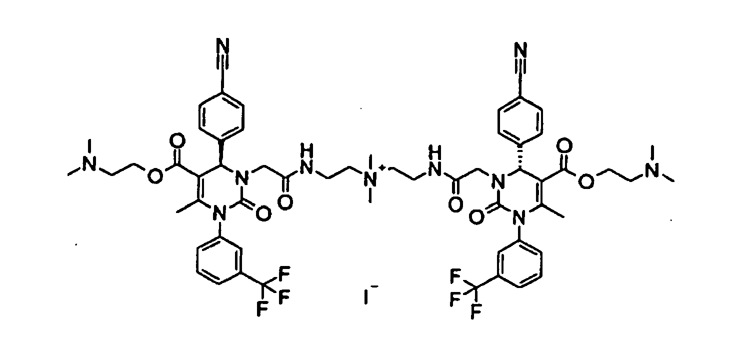

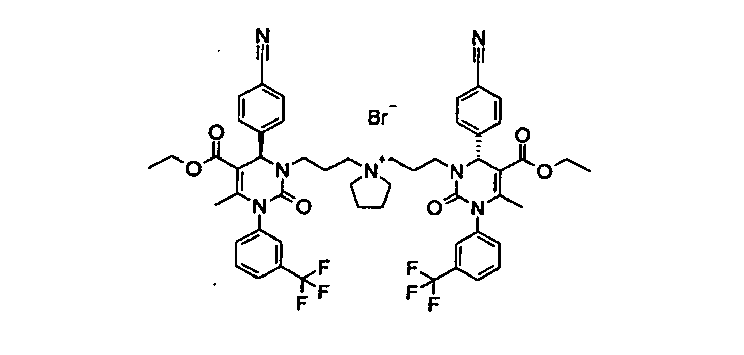

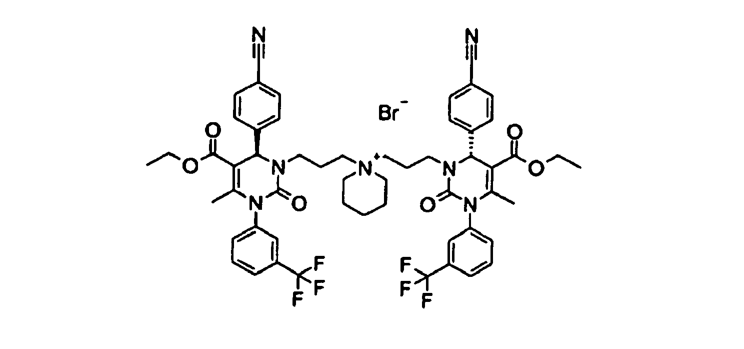

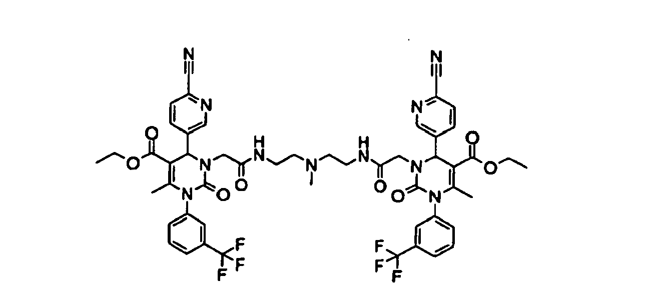

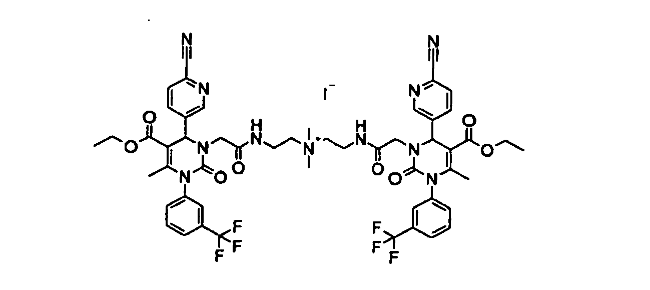

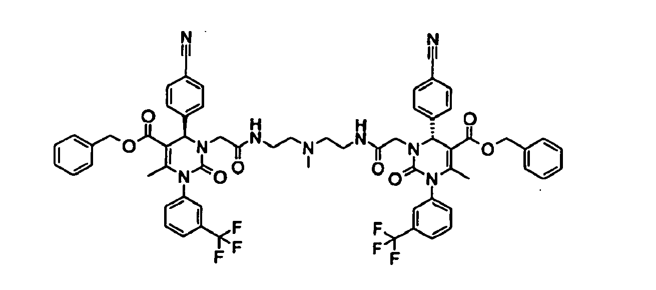

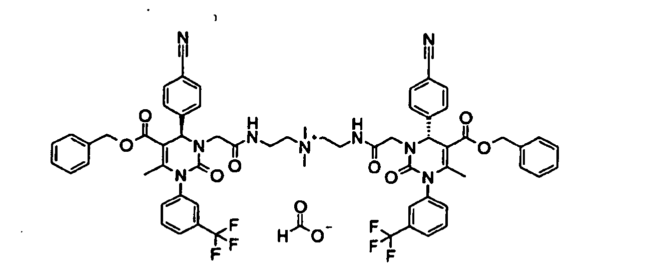

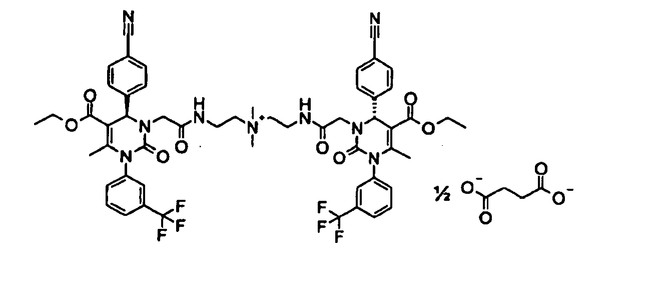

22. Composé selon la revendication 1, qui est l'un quelconque des 17 premiers composés selon la revendication 21.

23. Composé selon la revendication 1, qui est l'un quelconque des 22^{e}, 24^{e}, 33^{e}, 35^{e}, 36^{e}, 38^{e}, 41^{e}, 46^{e}, 65^{e}, 69^{e}, 70^{e}, 71^{e} et 76^{e} composés selon la revendication 21.

24. Composé selon l'une quelconque des revendications précédentes, destiné à un usage thérapeutique.

25. Composé pharmaceutique comprenant un composé selon l'une quelconque des revendications 1 à 23 ainsi qu'un vecteur ou excipient de qualité pharmaceutique.

26. Utilisation d'un composé selon l'une quelconque des revendications 1 à 23, pour la fabrication d'un médicament à utiliser pour la thérapie d'un état sélectionné parmi : asthme, syndromes du côlon irritable, bronchopneumopathie chronique obstructive (BPCO), bronchite chronique, fibrose pulmonaire, pneumonie, syndrome de détresse respiratoire aigüe (SDRA), emphysème pulmonaire, emphysème du fumeur, sarcoïdose, bronchectasie ou mucoviscidose.

27. Utilisation selon la revendication 26, dans lequel l'état est BPCO.

28. Utilisation selon la revendication 26, dans lequel l'état est la mucoviscidose.

29. Utilisation selon la revendication 26, dans lequel l'état est la colite ulcéreuse ou maladie de Crohn.

30. Utilisation selon l'une quelconque des revendications 26 à 29, dans lequel l'état est respiratoire et le médicament doit être administré par voie respiratoire.
